(19) Europäisches Patentamt / European Patent Office / Office européen des brevets

(11) EP 3 134 439 B1

(12) **EUROPEAN PATENT SPECIFICATION**

(45) Date of publication and mention of the grant of the patent:
**26.12.2018 Bulletin 2018/52**

(21) Application number: **15720854.7**

(22) Date of filing: **21.04.2015**

(51) Int Cl.:
***C07K 16/30*** *(2006.01)* ***C07K 16/40*** *(2006.01)*
***A61K 39/00*** *(2006.01)*

(86) International application number:
**PCT/US2015/026806**

(87) International publication number:
**WO 2015/164330 (29.10.2015 Gazette 2015/43)**

(54) **ANTI-PSYK ANTIBODY MOLECULES AND USE OF SAME FOR SYK-TARGETED THERAPY**

ANTI-PSYK-ANTIKÖRPERMOLEKÜLE UND VERWENDUNG DAVON FÜR EINE AUF SYK ABZIELENDE THERAPIE

MOLÉCULES D'ANTICORPS ANTI-PSYK ET LEUR UTILISATION POUR LA THÉRAPIE CIBLÉE SUR SYK

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.04.2014 US 201461982098 P**
**29.05.2014 US 201462004496 P**

(43) Date of publication of application:
**01.03.2017 Bulletin 2017/09**

(73) Proprietor: **Millennium Pharmaceuticals, Inc.**
**Cambridge, MA 02139 (US)**

(72) Inventors:
- **BRAKE, Rachael L.**
  **Natick,**
  **Massachusetts 01760 (US)**
- **BURKHARDT, Anne L.**
  **Ipswich,**
  **Massachusetts 01938 (US)**
- **HE MCDOUGALL, Helen D.**
  **Wellesley,**
  **Massachusetts 02481 (US)**
- **KANNAN, Karuppiah**
  **Newton,**
  **Massachusetts 02465 (US)**
- **THEISEN, Matthew**
  **Wakefield,**
  **Massachusetts 01880 (US)**
- **TIRRELL, Stephen M.**
  **Wellesley,**
  **Massachusetts 02482 (US)**

(74) Representative: **Jones Day**
**Rechtsanwälte, Attorneys-at-Law, Patentanwälte**
**Prinzregentenstrasse 11**
**80538 München (DE)**

(56) References cited:
**WO-A2-2011/045352**

- **Katsue Suzuki-Inoue ET AL: "Glycoproteins VI and Ib-IX-V stimulate tyrosine phosphorylation of tyrosine kinase Syk and phospholipase C[gamma] 2 at distinct sites", Biochem. J, 1 January 2004 (2004-01-01), pages 1023-1029, XP055197909, Retrieved from the Internet: URL:http://www.ncbi.nlm.nih.gov/pmc/articles/PMC1224016/pdf/14656219.pdf [retrieved on 2015-06-24]**
- **J. ZHANG ET AL: "Phosphorylation of Syk Activation Loop Tyrosines Is Essential for Syk Function: AN IN VIVO STUDY USING A SPECIFIC ANTI-Syk ACTIVATION LOOP PHOSPHOTYROSINE ANTIBODY", JOURNAL OF BIOLOGICAL CHEMISTRY, vol. 275, no. 45, 7 August 2000 (2000-08-07), pages 35442-35447, XP055197912, ISSN: 0021-9258, DOI: 10.1074/jbc.M004549200**
- **R M LARIVE ET AL: "Phosphoproteomic analysis of Syk kinase signaling in human cancer cells reveals its role in cell-cell adhesion", ONCOGENE, vol. 28, no. 24, 4 May 2009 (2009-05-04), pages 2337-2347, XP055197947, ISSN: 0950-9232, DOI: 10.1038/onc.2009.99**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MAIKE BUCHNER ET AL: "Spleen Tyrosine Kinase Is Overexpressed and Represents a Potential Therapeutic Target in Chronic Lymphocytic Leukemia", CANCER RESEARCH, AMERICAN ASSOCIATION FOR CANCER RESEARCH, US, vol. 69, no. 13, 1 July 2009 (2009-07-01), pages 5424-5432, XP002662991, ISSN: 0008-5472, DOI: 10.1158/0008-5472.CAN-08-4252
- S. E. SPURGEON ET AL: "The Selective Syk Inhibitor P505-15 (PRT062607) Inhibits B Cell Signaling and Function In Vitro and In Vivo and Augments the Activity of Fludarabine in Chronic Lymphocytic Leukemia", JOURNAL OF PHARMACOLOGY AND EXPERIMENTAL THERAPEUTICS, vol. 344, no. 2, 1 February 2013 (2013-02-01), pages 378-387, XP055198122, DOI: 10.1124/jpet.112.200832
- CHEN LINFENG ET AL: "SYK Inhibition Modulates Distinct PI3K/AKT- Dependent Survival Pathways and Cholesterol Biosynthesis in Diffuse Large B Cell Lymphomas", CANCER CELL, CELL PRESS, US, vol. 23, no. 6, 10 June 2013 (2013-06-10), pages 826-838, XP028566587, ISSN: 1535-6108, DOI: 10.1016/J.CCR.2013.05.002
- BURKE RUSSELL T ET AL: "A potential therapeutic strategy for chronic lymphocytic leukemia by combining Idelalisib and GS-9973, a novel spleen tyrosine kinase (Syk) inhibitor.", ONCOTARGET 28 FEB 2014, vol. 5, no. 4, 28 February 2014 (2014-02-28), pages 908-915, XP002741384, ISSN: 1949-2553
- T. KUROSAKI ET AL: "Role of the Syk autophosphorylation site and SH2 domains in B cell antigen receptor signaling", JOURNAL OF EXPERIMENTAL MEDICINE, vol. 182, no. 6, 1 December 1995 (1995-12-01), pages 1815-1823, XP055197915, ISSN: 0022-1007, DOI: 10.1084/jem.182.6.1815
- J. ZHANG ET AL: "Phosphorylation of Tyr342 in the Linker Region of Syk Is Critical for Fc RI Signaling in Mast Cells", MOLECULAR AND CELLULAR BIOLOGY, vol. 22, no. 23, 1 December 2002 (2002-12-01), pages 8144-8154, XP055197913, ISSN: 0270-7306, DOI: 10.1128/MCB.22.23.8144-8154.2002
- ROBERT L. GEAHLEN: "Getting Syk: spleen tyrosine kinase as a therapeutic target", TRENDS IN PHARMACOLOGICAL SCIENCES, vol. 35, no. 8, 1 August 2014 (2014-08-01) , pages 414-422, XP055197945, ISSN: 0165-6147, DOI: 10.1016/j.tips.2014.05.007
- "Anti-Syk (phospho Y525) antibody [EP575(2)Y] ab62350", , 24 June 2015 (2015-06-24), XP055198073, Retrieved from the Internet: URL:http://www.abcam.com/Syk-phospho-Y525 - antibody-EP5752Y-ab62350.pdf [retrieved on 2015-06-24]

SEQUENCE LISTING

<110> Millennium Pharmaceuticals, Inc.

<120> Anti-pSYK Antibody Molecules and Use of Same For SYK-Targeted Therapy

<130> 223266-370406

<150> US 61/982,098
<151> 2014-04-21

<150> US 62/004,496
<151> 2014-05-29

<160> 28

<170> PatentIn version 3.5

<210> 1
<211> 5079
<212> DNA
<213> Homo sapiens

<400> 1

```
acactgggag gaagtgcggg ccgcctgccc gggcgcgtta aggaagttgc ccaaaatgag      60
gaagagccgc gggcccggcg gctgaggcca ccccggcggc ggctggagag cgaggaggag     120
cgggtggccc cgcgctgcgc ccgccctcgc ctcacctggc gcaggtggac acctgcgcag     180
gtgtgtgccc tccggcccct gaagcatggc cagcagcggc atggctgaca gcgccaacca     240
cctgcccttc tttttcggca acatcacccg ggaggaggca gaagattacc tggtccaggg     300
gggcatgagt gatgggcttt atttgctgcg ccagagccgc aactacctgg gtggcttcgc     360
cctgtccgtg gcccacggga ggaaggcaca ccactacacc atcgagcggg agctgaatgg     420
cacctacgcc atcgccggtg gcaggaccca tgccagcccc gccgacctct gccactacca     480
ctcccaggag tctgatggcc tggtctgcct cctcaagaag cccttcaacc ggccccaagg     540
ggtgcagccc aagactgggc cctttgagga tttgaaggaa aacctcatca gggaatatgt     600
gaagcagaca tggaacctgc agggtcaggc tctggagcag gccatcatca gtcagaagcc     660
tcagctggag aagctgatcg ctaccacagc ccatgaaaaa atgccttggt tccatggaaa     720
aatctctcgg gaagaatctg agcaaattgt cctgatagga tcaaagacaa atggaaagtt     780
cctgatccga gccagagaca acaacggctc ctacgccctg tgcctgctgc acgaagggaa     840
ggtgctgcac tatcgcatcg acaaagacaa gacagggaag ctctccatcc ccgagggaaa     900
gaagttcgac acgctctggc agctagtcga gcattattct tataaagcag atggtttgtt     960
aagagttctt actgtcccat gtcaaaaaat cggcacacag ggaaatgtta attttggagg    1020
ccgtccacaa cttccaggtt cccatcctgc gacttggtca gcgggtggaa taatctcaag    1080
aatcaaatca tactccttcc caaagcctgg ccacagaaag tcctcccctg cccaagggaa    1140
ccggcaagag agtactgtgt cattcaatcc gtatgagcca gaacttgcac cctgggctgc    1200
agacaaaggc ccccagagag aagccctacc catggacaca gaggtgtacg agagccccta    1260
```

cgcggacccc gaggagatca ggcccaagga ggtttacctg gaccgaaagc tgctgacgct 1320
ggaagacaaa gaactgggct ctggtaattt tggaactgtg aaaaagggct actaccaaat 1380
gaaaaaagtt gtgaaaaccg tggctgtgaa aatactgaaa aacgaggcca atgaccccgc 1440
tcttaaagat gagttattag cagaagcaaa tgtcatgcag cagctggaca acccgtacat 1500
cgtgcggatg atcgggatat gcgaggccga gtcctggatg ctggttatgg agatggcaga 1560
acttggtccc ctcaataagt atttgcagca gaacagacat gtcaaggata agaacatcat 1620
agaactggtt catcaggttt ccatgggcat gaagtacttg gaggagagca attttgtgca 1680
cagagatctg gctgcaagaa atgtgttgct agttacccaa cattacgcca agatcagtga 1740
tttcggactt tccaaagcac tgcgtgctga tgaaaactac tacaaggccc agacccatgg 1800
aaagtggcct gtcaagtggt acgctccgga atgcatcaac tactacaagt tctccagcaa 1860
aagcgatgtc tggagctttg gagtgttgat gtgggaagca ttctcctatg ggcagaagcc 1920
atatcgaggg atgaaaggaa gtgaagtcac cgctatgtta gagaaaggag agcggatggg 1980
gtgccctgca gggtgtccaa gagagatgta cgatctcatg aatctgtgct ggacatacga 2040
tgtggaaaac aggcccggat tcgcagcagt ggaactgcgg ctgcgcaatt actactatga 2100
cgtggtgaac taaccgctcc cgcacctgtc ggtggctgcc tttgatcaca ggagcaatca 2160
caggaaaatg tatccagagg aattgattgt cagccacctc cctctgccag tcgggagagc 2220
caggcttgga tggaacatgc ccacaacttg tcacccaaag cctgtcccag gactcaccct 2280
ccacaaagca aaggcagtcc cgggagaaaa gacggatggc aggatccaag ggctagctg 2340
gatttgtttg ttttcttgtc tgtgtgattt tcatacaggt tatttttacg atctgtttcc 2400
aaatcccttt catgtctttc cacttctctg ggtcccgggg tgcatttgtt actcatcggg 2460
cccagggaca ttgcagagtg gcctagagca ctctcacccc aagcggcctt ttccaaatgc 2520
ccaaggatgc cttagcatgt gactcctgaa gggaaggcaa aggcagagga atttggctgc 2580
ttctacggcc atgagactga tccctggcca ctgaaaagct ttcctgacaa taaaaatgtt 2640
ttgaggcttt aaaaagaaaa tcaagtttga ccagtgcagt ttctaagcat gtagccagtt 2700
aaggaaagaa agaaagaaaa aaaaaaaagg cctggatact gcttttgctg tctctgttat 2760
gagatggaag acttacatgt ttgtgataaa aggggaccat gagaatgaat tggcttggct 2820
tactttcccc ctgaaatcct ctctcctgca gactgtcttg aagacctggt gactggtaaa 2880
taaagccctg catggaggct gcacagcagg ggcaagaggc ccatccccca gcatctcact 2940
gaggacagct tcaggctgcc ttcctctgaa cgtggtccac accttcctct cctccacaga 3000
gagggtgccg ccagaatccc ctgtcgcttt ctgtgtctgc aatgggggc agcacaggga 3060
tcaaagccat ctaaagagtt tccaaagaaa gtattaattc agaacaagcc aaagaccctg 3120
agcctcacca caaacaggcc ttttggagtg tgaatttgag ttgaagatac aagatcggag 3180
aatgattttc tggtcttaac taatcctcat cttcatgttt gatctttaag aagtcatcac 3240
ccattgattt cagttttgct gtacctcttg aaagttaaag agacatctca gcactttagg 3300

aggccgaggc gggtggatca cttgaggtaa ggagtttgag actagcctgg ccaatatggt 3360
aaaaccccat ctctactaaa aatacaaaaa ttagccgggc atggtggcat gtgtctgtag 3420
tcccagctac tcgggaggct gaggcaggag aatcgcttga acccaggaaa cggaggtcgc 3480
agtgagccaa gatcatgcca ctgcactcca gcttgggcat cacagcgaga ctctgtcaaa 3540
acaaacaaac aaaaaaacaa cttaaagagg taatttagcc atcattctta tgccagcaga 3600
tataaataaa cttggaccca tctggtcttc agctaaacct gagacatttt aaagtgcatg 3660
gacagccatg gacagcaggc cctcctctaa caggggatgc aaggcatgga gaaagacaat 3720
cagtacccaa gctcagccac agaagacagg agtcactcat ataacttgtg tttagaagtt 3780
tttggtagcc acgcacactt tctgaaatca cactatctgg tggtttaatc atatttttaa 3840
agacagaatc cctgagtgct gagcagattc tcaaaacaca tttagaatcc ctgaaattag 3900
aaagatcaat gacaaaatat ctgtcagcca ggccacaaac aggtgtaaaa ttatgaaagg 3960
agtggttgga tgtgccaagt ttggtaaagt ggtgactgca tctgagaaag aggctgtgag 4020
gctgaactct tggtggcttc cttctgtaac ttccagaggg agtcttcaac acaggccccg 4080
tgctcgtagg aatacggtag cacctatgta ggaagtgcgt ggagttttct gtcttctttc 4140
tgtgtgattt ttggcctttt tatcagcact tctcccctcc caggagcctg gggatgccaa 4200
acatccagaa tgtgatggga caagatgggg gcaggggcct cacctccctg cagaggtccg 4260
gccaggtctc cttgtccctg gacaatctcc tgagcctctc tgcttggtgg agcaggcacc 4320
tgtgtgcaga attcccactg tggccagcac gaggaagtct tttctagtga aaatgtgtct 4380
tgtggtcagg aataattatc ctttcccctg tagccaccaa ggagggcaaa tagagaaagg 4440
taacctaatt gaaggattgg tcatgtgaaa agggctacat ttgggaagct gggaaaggcc 4500
tccaggcttc tagagcagct agcttgggct ggattctcat acccaggctg ccccttggat 4560
tgttctaccc aagcttttcc ctggggtctg ggctcactcc ataaggtaag gtgcctttta 4620
ccttatggtc cttctttagc aggtaacaaa ggagcatcag gggcaggctg ccctggtggc 4680
atcacactgg ctagtgaggc cgtgaatatc ttgtccccca gcagggccga cagtttctat 4740
cacagaaaac agtgtgttca gtggtgaaaa tcgttgcatg catgttttca tctgagcgtg 4800
tccttctccc atactcccta tcagccagcc ctgcctgtag ctgctgtatg gtgattgcac 4860
ttggacatca gtccaatgac tgcaagtcgg cctggatttt cacttgcaga ggctacagct 4920
gcattgtcag gtctcccagc cctgcagaga gctccctcca ctggttagca gtgtgttgtg 4980
ttttccattc atttcagaag agctacattg tgtcactgga catttttaaa aactgtgatt 5040
tttaataaaa atttaaaatt tgctttgtga tgaaaaaaa 5079

<210> 2
<211> 635
<212> PRT
<213> Homo sapiens

<400> 2

Met Ala Ser Ser Gly Met Ala Asp Ser Ala Asn His Leu Pro Phe Phe
1 5 10 15

Phe Gly Asn Ile Thr Arg Glu Glu Ala Glu Asp Tyr Leu Val Gln Gly
20 25 30

Gly Met Ser Asp Gly Leu Tyr Leu Leu Arg Gln Ser Arg Asn Tyr Leu
35 40 45

Gly Gly Phe Ala Leu Ser Val Ala His Gly Arg Lys Ala His His Tyr
50 55 60

Thr Ile Glu Arg Glu Leu Asn Gly Thr Tyr Ala Ile Ala Gly Gly Arg
65 70 75 80

Thr His Ala Ser Pro Ala Asp Leu Cys His Tyr His Ser Gln Glu Ser
85 90 95

Asp Gly Leu Val Cys Leu Leu Lys Lys Pro Phe Asn Arg Pro Gln Gly
100 105 110

Val Gln Pro Lys Thr Gly Pro Phe Glu Asp Leu Lys Glu Asn Leu Ile
115 120 125

Arg Glu Tyr Val Lys Gln Thr Trp Asn Leu Gln Gly Gln Ala Leu Glu
130 135 140

Gln Ala Ile Ile Ser Gln Lys Pro Gln Leu Glu Lys Leu Ile Ala Thr
145 150 155 160

Thr Ala His Glu Lys Met Pro Trp Phe His Gly Lys Ile Ser Arg Glu
165 170 175

Glu Ser Glu Gln Ile Val Leu Ile Gly Ser Lys Thr Asn Gly Lys Phe
180 185 190

Leu Ile Arg Ala Arg Asp Asn Asn Gly Ser Tyr Ala Leu Cys Leu Leu
195 200 205

His Glu Gly Lys Val Leu His Tyr Arg Ile Asp Lys Asp Lys Thr Gly
210 215 220

Lys Leu Ser Ile Pro Glu Gly Lys Lys Phe Asp Thr Leu Trp Gln Leu
225 230 235 240

Val Glu His Tyr Ser Tyr Lys Ala Asp Gly Leu Leu Arg Val Leu Thr
245 250 255

Val Pro Cys Gln Lys Ile Gly Thr Gln Gly Asn Val Asn Phe Gly Gly

```
            260                 265                 270

Arg Pro Gln Leu Pro Gly Ser His Pro Ala Thr Trp Ser Ala Gly Gly
        275                 280                 285

Ile Ile Ser Arg Ile Lys Ser Tyr Ser Phe Pro Lys Pro Gly His Arg
    290                 295                 300

Lys Ser Ser Pro Ala Gln Gly Asn Arg Gln Glu Ser Thr Val Ser Phe
305                 310                 315                 320

Asn Pro Tyr Glu Pro Glu Leu Ala Pro Trp Ala Ala Asp Lys Gly Pro
                325                 330                 335

Gln Arg Glu Ala Leu Pro Met Asp Thr Glu Val Tyr Glu Ser Pro Tyr
            340                 345                 350

Ala Asp Pro Glu Glu Ile Arg Pro Lys Glu Val Tyr Leu Asp Arg Lys
        355                 360                 365

Leu Leu Thr Leu Glu Asp Lys Glu Leu Gly Ser Gly Asn Phe Gly Thr
    370                 375                 380

Val Lys Lys Gly Tyr Tyr Gln Met Lys Lys Val Val Lys Thr Val Ala
385                 390                 395                 400

Val Lys Ile Leu Lys Asn Glu Ala Asn Asp Pro Ala Leu Lys Asp Glu
                405                 410                 415

Leu Leu Ala Glu Ala Asn Val Met Gln Gln Leu Asp Asn Pro Tyr Ile
            420                 425                 430

Val Arg Met Ile Gly Ile Cys Glu Ala Glu Ser Trp Met Leu Val Met
        435                 440                 445

Glu Met Ala Glu Leu Gly Pro Leu Asn Lys Tyr Leu Gln Gln Asn Arg
    450                 455                 460

His Val Lys Asp Lys Asn Ile Ile Glu Leu Val His Gln Val Ser Met
465                 470                 475                 480

Gly Met Lys Tyr Leu Glu Glu Ser Asn Phe Val His Arg Asp Leu Ala
                485                 490                 495

Ala Arg Asn Val Leu Leu Val Thr Gln His Tyr Ala Lys Ile Ser Asp
            500                 505                 510

Phe Gly Leu Ser Lys Ala Leu Arg Ala Asp Glu Asn Tyr Tyr Lys Ala
        515                 520                 525
```

```
Gln Thr His Gly Lys Trp Pro Val Lys Trp Tyr Ala Pro Glu Cys Ile
    530                 535                 540

Asn Tyr Tyr Lys Phe Ser Ser Lys Ser Asp Val Trp Ser Phe Gly Val
545                 550                 555                 560

Leu Met Trp Glu Ala Phe Ser Tyr Gly Gln Lys Pro Tyr Arg Gly Met
                565                 570                 575

Lys Gly Ser Glu Val Thr Ala Met Leu Glu Lys Gly Glu Arg Met Gly
            580                 585                 590

Cys Pro Ala Gly Cys Pro Arg Glu Met Tyr Asp Leu Met Asn Leu Cys
        595                 600                 605

Trp Thr Tyr Asp Val Glu Asn Arg Pro Gly Phe Ala Ala Val Glu Leu
    610                 615                 620

Arg Leu Arg Asn Tyr Tyr Tyr Asp Val Val Asn
625                 630                 635
```

<210> 3
<211> 1377
<212> DNA
<213> Artificial Sequence

<220>
<223> Synthetic Polynucleotide

<400> 3

atggagactg ggctgcgctg gcttctcctg gtcgctgtgc tcaaaggtgt ccagtgtcag 60
tcggtggagg agtccggggg tcgcctggtc acgcctggga cacccctgac actcacctgc 120
acagtctctg gaatcgacct caatagttat ataatgagtt gggtccgcca ggctccaggg 180
aaggggctgg aatggatcgg aatcattagt cgtcgtggta acacatacta cgcgagctgg 240
ccgaaaggcc gattcaccat ctccaaaacc tcgaccacgg tggatctgaa aatcaccagt 300
ccgacaaccg aggacacggc cacctatttc tgtgccagag catatcttta tactagtggt 360
acgatgagcg tctggggccc aggcaccctg gtcaccgtct cctcagggca acctaaggct 420
ccatcagtct tcccactggc cccctgctgc ggggacacac ccagctccac ggtgaccctg 480
ggctgcctgg tcaaagggta cctcccggag ccagtgaccg tgacctggaa ctcgggcacc 540
ctcaccaatg ggtacgcac cttcccgtcc gtccggcagt cctcaggcct ctactcgctg 600
agcagcgtgg tgagcgtgac ctcaagcagc cagcccgtca cctgcaacgt ggcccaccca 660
gccaccaaca ccaaagtgga caagaccgtt gcgccctcga catgcagcaa gcccacgtgc 720
ccaccccctg aactcctggg gggaccgtct gtcttcatct tcccccaaa acccaaggac 780
accctcatga tctcacgcac ccccgaggtc acatgcgtgg tggtggacgt gagccaggat 840
gaccccgagg tgcagttcac atggtacata aacaacgagc aggtgcgcac cgcccggccg 900

ccgctacggg agcagcagtt caacagcacg atccgcgtgg tcagcaccct ccccatcgcg 960
caccaggact ggctgagggg caaggagttc aagtgcaaag tccacaacaa ggcactcccg 1020
gcccccatcg agaaaaccat ctccaaagcc agagggcagc ccctggagcc gaaggtctac 1080
accatgggcc ctccccggga ggagctgagc agcaggtcgg tcagcctgac ctgcatgatc 1140
aacggcttct acccttccga catctcggtg gagtgggaga agaacgggaa ggcagaggac 1200
aactacaaga ccacgccggc cgtgctggac agcgacggct cctacttcct ctacagcaag 1260
ctctcagtgc ccacgagtga gtggcagcgg ggcgacgtct tcacctgctc cgtgatgcac 1320
gaggccttgc acaaccacta cacgcagaag tccatctccc gctctccggg taaatga 1377

<210> 4
<211> 458
<212> PRT
<213> Artificial Sequence

<220>
<223> Synthetic Polypeptide

<400> 4

```
Met Glu Thr Gly Leu Arg Trp Leu Leu Leu Val Ala Val Leu Lys Gly
1               5                   10                  15

Val Gln Cys Gln Ser Val Glu Glu Ser Gly Gly Arg Leu Val Thr Pro
            20                  25                  30

Gly Thr Pro Leu Thr Leu Thr Cys Thr Val Ser Gly Ile Asp Leu Asn
        35                  40                  45

Ser Tyr Ile Met Ser Trp Val Arg Gln Ala Pro Gly Lys Gly Leu Glu
    50                  55                  60

Trp Ile Gly Ile Ile Ser Arg Arg Gly Asn Thr Tyr Tyr Ala Ser Trp
65                  70                  75                  80

Pro Lys Gly Arg Phe Thr Ile Ser Lys Thr Ser Thr Thr Val Asp Leu
                85                  90                  95

Lys Ile Thr Ser Pro Thr Thr Glu Asp Thr Ala Thr Tyr Phe Cys Ala
            100                 105                 110

Arg Ala Tyr Leu Tyr Thr Ser Gly Thr Met Ser Val Trp Gly Pro Gly
        115                 120                 125

Thr Leu Val Thr Val Ser Ser Gly Gln Pro Lys Ala Pro Ser Val Phe
    130                 135                 140

Pro Leu Ala Pro Cys Cys Gly Asp Thr Pro Ser Ser Thr Val Thr Leu
145                 150                 155                 160
```

Gly Cys Leu Val Lys Gly Tyr Leu Pro Glu Pro Val Thr Val Thr Trp
165 170 175

Asn Ser Gly Thr Leu Thr Asn Gly Val Arg Thr Phe Pro Ser Val Arg
180 185 190

Gln Ser Ser Gly Leu Tyr Ser Leu Ser Ser Val Val Ser Val Thr Ser
195 200 205

Ser Ser Gln Pro Val Thr Cys Asn Val Ala His Pro Ala Thr Asn Thr
210 215 220

Lys Val Asp Lys Thr Val Ala Pro Ser Thr Cys Ser Lys Pro Thr Cys
225 230 235 240

Pro Pro Pro Glu Leu Leu Gly Gly Pro Ser Val Phe Ile Phe Pro Pro
245 250 255

Lys Pro Lys Asp Thr Leu Met Ile Ser Arg Thr Pro Glu Val Thr Cys
260 265 270

Val Val Val Asp Val Ser Gln Asp Asp Pro Glu Val Gln Phe Thr Trp
275 280 285

Tyr Ile Asn Asn Glu Gln Val Arg Thr Ala Arg Pro Pro Leu Arg Glu
290 295 300

Gln Gln Phe Asn Ser Thr Ile Arg Val Val Ser Thr Leu Pro Ile Ala
305 310 315 320

His Gln Asp Trp Leu Arg Gly Lys Glu Phe Lys Cys Lys Val His Asn
325 330 335

Lys Ala Leu Pro Ala Pro Ile Glu Lys Thr Ile Ser Lys Ala Arg Gly
340 345 350

Gln Pro Leu Glu Pro Lys Val Tyr Thr Met Gly Pro Pro Arg Glu Glu
355 360 365

Leu Ser Ser Arg Ser Val Ser Leu Thr Cys Met Ile Asn Gly Phe Tyr
370 375 380

Pro Ser Asp Ile Ser Val Glu Trp Glu Lys Asn Gly Lys Ala Glu Asp
385 390 395 400

Asn Tyr Lys Thr Thr Pro Ala Val Leu Asp Ser Asp Gly Ser Tyr Phe
405 410 415

Leu Tyr Ser Lys Leu Ser Val Pro Thr Ser Glu Trp Gln Arg Gly Asp
420 425 430

```
Val Phe Thr Cys Ser Val Met His Glu Ala Leu His Asn His Tyr Thr
        435                 440                 445

Gln Lys Ser Ile Ser Arg Ser Pro Gly Lys
    450                 455
```

<210> 5
<211> 714
<212> DNA
<213> Artificial Sequence

<220>
<223> Synthetic Polynucleotide

<400> 5

```
atggacacga gggcccccac tcagctgctg gggctcctgc tgctctggct cccaggtgcc      60
agatgtgctg acattgtgat gacccagact ccatcccccg tggaggcagc tgtgggaggc     120
acagtcacca tcaagtgcca ggccagtgag agcattagta gttacttatc ctggtatcag     180
cagaaaccag ggcagcctcc caaactcctg atctacaggg catccactct ggtatctggg     240
gtcccatcgc ggttcaaagg cagtggatct gggacagatt tcactctcac catcagcgac     300
ctggagtgtg ccgatgctgc cacttactat tgtcaacata cttattttgg tagtgattat     360
gttggtggtt tcggcggagg gaccgaggtg gtggtcaaag gtgatccagt tgcacctact     420
gtcctcatct tcccaccagc tgctgatcag gtggcaactg gaacagtcac catcgtgtgt     480
gtggcgaata aatactttcc cgatgtcacc gtcacctggg aggtggatgg caccacccaa     540
acaactggca tcgagaacag taaaacaccg cagaattctg cagattgtac ctacaacctc     600
agcagcactc tgacactgac cagcacacag tacaacagcc acaaagagta cacctgcaag     660
gtgacccagg gcacgacctc agtcgtccag agcttcaata ggggtgactg ttag           714
```

<210> 6
<211> 237
<212> PRT
<213> Artificial Sequence

<220>
<223> Synthetic Polypeptide

<400> 6

```
Met Asp Thr Arg Ala Pro Thr Gln Leu Leu Gly Leu Leu Leu Leu Trp
1               5                   10                  15

Leu Pro Gly Ala Arg Cys Ala Asp Ile Val Met Thr Gln Thr Pro Ser
            20                  25                  30

Pro Val Glu Ala Ala Val Gly Gly Thr Val Thr Ile Lys Cys Gln Ala
        35                  40                  45

Ser Glu Ser Ile Ser Ser Tyr Leu Ser Trp Tyr Gln Gln Lys Pro Gly
    50                  55                  60

Gln Pro Pro Lys Leu Leu Ile Tyr Arg Ala Ser Thr Leu Val Ser Gly
65                  70                  75                  80

Val Pro Ser Arg Phe Lys Gly Ser Gly Ser Gly Thr Asp Phe Thr Leu
                85                  90                  95

Thr Ile Ser Asp Leu Glu Cys Ala Asp Ala Ala Thr Tyr Tyr Cys Gln
            100                 105                 110

His Thr Tyr Phe Gly Ser Asp Tyr Val Gly Gly Phe Gly Gly Gly Thr
        115                 120                 125

Glu Val Val Val Lys Gly Asp Pro Val Ala Pro Thr Val Leu Ile Phe
    130                 135                 140

Pro Pro Ala Ala Asp Gln Val Ala Thr Gly Thr Val Thr Ile Val Cys
145                 150                 155                 160

Val Ala Asn Lys Tyr Phe Pro Asp Val Thr Val Thr Trp Glu Val Asp
                165                 170                 175

Gly Thr Thr Gln Thr Thr Gly Ile Glu Asn Ser Lys Thr Pro Gln Asn
            180                 185                 190

Ser Ala Asp Cys Thr Tyr Asn Leu Ser Ser Thr Leu Thr Leu Thr Ser
        195                 200                 205

Thr Gln Tyr Asn Ser His Lys Glu Tyr Thr Cys Lys Val Thr Gln Gly
    210                 215                 220

Thr Thr Ser Val Val Gln Ser Phe Asn Arg Gly Asp Cys
225                 230                 235
```

<210> 7
<211> 360
<212> DNA
<213> Artificial Sequence

<220>
<223> Synthetic Polynucleotide

<400> 7

```
cagtgtcagt cggtggagga gtccgggggt cgcctggtca cgcctgggac acccctgaca        60
ctcacctgca cagtctctgg aatcgacctc aatagttata taatgagttg ggtccgccag       120
gctccaggga aggggctgga atggatcgga atcattagtc gtcgtggtaa cacatactac       180
gcgagctggc cgaaaggccg attcaccatc tccaaaacct cgaccacggt ggatctgaaa       240
atcaccagtc cgacaaccga ggacacggcc acctatttct gtgccagagc atatctttat       300
actagtggta cgatgagcgt ctggggccca ggcaccctgg tcaccgtctc ctcagggcaa       360
```

<210> 8
<211> 120
<212> PRT
<213> Artificial Sequence

<220>
<223> Synthetic Polypeptide

<400> 8

```
Gln Cys Gln Ser Val Glu Glu Ser Gly Gly Arg Leu Val Thr Pro Gly
1               5                   10                  15

Thr Pro Leu Thr Leu Thr Cys Thr Val Ser Gly Ile Asp Leu Asn Ser
            20                  25                  30

Tyr Ile Met Ser Trp Val Arg Gln Ala Pro Gly Lys Gly Leu Glu Trp
        35                  40                  45

Ile Gly Ile Ile Ser Arg Arg Gly Asn Thr Tyr Tyr Ala Ser Trp Pro
    50                  55                  60

Lys Gly Arg Phe Thr Ile Ser Lys Thr Ser Thr Thr Val Asp Leu Lys
65                  70                  75                  80

Ile Thr Ser Pro Thr Thr Glu Asp Thr Ala Thr Tyr Phe Cys Ala Arg
                85                  90                  95

Ala Tyr Leu Tyr Thr Ser Gly Thr Met Ser Val Trp Gly Pro Gly Thr
            100                 105                 110

Leu Val Thr Val Ser Ser Gly Gln
        115                 120
```

<210> 9
<211> 339
<212> DNA
<213> Artificial Sequence

<220>
<223> Synthetic Polynucleotide

<400> 9

```
gctgacattg tgatgaccca gactccatcc cccgtggagg cagctgtggg aggcacagtc      60
accatcaagt gccaggccag tgagagcatt agtagttact tatcctggta tcagcagaaa     120
ccagggcagc ctcccaaact cctgatctac agggcatcca ctctggtatc tggggtccca     180
tcgcggttca aaggcagtgg atctgggaca gatttcactc tcaccatcag cgacctggag     240
tgtgccgatg ctgccactta ctattgtcaa catacttatt ttggtagtga ttatgttggt     300
ggtttcggcg gagggaccga ggtggtggtc aaaggtgat                            339
```

<210> 10
<211> 113
<212> PRT
<213> Artificial Sequence

<220>
<223> Synthetic Polypeptide

<400> 10

```
Ala Asp Ile Val Met Thr Gln Thr Pro Ser Pro Val Glu Ala Ala Val
1               5                   10                  15

Gly Gly Thr Val Thr Ile Lys Cys Gln Ala Ser Glu Ser Ile Ser Ser
            20                  25                  30

Tyr Leu Ser Trp Tyr Gln Gln Lys Pro Gly Gln Pro Pro Lys Leu Leu
        35                  40                  45

Ile Tyr Arg Ala Ser Thr Leu Val Ser Gly Val Pro Ser Arg Phe Lys
    50                  55                  60

Gly Ser Gly Ser Gly Thr Asp Phe Thr Leu Thr Ile Ser Asp Leu Glu
65                  70                  75                  80

Cys Ala Asp Ala Ala Thr Tyr Tyr Cys Gln His Thr Tyr Phe Gly Ser
                85                  90                  95

Asp Tyr Val Gly Gly Phe Gly Gly Gly Thr Glu Val Val Val Lys Gly
            100                 105                 110

Asp
```

<210> 11
<211> 9
<212> PRT
<213> Artificial Sequence

<220>
<223> Synthetic Peptide

<400> 11

Ile Asp Leu Asn Ser Tyr Ile Met Ser
1 5

<210> 12
<211> 16
<212> PRT
<213> Artificial Sequence

<220>
<223> Synthetic Peptide

<400> 12

Ile Ile Ser Arg Arg Gly Asn Thr Tyr Tyr Ala Ser Trp Pro Lys Gly
1 5 10 15

<210> 13
<211> 11
<212> PRT
<213> Artificial Sequence

<220>
<223> Synthetic Peptide

<400> 13

Ala Tyr Leu Tyr Thr Ser Gly Thr Met Ser Val
1 5 10

<210> 14
<211> 11
<212> PRT
<213> Artificial Sequence

<220>
<223> Synthetic Peptide

<400> 14

Gln Ala Ser Glu Ser Ile Ser Ser Tyr Leu Ser
1 5 10

<210> 15
<211> 7
<212> PRT
<213> Artificial Sequence

<220>
<223> Synthetic Peptide

<400> 15

Arg Ala Ser Thr Leu Val Ser
1 5

<210> 16
<211> 12
<212> PRT
<213> Artificial Sequence

<220>
<223> Synthetic Peptide

<400> 16

Gln His Thr Tyr Phe Gly Ser Asp Tyr Val Gly Gly
1               5                   10

<210> 17
<211> 27
<212> DNA
<213> Artificial Sequence

<220>
<223> synthetic oligonucleotide

<400> 17
atcgacctca atagttatat aatgagt 27

<210> 18
<211> 48
<212> DNA
<213> Artificial Sequence

<220>
<223> synthetic oligonucleotide

<400> 18
atcattagtc gtcgtggtaa cacatactac gcgagctggc cgaaaggc 48

<210> 19
<211> 33
<212> DNA
<213> Artificial Sequence

<220>
<223> synthetic oligonucleotide

<400> 19
gcatatcttt atactagtgg tacgatgagc gtc 33

<210> 20
<211> 33
<212> DNA
<213> Artificial Sequence

<220>
<223> synthetic oligonucleotide

<400> 20
caggccagtg agagcattag tagttactta tcc 33

<210> 21

<211> 21
<212> DNA
<213> Artificial Sequence

<220>
<223> synthetic oligonucleotide

<400> 21
agggcatcca ctctggtatc t 21

<210> 22
<211> 36
<212> DNA
<213> Artificial Sequence

<220>
<223> synthetic oligonucleotide

<400> 22
caacatactt attttggtag tgattatgtt ggtggt 36

<210> 23
<211> 12
<212> PRT
<213> Artificial Sequence

<220>
<223> Synthetic Peptide

<400> 23

```
Lys Lys Lys Lys Glu Glu Ile Tyr Phe Phe Phe Gly
1               5                   10
```

<210> 24
<211> 289
<212> PRT
<213> Artificial Sequence

<220>
<223> Synthetic Polypeptide

<400> 24

Met Ala Lys Glu Glu Ile Arg Pro Lys Glu Val Tyr Leu Asp Arg Lys
1               5                   10                  15

Leu Leu Thr Leu Glu Asp Lys Glu Leu Gly Ser Gly Asn Phe Gly Thr
            20                  25                  30

Val Lys Lys Gly Tyr Tyr Gln Met Lys Lys Val Val Lys Thr Val Ala
        35                  40                  45

Val Lys Ile Leu Lys Asn Glu Ala Asn Asp Pro Ala Leu Lys Asp Glu
    50                  55                  60

Leu Leu Ala Glu Ala Asn Val Met Gln Gln Leu Asp Asn Pro Tyr Ile
65                  70                  75                  80

Val Arg Met Ile Gly Ile Cys Glu Ala Glu Ser Trp Met Leu Val Met
                85                  90                  95

Glu Met Ala Glu Leu Gly Pro Leu Asn Lys Tyr Leu Gln Gln Asn Arg
            100                 105                 110

His Val Lys Asp Lys Asn Ile Ile Glu Leu Val His Gln Val Ser Met
        115                 120                 125

Gly Met Lys Tyr Leu Glu Glu Ser Asn Phe Val His Arg Asp Leu Ala
    130                 135                 140

Ala Arg Asn Val Leu Leu Val Thr Gln His Tyr Ala Lys Ile Ser Asp
145                 150                 155                 160

Phe Gly Leu Ser Lys Ala Leu Arg Ala Asp Glu Asn Tyr Tyr Lys Ala
                165                 170                 175

Gln Thr His Gly Lys Trp Pro Val Lys Trp Tyr Ala Pro Glu Cys Ile
            180                 185                 190

Asn Tyr Tyr Lys Phe Ser Ser Lys Ser Asp Val Trp Ser Phe Gly Val
195 200 205

Leu Met Trp Glu Ala Phe Ser Tyr Gly Gln Lys Pro Tyr Arg Gly Met
210 215 220

Lys Gly Ser Glu Val Thr Ala Met Leu Glu Lys Gly Glu Arg Met Gly
225 230 235 240

Cys Pro Ala Gly Cys Pro Arg Glu Met Tyr Asp Leu Met Asn Leu Cys
245 250 255

Trp Thr Tyr Asp Val Glu Asn Arg Pro Gly Phe Ala Ala Val Glu Leu
260 265 270

Arg Leu Arg Asn Tyr Tyr Tyr Asp Val Val Asn His His His His His
275 280 285

His

<210> 25
<211> 10
<212> PRT
<213> Artificial Sequence

<220>
<223> Synthetic Peptide

<400> 25

Arg Ala Asp Glu Asn Tyr Tyr Lys Ala Gln
1 5 10

<210> 26
<211> 659
<212> PRT
<213> Homo sapiens

<400> 26

```
Met Ala Ala Val Ile Leu Glu Ser Ile Phe Leu Lys Arg Ser Gln Gln
1               5                   10                  15

Lys Lys Lys Thr Ser Pro Leu Asn Phe Lys Lys Arg Leu Phe Leu Leu
            20                  25                  30

Thr Val His Lys Leu Ser Tyr Tyr Glu Tyr Asp Phe Glu Arg Gly Arg
        35                  40                  45

Arg Gly Ser Lys Lys Gly Ser Ile Asp Val Glu Lys Ile Thr Cys Val
    50                  55                  60

Glu Thr Val Val Pro Glu Lys Asn Pro Pro Pro Glu Arg Gln Ile Pro
65                  70                  75                  80
```

Arg Arg Gly Glu Glu Ser Ser Glu Met Glu Gln Ile Ser Ile Ile Glu
85 90 95

Arg Phe Pro Tyr Pro Phe Gln Val Val Tyr Asp Glu Gly Pro Leu Tyr
100 105 110

Val Phe Ser Pro Thr Glu Glu Leu Arg Lys Arg Trp Ile His Gln Leu
115 120 125

Lys Asn Val Ile Arg Tyr Asn Ser Asp Leu Val Gln Lys Tyr His Pro
130 135 140

Cys Phe Trp Ile Asp Gly Gln Tyr Leu Cys Cys Ser Gln Thr Ala Lys
145 150 155 160

Asn Ala Met Gly Cys Gln Ile Leu Glu Asn Arg Asn Gly Ser Leu Lys
165 170 175

Pro Gly Ser Ser His Arg Lys Thr Lys Lys Pro Leu Pro Pro Thr Pro
180 185 190

Glu Glu Asp Gln Ile Leu Lys Lys Pro Leu Pro Pro Glu Pro Ala Ala
195 200 205

Ala Pro Val Ser Thr Ser Glu Leu Lys Lys Val Val Ala Leu Tyr Asp
210 215 220

Tyr Met Pro Met Asn Ala Asn Asp Leu Gln Leu Arg Lys Gly Asp Glu
225 230 235 240

Tyr Phe Ile Leu Glu Glu Ser Asn Leu Pro Trp Trp Arg Ala Arg Asp
245 250 255

Lys Asn Gly Gln Glu Gly Tyr Ile Pro Ser Asn Tyr Val Thr Glu Ala
260 265 270

Glu Asp Ser Ile Glu Met Tyr Glu Trp Tyr Ser Lys His Met Thr Arg
275 280 285

Ser Gln Ala Glu Gln Leu Leu Lys Gln Glu Gly Lys Glu Gly Gly Phe
290 295 300

Ile Val Arg Asp Ser Ser Lys Ala Gly Lys Tyr Thr Val Ser Val Phe
305 310 315 320

Ala Lys Ser Thr Gly Asp Pro Gln Gly Val Ile Arg His Tyr Val Val
325 330 335

Cys Ser Thr Pro Gln Ser Gln Tyr Tyr Leu Ala Glu Lys His Leu Phe

340 345 350

Ser Thr Ile Pro Glu Leu Ile Asn Tyr His Gln His Asn Ser Ala Gly
355 360 365

Leu Ile Ser Arg Leu Lys Tyr Pro Val Ser Gln Gln Asn Lys Asn Ala
370 375 380

Pro Ser Thr Ala Gly Leu Gly Tyr Gly Ser Trp Glu Ile Asp Pro Lys
385 390 395 400

Asp Leu Thr Phe Leu Lys Glu Leu Gly Thr Gly Gln Phe Gly Val Val
405 410 415

Lys Tyr Gly Lys Trp Arg Gly Gln Tyr Asp Val Ala Ile Lys Met Ile
420 425 430

Lys Glu Gly Ser Met Ser Glu Asp Glu Phe Ile Glu Glu Ala Lys Val
435 440 445

Met Met Asn Leu Ser His Glu Lys Leu Val Gln Leu Tyr Gly Val Cys
450 455 460

Thr Lys Gln Arg Pro Ile Phe Ile Ile Thr Glu Tyr Met Ala Asn Gly
465 470 475 480

Cys Leu Leu Asn Tyr Leu Arg Glu Met Arg His Arg Phe Gln Thr Gln
485 490 495

Gln Leu Leu Glu Met Cys Lys Asp Val Cys Glu Ala Met Glu Tyr Leu
500 505 510

Glu Ser Lys Gln Phe Leu His Arg Asp Leu Ala Ala Arg Asn Cys Leu
515 520 525

Val Asn Asp Gln Gly Val Val Lys Val Ser Asp Phe Gly Leu Ser Arg
530 535 540

Tyr Val Leu Asp Asp Glu Tyr Thr Ser Ser Val Gly Ser Lys Phe Pro
545 550 555 560

Val Arg Trp Ser Pro Pro Glu Val Leu Met Tyr Ser Lys Phe Ser Ser
565 570 575

Lys Ser Asp Ile Trp Ala Phe Gly Val Leu Met Trp Glu Ile Tyr Ser
580 585 590

Leu Gly Lys Met Pro Tyr Glu Arg Phe Thr Asn Ser Glu Thr Ala Glu
595 600 605

His Ile Ala Gln Gly Leu Arg Leu Tyr Arg Pro His Leu Ala Ser Glu
610 615 620

Lys Val Tyr Thr Ile Met Tyr Ser Cys Trp His Glu Lys Ala Asp Glu
625 630 635 640

Arg Pro Thr Phe Lys Ile Leu Leu Ser Asn Ile Leu Asp Val Met Asp
645 650 655

Glu Glu Ser

<210> 27
<211> 456
<212> PRT
<213> Homo sapiens

<400> 27

Met Asp Lys Leu Asn Lys Ile Thr Val Pro Ala Ser Gln Lys Leu Arg
1 5 10 15

Gln Leu Gln Lys Met Val His Asp Ile Lys Asn Asn Glu Gly Gly Ile
20 25 30

Met Asn Lys Ile Lys Lys Leu Lys Val Lys Ala Pro Pro Ser Val Pro
35 40 45

Arg Arg Asp Tyr Ala Ser Glu Ser Pro Ala Asp Glu Glu Glu Gln Trp
50 55 60

Ser Asp Asp Phe Asp Ser Asp Tyr Glu Asn Pro Asp Glu His Ser Asp
65 70 75 80

Ser Glu Met Tyr Val Met Pro Ala Glu Glu Asn Ala Asp Asp Ser Tyr
85 90 95

Glu Pro Pro Pro Val Glu Gln Glu Thr Arg Pro Val His Pro Ala Leu
100 105 110

Pro Phe Ala Arg Gly Glu Tyr Ile Asp Asn Arg Ser Ser Gln Arg His
115 120 125

Ser Pro Pro Phe Ser Lys Thr Leu Pro Ser Lys Pro Ser Trp Pro Ser
130 135 140

Glu Lys Ala Arg Leu Thr Ser Thr Leu Pro Ala Leu Thr Ala Leu Gln
145 150 155 160

Lys Pro Gln Val Pro Pro Lys Pro Lys Gly Leu Leu Glu Asp Glu Ala
165 170 175

```
Asp Tyr Val Val Pro Val Glu Asp Asn Asp Glu Asn Tyr Ile His Pro
            180                 185                 190

Thr Glu Ser Ser Ser Pro Pro Pro Glu Lys Ala Pro Met Val Asn Arg
        195                 200                 205

Ser Thr Lys Pro Asn Ser Ser Thr Pro Ala Ser Pro Pro Gly Thr Ala
    210                 215                 220

Ser Gly Arg Asn Ser Gly Ala Trp Glu Thr Lys Ser Pro Pro Pro Ala
225                 230                 235                 240

Ala Pro Ser Pro Leu Pro Arg Ala Gly Lys Lys Pro Thr Thr Pro Leu
                245                 250                 255

Lys Thr Thr Pro Val Ala Ser Gln Gln Asn Ala Ser Ser Val Cys Glu
            260                 265                 270

Glu Lys Pro Ile Pro Ala Glu Arg His Arg Gly Ser Ser His Arg Gln
        275                 280                 285

Glu Ala Val Gln Ser Pro Val Phe Pro Pro Ala Gln Lys Gln Ile His
    290                 295                 300

Gln Lys Pro Ile Pro Leu Pro Arg Phe Thr Glu Gly Gly Asn Pro Thr
305                 310                 315                 320

Val Asp Gly Pro Leu Pro Ser Phe Ser Ser Asn Ser Thr Ile Ser Glu
                325                 330                 335

Gln Glu Ala Gly Val Leu Cys Lys Pro Trp Tyr Ala Gly Ala Cys Asp
            340                 345                 350

Arg Lys Ser Ala Glu Glu Ala Leu His Arg Ser Asn Lys Asp Gly Ser
        355                 360                 365

Phe Leu Ile Arg Lys Ser Ser Gly His Asp Ser Lys Gln Pro Tyr Thr
    370                 375                 380

Leu Val Val Phe Phe Asn Lys Arg Val Tyr Asn Ile Pro Val Arg Phe
385                 390                 395                 400

Ile Glu Ala Thr Lys Gln Tyr Ala Leu Gly Arg Lys Lys Asn Gly Glu
                405                 410                 415

Glu Tyr Phe Gly Ser Val Ala Glu Ile Ile Arg Asn His Gln His Ser
            420                 425                 430

Pro Leu Val Leu Ile Asp Ser Gln Asn Asn Thr Lys Asp Ser Thr Arg
        435                 440                 445
```

Leu Lys Tyr Ala Val Lys Val Ser
450 455

<210> 28
<211> 993
<212> PRT
<213> Homo sapiens

<400> 28

Met Pro Ala Leu Ala Arg Asp Gly Gly Gln Leu Pro Leu Leu Val Val
1 5 10 15

Phe Ser Ala Met Ile Phe Gly Thr Ile Thr Asn Gln Asp Leu Pro Val
20 25 30

Ile Lys Cys Val Leu Ile Asn His Lys Asn Asn Asp Ser Ser Val Gly
35 40 45

Lys Ser Ser Ser Tyr Pro Met Val Ser Glu Ser Pro Glu Asp Leu Gly
50 55 60

Cys Ala Leu Arg Pro Gln Ser Ser Gly Thr Val Tyr Glu Ala Ala Ala
65 70 75 80

Val Glu Val Asp Val Ser Ala Ser Ile Thr Leu Gln Val Leu Val Asp
85 90 95

Ala Pro Gly Asn Ile Ser Cys Leu Trp Val Phe Lys His Ser Ser Leu
100 105 110

Asn Cys Gln Pro His Phe Asp Leu Gln Asn Arg Gly Val Val Ser Met
115 120 125

Val Ile Leu Lys Met Thr Glu Thr Gln Ala Gly Glu Tyr Leu Leu Phe
130 135 140

Ile Gln Ser Glu Ala Thr Asn Tyr Thr Ile Leu Phe Thr Val Ser Ile
145 150 155 160

Arg Asn Thr Leu Leu Tyr Thr Leu Arg Arg Pro Tyr Phe Arg Lys Met
165 170 175

Glu Asn Gln Asp Ala Leu Val Cys Ile Ser Glu Ser Val Pro Glu Pro
180 185 190

Ile Val Glu Trp Val Leu Cys Asp Ser Gln Gly Glu Ser Cys Lys Glu
195 200 205

Glu Ser Pro Ala Val Val Lys Lys Glu Glu Lys Val Leu His Glu Leu
210 215 220

Phe Gly Thr Asp Ile Arg Cys Cys Ala Arg Asn Glu Leu Gly Arg Glu
225 230 235 240

Cys Thr Arg Leu Phe Thr Ile Asp Leu Asn Gln Thr Pro Gln Thr Thr
245 250 255

Leu Pro Gln Leu Phe Leu Lys Val Gly Glu Pro Leu Trp Ile Arg Cys
260 265 270

Lys Ala Val His Val Asn His Gly Phe Gly Leu Thr Trp Glu Leu Glu
275 280 285

Asn Lys Ala Leu Glu Glu Gly Asn Tyr Phe Glu Met Ser Thr Tyr Ser
290 295 300

Thr Asn Arg Thr Met Ile Arg Ile Leu Phe Ala Phe Val Ser Ser Val
305 310 315 320

Ala Arg Asn Asp Thr Gly Tyr Tyr Thr Cys Ser Ser Ser Lys His Pro
325 330 335

Ser Gln Ser Ala Leu Val Thr Ile Val Glu Lys Gly Phe Ile Asn Ala
340 345 350

Thr Asn Ser Ser Glu Asp Tyr Glu Ile Asp Gln Tyr Glu Glu Phe Cys
355 360 365

Phe Ser Val Arg Phe Lys Ala Tyr Pro Gln Ile Arg Cys Thr Trp Thr
370 375 380

Phe Ser Arg Lys Ser Phe Pro Cys Glu Gln Lys Gly Leu Asp Asn Gly
385 390 395 400

Tyr Ser Ile Ser Lys Phe Cys Asn His Lys His Gln Pro Gly Glu Tyr
405 410 415

Ile Phe His Ala Glu Asn Asp Asp Ala Gln Phe Thr Lys Met Phe Thr
420 425 430

Leu Asn Ile Arg Arg Lys Pro Gln Val Leu Ala Glu Ala Ser Ala Ser
435 440 445

Gln Ala Ser Cys Phe Ser Asp Gly Tyr Pro Leu Pro Ser Trp Thr Trp
450 455 460

Lys Lys Cys Ser Asp Lys Ser Pro Asn Cys Thr Glu Glu Ile Thr Glu
465 470 475 480

Gly Val Trp Asn Arg Lys Ala Asn Arg Lys Val Phe Gly Gln Trp Val
485 490 495

Ser Ser Ser Thr Leu Asn Met Ser Glu Ala Ile Lys Gly Phe Leu Val
500 505 510

Lys Cys Cys Ala Tyr Asn Ser Leu Gly Thr Ser Cys Glu Thr Ile Leu
515 520 525

Leu Asn Ser Pro Gly Pro Phe Pro Phe Ile Gln Asp Asn Ile Ser Phe
530 535 540

Tyr Ala Thr Ile Gly Val Cys Leu Leu Phe Ile Val Val Leu Thr Leu
545 550 555 560

Leu Ile Cys His Lys Tyr Lys Lys Gln Phe Arg Tyr Glu Ser Gln Leu
565 570 575

Gln Met Val Gln Val Thr Gly Ser Ser Asp Asn Glu Tyr Phe Tyr Val
580 585 590

Asp Phe Arg Glu Tyr Glu Tyr Asp Leu Lys Trp Glu Phe Pro Arg Glu
595 600 605

Asn Leu Glu Phe Gly Lys Val Leu Gly Ser Gly Ala Phe Gly Lys Val
610 615 620

Met Asn Ala Thr Ala Tyr Gly Ile Ser Lys Thr Gly Val Ser Ile Gln
625 630 635 640

Val Ala Val Lys Met Leu Lys Glu Lys Ala Asp Ser Ser Glu Arg Glu
645 650 655

Ala Leu Met Ser Glu Leu Lys Met Met Thr Gln Leu Gly Ser His Glu
660 665 670

Asn Ile Val Asn Leu Leu Gly Ala Cys Thr Leu Ser Gly Pro Ile Tyr
675 680 685

Leu Ile Phe Glu Tyr Cys Cys Tyr Gly Asp Leu Leu Asn Tyr Leu Arg
690 695 700

Ser Lys Arg Glu Lys Phe His Arg Thr Trp Thr Glu Ile Phe Lys Glu
705 710 715 720

His Asn Phe Ser Phe Tyr Pro Thr Phe Gln Ser His Pro Asn Ser Ser
725 730 735

Met Pro Gly Ser Arg Glu Val Gln Ile His Pro Asp Ser Asp Gln Ile
740 745 750

Ser Gly Leu His Gly Asn Ser Phe His Ser Glu Asp Glu Ile Glu Tyr

755 760 765

Glu Asn Gln Lys Arg Leu Glu Glu Glu Glu Asp Leu Asn Val Leu Thr
770 775 780

Phe Glu Asp Leu Leu Cys Phe Ala Tyr Gln Val Ala Lys Gly Met Glu
785 790 795 800

Phe Leu Glu Phe Lys Ser Cys Val His Arg Asp Leu Ala Ala Arg Asn
805 810 815

Val Leu Val Thr His Gly Lys Val Val Lys Ile Cys Asp Phe Gly Leu
820 825 830

Ala Arg Asp Ile Met Ser Asp Ser Asn Tyr Val Val Arg Gly Asn Ala
835 840 845

Arg Leu Pro Val Lys Trp Met Ala Pro Glu Ser Leu Phe Glu Gly Ile
850 855 860

Tyr Thr Ile Lys Ser Asp Val Trp Ser Tyr Gly Ile Leu Leu Trp Glu
865 870 875 880

Ile Phe Ser Leu Gly Val Asn Pro Tyr Pro Gly Ile Pro Val Asp Ala
885 890 895

Asn Phe Tyr Lys Leu Ile Gln Asn Gly Phe Lys Met Asp Gln Pro Phe
900 905 910

Tyr Ala Thr Glu Glu Ile Tyr Ile Ile Met Gln Ser Cys Trp Ala Phe
915 920 925

Asp Ser Arg Lys Arg Pro Ser Phe Pro Asn Leu Thr Ser Phe Leu Gly
930 935 940

Cys Gln Leu Ala Asp Ala Glu Glu Ala Met Tyr Gln Asn Val Asp Gly
945 950 955 960

Arg Val Ser Glu Cys Pro His Thr Tyr Gln Asn Arg Arg Pro Phe Ser
965 970 975

Arg Glu Met Asp Leu Gly Leu Leu Ser Pro Gln Ala Gln Val Glu Asp
980 985 990

Ser

## Description

### Field of the Invention

Generally, the invention relates to antibody molecules which bind pSYK, and methods for using the same for diagnosis, prognosis, to select patients for treatment with a SYK-targeted therapy, or evaluate the pharmacodynamic profile of a SYK-targeted therapy.

### Sequence Listing

This application contains a Sequence Listing which is submitted in electronically readable format. The electronic Sequence Listing file was created on April 20, 2015, is named "223266-370406 MIL81 Sequence Listing_ST25.txt" and has a size of 47.4 KB (48,590 bytes). The entire contents of the Sequence Listing in the electronic "223266-370406 MIL81 Sequence Listing_ST25.txt" file are incorporated herein by this reference.

### Background

Spleen tyrosine kinase (SYK) is a 72 kDa non-receptor cytoplasmic tyrosine kinase. SYK has a primary amino acid sequence similar to that of zeta-associated protein-70 (ZAP-70) and is involved in receptor-mediated signal transduction. The N-terminal domain of SYK contains two Src-homology 2 (SH2) domains, which bind to diphosphorylated immunoreceptor tyrosine-based activation motifs (ITAMs) found in the cytoplasmic signaling domains of many immunoreceptor complexes. The C-terminus contains the catalytic domain, and includes several catalytic loop autophosphorylation sites that are responsible for receptor-induced SYK activation and subsequent downstream signal propagation. SYK is expressed in many cell types involved in adaptive and innate immunity, including lymphocytes (B cells, T cells, and NK cells), granulocytes (basophils, neutrophils, and eosinophils), monocytes, macrophages, dendritic cells, and mast cells. SYK is expressed in other cell types, including airway epithelium and fibroblasts in the upper respiratory system. See, e.g., Martin Turner et al., Immunology Today (2000) 21(3):148-54; and Michael P. Sanderson et al., Inflammation & Allergy-Drug Targets (2009) 8:87-95.

One of the continued problems with therapy in cancer patients is individual differences in response to therapies. While advances in development of successful cancer therapies progress, only a subset of patients respond to any particular therapy. With the narrow therapeutic index and the toxic potential of many available cancer therapies, such differential responses potentially contribute to patients undergoing unnecessary, ineffective and even potentially harmful therapy regimens. Suziki-Inque, K. *et al.* studied the phosphorylation of Syk and PLC$\gamma$2 by glycoproteins GPVI and GPIb-IX-V, concluding that these glycoproteins stimulate tyrosine phosphorylation of Syk and PLC$\gamma$2 at different sites. (Suzuki-Inque, K. et al., Biochem. J. (2004) 378. 1023-1029). If a designed therapy could be optimized to treat individual patients, such situations could be reduced or even eliminated. Furthermore, targeted designed therapy may provide more focused, successful patient therapy overall.

### Summary

SYK is involved in various signal transduction cascades in cells of the hematopoietic lineage including those involved in B-cell receptor (BCR) activation, B cell migration, and B cell polarization. Abnormal SYK activation has been implicated in several hematopoietic malignancies including acute myeloid leukemia (AML), chronic lymphocytic leukemia (CLL), peripheral T-cell lymphoma (PTCL), follicular lymphoma, mantle cell lymphoma and diffuse large B-cell lymphoma (DLBCL). There is a need to identify patients with abnormal SYK activation as part of effective management of SYK-related disease.

The disclosure is based, at least in part, on the discovery of novel anti-phospho-spleen tyrosine kinase (pSYK) antibodies. The present disclosure relates to prognosis and selecting for treatment of cancer by detection and/or measurement of pSYK by methods described herein. The disclosure further relates to the discovery that subjects with cancer respond to treatment with a SYK inhibitor. In one aspect, the invention generally relates to increased expression of pSYK, e.g., SYK phosphorylated at tyrosine 525 and/or 526 (pSYK Y525/526) in biological samples comprising cells obtained from subjects with cancer. Accordingly, in certain embodiments, the invention relates to treating cancer patients with a SYK inhibitor if a sample from the patient demonstrates an elevated level of pSYK Y525/526.

The invention provides anti-pSYK antibody molecules comprising three heavy chain complementarity determining regions (CDR1, CDR2, and CDR3) comprising amino acid sequences SEQ ID NOs:11, 12 and 13, respectively; and three light chain complementarity determining regions (CDR1, CDR2, and CDR3) comprising amino acid sequences SEQ ID NOs:14, 15 and 16, respectively.

In some embodiment the anti-pSYK antibody molecules of the invention are monoclonal antibodies. In some embodiments, the anti-pSYK antibody molecules of the invention are rabbit or rabbit-derived antibodies, preferably rabbit monoclonal antibodies. In other embodiments, the anti-pSYK antibody molecules of the invention comprise a heavy chain variable region comprising an amino acid sequence according to SEQ ID NO:8, and a light chain variable region comprising an amino acid sequence according to SEQ ID NO:10.
In some embodiments, the anti-pSYK antibody molecules of the invention are conjugated to a detectable label. In specific embodiments, the detectable label is: (i) selected from the group consisting of horseradish peroxidase (HRP), alkaline phosphatase, galactosidase, glucoamylase, lysozyme, saccharide oxidases, heterocyclic oxidases, coupled with an enzyme that employs hydrogen peroxide to oxidize a dye, biotin/avidin, spin labels, bacteriophage labels, and stable free radicals; (ii) a fluorophore selected from fluorescein or a derivatives thereof, rhodamine or a derivative thereof, dansyl, umbelliferone, a luciferase, luciferin, and 2,3-dihydrophthalazinediones; or (iii) a radioactive agent selected from the group consisting of $^{32}P$, $^{3}H$, $^{14}C$, $^{188}Rh$ , $^{43}K$, $^{52}Fe$, $^{57}Co$, $^{67}Cu$, $^{67}Ga$, $^{68}Ga$, $^{77}Br$, $^{81}Rb/^{81M}Kr$, $^{87M}Sr$, $^{99}Tc$, $^{11}In$, $^{113M}In$, $^{123}I$, $^{125}I$, $^{127}Cs$, $^{129}Cs$, $^{131}I$, $^{132}I$, $^{197}Hg$, $^{203}Pb$, $^{206}Bi$, and $^{213}Bi$.

In another aspect, the invention provides isolated nucleic acid sequences that encode the anti-pSYK antibody molecules of the invention. The invention also provides cells comprising the isolated nucleic acid sequence of the invention. In yet another aspect, the invention provides methods of producing the anti-pSYK antibody molecule the inventon, comprising culturing the cell of the invention under conditions that allow production of the anti-pSYK antibody molecule.

In another aspect, the invention provides SYK-targeted therapeutic agents for use in a method of treating a patient having a disease characterized by one or more pSYK-expressing cells, said method comprising: a. detecting pSYK protein expression in a biological sample obtained from the patient using a method comprising: contacting the biological sample with the anti-pSYK antibody molecule of any one of claims 1 to 7; and detecting formation of a complex between the anti-pSYK antibody molecule and pSYK protein; and b. administering the SYK-targeted therapeutic agent to the patient if the biological sample expresses pSYK.
In some embodiments, the SYK-targeted therapeutic agent for use in the invention comprises 6-((1S,2R)-2-aminocyclohexylamino)-7-fluoro-4-(1-methyl-1H-pyrazol-4-yl)-1H-pyrrolo[3,4-c]pyridin-3(2H)-one or a pharmaceutically acceptable salt thereof.
In another embodiment of the SYK-targeted therapeutic agent for use in the invention the patient is a cancer patient, and wherein the biological sample is a sample from tumor cells obtained from the cancer patient. In some embodiments, the disease is acute myeloid leukemia, or diffuse large B-cell lymphoma (DLBCL).

In another aspect, the invention provides methods of evaluating the pharmacodynamics of a SYK-targeted therapy, said method comprising the steps of: a. contacting the anti-pSYK antibody molecule of the invention with a biological sample comprising one or more cells suspected of expressing pSYK from a patient who has received the SYK-targeted therapy according to a dosing regimen; b. detecting formation of a complex between the anti-pSYK antibody molecule and pSYK protein in the biological sample; c. quantifying pSYK expression in the biological sample to determine a pSYK expression level; and d. comparing the pSYK expression level against a database comprising the SYK-targeted therapy. In a specific embodiment, the method further comprises adjusting the dosing regimen based on the pSYK expression level.

Hereinafter, reference to an anti-pSYK antibody molecule or any embodiment thereof in the context of the invention, shall mean the anti-SYK antibody molecule comprising the three heavy chain CDRs and the three light chain CDRs, as defined above.

The anti-pSYK antibody molecules may be useful as naked antibody molecules and as components of immunoconjugates. Accordingly, in another aspect, the invention features immunoconjugates comprising an anti-pSYK antibody molecule of the invention and a therapeutic agent or label. The invention also features methods of using the anti-pSYK antibody molecules and immunoconjucates described herein, e.g., for detection of pSYK and of cells or tissues that express pSYK. Such methods are useful, inter alia, for diagnosis, prognosis, imaging, or staging of a SYK-mediated disease. Accordingly, in some aspects, the invention features methods of selecting a subject for treatment with a SYK-targeted therapy, e.g., an anti-pSYK antibody therapy or a therapeutic regimen comprising a therapeutic agent such as a SYK inhibitor. The invention also provides a SYK-targeted therapeutic agent for use in a method of treating a patient having a disease characterized by one or more pSYK-expressing cells, the method comprising determining if a subject having a disease is a potential candidate for a SYK-targeted therapy, e.g., a SYK-targeted therapy described herein. In some aspects, the treatment includes acquiring knowledge and/or evaluating a sample or subject to determine pSYK expression levels, and if the sample or subject expresses pSYK, then administering a SYK-targeted therapy, e.g., a SYK targeted therapy described herein such as a small molecule inhibitor of SYK. In other aspects, the method features generating a personalized treatment report, e.g., a SYK targeted treatment report, by obtaining a sample from a subject and determining pSYK expression levels or activation status, e.g., by a detection or measurement method described herein such as using an anti-pSYK antibody described herein, and based upon the determination, selecting a targeted treatment for the subject.

Anti-pSYK antibodies, e.g., the anti-pSYK antibodies of the invention, are also useful for evaluating the pharmacodynamics of a SYK-targeted therapy. In some such embodiments, the dosage of the SYK-targeted therapy may be adjusted based on the level of pSYK expression.

In another embodiment, the invention also relates to isolated and/or recombinant nucleic acids encoding anti-pSYK antibody molecule amino acid sequences, as well as host cells comprising such nucleic acids, and methods for producing the anti-pSYK antibody molecules. Also featured herein are reaction mixtures and kits comprising the anti-pSYK antibodies, e.g., an immunoconjugate, described herein, as well as in vitro assays, e.g., comprising an anti-pSYK antibody described herein, to detect pSYK expression.

Specifically, the invention provides an anti-pSYK antibody molecule comprising three heavy chain complementarity determining regions (CDR1, CDR2, and CDR3) comprising amino acid sequences SEQ ID NOs: 11, 12 and 13, respectively; and three light chain complementarity determining regions (CDR1, CDR2, and CDR3) comprising amino acid sequences SEQ ID NOs: 14, 15 and 16, respectively. In some embodiments, the anti-pSYK antibody molecule is a monoclonal antibody. In some embodiments, the anti-pSYK antibody molecule is a rabbit or rabbit-derived antibody. In some embodiments, the antibody is a rabbit monoclonal antibody.

In some embodiments, the anti-pSYK antibody molecule further comprises a heavy chain variable region comprising an amino acid sequence according to SEQ ID NO:8, and a light chain variable region comprising an amino acid sequence according to SEQ ID NO:10.

In some embodiments, the anti-pSYK antibody molecule is conjugated to a detectable label. In some embodiments, the detectable label is selected from the group consisting of horseradish peroxidase (HRP), alkaline phosphatase, galactosidase, glucoamylase, lysozyme, saccharide oxidases, heterocyclic oxidases, coupled with an enzyme that employs hydrogen peroxide to oxidize a dye, biotin/avidin, spin labels, bacteriophage labels, and stable free radicals. In some embodiments, the detectable label is a fluorophores selected from fluorescein or a derivatives thereof, rhodamine or a derivative thereof, dansyl, umbelliferone, a luceriferase, luciferin, and 2,3-dihydrophthalazinediones. In some embodiments, the detectable label is a radioactive agent selected from the group consisting of $^{32}$P, $^{3}$H, $^{14}$C, $^{188}$Rh, $^{43}$K, $^{52}$Fe, $^{57}$Co, $^{67}$Cu, 67Ga, 68Ga, $^{77}$Br, $^{81}$Rb/$^{81M}$Kr, $^{87M}$Sr, $^{99}$Tc, $^{111}$In, $^{113M}$In, $^{123}$I, $^{125}$I, $^{127}$Cs, $^{129}$Cs, $^{131}$I, $^{132}$I, $^{197}$Hg, $^{203}$Pb, $^{206}$Bi, and $^{213}$Bi.

In one embodiment, the invention provides an isolated nucleic acid sequence that encodes the anti-pSYK antibody molecule. In one embodiment, the invention provides a cell comprising the isolated nucleic acid. In one embodiment, the invention provides a method of producing an anti-pSYK antibody molecule, comprising culturing the cell under conditions that allow production of an antibody molecule. Described herein is also a vector comprising one or both of the light chain and heavy chain the anti-pSYK antibody molecule. In one embodiment, the invention provides the use of a SYK-targeted therapeutic agent in a method of treating a patient having a disease characterized by one or more pSYK-expressing cells, said method comprising detecting a pSYK molecule in a biological sample comprising a) contacting the biological sample with the antibody molecule and b) determining if said antibody molecule binds to said pSYK molecule. In some embodiments, the method of detection comprises an immunohistochemistry assay. In some embodiments, the biological sample is a tumor biopsy derived from a patient suspected of having a pSYK expressing cancer. In some embodiments, the method further comprises the step of quantifying pSYK expression in said biological sample. In some embodiments, the quantification of pSYK expression comprises cytoplasmic pSYK expression in said biological sample. In some embodiments, the quantification step comprises an H-score approach.

In some embodiments, the pSYK expressing cancer is a hematological malignancy, selected from a leukemia and a lymphoma. In some embodiments, the hematological malignancy is chronic lymphocytic leukemia (CLL). In some embodiments, the hematological malignancy is acute myeloid leukemia. In some embodiments, the hematological malignancy is diffuse large B-cell lymphoma (DLBCL).

Disclosed herein is also a kit comprising the anti-pSYK antibody molecule and instructions for use. In some examples, the kit further comprises a SYK-targeted therapeutic agent. In some embodiments, the SYK-targeted therapeutic agent comprises a fused heteroaromatic pyrrolidinone. In some embodiments, the SYK-targeted therapeutic agent comprises 6-((1R,2S)-2-Aminocyclohexylamino)-7-fluoro-4-(1-methyl-1H-pyrazol-4-yl)-1H-pyrrolo[3,4-c]pyridin-3(2H)-one or a pharmaceutically acceptable salt thereof. In some embodiments, the SYK-targeted therapeutic agent comprises 6-((1S,2R)-2-Aminocyclohexylamino)-7-fluoro-4-(1-methyl-1H-pyrazol-4-yl)-1H-pyrrolo[3,4-c]pyridin-3(2H)-one or a pharmaceutically acceptable salt thereof. In some embodiments, the SYK-targeted therapeutic agent comprises 6-((1R,2S)-2-Aminocyclohexylamino)-4-(1-(difluoromethyl)-1H-pyrazol-4-yl)-7-fluoro-1H-pyrrolo[3,4-c]pyridin-3(2H)-one or a pharmaceutically acceptable salt thereof. In some embodiments, the SYK-targeted therapeutic agent comprises cis-6-(2-Aminocyclohexylamino)-7-fluoro-4-(1-methyl-1H-pyrazol-4-yl)-1H-pyrrolo[3,4-c]pyridin-3 (2H)-one or a pharmaceutically acceptable salt thereof. In some embodiments, the SYK-targeted therapeutic agent comprises 6-((3R,4R)-3-aminotetrahydro-2H-pyran-4-ylamino)-4-(1-(difluoromethyl)-1H-pyrazol-4-yl)-7-fluoro-1H-pyrrolo[3,4-c]pyridin-3(2H)-one or a pharmaceutically acceptable salt thereof. In some embodiments, the SYK-targeted therapeutic agent comprises 6-((3R,4R)-3-Aminotetrahydro-2H-pyran-4-ylamino)-7-fluoro-4-(3-methylisothiazol-5-yl)-1H-pyrrolo[3,4-c]pyridin-3(2H)-one or a pharmaceutically acceptable salt thereof.

The invention provides a SYK-targeted therapeutic agent for use in a method of treating a patient having a disease characterized by one or more pSYK-expressing cells, comprising: a. detecting pSYK protein expression in a biological sample obtained from the patient; and b. administering a SYK-targeted therapeutic agent to the patient if the biological sample expresses pSYK. In some embodiments, the detection step comprises the steps of: a) contacting the biological sample with an anti-pSYK antibody molecule comprising three heavy chain complementarity determining regions (CDR1, CDR2, and CDR3) comprising amino acid sequences according to SEQ ID NOs: 11, 12 and 13, respectively; and three light chain complementarity determining regions (CDR1, CDR2, and CDR3) comprising amino acid sequences according to SEQ ID NOs: 14, 15 and 16, respectively; and b) detecting formation of a complex between the anti-pSYK antibody molecule and pSYK protein. In some embodiments, the detection step is performed via immunohistochemistry.

In some embodiments, the SYK-targeted therapeutic agent comprises a fused heteroaromatic pyrrolidinone. In some embodiments, the SYK-targeted therapeutic agent comprises 6-((1R,2S)-2-Aminocyclohexylamino)-7-fluoro-4-(1-methyl-1H-pyrazol-4-yl)-1H-pyrrolo [3,4-c]pyridin-3(2H)-one or a pharmaceutically acceptable salt thereof. In some embodiments, the SYK-targeted therapeutic agent comprises 6-((1S,2R)-2-Aminocyclohexylamino)-7-fluoro-4-(1-methyl-1H-pyrazol-4-yl)-1H-pyrrolo[3,4-c]pyridin-3(2H)-one or a pharmaceutically acceptable salt thereof. In some embodiments, the SYK-targeted therapeutic agent comprises 6-((1R,2S)-2-Aminocyclohexylamino)-4-(1-(difluoromethyl)-1H-pyrazol-4-yl)-7-fluoro-1H-pyrrolo[3,4-c]pyridin-3(2H)-one or a pharmaceutically acceptable salt thereof. In some embodiments, the SYK-targeted therapeutic agent comprises cis-6-(2-Aminocyclohexylamino)-7-fluoro-4-(1-methyl-1H-pyrazol-4-yl)-1H-pyrrolo[3,4-c]pyridin-3 (2H)-one or a pharmaceutically acceptable salt thereof. In some embodiments, the SYK-targeted therapeutic agent comprises 6-((3R,4R)-3-aminotetrahydro-2H-pyran-4-ylamino)-4-(1-(difluoromethyl)-1H-pyrazol-4-yl)-7-fluoro-1H-pyrrolo[3,4-c]pyridin-3(2H)-one or a pharmaceutically acceptable salt thereof. In some embodiments, the SYK-targeted therapeutic agent comprises 6-((3R,4R)-3-Aminotetrahydro-2H-pyran-4-ylamino)-7-fluoro-4-(3-methylisotbiazol-5-yl)-1H-pyrrolo[3,4-c]pyridin-3(2H)-one or a pharmaceutically acceptable salt thereof.

In some embodiments, the disease characterized by one or more pSYK-expressing cells is a cancer. In some embodiments, the pSYK expressing cancer is a hematological malignancy selected from a leukemia and a lymphoma. In some embodiments, the hematological malignancy is chronic lymphocytic leukemia (CLL). In some embodiments, the hematological malignancy is acute myeloid leukemia (AML). In some embodiments, the hematological malignancy is diffuse large B-cell lymphoma (DLBCL).

In some embodiments, the biological sample is a cell or a tissue biopsy. In some embodiments, the cell or tissue biopsy is a tumor biopsy.

In one embodiment, the SYK-targeted therapeutic agent for use in the invention comprises determining sensitivity of cancer cells to a SYK-targeted therapeutic agent, the method comprising the steps of: a) providing a sample from cancer cells from a patient that has cancer; b) contacting the sample with an anti-pSYK antibody molecule comprising three heavy chain complementarity determining regions (CDR1, CDR2, and CDR3) comprising amino acid sequences according to SEQ ID NOs: 11, 12 and 13, respectively; and three light chain complementarity determining regions (CDR1, CDR2, and CDR3) comprising amino acid sequences according to SEQ ID NOs: 14, 15 and 16, respectively; and c) detecting formation of a complex between the anti-pSYK antibody molecule and pSYK protein, thereby determining the sensitivity of the cancer to the SYK-targeted therapeutic agent, and/or determining if a subject is a candidate for treatment with a SYK-targeted therapy.

In one embodiment, the SYK-targeted therapeutic agent for use in the invention comprise evaluating whether a subject is a potential candidate for a SYK-targeted therapy, the method comprising the steps of: a) providing a sample from cancer cells from a patient that has cancer; b) contacting the sample with an anti-pSYK antibody molecule comprising three heavy chain complementarity determining regions (CDR1, CDR2, and CDR3) comprising amino acid sequences according to SEQ ID NOs: 11, 12 and 13, respectively; and three light chain complementarity determining regions (CDR1, CDR2, and CDR3) comprising amino acid sequences according to SEQ ID NOs: 14, 15 and 16, respectively; and c) detecting formation of a complex between the anti-pSYK antibody molecule and pSYK protein, thereby determining the sensitivity of the cancer to the SYK-targeted therapeutic agent, and/or determining if a subject is or identifying the subject as a candidate for treatment with a SYK-targeted therapy. In some embodiments, the detection step is performed via immunohistochemistry.

In some embodiments, the cancer is a hematological malignancy. In some embodiments, the hematological malignancy is chronic lymphocytic leukemia (CLL). In some embodiments, the hematological malignancy is acute myeloid leukemia (AML). In some embodiments, the hematological malignancy is diffuse large B-cell lymphoma (DLBCL).

In some embodiments, the SYK-targeted therapeutic agent for use in the invention further comprises the step of administering the SYK-targeted therapeutic agent to the patient. In some embodiments, the SYK-targeted therapeutic agent comprises a fused heteroaromatic pyrrolidinone. In some embodiments, the SYK-targeted therapeutic agent comprises 6-((1R,2S)-2-Aminocyclohexylamino)-7-fluoro-4-(1-methyl-1H-pyrazol-4-yl)-1H-pyrrolo[3,4-c]pyridin-3(2H)-one or a pharmaceutically acceptable salt thereof. In some embodiments, the SYK-targeted therapeutic agent comprises 6-((1S,2R)-2-Aminocyclohexylamino)-7-fluoro-4-(1-methyl-1H-pyrazol-4-yl)-1H-pyrrolo[3,4-c]pyridin-3(2H)-one or a pharmaceutically acceptable salt thereof. In some embodiments, the SYK-targeted therapeutic agent comprises 6-((1R,2S)-2-Aminocyclohexylamino)-4-(1-(difluoromethyl)-1H-pyrazol-4-yl)-7-fluoro-1H-pyrrolo[3,4-c]pyridin-3(2H)-one or a pharmaceutically acceptable salt thereof. In some embodiments, the SYK-targeted therapeutic agent comprises cis-6-(2-Aminocyclohexylamino)-7-fluoro-4-(1-methyl-1H-pyrazol-4-yl)-1H-pyrrolo[3,4-c]pyridin-3 (2H)-one or a pharmaceutically acceptable salt thereof. In some embodiments, the SYK-targeted therapeutic agent comprises 6-((3R,4R)-3-aminotetrahydro-2H-pyran-4-ylamino)-4-(1-(difluoromethyl)-1H-pyrazol-4-yl)-7-fluoro-1H-pyrrolo[3,4-c]pyridin-3(2H)-one or a pharmaceutically acceptable salt thereof. In some embodiments, the SYK-targeted therapeutic agent comprises 6-((3R,4R)-3-Aminotetrahydro-2H-pyran-4-ylamino)-7-fluoro-4-(3-methylisothiazol-5-yl)-1H-pyrrolo[3,4-c]pyridin-3(2H)-one or a pharmaceutically acceptable salt thereof.

The use of a SYK-targeted therapeutic agent for treatment and the method of evaluating the pharmacodynamics of a SYK-targeted therapy use a reaction mixture comprising a biological sample and an anti-pSYK antibody molecule comprising three heavy chain complementarity determining regions (CDR1, CDR2, and CDR3) comprising amino acid sequences according to SEQ ID NOs: 11, 12 and 13, respectively; and three light chain complementarity determining regions (CDR1, CDR2, and CDR3) comprising amino acid sequences according to SEQ ID NOs: 14, 15 and 16, respectively. In some embodiments, the biological sample comprises one or more cells. In some embodiments, the biological sample comprises cancer cells obtained from the patient. In some embodiments, the biological sample comprises a tissue sample. In some embodiments, the tissue sample is a paraffin-embedded tissue sample. In some embodiments, the biological sample is a primary or metastatic tumor biopsy sample. In some embodiments, the biological sample is mounted on a slide.

In some embodiments, the cell is a chronic lymphocytic leukemia cell. In some embodiments, the cell is an acute myeloid leukemia cell. In some embodiments, the cell is a diffuse large B-cell lymphoma cell. In some embodiments, the cell is a peripheral T-cell lymphoma cell. In some embodiments, the biological sample is suspected of containing pSYK protein.

In some embodiments, the reaction mixture further comprises a reagent suitable for detecting formation of a complex between the anti-pSYK antibody and pSYK protein.

In one embodiment, the invention provides the use of a SYK-targeted therapeutic agent in a method of treating a patient having a disease characterized by one or more pSYK-expressing cells, said method comprising generating a personalized cancer treatment report, said method comprising the steps of: a) contacting a biological sample comprising one or more cancer cells obtained from a cancer patient suspected of having a pSYK-expressing cancer with an anti-pSYK antibody molecule comprising three heavy chain complementarity determining regions (CDR1, CDR2, and CDR3) comprising amino acid sequences according to SEQ ID NOs: 11, 12 and 13, respectively; and three light chain complementarity determining regions (CDR1, CDR2, and CDR3) comprising amino acid sequences according to SEQ ID NOs: 14, 15 and 16, respectively; b) detecting formation of a complex between the anti-pSYK molecule and pSYK protein in the biological sample; c) quantifying pSYK expression in the biological sample; d) comparing the pSYK expression level against a database comprising expression levels from a selection of SYK-targeted therapies; and e) selecting a SYK-targeted therapy and, optionally, a dosing regimen based on the pSYK expression level.

In some embodiments, the pSYK-expressing cancer is is a hematological malignancy selected from a leukemia and a lymphoma. In some embodiments, the hematological malignancy is chronic lymphocytic leukemia (CLL). In some embodiments, the hematological malignancy is acute myeloid leukemia (AML). In some embodiments, the hematological malignancy is diffuse large B-cell lymphoma (DLBCL).

In some embodiments, the detection step is performed via immunohistochemistry. In some embodiments, the quantification of pSYK expression comprises cytoplasmic pSYK expression in said biological sample. In some embodiments, the quantification step comprises an H-score approach.

In one embodiment, the invention provides a method of evaluating the pharmacodynamics of a SYK-targeted therapy, said method comprising the steps of: a) administering to a patient a SYK-targeted therapy; b) obtaining a biological sample comprising one or more cells suspected of expressing pSYK from the patient; c) contacting the biological sample with an anti-pSYK antibody molecule comprising three heavy chain complementarity determining regions (CDR1, CDR2, and CDR3) comprising amino acid sequences according to SEQ ID NOs: 11, 12 and 13, respectively; and three light chain complementarity determining regions (CDR1, CDR2, and CDR3) comprising amino acid sequences according to SEQ ID NOs: 14, 15 and 16, respectively; d) detecting formation of a complex between the anti-pSYK molecule and pSYK protein in the biological sample; e) quantifying pSYK expression in the biological sample; f) comparing the pSYK expression level against a database comprising SYK-targeted therapy; and g) optionally, adjusting the dosing regimen based on the pSYK expression level.

In some embodiments, the pSYK-expressing cancer is a hematological malignancy selected from a leukemia and a lymphoma. In some embodiments, the hematological malignancy is chronic lymphocytic leukemia (CLL). In some embodiments, the hematological malignancy is acute myeloid leukemia (AML). In some embodiments, the hematological malignancy is diffuse large B-cell lymphoma (DLBCL).

In some embodiments, the detection step is performed via immunohistochemistry. In some embodiments, the quantification of pSYK expression comprises cytoplasmic pSYK expression in said biological sample. In some embodiments, the quantification step comprises an H-score approach.

In some embodiments, the SYK-targeted therapy comprises a fused heteroaromatic pyrrolidinone. In some embodiments, the SYK-targeted therapy comprises 6-((1R,2S)-2-Aminocyclohexylamino)-7-fluoro-4-(1-methyl-1H-pyrazol-4-yl)-1H-pyrrolo[3,4-c]pyridin-3(2H)-one or a pharmaceutically acceptable salt thereof In some embodiments, the SYK-targeted therapy comprises 6-((1S,2R)-2-Aminocyclohexylamino)-7-fluoro-4-(1-methyl-1H-pyrazol-4-yl)-1H-pyrrolo[3,4-c]pyridin-3(2H)-one or a pharmaceutically acceptable salt thereof. In some embodiments, the SYK-targeted therapy comprises 6-((1R,2S)-2-Aminocyclohexylamino)-4-(1-(difluoromethyl)-1H-pyrazol-4-yl)-7-fluoro-1H-pyrrolo[3,4-c]pyridin-3(2H)-one or a pharmaceutically acceptable salt thereof. In some embodiments, the SYK-targeted therapy comprises cis-6-(2-Aminocyclohexylamino)-7-fluoro-4-(1-methyl-1H-pyrazol-4-yl)-1H-pyrrolo[3,4-c]pyridin-3 (2H)-one or a pharmaceutically acceptable salt thereof. In some embodiments, the SYK-targeted therapy comprises 6-((3R,4R)-3-aminotetrahydro-2H-pyran-4-ylamin-(difluoromethyl)- 1H-pyrazol-4-yl)-7-fluoro-1H-pyrrolo[3,4-c]pyridin-3(2H)-one or a pharmaceutically acceptable salt thereof. In some embodiments, the SYK-targeted therapy comprises 6-((3R,4R)-3-Aminotetrahydro-2H-pyran-4-ylamino)-7-fluoro-4-(3-methylisothiazol-5-yl)-1H-pyrrolo[3,4-c]pyridin-3(2H)-one or a pharmaceutically acceptable salt thereof.

Disclosesd herein is also a method for identifying a compound as a SYK inhibitor, comprising: a) contacting a cell comprising pSYK with a test compound; and b) measuring the effect of the test compound on the phosphorylation or Y525/526 in the cell, and wherein the test compound is a SYK inhibitor if it inhibits the phosphorylation of Y525/526.

Futher, disclosed herein is a method for paying for the treatment of cancer with an SYK inhibitor comprising: a) recording the activation status of SYK in a patient sample comprising tumor cells, and b) paying for the SYK inhibitor treatment if the SYK activation status indicates a favorable outcome.

Other features, objects, and advantages of the invention(s) disclosed herein will be apparent from the description and drawings, and from the claims.

## Brief Description of the Drawings

Figure 1 depicts a SYK RNA baseline screen in cells, xenografts and primary tumors of hematologic and lymphoma malignancies. HBL1 was added in a later experiment. The arrows point to examples of cells with low, medium, and high expression of SYK RNA (LY10, HBL1, PHTX-95L, respectively).

Figure 2 depicts pSYK IHC staining of PHTX 95L Xenograft Tissue using Epitomics anti-pSYK Y525/6 antibody (Epitomics 2175-1) compared to antibodies from MIL81 hybridoma subclones, MIL81-1-8, MIL81-2-1, and MIL81-99-1. The xenograft-implanted mice were either untreated or treated with a SYK inhibitor (Compound A).

Figure 3 quantifies pSYK IHC staining of PHTX 95L xenograft tissue using Epitomics 2175-1 antibody compared to antibody from MIL81 hybridoma subclones. The xenograft-implanted mice were either untreated (Control) or treated with a SYK inhibitor (Compound A). MIL81-1-8 is the best due to its intensity and signal to noise ratio.

Figure 4 depicts pSYK IHC background staining of PHTX 95L xenograft tissue using Epitomics 2175-1 antibody compared to MIL81-1-8 antibody.

Figure 5 depicts pSYK IHC staining of HBL1 xenograft tissue using Epitomics anti-pSYK Y525/6 antibody compared to MIL81-1-8 antibody. The xenograft-implanted mice were either untreated or treated with a SYK inhibitor (Compound A).

Figure 6 depicts pSYK IHC staining of OCI LY10 xenograft tissue using Epitomics anti-pSYK Y525/6 antibody compared to MIL81-1-8 antibody. The xenograft-implanted mice were either untreated or treated with a SYK inhibitor (Compound A).

Figure 7 quantifies pSYK IHC staining of three xenograft models with different levels of SYK expression (PHTX-95L, HBL1, and OCI-LY10) using Epitomics 2175-1 compared to MIL81-1-8 antibody. The xenograft-implanted mice were either untreated or treated with a SYK inhibitor (Compound A, 120 or 90 mg/kg).

Figure 8 depicts the MIL81-1-8 antibody IHC staining pattern compared to staining of other markers Epitomics 2175-1 (pSYK Y525/526), Epitomics #2173 (SYK pY323) and Epitomics #1688 (total SYK) in DLBCL Tissue Micro Array (TMA) Cores. This shows that MIL81-1-8 has an off-target (nuclear staining) effect that is seen with a number of pSYK antibodies on these samples.

Figure 9 depicts peptide blocking for MIL81-1-8 validation testing with a pellet of pervanadate-treated WSU-DLCL cells, PHTX-95L xenograft or a TMA core of a DLBCL tumor. When peptide immunogen is added to the antibody prior to incubation with the tissue, the pSYK IHC staining is blocked.

Figure 10 depicts phosphatase treatment for MIL81-1-8 validation testing with a pellet of pervanadate-treated WSU-DLCL cells, PHTX-95L xenograft or a sample of normal spleen tissue. Prior to antibody staining, some slides were treated with phosphatase. MIL81-1-8 does not stain phosphatase-treated tissue, except some cells in normal spleen.

Figure 11 depicts MIL81-1-8 IHC cytoplasmic and nuclear staining on DLBCL tissue biopsies. Cytoplasmic staining predominates in the periphery of the biopsy, while nuclear staining predominates in the interior portion of the biopsy.

Figure 12 depicts cytoplasmic IHC staining of a peripheral portion of a DLBCL biopsy tissue by MIL81-1-8 antibody, Epitomics 2175-1 (pSYK Y525/526) antibody, Epitomics #2173 (SYK pY323) antibody and Epitomics #1688 (total SYK) antibody.

Figure 13 depicts nuclear IHC staining of an interior portion of a DLBCL biopsy by MIL81-1-8 antibody, Epitomics 2175-1 (pSYK Y525/526) antibody, Epitomics #2173 (SYK pY323) antibody and Epitomics #1688 (total SYK) antibody.

Figure 14 depicts MIL81-1-8 compared to commercially available SYK antibodies (Epitomics #2173 (SYK pY323) antibody and Epitomics 2175-1 (pSYK Y525/526) antibody) on DLBCL tissue biopsies. This shows that MIL81-1-8 provides an improvement over commercially available Epitomics 2175-1.

Figure 15 depicts pathology scores of MIL81-1-8 and commercially available SYK antibodies (Epitomics #1688 (total SYK) antibody, Epitomics #2173 (SYK pY323) antibody and Epitomics 2175-1 (pSYK Y525/526) antibody) on DLBCL tissue biopsies (the biopsies of Fig. 14 as well as additional biopsies). This shows that MIL81-1-8 provides an improvement over commercially available Epitomics 2175-1.

Figure 16 depicts staining of a xenograft of a SYK negative line (H1650 lung adenocarcinoma) by MIL81-1-8 antibody, Epitomics 2175-1 (pSYK Y525/526) antibody, Epitomics #2173 (SYK pY323) antibody and Epitomics #1688 (total SYK) antibody. Note no staining by Epitomics #1688, even at 1:100 dilution (4x the normal concentration).

Figure 17 depicts staining of a xenograft of a SYK negative line (H1650 lung adenocarcinoma) by MIL81-1-8 antibody, Epitomics 2175-1 (pSYK Y525/526) antibody, Epitomics #2173 (SYK pY323) antibody and Cell Signaling Technologies 2711 pSYK Y525/526 antibody.

Figure 18 depicts a MIL81-1-8 hybridoma subclone antibody compared to MIL81-1-8 molecular clone antibody staining of WSU-DLCL cell pellets, xenograft of TMD8 lymphoma cell line and xenograft of TMD8 lymphoma cell line grown in mice treated with Compound A.

Figure 19 depicts a MIL81-1-8 hybridoma subclone antibody compared to MIL81-1-8 molecular clone antibody staining of HBL1 xenograft, PHTX95L xenograft, and xenograft of PHTX95L tumor from mice treated with Compound A.

Figure 20 depicts a MIL81-1-8 hybridoma subclone antibody compared to MIL81-1-8 molecular clone antibody using human biopsies of DLBCL tumors or normal lymph node.

Figure 21 depicts the $EC_{50}$ for Compound A following 72 hours of treatment in a variety of DLBCL cell lines.

Figure 22 depicts the anti-tumor activity in the OCI-LY10 (ABC) xenograft model for Compound A.

Figure 23 depicts the anti-tumor activity in the PHTX-95L primary DLBCL model for Compound A.

Figure 24 depicts the anti-tumor activity in the OCI-LY19 (GCB) xenograft model for Compound A.

Figure 25 depicts the staining differences in PHTX-95L (DLBCL) xenograft model when stained with AMP HQ IHC compared to non amplified IHC and the result after treatment of the mouse with Compound A.

Figure 26 depicts the decrease in nuclear off target staining seen in human DLBCL biopsy samples when stained with AMP HQ IHC (Figs. 26 B, D, F) compared to non amplified IHC (Figs 26 A, C, E). Fig. 26 A and B, 1x magnification; C and D, 5x magnification; E and F, 20x magnification.

Figure 27 depicts the staining pattern of the Dual TSA immunofluorescent staining on MV-4-11 (AML) cell pellets, NCI-H82 (lung) cell pellets, and KG-1 (AML) xenograft tissue.

Figure 28 depicts the results of an IF assay using a dual MIL81-1-8 CD34/CD117 to detect elevated pSYK Y525/526.

## Detailed Description

The invention provides a SYK inhibitor for use in a method of treating a cancer characterized by one or more pSYK-expressing cells, comprising administering to a patient a therapeutically effective amount of said SYK inhibitor, such as a small molecule inhibitor of SYK or a pharmaceutically acceptable salt or pharmaceutical composition thereof. In some embodiments, the tumor sample of the cancer patient is characterized by having an elevated level of pSYK. In some embodiments, the biological sample of the cancer patient e.g., tumor sample is characterized by having an elevated level of pSYK. In some embodiments, the tumor sample of the cancer patient is characterized by having pSYK Y525/526, e.g., as measured by a pSYK immunohistochemistry (IHC) assay. In some embodiments, the present invention provides the use of a SYK-targeted therapeutic agent in a method of treating a patient having a disease characterized by one or more pSYK-expressing cells, said method comprising continuing a therapeutic regimen comprising a SYK inhibitor, comprising obtaining after some treatment with the SYK inhibitor a second biological sample from the patient, measuring the level of pSYK and continuing the treatment if the second sample is characterized as having an elevated level of pSYK or less pSYK than in the first sample.

In some embodiments, the methods further include detecting an additional phosphoprotein, such as phosphorylated B-Cell Linker protein (pBLNK) and/or phosphorylated Bruton agammaglobulinemia Tyrosine Kinase (pBTK) and/or total SYK, such as SYK detected regardless of the presence of phosphate on any amino acid residue(s) (e.g.,

using Epitomics 1688-1 antibody). In certain embodiments, the methods further include detecting phosphorylated Fms-related Tyrosine Kinase 3 (pFLT3).

In certain embodiments, the SYK-targeted therapeutic agent for use in the invention relates to therapeutic methods which further include the step of beginning, continuing, or commencing a therapy accordingly where the presence of pSYK, such as pSYK Y525/526 is measured. In addition, the methods include therapeutic methods which further include the step of stopping, discontinuing, altering or halting a SYK-targeted therapy accordingly where the presence or reappearance of pSYK, such as pSYK Y525/526 indicates that the patient is expected to demonstrate an unfavorable outcome with the treatment, e.g., with the SYK inhibitor, e.g., as compared to a patient identified as having a favorable outcome receiving the same therapeutic regimen. In another aspect, methods encompass analysis of a patient not yet being treated with a therapy, e.g., a SYK inhibitor, and identification of the patient for treatment and prediction of treatment outcome based upon the presence of pSYK as described herein. Such methods can include not being treated with the therapy, e.g., SYK inhibitor, being treated with therapy, e.g., SYK inhibitor, being treated with a SYK inhibitor in combination with one more additional therapies, being treated with an alternative therapy to a SYK inhibitor, or being treated with a more aggressive dosing and/or administration regimen of a therapy, e.g., SYK inhibitor, e.g., as compared to the dosing and/or administration regimen of a patient identified as having a favorable outcome to standard SYK inhibitor therapy. Thus, the invention can eliminate ineffective or inappropriate use of therapy, e.g., SYK inhibitor therapy regimens.

Disclosed herein are also methods to determine the activity of an agent, the efficacy of an agent, or identify new therapeutic agents or combinations. Such methods include methods to identify an agent as useful, e.g., as a SYK inhibitor for treating a cancer, e.g., a hematological cancer or a solid tumor cancer, based on its ability to affect the presence or amount of a pSYK, e.g., as detected or measured by an antibody or method described herein. An inhibitor which decreases the presence of pSYK, e.g., pSYK Y526/526 (i.e., in a cell population, the inhibitor selects against cells comprising pSYK) in a manner that indicates favorable outcome of a patient having cancer would be a candidate agent for the treatment of cancer. Also, an agent which is able to decrease the viability of a tumor cell comprising pSYK or decrease an unfavorably high amount of pSYK would be a candidate therapeutic agent for treating the cancer.

Disclosed herein is a method to evaluate whether to treat or pay for the treatment of cancer, e.g., hematological cancer or solid tumor cancer by reviewing the amount of a patient's pSYK for indication of outcome to a cancer therapy, e.g., a SYK inhibitor therapy regimen, determining whether payment should be made and paying for cancer therapy of the patient if a favorable outcome is indicated.

SYK is a key mediator of signaling through immune receptors (B-cell and Fc receptors), proteins associated with Epstein-Barr Virus (EBV) latency and transformation, and cell-cell and cell-matrix interactions. SYK is activated in lymphomas/leukemias, EBV-associated tumors and other solid tumors. SYK, BTK and PI3K$\delta$ are clinically validated targets in BCR-activated malignancies. SYK regulates several biological processes including innate and adaptive immunity, cell adhesion, osteoclast maturation, platelet activation and vascular development. SYK assembles into signaling complexes, e.g., signalosomes, with activated receptors at the plasma membrane via interaction between its SH2 domains and receptor tyrosine-phosphorylated immunoreceptor tyrosine-based activation motif (ITAM) domains. The phosphorylation of the ITAM domains is generally mediated by SRC subfamily kinases upon engagement of the receptor. More rarely signal transduction via SYK could be ITAM-independent. Direct downstream effectors phosphorylated by SYK include VAV1, phospholipase C gamma (PLC$\gamma$) such as PLCG1, PI-3-kinase, LCP2 and BLNK. Activated upon BCR engagement, SYK phosphorylates and activates BLNK, an adapter linking the activated BCR to downstream signaling adapters and effectors. It also phosphorylates and activates PLCG1 and the PKC signaling pathway. It also phosphorylates BTK and regulates its activity in B-cell antigen receptor (BCR)-coupled signaling. SYK also plays a role in T-cell receptor signaling and innate immune response to fungal, bacterial and viral pathogens. SYK also plays also a role in non-immune processes, including vascular development where it may regulate blood and lymphatic vascular separation. SYK is required for osteoclast development and function, and functions in the activation of platelets by collagen.

Nucleotide sequence for human SYK (GenBank Accession No. NM_003177; SEQ ID NO:1):

acactgggag gaagtgcggg ccgcctgccc gggcgcgtta aggaagttgc ccaaaatgag
gaagagccgc gggcccggcg gctgaggcca ccccggcggc ggctggagag cgaggaggag
cgggtggccc cgcgctgcgc ccgccctcgc ctcacctggc gcaggtggac acctgcgcag
gtgtgtgccc tccggcccct gaagcatggc cagcagcggc atggctgaca gcgccaacca
cctgcccttc tttttcggca acatcacccg ggaggaggca gaagattacc tggtccaggg
gggcatgagt gatgggcttt atttgctgcg ccagagccgc aactacctgg gtggcttcgc
cctgtccgtg gcccacggga ggaaggcaca ccactacacc atcgagcggg agctgaatgg
cacctacgcc atcgccggtg gcaggaccca tgccagcccc gccgacctct gccactacca
ctcccaggag tctgatggcc tggtctgcct cctcaagaag cccttcaacc ggccccaagg
ggtgcagccc aagactgggc cctttgagga tttgaaggaa aacctcatca gggaatatgt
gaagcagaca tggaacctgc agggtcaggc tctggagcag gccatcatca gtcagaagcc
tcagctggag aagctgatcg ctaccacagc ccatgaaaaa atgccttggt tccatggaaa
aatctctcgg gaagaatctg agcaaattgt cctgatagga tcaaagacaa atggaaagtt
cctgatccga gccagagaca acaacggctc ctacgccctg tgcctgctgc acgaagggaa
ggtgctgcac tatcgcatcg acaaagacaa gacagggaag ctctccatcc ccgagggaaa
gaagttcgac acgctctggc agctagtcga gcattattct tataaagcag atggtttgtt
aagagttctt actgtcccat gtcaaaaaat cggcacacag ggaaatgtta attttggagg
ccgtccacaa cttccaggtt cccatcctgc gacttggtca gcgggtggaa taatctcaag
aatcaaatca tactccttcc caaagcctgg ccacagaaag tcctcccctg cccaagggaa
ccggcaagag agtactgtgt cattcaatcc gtatgagcca gaacttgcac cctgggctgc
agacaaaggc ccccagagag aagccctacc catggacaca gaggtgtacg agagccccta
cgcggacccc gaggagatca ggcccaagga ggtttacctg gaccgaaagc tgctgacgct
ggaagacaaa gaactgggct ctggtaattt tggaactgtg aaaaagggct actaccaaat
gaaaaaagtt gtgaaaaccg tggctgtgaa aatactgaaa aacgaggcca atgaccccgc
tcttaaagat gagttattag cagaagcaaa tgtcatgcag cagctggaca acccgtacat
cgtgcggatg atcgggatat gcgaggccga gtcctggatg ctggttatgg agatggcaga
acttggtccc ctcaataagt atttgcagca gaacagacat gtcaaggata agaacatcat
agaactggtt catcaggttt ccatgggcat gaagtacttg gaggagagca attttgtgca
cagagatctg gctgcaagaa atgtgttgct agttacccaa cattacgcca agatcagtga
tttcggactt tccaaagcac tgcgtgctga tgaaaactac tacaaggccc agacccatgg
aaagtggcct gtcaagtggt acgctccgga atgcatcaac tactacaagt tctccagcaa
aagcgatgtc tggagctttg gagtgttgat gtgggaagca ttctcctatg ggcagaagcc
atatcgaggg atgaaaggaa gtgaagtcac cgctatgtta gagaaaggag agcggatggg
gtgccctgca gggtgtccaa gagagatgta cgatctcatg aatctgtgct ggacatacga
tgtggaaaac aggcccggat tcgcagcagt ggaactgcgg ctgcgcaatt actactatga
cgtggtgaac taaccgctcc cgcacctgtc ggtggctgcc tttgatcaca ggagcaatca
caggaaaatg tatccagagg aattgattgt cagccacctc cctctgccag tcgggagagc
caggcttgga tggaacatgc ccacaacttg tcacccaaag cctgtcccag gactcaccct
ccacaaagca aaggcagtcc cgggagaaaa gacggatggc aggatccaag ggctagctg

gatttgtttg ttttcttgtc tgtgtgattt tcatacaggt tatttttacg atctgtttcc
aaatcccttt catgtctttc cacttctctg ggtcccgggg tgcatttgtt actcatcggg
cccagggaca ttgcagagtg gcctagagca ctctcacccc aagcggcctt ttccaaatgc
ccaaggatgc cttagcatgt gactcctgaa gggaaggcaa aggcagagga atttggctgc
ttctacggcc atgagactga tccctggcca ctgaaaagct ttcctgacaa taaaaatgtt
ttgaggcttt aaaaagaaaa tcaagtttga ccagtgcagt ttctaagcat gtagccagtt
aaggaaagaa agaaagaaaa aaaaaaaagg cctggatact gcttttgctg tctctgttat
gagatggaag acttacatgt ttgtgataaa aggggaccat gagaatgaat tggcttggct
tactttcccc ctgaaatcct ctctcctgca gactgtcttg aagacctggt gactggtaaa
taaagccctg catggaggct gcacagcagg ggcaagaggc ccatccccca gcatctcact
gaggacagct tcaggctgcc ttcctctgaa cgtggtccac accttcctct cctccacaga
gagggtgccg ccagaatccc ctgtcgcttt ctgtgtctgc aatggggggc agcacaggga
tcaaagccat ctaaagagtt tccaaagaaa gtattaattc agaacaagcc aaagaccctg
agcctcacca caaacaggcc ttttggagtg tgaatttgag ttgaagatac aagatcggag
aatgattttc tggtcttaac taatcctcat cttcatgttt gatctttaag aagtcatcac
ccattgattt cagttttgct gtacctcttg aaagttaaag agacatctca gcactttagg
aggccgaggc gggtggatca cttgaggtaa ggagtttgag actagcctgg ccaatatggt
aaaaccccat ctctactaaa aatacaaaaa ttagccgggc atggtggcat gtgtctgtag
tcccagctac tcgggaggct gaggcaggag aatcgcttga acccaggaaa cggaggtcgc
agtgagccaa gatcatgcca ctgcactcca gcttgggcat cacagcgaga ctctgtcaaa
acaaacaaac aaaaaaacaa cttaaagagg taatttagcc atcattctta tgccagcaga
tataaataaa cttggaccca tctggtcttc agctaaacct gagacatttt aaagtgcatg
gacagccatg gacagcaggc cctcctctaa caggggatgc aaggcatgga gaaagacaat
cagtacccaa gctcagccac agaagacagg agtcactcat ataacttgtg tttagaagtt
tttggtagcc acgcacactt tctgaaatca cactatctgg tggtttaatc atatttttaa
agacagaatc cctgagtgct gagcagattc tcaaaacaca tttagaatcc ctgaaattag
aaagatcaat gacaaaatat ctgtcagcca ggccacaaac aggtgtaaaa ttatgaaagg
agtggttgga tgtgccaagt ttggtaaagt ggtgactgca tctgagaaag aggctgtgag
gctgaactct tggtggcttc cttctgtaac ttccagaggg agtcttcaac acaggccccg
tgctcgtagg aatacggtag cacctatgta ggaagtgcgt ggagttttct gtcttctttc
tgtgtgattt ttggcctttt tatcagcact tctcccctcc caggagcctg gggatgccaa
acatccagaa tgtgatggga caagatgggg gcaggggcct cacctccctg cagaggtccg
gccaggtctc cttgtccctg gacaatctcc tgagcctctc tgcttggtgg agcaggcacc
tgtgtgcaga attcccactg tggccagcac gaggaagtct tttctagtga aaatgtgtct
tgtggtcagg aataattatc ctttcccctg tagccaccaa ggagggcaaa tagagaaagg
taacctaatt gaaggattgg tcatgtgaaa agggctacat ttgggaagct gggaaaggcc
tccaggcttc tagagcagct agcttgggct ggattctcat acccaggctg ccccttggat
tgttctaccc aagcttttcc ctggggtctg ggctcactcc ataaggtaag gtgcctttta
ccttatggtc cttctttagc aggtaacaaa ggagcatcag gggcaggctg ccctggtggc
atcacactgg ctagtgaggc cgtgaatatc ttgtccccca gcagggccga cagtttctat
cacagaaaac agtgtgttca gtggtgaaaa tcgttgcatg catgttttca tctgagcgtg

```
tccttctccc atactcccta tcagccagcc ctgcctgtag ctgctgtatg gtgattgcac
ttggacatca gtccaatgac tgcaagtcgg cctggatttt cacttgcaga ggctacagct
gcattgtcag gtctcccagc cctgcagaga gctccctcca ctggttagca gtgtgttgtg
ttttccattc atttcagaag agctacattg tgtcactgga catttttaaa aactgtgatt
tttaataaaa atttaaaatt tgctttgtga tgaaaaaaa
```

Amino acid sequence for human SYK (UniProtKB/Swiss-Prot P43405 or GenPept NP_003168; SEQ ID NO:2):

```
MASSGMADSANHLPFFFGNITREEAEDYLVQGGMSDGLYLLRQSRNYLGGFALSVAHGRKAH
HYTIERELNGTYAIAGGRTHASPADLCHYHSQESDGLVCLLKKPFNRPQGVQPKTGPFEDLK
ENLIREYVKQTWNLQGQALEQAIISQKPQLEKLIATTAHEKMPWFHGKISREESEQIVLIGS
KTNGKFLIRARDNNGSYALCLLHEGKVLHYRIDKDKTGKLSIPEGKKFDTLWQLVEHYSYKA
DGLLRVLTVPCQKIGTQGNVNFGGRPQLPGSHPATWSAGGIISRIKSYSFPKPGHRKSSPAQ
GNRQESTVSFNPYEPELAPWAADKGPQREALPMDTEVYESPYADPEEIRPKEVYLDRKLLTL
EDKELGSGNFGTVKKGYYQMKKVVKTVAVKILKNEANDPALKDELLAEANVMQQLDNPYIVR
MIGICEAESWMLVMEMAELGPLNKYLQQNRHVKDKNIIELVHQVSMGMKYLEESNFVHRDLA
ARNVLLVTQHYAKISDFGLSKALRADENYYKAQTHGKWPVKWYAPECINYYKFSSKSDVWSF
GVLMWEAFSYGQKPYRGMKGSEVTAMLEKGERMGCPAGCPREMYDLMNLCWTYDVENRPGFA
AVELRLRNYYYDVVN
```

In quiescent or unstimulated cells, SYK is inactive and partially (incompletely) phosphorylated. In order to provide active SYK, several sites, such as Y296, S297, Y323, Y348, Y352, Y630, on SYK may be phosphorylated, resulting in phospho-SYK (pSYK) activation. As sites become phosphorylated, SYK binds signaling partners. Fully active pSYK is autophosphorylated at tyrosines Y525/526. Fully active pSYK then phosphorylates proteins downstream in the signaling cascade. In B-cells, stimulation of the B-cell receptor (BCR) and subsequent phosphorylation of the ITAM domain of CD79a and b leads to recruitment of SYK to the membrane and phosphorylation of SYK. Aberrant BCR-mediated SYK activation can lead to the development of diffuse large B-cell lymphoma (DLBCL), follicular lymphoma (FL), mantle cell lymphoma (MCL) or chronic lymphocytic leukemia (CLL). In myeloid cells, activation of SYK is mediated by the Fcγ receptor. Aberrant Fcγ receptor-mediated SYK activation can lead to the development of acute myeloid leukemia (AML) or myelodysplastic syndrome (MDS). In EBV-infected cells, SYK activation is mediated through human herpesvirus 4 latent membrane protein 2A (LMP2A). Aberrant LMP2A-mediated SYK activation can lead to the development of nasopharyngeal carcinoma, lymphoma or gastric carcinoma.

pSYK has been characterized as a protein involved in various cancers and can therefore serve as a diagnostic or therapeutic target. Antibody molecules directed to pSYK can be used in naked or labeled form, to detect pSYK-expressing cancerous cells. Anti-pSYK antibody molecules of the invention can bind human pSYK. In some embodiments, an anti-pSYK antibody molecule of the invention can inhibit the binding of a ligand to pSYK. In other embodiments, an anti-pSYK antibody molecule of the invention does not inhibit the binding of a ligand to pSYK.

The present invention is based, in part, on the recognition that commercially available anti-pSYK antibodies are deficient at detecting some tumor cells with activated SYK. In some embodiments, commercially available anti-pSYK antibodies are deficient at detecting pSYK Y525/526. In some embodiments, commercially available anti-pSYK antibodies are deficient at detecting pSYK Y525/526 in samples obtained from patients with cancer (e.g., PTCL, DLBCL, FL, MCL, CLL, AML, MDS, nasopharyngeal carcinoma, lymphoma, gastric carcinoma, breast cancer, ovarian cancer, lung cancer (e.g., small cell lung cancer) and post-transplant lymphoproliferative disorders (PT-LPD)). Commercially available antibodies specific for pSYK include Epitomics Catalog No. 2175-1 (pSYK Y525/6); Cell Signaling Technologies Catalog No. 2710 (pSYK Y525/6); Cell Signaling Technologies Catalog No. 2711 (pSYK Y525/6); Abgent Catalog No. AP3271a (pSYK Y525/6). Accordingly, in certain embodiments, the antibody molecules described are improved reagents which provide an improved method of detecting cancer cells. In some embodiments, the anti-SYK antibody molecules of the invention are improved reagents which provide an improved method for detecting or measuring the amount of pSYK Y525/526, such as in samples obtained from patients with cancer. In some embodiments, the anti-SYK antibody molecules of the invention are improved reagents which provide an improved method of detecting and/or measuring pSYK Y525/526 in a cell-based assay, such as immunocytochemistry, immunofluorescence, or immunohistochemistry. In certain such embodiments, the anti-SYK antibody molecules provide improved intensity. In certain such embodiments, the anti-SYK antibody molecules provide improved dynamic range. See, for example, Figures 2, 3, 7, 14. Quantification of the signal resulting from binding of anti-pSYK Y525/526 antibodies described herein demonstrates a greater signal-to-noise ratio and dynamic range, i.e., difference between lowest and highest values, than the signal resulting from binding of commercially available anti-pSYK Y525/526 antibodies. A large dynamic range of signal detection provides the ability to detect pSYK Y525/526 in samples comprising tumor cells from subjects, aged, damaged or archived tumor specimens, or tumors which do not express high amounts of pSYK Y525/526. This reduces the number of false negative results and provides higher certainty of correct therapeutic regimens.

Specific detection and/or quantitative determination of pSYK Y525/526 using anti-pSYK Y525/526 antibodies described herein in a sample comprising tumor cells from a subject indicates fully active SYK, i.e., pSYK which causes the phosphorylation of proteins such as BTK, BLNK or PLC$\gamma$, downstream in the signaling cascade. This reduces the number of false positive results, such as from detecting partially phosphorylated, partially activated or non-autophosphorylating pSYK, which may not indicate a tumor which is SYK-dependent. Contributing to the superiority of anti-pSYK Y525/526 antibodies described herein may be the selection using autophosphorylated pSYK, in combination with being raised against a pSYK Y525/526 peptide immunogen. Further selection comprised tests for performance of the antibodies in specific three-dimensional assays, such as immunocytochemistry (ICC), immunofluorescence (IF) and immunohistochemistry (IHC).

**Definitions**

Unless otherwise defined herein, scientific and technical terms used in connection with the present invention have the meanings that are commonly understood by those of ordinary skill in the art. Generally, nomenclature utilized in connection with, and techniques of, cell and tissue culture, molecular biology, and protein and oligo- or polynucleotide chemistry and hybridization described herein are those known in the art. GenBank or GenPept accession numbers and useful nucleic acid and peptide sequences can be found at the website maintained by the National Center for Biotechnological Information (NCBI), Bethesda MD. Macromolecule names written in all capitalized letters without or with numbers are the names of proteins or genes as listed in the Gene database of NCBI. Sequences corresponding to the gene database names described herein are incorporated herein by reference. Standard techniques are used for recombinant DNA, oligonucleotide synthesis, and tissue culture and transformation and transfection (e.g., electroporation, lipofection). Enzymatic reactions and purification techniques are performed according to manufacturer's specifications or as commonly accomplished in the art or as described herein. The foregoing techniques and procedures are generally performed according to methods known in the art, e.g., as described in various general and more specific references that are cited and discussed throughout the present specification. See e.g., Sambrook et al. Molecular Cloning: A Laboratory Manual (3rd ed., Cold Spring Harbor Laboratory Press, Cold Spring Harbor, N.Y. (2000)); Harlow, E. and Lane, D. (1988) Antibodies: A Laboratory Manual, Cold Spring Harbor Laboratory Press, Cold Spring Harbor, N.Y. or see generally, Buchwalow and Böcker, Immunohistochemistry Basics and Methods (2010, Springer-Verlag, Berlin, Germany). The nomenclatures utilized in connection with, and the laboratory procedures and techniques described herein are known in the art. Furthermore, unless otherwise required by context, singular terms shall include pluralities and plural terms shall include the singular.

The articles "a," "an" and "at least one" are used herein to refer to one or to more than one of the grammatical object of the article. By way of example, "an element" means one or more than one element, at least one element. In the case of conflict, the present specification, including definitions, will control.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%.

As used herein, "SYK," also known as "spleen tyrosine kinase" or "p72-Syk" protein refers to mammalian tyrosine-protein kinase SYK, such as human SYK protein. As used herein, "SYK" also refers to human SYK protein phosphorylated at sites other than Y525 and/or Y526. In certain such embodiments, particular phosphorylated residues are indicated with a "p" before the residue number (referring to SEQ ID NO:2) For example, SYK phosphorylated at tyrosine residue 323 is herein referred to as SYK pY323. Human SYK refers to the protein shown in SEQ ID NO:2 and naturally occurring isoforms or allelic protein variants thereof. The allele in SEQ ID NO:2 can be encoded by the nucleic acid sequence of SYK shown in SEQ ID NO:1. One SYK variant lacks an in-frame exon and encodes a polypeptide variant of SEQ ID NO:2 which does not include amino acid residues 282 to 305 of SEQ ID NO:2. Other variants are known in the art. For example, there are several single nucleotide polymorphisms (SNPs) of human SYK, as seen in the NCBI SNP database. Typically, a naturally occurring allelic variant has an amino acid sequence at least 90%, 95%, 97%, 98% or 99% identical to the SYK sequence of SEQ ID NO:2. The transcript encodes a protein product of 635 amino acids, and is described in GenBank accession no.: NM_003177. The SH2 domains mediate the interaction of SYK with the phosphorylated ITAM domains of transmembrane proteins. SYK protein is characterized as a cytoplasmic non-receptor tyrosine kinase, and is believed to play a critical role in several biological processes including innate and adaptive immunity, cell adhesion, osteoclast maturation, platelet activation and vascular development.

As used herein, the terms "antibody molecule", "antibody" "immunoglobulin" and "antibodies" broadly encompass naturally-occurring forms of antibodies, e.g., polyclonal antibodies (e.g., IgG, IgA, IgM, IgE) and monoclonal and recombinant antibodies or antibody peptides such as single-chain antibodies, two-chain and multi-chain proteins, chimeric, CDR-grafted, human and humanized antibodies and multi-specific antibodies, as well as fragments and derivatives of all of the foregoing, which fragments (e.g., dAbs, scFv, Fv, Fab, $F(ab)'_2$, Fab') and derivatives such as functional heavy chain antibodies, nanobodies, e.g., derivatives when paired with a complete variable region of the other chain, e.g., the light chain, it will allow binding of at least 25, 50, 75, 85 or 90% of that seen with the whole heavy and light variable region, as well as any portion of an antibody having specificity toward at least one desired epitope, that competes with the intact antibody for specific binding (e.g., a fragment having sufficient CDR sequences and having sufficient framework sequences so as to bind specifically to an epitope). The term "antibody" also includes synthetic and genetically engineered variants, such as monobodies and diabodies. Although not within the term "antibody molecules," the invention also includes derivatives such as "antibody analog(s)," other non-antibody molecule protein-based scaffolds, e.g., fusion proteins and/or immunoconjugates that use CDRs to provide specific antigen binding.

As used herein, the term "pSYK," "p-SYK" and "phospho-SYK" refer to phosphorylated Spleen Tyrosine Kinase (SYK). An "anti-pSYK antibody molecule" refers to an antibody molecule (i.e., an antibody, antigen-binding fragment of an antibody or antibody analog) which interacts with or recognizes, e.g., binds (e.g., binds specifically) to phosphorylated SYK, e.g., human phosphorylated SYK. Exemplary anti-pSYK antibody molecules are such as those summarized in Tables 1 and 2. In certain such embodiments, as used herein "pSYK", "p-SYK" and "phospho-SYK" refer in particular to SYK that has been phosphorylated at tyrosine 525 and/or 526 of SEQ ID NO:2.

As used herein, the term "pBTK" or "phospho-Bruton agammaglobulinemia tyrosine kinase" means the polypeptide represented by GenPept Accession No. NP_000052, SEQ ID NO:26, or an isoform thereof, phosphorylated at at least one of its amino acid residues. In some embodiments, pBTK is phosphorylated at tyrosine 551 of SEQ ID NO:26 ("BTK pY551").

In other embodiments, pBTK is phosphorylated at tyrosine 223 of SEQ ID NO:26 ("BTK pY223 ").

As used herein, the term "pBLNK" or "phospho-B-cell linker protein means the polypeptide represented by GenPept Accession No. NP_037446, SEQ ID NO:27 or an isoform thereof, phosphorylated at at least one of its amino acid residues. In some embodiments, pBTK is phosphorylated at tyrosine 96 of SEQ ID NO:27 ("BLNK pY96"). In other embodiments, pBTK is phosphorylated at tyrosine 84 of SEQ ID NO:27 ("BLNK pY84").

As used herein, the term "pFLT3" or "phospho-fms-related tyrosine kinase 3 means the polypeptide represented by GenPept Accession No. NP_004110, SEQ ID NO:28 or an isoform thereof, phosphorylated at at least one of its amino acid residues. In some embodiments, pFLT3 is phosphorylated at tyrosine 591 of SEQ ID NO:28 ("FLT3 pY591"). In other embodiments, pFLT3 is phosphorylated at tyrosine 969 of SEQ ID NO:28 ("FLT3 pY969").

As used herein, a "SYK inhibitor," a "SYK-targeted therapeutic agent" or "SYK targeted therapy" is a compound which inhibits activation of SYK, inhibits the activity of SYK or pSYK or inhibits the autophosphorylation of SYK. In some embodiments, a SYK inhibitor inhibits the autophosphorylation of pSYK. In some embodiments, the autophosphorylation inhibited by a SYK inhibitor is the autophosphorylation at tyrosine residue 525 and/or tyrosine residue 526 of SEQ ID NO:2. A SYK inhibitor can be one of several known in the art or may be selected from the group consisting of an ATP-competitor such as R406 or a non-hydrolyzable ATP analog, piceatannol, fostamatinib, a diaminopyrimidine carboxamide, e.g., as described in WO1999/31073 or WO2009/136995, a pyrrolopyrimidine, e.g., as described in WO2009/131687, and a fused heteroaromatic pyrrolidinone. In certain embodiments, a SYK inhibitor may be a fused heteroaromatic pyrrolidinone. In certain embodiments, a fused heteroaromatic pyrrolidinone may be a pyrrolopyrimidinone (e.g., a 6,7-dihydro-5H-pyrrolo[3,4-c]pyrimidin-5-one) or a pyrrolopyridinone (e.g., a 1H-pyrrolo[3,4-c]pyridine-3(2H)-one) compound described in U.S. Patent No. 8,440,689. Some fused heteroaromatic pyrrolidinone compounds are described herein. One such fused heteroaromatic pyrrolidinone is described herein as Compound A. A therapeutic regimen comprising a SYK inhibitor may comprise treating a subject with a fused heteroaromatic pyrrolidinone, such as Compound A.

The term, "antigen binding constellation of CDRs" or "a number of CDRs sufficient to allow binding" (and similar language), as used herein, refers to sufficient CDRs of a chain, e.g., the heavy chain, such that when placed in a framework and paired with a complete variable region of the other chain, or with a portion of the other chain's variable region of similar length and having the same number of CDRs, e.g., the light chain, will allow binding, e.g., of at least 25, 50, 75, 85 or 90% of that seen with the whole heavy and light variable region. As used herein, the term "humanized antibody" refers to an antibody that is derived from a non-human antibody e.g., rabbit, rodent (e.g., murine), sheep or goat, that retains or substantially retains the antigen-binding properties of the parent antibody but is less immunogenic in humans. Humanized as used herein is intended to include deimmunized antibodies. Typically, humanized antibodies include non-human CDRs and human or human derived framework and constant regions.

The term "modified" antibody, as used herein, refers to antibodies that are prepared, expressed, created or isolated by recombinant means, such as antibodies expressed using a recombinant expression vector transfected into a host cell, antibodies isolated from a recombinant, combinatorial antibody library, antibodies isolated from a non-human animal (e.g., a rabbit, mouse, rat, sheep or goat) that is transgenic for human immunoglobulin genes or antibodies prepared, expressed, created or isolated by any other means that involves splicing of human immunoglobulin gene sequences to other DNA sequences. Such modified antibodies include humanized, CDR grafted (e.g., an antibody having CDRs from a first antibody and a framework region from a different source, e.g., a second antibody or a consensus framework), chimeric, in vitro generated (e.g., by phage display) antibodies, and may optionally include variable or constant regions derived from human germline immunoglobulin sequences or human immunoglobulin genes or antibodies which have been prepared, expressed, created or isolated by any means that involves splicing of human immunoglobulin gene sequences to alternative immunoglobulin sequences. In certain embodiments a modified antibody molecule includes an antibody molecule having a sequence change from a reference antibody.

The term "monospecific antibody" refers to an antibody or antibody preparation that displays a single binding specificity and affinity for a particular epitope. This term includes a "monoclonal antibody" or "monoclonal antibody composition."

The term "bispecific antibody" or "bifunctional antibody" refers to an antibody that displays dual binding specificity for two epitopes, where each binding site differs and recognizes a different epitope.

The terms "non-conjugated antibody" and "naked antibody" are used interchangeably to refer to an antibody molecule that is not conjugated to a non-antibody moiety, e.g., an agent or a label.

Each of the terms "immunoconjugate," "antibody-drug conjugate" and "antibody conjugate" are used interchangeably and refer to an antibody that is conjugated to a non-antibody moiety, e.g., an agent or a label. The term "agent" is used herein to denote a chemical compound, a mixture of chemical compounds, a biological macromolecule, or an extract made from biological materials. The term "therapeutic agent" refers to an agent that has biological activity. Exemplary therapeutic agents are chemotherapeutic agents.

"Cytotoxic agents" refer to compounds which cause cell death primarily by interfering directly with the cell's functioning, including, but not limited to, alkylating agents, tumor necrosis factor inhibitors, intercalators, microtubule inhibitors, kinase inhibitors, proteasome inhibitors and topoisomerase inhibitors. A "toxic payload" as used herein refers to a sufficient amount of cytotoxic agent which, when delivered to a cell results in cell death. Delivery of a toxic payload may be accomplished by administration of a sufficient amount of immunoconjugate comprising an antibody or antigen binding fragment of the invention and a cytotoxic agent. Delivery of a toxic payload may also be accomplished by administration of a sufficient amount of an immunoconjugate comprising a cytotoxic agent, wherein the immunoconjugate comprises a secondary antibody or antigen binding fragment thereof which recognizes and binds an antibody or antigen binding fragment of the invention.

As used herein the phrase, a sequence "derived from" or "specific for a designated sequence" refers to a sequence that comprises a contiguous sequence of approximately at least 6 nucleotides or at least 2 amino acids, at least about 9 nucleotides or at least 3 amino acids, at least about 10-12 nucleotides or 4 amino acids, or at least about 15-21 nucleotides or 5-7 amino acids corresponding, i.e., identical or complementary to, e.g., a contiguous region of the designated sequence. In certain embodiments, the sequence comprises all of a designated nucleotide or amino acid sequence. The sequence may be complementary (in the case of a polynucleotide sequence) or identical to a sequence region that is unique to a particular sequence as determined by techniques known in the art. Regions from which sequences may be derived, include but are not limited to, regions encoding specific epitopes, regions encoding CDRs, regions encoding framework sequences, regions encoding constant domain regions, regions encoding variable domain regions, as well as non-translated and/or non-transcribed regions. The derived sequence will not necessarily be derived physically from the sequence of interest under study, but may be generated in any manner, including, but not limited to, chemical synthesis, replication, reverse transcription or transcription, that is based on the information provided by the sequence of bases in the region(s) from which the polynucleotide is derived. As such, it may represent either a sense or an antisense orientation of the original polynucleotide. In addition, combinations of regions corresponding to that of the designated sequence may be modified or combined in ways known in the art to be consistent with the intended use. For example, a sequence may comprise two or more contiguous sequences which each comprise part of a designated sequence, and are interrupted with a region which is not identical to the designated sequence but is intended to represent a sequence derived from the designated sequence. With regard to antibody molecules, "derived therefrom" includes an antibody molecule which is functionally or structurally related to a comparison antibody, e.g., "derived therefrom" includes an antibody molecule having similar or substantially the same sequence or structure, e.g., having the same or similar CDRs, framework or variable regions. "Derived therefrom" for an antibody also includes residues, e.g., one or more, e.g., 2, 3, 4, 5, 6 or more residues, which may or may not be contiguous, but are defined or identified according to a numbering scheme or homology to general antibody structure or three-dimensional proximity, i.e., within a CDR or a framework region, of a comparison sequence. The term "derived therefrom" is not limited to physically derived therefrom, but includes generation by any manner, e.g., by use of sequence information from a comparison antibody to design another antibody and can refer merely to sequence similarity. As used herein, the phrase "encoded by" refers to a nucleic acid sequence that codes for a polypeptide sequence, wherein the polypeptide sequence or a portion thereof contains an amino acid sequence of at least 3 to 5 amino acids, at least 8 to 10 amino acids, or at least 15 to 20 amino acids from a polypeptide encoded by the nucleic acid sequence.

Calculations of "homology" between two sequences can be performed as follows. The sequences are aligned for optimal comparison purposes (e.g., gaps can be introduced in one or both of a first and a second amino acid or nucleic acid sequence for optimal alignment and nonhomologous sequences can be disregarded for comparison purposes). The length of a reference sequence aligned for comparison purposes is at least 30%, 40%, or 50%, at least 60%, or at least 70%, 80%, 90%, 95%, or 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent homology between two sequences may be accomplished using a mathematical algorithm. The percent homology between two amino acid sequences may be determined using any method known in the art. For example, the Needleman and Wunsch, J. Mol. Biol. 48:444-453 (1970), algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6. The percent homology between two nucleotide sequences can also be determined using the GAP program in the GCG software package (Accelerys, Inc. San Diego, Calif.), using an NWSgapdna.CMP matrix and a gap weight of 40, 50, 60, 70, or 80 and a length weight of 1, 2, 3, 4, 5, or 6. An exemplary set of parameters for determination of homology are a Blossum 62 scoring matrix with a gap penalty of 12, a gap extend penalty of 4, and a frameshift gap penalty of 5.

As used herein, the term "hybridizes under stringent conditions" describes conditions for hybridization and washing. Guidance for performing hybridization reactions may be found in Current Protocols in Molecular Biology, John Wiley & Sons, N.Y. (1989), 6.3.1-6.3.6. Aqueous and nonaqueous methods are described in that reference and either may be used. Specific hybridization conditions referred to herein are as follows: 1) low stringency hybridization conditions in 6X sodium chloride/sodium citrate (SSC) at about 45° C, followed by two washes in 0.2X SSC, 0.1% SDS at least at 50° C, (the temperature of the washes may be increased to 55° C for low stringency conditions); 2) medium stringency hybridization conditions in 6X SSC at about 45° C, followed by one or more washes in 0.2X SSC, 0.1% SDS at 60° C; 3) high stringency hybridization conditions in 6X SSC at about 45° C, followed by one or more washes in 0.2X SSC, 0.1% SDS at 65° C; and 4) very high stringency hybridization conditions are 0.5M sodium phosphate, 7% SDS at 65° C, followed by one or more washes at 0.2X SSC, 1% SDS at 65° C. Very high stringency conditions (4) are often the preferred conditions and the ones that should be used unless otherwise specified. It is understood that the antibodies and antigen binding fragment thereof of the invention may have additional conservative or non-essential amino acid substitutions, which do not have a substantial effect on the polypeptide functions. Whether or not a particular substitution will be tolerated, i.e., will not adversely affect desired biological properties, such as binding activity, may be determined as described in Bowie, J U et al. Science 247: 1306-1310 (1990) or Padlan et al. FASEB J. 9: 133-139 (1995). A "conservative amino acid substitution" is one in which the amino acid residue is replaced with an amino acid residue having a similar side chain. Families of amino acid residues having similar side chains have been defined in the art.

These families include amino acids with basic side chains (e.g., lysine, arginine, histidine), acidic side chains (e.g., aspartic acid, glutamic acid), uncharged polar side chains (e.g., asparagine, glutamine, serine, threonine, tyrosine, cysteine), nonpolar side chains (e.g., glycine, alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

A "non-essential" amino acid residue is a residue that may be altered from the wild-type sequence of the binding agent, e.g., the antibody, without abolishing or, without substantially altering a biological activity, whereas an "essential" amino acid residue results in such a change. In an antibody, an essential amino acid residue may be a specificity determining residue (SDR).

As used herein, the term "isolated" refers to material that is removed from its original environment (e.g., the natural environment if it is naturally occurring). For example, a naturally occurring polynucleotide or polypeptide present in a living animal is not isolated, but the same polynucleotide or DNA or polypeptide, separated from some or all of the coexisting materials in the natural system, is isolated. Such polynucleotide or polypeptide could be part of a vector and/or such polynucleotide or polypeptide could be part of a composition, e.g., a mixture, solution or suspension or comprising an isolated cell or a cultured cell which comprises the polynucleotide or polypeptide, and still be isolated in that the vector or composition is not part of its natural environment.

As used herein, the term "operably linked" refers to a situation wherein the components described are in a relationship permitting them to function in their intended manner. Thus, for example, a control sequence "operably linked" to a coding sequence is ligated in such a manner that expression of the coding sequence is achieved under conditions compatible with the control sequence.

As used herein, the term "vector" refers to a replicon in which another polynucleotide segment is attached, such as to bring about the replication and/or expression of the attached segment.

As used herein, the term "control sequence" refers to a polynucleotide sequence that is necessary to effect the expression of a coding sequence to which it is ligated. The nature of such control sequences differs depending upon the host organism. In prokaryotes, such control sequences generally include a promoter, a ribosomal binding site and terminators and, in some instances, enhancers. The term "control sequence" thus is intended to include at a minimum all components whose presence is necessary for expression, and also may include additional components whose presence is advantageous, for example, leader sequences.

As used herein, the term "purified product" refers to a preparation of the product which has been isolated from the cellular constituents with which the product is normally associated and/or from other types of cells that may be present in the sample of interest.

As used herein, the term "epitope" refers to a protein determinate capable of binding specifically to an antibody. Epitopic determinants usually consist of chemically active surface groupings of molecules such as amino acids or sugar side chains and usually have specific three dimensional structural characteristics, as well as specific charge characteristics. Some epitopes are linear epitopes while others are conformational epitopes. A linear epitope is an epitope wherein a contiguous amino acid primary sequence comprises the epitope recognized. A linear epitope typically includes at least 3, and more usually, at least 5, for example, about 8 to about 10 contiguous amino acids. A conformational epitope can result from at least two situations, such as: a) a linear sequence which is only exposed to antibody binding in certain protein conformations, e.g., dependent on ligand binding, or dependent on modification (e.g., phosphorylation) by signaling molecules; or b) a combination of structural features from more than one part of the protein, or in multisubunit proteins, from more than one subunit, wherein the features are in sufficiently close proximity in 3-dimensional space to participate in binding.

As used herein, "isotype" refers to the antibody class (e.g., IgM, IgA, IgE or IgG) that is encoded by heavy chain constant region genes.

As used herein, the terms "detectable agent," "label" or "labeled" are used to refer to incorporation of a detectable marker on a polypeptide or glycoprotein. Various methods of labeling polypeptides and glycoproteins are known in the art and may be used. Examples of labels for polypeptides include, but are not limited to, the following: radioisotopes or radionuclides (e.g., indium ($^{111}In$), iodine ($^{131}I$ or $^{125}I$), yttrium ($^{90}Y$), lutetium ($^{177}Lu$), actinium ($^{225}Ac$), bismuth ($^{212}Bi$ or $^{213}Bi$), sulfur ($^{35}S$), carbon ($^{14}C$), tritium ($^{3}H$), rhodium ($^{188}Rh$), technetium ($^{99}mTc$), praseodymium, or phosphorous ($^{32}P$) or a positron-emitting radionuclide, e.g., carbon-11 ($^{11}C$), potassium-40 ($^{40}K$), nitrogen-13 ($^{13}N$), oxygen-15 ($^{15}O$), fluorine-18 ($^{18}F$), gallium-68 ($^{68}Ga$), and iodine- 121 ($^{121}I$)), fluorescent labels (e.g., FITC, rhodamine, lanthanide phosphors, cyanine (Cy) fluorescent dyes such as Cy3, or Cy5), Alexa Fluor 488, Alexa Fluor 592, Oregon green, enzymatic labels (e.g., horseradish peroxidase, beta-galactosidase, luciferase, alkaline phosphatase), chemiluminescent, biotinyl groups (which may be detected by a marked avidin, e.g., a molecule containing a streptavidin moiety and a fluorescent marker or an enzymatic activity that may be detected by optical or calorimetric methods), and predetermined polypeptide epitopes recognized by a secondary reporter (e.g., leucine zipper pair sequences, binding sites for secondary antibodies, metal binding domains, epitope tags). In some embodiments, labels are attached by spacer arms of various lengths to reduce potential steric hindrance.

As used herein, "specific binding," "bind(s) specifically" or "binding specificity" means, for an anti-pSYK antibody molecule, that the antibody molecule binds to pSYK, e.g., human phosphorylated SYK protein, with greater affinity than it does to a non-phosphorylated SYK, phosphatase-treated (PPase) SYK or pSYK or non-SYK protein, e.g., bovine serum albumin (BSA), a rat sarcoma viral oncogene homolog (RAS), or human actin. Typically an anti-pSYK molecule will have a $K_d$ for the non-phosphorylated SYK or non-SYK protein, e.g., BSA, RAS or actin, which is greater than 2, greater than 10, greater than 100, greater than 1,000 times, greater than $10^4$, greater than $10^5$, or greater than $10^6$ times its $K_d$ for phosphorylated SYK, e.g., human phosphorylated SYK protein. In determination of $K_d$, the $K_d$ for phosphorylated SYK and the non-phosphorylated SYK or non-SYK protein, e.g., BSA, RAS or actin, should be done under the same conditions. In some embodiments, an anti-pSYK antibody having specific binding to pSYK Y525/526 binds to pSYK Y525/526 with greater affinity than it does to SYK phosphorylated at any other tyrosine, serine or threonine of SEQ ID NO:2.

The term "affinity" or "binding affinity" refers to the apparent association constant or $K_a$. The $K_a$ is the reciprocal of the dissociation constant ($K_d$). An antibody may, for example, have a binding affinity of at least $10^5$, $10^6$, $10^7$, $10^8$, $10^9$, $10^{10}$ and $10^{11}$ $M^{-1}$ for a particular target molecule. Higher affinity binding of an antibody to a first target relative to a second target may be indicated by a higher $K_A$ (or a smaller numerical value $K_D$) for binding the first target than the $K_A$ (or numerical value $K_D$) for binding the second target. In such cases, the antibody has specificity for the first target (e.g., a protein in a first conformation or mimic thereof) relative to the second target (e.g., the same protein in a second conformation or mimic thereof; or a second protein). Differences in binding affinity (e.g., for specificity or other comparisons) may be at least 1.5, 2, 3, 4, 5, 10, 15, 20, 37.5, 50, 70, 80, 91, 100, 500, 1000, or $10^5$ fold. Binding affinity may be determined by a variety of methods including equilibrium dialysis, equilibrium binding, gel filtration, ELISA, surface plasmon resonance, or spectroscopy (e.g., using a fluorescence assay). For example, relative affinity of an anti-pSYK antibody molecule may be measured from ELISA measurements against pSYK protein, pSYK Y525/526 peptide (e.g., the immunogen used to raise anti-pSYK antibody molecules), by FACS measurements with pSYK expressing cells.

Exemplary conditions for evaluating binding affinity are in TRIS-buffer (50mM TRIS, 150mM NaCl, 5mM $CaCl_2$ at pH7.5). These techniques may be used to measure the concentration of bound and free binding protein as a function of binding protein (or target) concentration. The concentration of bound binding protein ([Bound]) is related to the concentration of free binding protein ([Free]) and the concentration of binding sites for the binding protein on the target where (N) is the number of binding sites per target molecule by the following equation:

$$[\text{Bound}] = \text{N} \cdot [\text{Free}]/((1/\text{K}_\text{A}) + [\text{Free}]).$$

It is not always necessary to make an exact determination of $K_A$, though, since sometimes it is sufficient to obtain a quantitative measurement of affinity, e.g., determined using a method such as ELISA or FACS analysis, is proportional to $K_A$, and thus may be used for comparisons, such as determining whether a higher affinity is, e.g., 2-fold higher, to obtain a qualitative measurement of affinity, or to obtain an inference of affinity, e.g., by activity in a functional assay, e.g., an in vitro or in vivo assay. Affinity of anti-pSYK antibody molecules can also be measured using a technology such as real-time Biomolecular Interaction Analysis (BIA) (see, e.g., Sjolander, S, and Urbaniczky, C, 1991, Anal. Chem. 63:2338-2345 and Szabo et al., 1995, Curr. Opin. Struct. Biol. 5:699-705). As used herein, "BIA" or "surface plasmon resonance" is a technology for studying biospecific interactions in real time, without labeling any of the interactants (e.g., BIACORE™). Changes in the mass at the binding surface (indicative of a binding event) result in alterations of the refractive index of light near the surface (the optical phenomenon of surface plasmon resonance (SPR)), resulting in a detectable signal which may be used as an indication of real-time reactions between biological molecules.

The measurement of affinity of anti-pSYK antibody molecules using a BIACORE™ T100 system (GE Healthcare, Piscataway, N.J.) is described briefly. An anti-pSYK antibody (Prep A) may be diluted to an appropriate concentration (e.g., 20 g/mL) in 10 nM sodium acetate, pH 4.0 and a reference/control antibody (Prep B) may be diluted to an appropriate concentration (e.g., 10 g/mL) in 10 mM sodium acetate, pH 4.0. Each antibody may then be covalently immobilized to several CM4 BIACORE chips using standard amine coupling. For each CM4 chip prepared, Prep A antibody may be immobilized over two flow cells at around 75-100 RU while Prep B antibody may be immobilized to one flow cell at around 70-80 RU. The remaining fourth flow cell of each CM4 chip may be used as the reference flow cell.

As used herein, the term "treat" or "treatment" is defined as the administration of a therapeutic agent to modify a material or subject. In the context of cancer, "treatment" shall mean the use of a therapy to prevent or inhibit further tumor growth, as well as to cause shrinkage of a tumor. Such use can provide longer survival times. Treatment is also intended to include prevention of metastasis of tumor. A tumor is "inhibited" or "treated" if at least one symptom (as determined by responsiveness/non-responsiveness, time to progression, or indicators known in the art and described herein) of the cancer or tumor is alleviated, terminated, slowed, minimized, or prevented. Any amelioration of any symptom, physical or otherwise, of a tumor pursuant to treatment using a therapeutic regimen (e.g., comprising a SYK inhibitor) as further described herein, is within the scope of the invention, The treatment may be to cure, heal, alleviate, relieve, alter, remedy, ameliorate, palliate, improve or affect the disorder, the symptoms of the disorder or the predisposition toward the disorder, e.g., a cancer. While not wishing to be bound by theory, treating is believed to cause the inhibition, ablation, or killing of a cell in vitro or in vivo, or otherwise reducing capacity of a cell, e.g., an aberrant cell, to mediate a disorder, e.g., a disorder as described herein (e.g., a cancer). Administration of a SYK-targeted therapy, can be providing an anti-pSYK antibody molecule or a SYK inhibitor to a subject, e.g., a patient, or administration, e.g., by application, to an isolated tissue or cell from a subject which is returned to the subject. The anti-pSYK antibody molecule or SYK inhibitor may be administered alone or in combination with a second agent. As used herein, the term "subject" is intended to include mammals, primates, humans and non-human animals. For example, a subject may be a patient (e.g., a human patient or a veterinary patient), having a disorder, disease, or condition, such as a disorder, disease, or condition mediated by SYK, e.g., aberrant BCR-mediated SYK activation disorders (e.g., DLBCL, follicular lymphoma, mantle cell lymphoma, or chronic lymphocytic leukemia), aberrant Fcγ receptor-mediated SYK activation disorders (e.g., acute myeloid leukemia or myelodysplastic syndrome) or aberrant LMP2A-mediated SYK activation disorders (e.g., nasopharyngeal carcinoma, lymphoma, or gastric cancer) as described above. In certain such embodiments the disorder, disease, or condition is related to abnormal cell growth, including hematological cancer, leukemia, lymphoma or myeloma malignancies, such as acute myeloid leukemia (AML), B-cell chronic lymphocytic leukemia (BCLL), B-cell lymphoma (e.g., mantle cell lymphoma (MCL), diffuse large B-cell lymphoma (DLBCL)), follicular lymphoma, and T-cell lymphoma (e.g., peripheral T-cell lymphoma), as well as epithelial cancers (i.e., carcinomas), such as lung cancer (small cell lung cancer and non-small cell lung cancer), pancreatic cancer, and colon cancer. In addition to the hematological malignancies and epithelial cancers noted above, in some embodiments, the condition may include other types of cancer, including leukemia (chronic myelogenous leukemia and chronic lymphocytic leukemia (CLL)) and breast cancer, genitourinary cancer, skin cancer, bone cancer, prostate cancer, and liver cancer; brain cancer; cancer of the larynx, gall bladder, rectum, parathyroid, thyroid, adrenal, neural tissue, bladder, head, neck, stomach, bronchi, and kidneys; basal cell carcinoma, squamous cell carcinoma, metastatic skin carcinoma, osteosarcoma, Ewing's sarcoma, veticulum cell sarcoma, and Kaposi's sarcoma; myeloma, EBV-associated tumors and other solid tumors, giant cell tumor, islet cell tumor, acute and chronic lymphocytic and granulocytic tumors, hairy-cell tumor, adenoma, medullary carcinoma, pheochromocytoma, mucosal neuromas, intestinal ganglioneuromas, hyperplastic corneal nerve tumor, marfanoid habitus tumor, Wilms' tumor, seminoma, ovarian tumor, leiomyomater tumor, cervical dysplasia, neuroblastoma, retinoblastoma, myelodysplastic syndrome (MDS), rhabdomyosarcoma, astrocytoma, non-Hodgkin's lymphoma, malignant hypercalcemia, polycythermia vera, adenocarcinoma, glioblastoma multiforma, glioma, lymphomas, and malignant melanomas, among others. In certain embodiments, the disorder, disease or condition is selected from PTCL, DLBCL, FL, MCL, CLL, AML, MDS, nasopharyngeal carcinoma, lymphoma, gastric carcinoma, breast cancer, ovarian cancer, lung cancer (e.g., small cell lung cancer) and PT-LPD. In some embodiments, the DLBCL is classified as a subtype selected from the group consisting of germinal center B cell-like (GCB) subtype, activated B cell-like (ABC) subtype and non-germinal center B cell-like (non-GCB) subtype. In addition to cancer, in some embodiments, the condition may include other diseases related to abnormal cell growth, including non-malignant proliferative diseases such as benign prostatic hypertrophy, restinosis, hyperplasia, synovial proliferation disorder, retinopathy or other neovascular disorders of the eye, among others. In some embodiments, the condition may include a symptom of such SYK expressing, pSYK expressing or SYK-activating conditions; or a predisposition toward such SYK-expressing, pSYK expressing or SYK-activating conditions. SYK, BTK and PI3K$\delta$ are clinically validated targets in BCR-activated malignancies. The term "non-human animals" of the invention includes all non-human vertebrates, e.g., non-human mammals and non-mammals, such as non-human primates, sheep, dog, cow, chickens, amphibians, reptiles, mouse, rat, rabbit or goat etc., unless otherwise noted. In an embodiment, "subject" excludes one or more or all of a mouse, rat, rabbit or goat.

As used herein, an amount of an anti-pSYK antibody molecule or a therapeutic agent "effective" or "sufficient" to treat a disorder, or a "therapeutically effective amount" or "therapeutically sufficient amount" refers to an amount of the antibody molecule which is effective, upon single or multiple dose administration to a subject, (a) to cause a detectable decrease in the severity of the disorder or disease state being treated; (b) to ameliorate or alleviate the patient's symptoms of the disease or disorder; or (c) to slow or prevent advancement of, or otherwise stabilize or prolong stabilization of, the disorder or disease state being treated (e.g., prevent additional tumor growth of a cancer) or a cell, e.g., cancer cell (e.g., a SYK-expressing, pSYK expressing or SYK-activated tumor cell), or in prolonging curing, alleviating, relieving or improving a subject with a disorder as described herein beyond that expected in the absence of such treatment. As used herein, "inhibiting the growth" of the tumor or cancer refers to slowing, interrupting, arresting or stopping its growth and/or metastases and does not necessarily indicate a total elimination of the tumor growth. A specific dosage and treatment regimen for any particular patient may depend upon a variety of factors, including the activity of the specific compound employed, the age, body weight, general health, sex, and diet of the patient, time of administration, rate of excretion, drug combinations, the judgment of the treating physician, and the severity of the particular disease being treated.

The H-score approach provides optimal data resolution for determining variation in intensity and tumor percentage of staining within and among tumor types. It also provides a good tool for determining thresholds for positive staining. In this method, the percentage of cells (0-100) within a tumor with staining intensities ranging from 0-3+ are provided. With the instant method, scores with intensities of 0, 0.5, 1, 2 and 3 were provided. Depending on the marker, 0.5 staining may be scored as positive or negative, and reflects light but perceptible staining for the marker. To obtain an H-score, the percentage of tumor cells are multiplied by each intensity and added together:

$$\text{H score} = (\%\ \text{tumor}^*\ 1) + (\%\ \text{tumor}^*2) + (\%\ \text{tumor}^*3).$$

For example, if a tumor is 20% negative (0), 30% +1, 10% +2, 40% +3, this would give an H score of 170.

The maximum H-score is 300 (100% * +3), per sub-cellular localization (i.e., apical or cytoplasmic), if 100% of tumor cells label with 3+ intensity. Initially, as a control, the total H-score alone was not be used to compare samples, but evaluated in addition to a review of the break-down of the percentage of cells at each intensity. For example, a score of 90 could represent 90% of tumor cells staining with 1+ intensity or 30% of cells with 3+ intensity. These samples have the same H-score but very different pSYK expression. The percentage of cells to be scored at each intensity may vary, but are normally scored in increments of 10%; however, a small percentage of scoring of a single component may be estimated at 1% and 5% as well in order to demonstrate that some level of staining is present. For pSYK, apical staining may be considered for evaluating at low level increments, such as 1 and 5%.

Anti-pSYK Antibodies The anti-pSYK antibody molecules of the invention are useful, inter alia, to detect pSYK expression. In some embodiments, the anti-pSYK antibody molecules are useful for detecting autophosphorylated pSYK. In some embodiments, the anti-pSYK antibody molecules are useful for detecting pSYK Y525/526. In some embodiments, the anti-pSYK antibody molecules are useful for detecting activated pSYK, e.g., SYK which can lead to the phosphorylation of BLNK, BTK, or PLCγ. The anti-pSYK antibody molecules, e.g., useful for pSYK detection, may include non-human anti-pSYK antibody molecules (e.g., non-human and non- murine antibody molecules) that specifically bind to pSYK, e.g., with a binding affinity of at least $10^3$, $10^4$, $10^5$, $10^6$, $10^7$,$10^8$, $10^9$, $10^{10}$ or $10^{11}$ $M^{-1}$ for pSYK, e.g., for pSYK Y525/526. The anti-pSYK antibody molecule may be a non-human, non-murine and non-rat antibody molecule, e.g., a rabbit anti-pSYK antibody molecule, e.g., as described herein.

In certain aspects, the invention relates to anti-pSYK antibody molecules that include features such as those summarized in Tables 1 and 2. In other aspects, the invention relates to anti-pSYK antibody molecules that include features such as those summarized in Tables 3, 4, 5 and/or 6.

In an embodiment, the anti-pSYK antibody molecule is a rabbit hybridoma antibody and is one of antibody MIL81-1-8. In an embodiment, the anti-pSYK antibody molecule is derived from antibody MIL81-1-8. In another embodiment, the anti-pSYK antibody molecule is a rabbit hybridoma antibody and is MIL81-2-1. In an embodiment, the anti-pSYK antibody molecule is derived from antibody MIL81-2-1. In another embodiment, the anti-pSYK antibody molecule is a rabbit hybridoma antibody and is MIL81-99-1. In an embodiment, the anti-pSYK antibody molecule is derived from antibody MIL81-99-1.

In an embodiment an anti-pSYK antibody molecule has an affinity for pSYK, e.g., as measured by direct binding or competition binding assays. In an embodiment the anti-pSYK antibody molecule has a $K_d$ of less than $1x10^{-6}$ M, less than $1x10^{-7}$ M, less than $1x10^{-8}$ M, less than $1x10^{-9}$ M, less than $1x10^{-10}$ M, less than $1x 10^{-11}$ M, less than $1x10^{-12}$ M, or less than $1x10^{-13}$ M. In an embodiment the antibody molecule is an IgG, or antigen-binding fragment thereof, and has a $K_d$ of less than $1x10^{-6}$ M, less than $1x10^{-7}$ M, less than $1x10^{-8}$ M, or less than $1x10^{-9}$ M. In an embodiment, an anti-pSYK antibody molecule, e.g., a MIL81-1-8 antibody or antibody derived therefrom has a $K_d$ of about 80 to about 200 pM, about 50 to about 400 pM, about 100 to about 300 pM, about 100 to about 150 pM or about 120 pM. In an embodiment, an anti-pSYK antibody molecule, e.g., a MIL81-1-8 antibody or antibody derived therefrom has a $k_a$ of about 0.9 to about $1.25x10^5$ $M^{-1}$ $s^{-1}$, or about $1.1x10^s$ $M^{-1}$ $s^{-1}$. In an embodiment the antibody molecule is an ScFv and has a $K_d$ of less than $1x10^{-6}$ M, less than $1x10^{-7}$ M, less than $1x10^{-8}$ M, less than $1x10^{-9}$ M, less than $1x10^{-10}$ M, $1x10^{-11}$ M, less than $1x10^{-12}$ M, or less than $1x10^{-13}$ M.

The naturally occurring mammalian antibody structural unit is typified by a tetramer. Each tetramer is composed of two pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains can be classified as kappa and lambda light chains. Heavy chains may be classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989)). The variable regions of each light/heavy chain pair form the antibody binding site. In certain embodiments the isotypes for the anti-pSYK antibody molecules are IgG immunoglobulins, which may be classified into four subclasses, IgGl, IgG2, IgG3 and IgG4, having different gamma heavy chains. Most therapeutic antibodies are human, chimeric, or humanized antibodies of the IgG1 isotype. In a particular embodiment, the anti-pSYK antibody molecule is a rabbit IgG antibody.

The variable regions of each heavy and light chain pair form the antigen binding site. Thus, an intact IgG antibody has two binding sites which are the same. However, bifunctional or bispecific antibodies are artificial hybrid constructs which have two different heavy/light chain pairs, resulting in two different binding sites.

The chains all exhibit the same general structure of relatively conserved framework regions (FR) joined by three hypervariable regions, also called complementarity determining regions or CDRs. The CDRs from the two chains of each pair are aligned by the framework regions, enabling binding to a specific epitope. From N-terminal to C-terminal, both light and heavy chains comprise the domains FR1, CDR1, FR2, CDR2, FR3, CDR3 and FR4. The assignment of amino acids to each domain is in accordance with the definitions of Kabat Sequences of Proteins of Immunological Interest (National Institutes of Health, Bethesda, Md. (1987 and 1991)), or Chothia & Lesk J. Mol. Biol. 196:901-917 (1987); Chothia et al. Nature 342:878-883 (1989). As used herein, CDRs are referred to for each of the heavy (HCDR1, HCDR2, HCDR3) and light (LCDR1, LCDR2, LCDR3) chains.

An anti-pSYK antibody molecule can comprise all, or an antigen binding subset of the CDRs, of one or both, the heavy and light chain, of one of the above-referenced rabbit antibodies. Amino acid sequences of rabbit hybridoma antibodies, including variable regions and CDRs, may be found in Table 3 and Table 5.

Thus, the antibody molecule may include one or both of: (a) one, two, three, or an antigen binding number of, light chain CDRs (LCDR1, LCDR2 and/or LCDR3) of one of the above-referenced rabbit hybridoma antibodies, or the CDR(s) may comprise an amino acid sequence of one or more or all of LCDR1-3 as follows: LCDR1, or modified LCDR1 wherein one to seven amino acids are conservatively substituted) LCDR2, or modified LCDR2 wherein one or two amino acids are conservatively substituted); or LCDR3, or modified LCDR3 wherein one or two amino acids are conservatively substituted; and (b) one, two, three, or an antigen binding number of, heavy chain CDRs (HCDR1, HCDR2 and/or HCDR3) of one of the above-referenced rabbit hybridoma antibodies. In certain examples the CDR(s) may comprise an amino acid sequence of one or more or all of HCDR1-3 as follows: HCDR1, or modified HCDR1 wherein one or two amino acids are conservatively substituted; HCDR2, or modified HCDR2 wherein one to four amino acids are conservatively substituted; or HCDR3, or modified HCDR3 wherein one or two amino acids are conservatively substituted.

Useful immunogens for production of anti-pSYK antibody molecules include pSYK e.g., human pSYK-expressing cells; membrane fractions of pSYK-expressing cells; recombinant cells expressing pSYK; isolated or purified pSYK, e.g., human pSYK protein (e.g., biochemically isolated pSYK, or a portion thereof (e.g., a portion or peptide comprising the phosphorylation sites of pSYK, e.g., comprising at least about 8, 10, 12, 14, 16, 20, 24, 28 or 32 amino acid residues of SEQ ID NO:2)). In some examples, an immunogen to generate an anti-pSYK antibody is a synthetic phosphopeptide corresponding to residues surrounding Tyr525/526 of human SYK. For example, the peptide immunogen is amino acids 520 to 529 of SEQ ID NO:2, modified with phosphate on tyrosine 525 and/or 526.

Immunogens may be fused to heterologous sequences to aid in biochemical manipulation, purification, immunization or antibody titer measurement. Such immunogens may comprise a portion of pSYK, e.g., the phosphorylation domain, and a portion comprising a non-pSYK polypeptide. Many options exist for constructing a fusion protein for ease of purification or immobilization onto a solid support, e.g., an affinity column or a microtiter plate or other suitable assay substrate/chip. For example, a fusion moiety can add a domain, e.g., glutathione-S -transferase/kinase (GST), which can bind glutathione; an Fc region of an immunoglobulin, which can bind to protein A or protein G; amino acid residues, e.g., two, three, four, five, or six histidine residues which can bind nickel or cobalt on an affinity column; an epitope tag, e.g., a portion of c-myc oncogene (myc-tag), a FLAG tag (U.S. Pat. No. 4,703,004), a hemagglutinin (HA) tag, a T7 gene 10 tag, a V5 tag, an HSV tag, or a VSV-G tag which can bind a tag-specific antibody; or a cofactor, e.g., biotin, which can bind streptavidin. In some examples, a peptide immunogen has a terminal cysteine so the peptide may be conjugated to a hapten or carrier protein. In some examples, the peptide immunogen has an N-terminal cysteine.

Immunogens which comprise the Fc portion of an immunoglobulin can hold the pSYK, either in solution or attached to a cell, in a configuration which allows structural access to pSYK epitopes by the host immune surveillance components for efficient antibody generation. Because immunoglobulin heavy chains comprising the Fc regions associate into dimers through interchain disulfide bonds, immunogens resulting from fusion with Fc regions are dimers.

An Fc portion derived from a non-host species, e.g., human Ig Fc region, for fusing to an immunogen for immunization in a host species, e.g., mouse, rat, rabbit, goat, acts as an adjuvant. This adjuvant function can trigger specific antibodies against both Fc and pSYK epitopes. Fc-reactive antibodies may be identified and discarded during screening. The Fc portion may have a wild type sequence or a sequence which is mutated to modify effector function. For example, a mutated constant region (variant) may be incorporated into a fusion protein to minimize binding to Fc receptors and/or ability to fix complement (see e.g. Winter et al, GB 2,209,757 B; Morrison et al., WO 89/07142; Morgan et al., WO 94/29351). In a certain embodiments, lysine 235 and glycine 237, numbered according to Fc region standards, are mutated, e.g., to alanine. An immunogen/fusion protein with Fc-mutated IgG can have reduced interaction with Fc receptors in the host.

Useful epitopes, e.g., reference epitopes, from the pSYK molecule, to which the anti-pSYK antibody molecules, e.g., rabbit monoclonal antibodies, or humanized versions thereof, as described herein, may bind, may be found in permeabilized cells, membrane fractions, cell lysates, and in tissue sections.

For example, an epitope for an anti-pSYK antibody molecule may reside within, or include a residue(s) from, residues 500-550 of SEQ ID NO:2; residues 510-540 of SEQ ID NO:2; residues 515-530 of SEQ ID NO:2; or fragments thereof that bind an anti-pSYK antibody molecule of the invention, e.g., a MIL81-1-8-binding fragment thereof. Such fragments may comprise residues 525/526 and surrounding residues of SEQ ID NO:2, and may be phosphorylated e.g. phosphorylated at tyrosine 525 and/or phosphorylated tyrosine 526. In some embodiments, an epitope for an anti-pSYK antibody molecule, e.g., a MIL81-1-8 antibody, is a conformational epitope further comprising one or more additional amino acid residues in the SYK amino acid sequence, i.e., selected from about residue 1 to 635 of SEQ ID NO:2.

Antibodies raised against such epitopes or the phosphorylation domain, e.g., epitopes that reside within, or include a residue(s) from amino acid residues 500-550, 510-540, 515-530 or 520-529 of SEQ ID NO:2, or antibody molecules derived therefrom, may be useful as therapeutic or diagnostic antibodies, as described herein.

In an embodiment, the anti-pSYK antibody molecule has one or more of the following properties: a) it competes for binding, e.g., binding to cytoplasmic pSYK or purified pSYK, with one of the above-referenced anti-pSYK antibody molecules summarized in Tables 1 and 2 or rabbit hybridoma antibodies (e.g., MIL81-1-8, MIL81-2-1 or MIL81-99-1); b) it binds to the same, or substantially the same, epitope on pSYK as one of the above-referenced anti-pSYK antibody molecules summarized in Tables 1 and 2, or rabbit hybridoma antibodies (e.g., MIL81-1-8, MIL81-2-1 or MIL81-99-1). In an embodiment, the antibody binds the same epitope, as determined by one or more of a peptide array assay or by binding to truncation mutants, chimeras or point mutants of SEQ ID NO:2 expressed in the cytoplasm or membrane preparations, e.g., as those assays are described herein; c) it binds to an epitope which has at least 1, 2, 3, 4, 5, 8, 10, 15 or 20 contiguous amino acid residues in common with the epitope of one of the above-referenced anti-pSYK antibody molecules summarized in Tables 1 and 2, or rabbit hybridoma antibodies (e.g., MIL81-1-8, MIL81-2-1 or MIL81-99-1); d) it binds a region of human pSYK that is bound by an anti-pSYK antibody of the invention, wherein the region e.g., cytoplasmic region, is 10-15, 10-20, 20-30, or 20-40 residues in length, and binding is determined, e.g., by binding to truncation mutants. In an embodiment the anti-pSYK antibody molecule binds the phosphorylation domain of human pSYK. In an embodiment an anti-pSYK antibody molecule may bind the human pSYK portion defined by amino acid residues 500-550, 510-540, 515-530 or 520-529 of SEQ ID NO:2. In an embodiment an anti-pSYK antibody molecule binds the phosphorylation site at amino acid residues 525 and/or 526 of SEQ ID NO:2; or e) it binds to a reference epitope described herein.

In an embodiment, the anti-pSYK antibody molecule binds the SYK sequence: RADEN-pY-pY-KAQ (amino acids 520 to 529 of SEQ ID NO:2, modified with phosphate on tyrosine 525 and 526).

In some embodiments, the antibody molecule binds a conformational epitope. In other embodiments, an antibody molecule binds a linear epitope.

The anti-pSYK antibody molecules may be polyclonal antibodies, monoclonal antibodies, monospecific antibodies, chimeric antibodies (See U.S. Pat. No. 6,020,153) or humanized antibodies or antibody fragments or derivatives thereof. Synthetic and genetically engineered variants (See U.S. Pat. No. 6,331,415) of any of the foregoing are also contemplated by the present invention. Monoclonal antibodies may be produced by a variety of techniques, including conventional murine monoclonal antibody methodology (e.g., the standard somatic cell hybridization technique of Kohler and Milstein, Nature 256: 495 (1975); see generally, Harlow, E. and Lane, D. (1988) Antibodies: A Laboratory Manual, Cold Spring Harbor Laboratory Press, Cold Spring Harbor, N.Y), and the rabbit monoclonal antibody technology and services provided by Epitomics (Burlingame, CA) which produces custom rabbit monoclonal antibodies (RabMAbs®) using rabbit-rabbit hybridomas generated by fusing isolated B-cells from an immunized rabbit with a fusion partner cell line, as described in U.S. Patents 7,402,409, 7,429,487, 7,462,697, 7,575,896, 7,732,168, and 8,062,867.

Immunization with protein, e.g., pSYK or a soluble portion, or fusion protein comprising a portion of pSYK, such as immunogen peptide, SEQ ID NO:25, or cells or membrane fractions therefrom, e.g., cells expressing surface-exposed pSYK or a portion thereof, may be performed with the immunogen prepared for injection in a manner to induce a response, e.g., with adjuvant, e.g., complete Freund's adjuvant. Other suitable adjuvants include TITERMAX GOLD® adjuvant (CYTRX Corporation, Los Angeles, Calif.) and alum. Small peptide immunogens, such as SEQ ID NO:25, may be linked to a larger molecule, such as keyhole limpet hemocyanin (KLH). Mice or rabbits may be injected in a number of manners, e.g., subcutaneous, intravenous or intramuscular at a number of sites, e.g., in the peritoneum (i.p.), base of the tail, or foot pad, or a combination of sites, e.g., iP and base of tail (BIP). Booster injections can include the same or a different immunogen and can additionally include adjuvant, e.g., incomplete Freund's adjuvant. Immunization with DNA, e.g., DNA encoding SYK or a portion thereof or fusion protein comprising pSYK or a portion thereof may be injected using gene gun technology. For example, DNA is loaded onto microscopic gold particles and injected into mice or rabbits at frequent intervals over a brief period. Generally, where a monoclonal antibody is desired, a hybridoma is produced by fusing a suitable cell from an immortal cell line (e.g., a myeloma cell line such as SP2/0, P3X63Ag8.653 or a heteromyeloma) with antibody-producing cells. Antibody-producing cells may be obtained from the peripheral blood or the spleen or lymph nodes, of humans, human-antibody transgenic animals or other suitable animals (e.g., rabbits) immunized with the antigen of interest. Cells that produce antibodies of human origin (e.g., a human antibody) may be produced using suitable methods, for example, fusion of a human antibody-producing cell and a heteromyeloma or trioma, or immortalization of an activated human B cell via infection with Epstein Barr virus. (See, e.g., U.S. Pat. No. 6,197,582 (Trakht); Niedbala et al., Hybridoma, 17:299-304 (1998); Zanella et al., J Immunol Methods, 156:205-215 (1992); and Gustafsson et al., Hum Antibodies Hybridomas, 2:26-32 (1991)). In some examples, the fusion is performed using the fusion partner cell line 240E-1 from U.S. Patent No. 5,675,063 or 240E-W from U.S. Patent No. 7,429,487.

The fused or immortalized antibody-producing cells (hybridomas) may be isolated using selective culture conditions, and cloned by limiting dilution. Cells which produce antibodies with the desired specificity may be identified using a suitable assay (e.g., ELISA (e.g., with immunogen, immobilized on the microtiter well) or by FACS on a cell expressing pSYK or a portion thereof). For example, if the pSYK-immunogen comprises a fusion moiety that is an affinity reagent, this moiety can allow the fusion protein comprising pSYK or a portion thereof to be bound to a matrix, e.g., protein G-coated, streptavidin-coated, glutathione-derivatized or antibody-coated microtitre plates or assay chips, which are then combined with the immune serum or conditioned medium from a hybridoma or antibody-expressing recombinant cell, and the mixture incubated under conditions conducive to complex formation (e.g., at physiological conditions for salt and pH). Following incubation, the microtitre plate wells or chip cells are washed to remove any unbound components and binding by anti-pSYK antibody is measured.

In some examples, the screening to identify the anti-pSYK antibody measures binding to the immunogen. In some examples, the screening to identify the anti-pSYK antibody measures binding to pSYK. In some examples, the pSYK is GST-tagged recombinant SYK. In some examples, the GST-tagged recombinant SYK is autophosphorylated.

In some examples, the pSYK is a component of a lysate from a cell comprising activated SYK. Control reagents useful to identify anti-pSYK antibodies and ensure specificity include using unstimulated cells, sample comprising SYK phosphorylated at sites other than Y525 or Y526 or phosphatase-treated sample, such as a sample treated with T-cell protein phosphatase (New England Biolabs, Ipswich, MA). In some embodiments, the screening to identify the anti-pSYK antibody compares the binding to a composition comprising autophosphorylated pSYK with binding to a composition comprising phosphatase treated pSYK and selects an antibody whose binding is reduced or eliminated in the phosphatase-treated composition.

In certain embodiments, e.g., for in vivo or therapeutic applications, the antibodies of the present invention are humanized antibodies. The advantage of humanized antibodies is that they potentially decrease or eliminate the immunogenicity of the antibody in a subject recipient, thereby permitting an increase in the bioavailability and a reduction in the possibility of adverse immune reaction, thus potentially enabling multiple antibody administrations.

Humanization and Display Technologies and Modifications to Antibodies

Humanized antibody molecules may minimize the immunogenic and allergic responses intrinsic to non-human or non-human-derivatized mAbs and thus to increase the efficacy and safety of the antibodies administered to human subjects. The use of humanized antibody molecules may provide a substantial advantage in the treatment of chronic and recurring human diseases, such as inflammation, autoimmunity, and cancer, which require repeated antibody administrations.

The production of humanized antibodies with reduced immunogenicity may be accomplished in connection with techniques of humanization and display techniques using appropriate libraries. It will be appreciated that antibodies from non-human species, such as mice, rats, rabbits, sheep, goats, etc., may be humanized or primatized using techniques known in the art. See e.g., Winter and Harris Immunol Today 14:43-46 (1993) and Wright et al. Crit. Reviews in Immunol. 12125-168 (1992). The antibody of interest may be engineered by recombinant DNA techniques to substitute the CH1, CH2, CH3, hinge domains, and/or the framework domain with the corresponding human sequence (see WO 92/02190 and U.S. Pat. Nos. 5,530,101, 5,585,089, 5,693,761, 5,693,792, 5,714,350, and 5,777,085). Also, the use of Ig cDNA for construction of chimeric immunoglobulin genes is known in the art (Liu et al. Proc Natl Acad Sci USA. 84:3439 (1987) and J. Immunol. 139:3521 (1987)). mRNA is isolated from a hybridoma or other cell producing the antibody and used to produce cDNA: The cDNA of interest may be amplified by the polymerase chain reaction using specific primers (U.S. Pat. Nos. 4,683,195 and 4,683,202).

Alternatively, phage display technology (see, e.g., McCafferty et al, Nature, 348:552-553 (1990)) may be used to produce human antibodies or antibodies from other species, as well as antibody fragments in vitro, from immunoglobulin variable (V) domain genes, e.g., from repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display may be performed in a variety of formats; for their review see, e.g., Johnson and Chiswell, Current Opinion in Structural Biology, 3:564-571 (1993).

Several sources of V-gene segments may be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors may be constructed and antibodies to a diverse array of antigens (including self-antigens) may be isolated essentially following the techniques described by Marks et al., J. Mol. Biol, 222:581-597 (1991), or Griffith et al, EMBO J., 12:725-734 (1993). See, also, U.S. Pat. Nos. 5,565,332 and 5,573,905. Display libraries may contain antibodies or antigen-binding fragments of antibodies that contain artificial amino acid sequences. For example, the library may contain Fab fragments which contain artificial CDRs (e.g., random amino acid sequences) and human framework regions. (See, for example, U.S. Pat. No. 6,300,064 (Knappik, et al.).)

The sequences of human constant region genes may be found in Kabat et al. (1991) Sequences of Proteins of Immunological Interest, N.I.H. publication no. 91-3242, the NCBI IgBLAST database or the Abysis antibody database maintained by the bioinformatics group at University College London, UK. Human C region genes are readily available from known clones. The choice of isotype will be guided by the desired effector functions, such as complement fixation, or activity in antibody-dependent cellular cytotoxicity. Isotypes may be IgGl, IgG2, IgG3 or IgG4. In particular embodiments, antibody molecules of the invention are IgG1 and IgG2. In certain embodiments, the antibody molecules of the invention are IgG2. Either of the human light chain constant regions, kappa or lambda, may be used. The chimeric, humanized antibody is then expressed by conventional methods.

In some embodiments, an anti-pSYK antibody molecule of the invention may draw antibody-dependent cellular cytotoxicity (ADCC) to a cell expressing pSYK, e.g., a tumor cell. Antibodies with the IgG1 and IgG3 isotypes are useful for eliciting effector function in an antibody-dependent cytotoxic capacity, due to their ability to bind the Fc receptor. Antibodies with the IgG2 and IgG4 isotypes are useful to minimize an ADCC response because of their low ability to bind the Fc receptor. In related embodiments substitutions in the Fc region or changes in the glycosylation composition of an antibody, e.g., by growth in a modified eukaryotic cell line, may be made to enhance the ability of Fc receptors to recognize, bind, and/or mediate cytotoxicity of cells to which anti-pSYK antibodies bind (see, e.g., U.S. Pat. Nos. 7,317,091, 5,624,821 and publications including WO 00/42072, Shields, et al. J. Biol. Chem. 276:6591-6604 (2001), Lazar et al. Proc. Natl. Acad. Sci. U.S.A. 103:4005-4010 (2006), Satoh et al. Expert Opin Biol. Ther. 6:1161-1173 (2006)). In certain embodiments, the antibody or antigen-binding fragment (e.g., antibody of human origin, human antibody) may include amino acid substitutions or replacements that alter or tailor function (e.g., effector function). For example, a constant region of human origin (e.g., γ1 constant region, γ2 constant region) may be designed to reduce complement activation and/or Fc receptor binding. (See, for example, U.S. Pat. Nos. 5,648,260 (Winter et al.), 5,624,821 (Winter et al.) and 5,834,597 (Tso et al.))

In certain embodiments, the amino acid sequence of a constant region of human origin that contains such amino acid substitutions or replacements is at least about 95% identical over the full length to the amino acid sequence of the unaltered constant region of human origin, or at least about 99% identical over the full length to the amino acid sequence of the unaltered constant region of human origin.

In still another embodiment, effector functions may also be altered by modulating the glycosylation pattern of the antibody. By altering is meant deleting one or more carbohydrate moieties found in the antibody, and/or adding one or more glycosylation sites that are not present in the antibody. For example, antibodies with enhanced ADCC activities with a mature carbohydrate structure that lacks fucose attached to an Fc region of the antibody are described in U.S. Patent Application Publication No. 2003/0157108 (Presta). See also U.S. Patent Application Publication No. 2004/0093621 (Kyowa Hakko Kogyo Co., Ltd). Glycofi has also developed yeast cell lines capable of producing specific glycoforms of antibodies. Humanized antibodies may also be made using a CDR-grafted approach. Techniques of generation of such humanized antibodies are known in the art. Generally, humanized antibodies are produced by obtaining nucleic acid sequences that encode the variable heavy and variable light sequences of an antibody that binds to pSYK, identifying the complementary determining region or "CDR" in the variable heavy and variable light sequences and grafting the CDR nucleic acid sequences on to human framework nucleic acid sequences. (See, for example, U.S. Pat. Nos. 4,816,567 and 5,225,539). The location of the CDRs and framework residues may be determined (see, Kabat, E. A., et al. (1991) Sequences of Proteins of Immunological Interest, Fifth Edition, U.S. Department of Health and Human Services, NIH Publication No. 91-3242, and Chothia, C. et al. J. Mol. Biol. 196:901-917 (1987)). Anti-pSYK antibody molecules described herein have the CDR amino acid sequences and nucleic acid sequences encoding CDRs listed in Tables 5 and 6. In some embodiments sequences from Tables 5 and 6 may be incorporated into molecules which recognize pSYK for use in the therapeutic or diagnostic methods described herein. The human framework that is selected is one that is suitable for in vivo administration, meaning that it does not exhibit immunogenicity. For example, such a determination may be made by prior experience with in vivo usage of such antibodies and studies of amino acid similarities. A suitable framework region may be selected from an antibody of human origin having at least about 65%, at least about 70%, at least about 80%, at least about 90% or at least about 95% amino acid sequence identity over the length of the framework region within the amino acid sequence of the equivalent portion (e.g., framework region) of the donor antibody, e.g., an anti-pSYK antibody molecule. Amino acid sequence identity may be determined using a suitable amino acid sequence alignment algorithm, such as CLUSTAL W, using the default parameters. (Thompson J. D. et al., Nucleic Acids Res. 22:4673-4680 (1994)).

Once the CDRs and FRs of the cloned antibody that are to be humanized are identified, the amino acid sequences encoding the CDRs are identified and the corresponding nucleic acid sequences grafted on to selected human FRs. This may be done using known primers and linkers, the selection of which are known in the art. All of the CDRs of a particular human antibody may be replaced with at least a portion of a non-human CDR or only some of the CDRs may be replaced with non-human CDRs. It is only necessary to replace the number of CDRs required for binding of the humanized antibody to a predetermined antigen. After the CDRs are grafted onto selected human FRs, the resulting "humanized" variable heavy and variable light sequences are expressed to produce a humanized Fv or humanized antibody that binds to pSYK. In certain cases, the CDR-grafted (e.g., humanized) antibody binds a pSYK protein with an affinity similar to, substantially the same as, or better than that of the donor antibody. Typically, the humanized variable heavy and light sequences are expressed as a fusion protein with human constant domain sequences so an intact antibody that binds to pSYK is obtained. However, a humanized Fv antibody may be produced that does not contain the constant sequences.

Also within the scope of the invention are humanized anti-SYK antibody molecules in which specific amino acids have been substituted, deleted or added. In particular, humanized antibodies may have amino acid substitutions in the framework region, such as to improve binding to the antigen. For example, a selected, small number of acceptor framework residues of the humanized immunoglobulin chain may be replaced by the corresponding donor amino acids. Locations of the substitutions include amino acid residues adjacent to the CDR, or which are capable of interacting with a CDR (see e.g., U.S. Pat. No. 5,585,089 or 5,859,205). The acceptor framework may be a mature human antibody framework sequence or a consensus sequence. As used herein, the term "consensus sequence" refers to the sequence found most frequently, or devised from the most common residues at each position in a sequence in a region among related family members. A number of human antibody consensus sequences are available, including consensus sequences for the different subgroups of human variable regions (see, Kabat, E. A., et al., Sequences of Proteins of Immunological Interest, Fifth Edition, U.S. Department of Health and Human Services, U.S. Government Printing Office (1991)). The Kabat database and its applications are freely available on line, e.g. via IgBLAST at the National Center for Biotechnology Information, Bethesda, Md. (also see, Johnson, G. and Wu, T. T., Nucleic Acids Research 29:205-206 (2001)).

Other techniques for humanizing antibodies are described in Padlan et al. EP 519596 A1, published on Dec. 23, 1992.

The anti-pSYK antibody molecule includes other humanized antibodies which may also be modified by specific deletion of human T cell epitopes or "deimmunization" by the methods disclosed in PCT Publication Nos. WO 98/52976 and WO 00/34317. Briefly, the rabbit, or other non-human species, heavy and light chain variable regions of an anti-pSYK antibody may be analyzed for peptides that bind to MHC Class II; these peptides represent potential T-cell epitopes. For detection of potential T-cell epitopes, a computer modeling approach termed "peptide threading" may be applied, and in addition a database of human MHC class II binding peptides may be searched for motifs present in the rabbit VH and VL sequences, as described in PCT Publication Nos. WO 98/52976 and WO 00/34317. These motifs bind to any of the 18 major MHC class II DR allotypes, and thus constitute potential T cell epitopes. Potential T-cell epitopes detected may be eliminated by substituting small numbers of amino acid residues in the variable regions or by single amino acid substitutions. After the deimmunized VH and VL of an anti-pSYK antibody are constructed by mutagenesis of the rabbit VH and VL genes, the mutagenized variable sequence may, optionally, be fused to a human constant region, e.g., human IgGI, IgG2 or K (kappa) constant regions.

Reduction of an immunogenic response by a CDR-grafted antibody may be achieved by changes, e.g., deletions, substitutions, of amino acid residues in CDRs (Kashmiri et al. Methods 36:25-34 (2005), U.S. Pat. No. 6,818,749, Tan et al. J. Immunol. 169:1119-1125 (2006)). For example, in certain embodiments residues at positions involved in contact with the antigen would not be changed. Typically, such residues, the specificity determining residues (SDRs), are in positions which display high levels of variability among antibodies. Consensus sequences derived, e.g., by the Clustal method (Higgins D. G. et al., Meth. Enzymol. 266:383-402 (1996)), from anti-pSYK antibody molecules, e.g., from antibodies described herein, aid in identifying SDRs. In the anti-pSYK antibody molecules described herein, the SDRs are the following, at least the first residue or in some embodiments, the first four residues of heavy chain CDR1; at least the N-terminal portion, e.g., the first seven, ten or 13 residues of heavy chain CDR2; nearly all of heavy chain CDR3; the C-terminal portion, e.g., after residue six, eight, or nine of light chain CDR1; about the first, middle and/or last residue of light chain CDR2; and most of light chain CDR3, or at least after residue two or three. Accordingly, to maintain binding to pSYK protein after humanization or modification of an anti-pSYK antibody molecule, such SDR residues in CDRs of the anti-pSYK antibody molecules are less amenable to changes, e.g., from rabbit residues to human consensus residues than are residues in other residues of the CDRs or the framework regions. Conversely, in certain embodiments, it may be beneficial to change residues in non-human, e.g., rabbit CDRs to residues identified as consensus in human CDRs, e.g., CDRs of anti-pSYK antibody.

Anti-pSYK antibodies that are not intact antibodies are also disclosed. Such antibodies may be derived from any of the antibodies described above. Useful antibody molecules of this type include (i) a Fab fragment, a monovalent fragment consisting of the VL, VH, CL and CHI domains; (ii) a $F(ab')_2$ fragment, a bivalent fragment comprising two Fab fragments linked by a disulfide bridge at the hinge region; (iii) an Fd fragment consisting of the VH and CHI domains; (iv) an Fv fragment consisting of the VL and VH domains of a single arm of an antibody, (v) a dAb fragment (Ward et al., Nature 341:544-546 (1989)), which consists of a VH domain; (vii) a single domain functional heavy chain antibody, which consists of a VHH domain (known as a nanobody) see e.g., Cortez-Retamozo, et al., Cancer Res. 64: 2853-2857 (2004), and references cited therein; and (vii) an isolated CDR, e.g., one or more isolated CDRs together with sufficient framework to provide an antigen binding fragment. Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain Fv (scFv); see e.g., Bird et al. Science 242:423-426 (1988); and Huston et al. Proc. Natl. Acad. Sci. USA 85:5879-5883 (1988). Such single chain antibodies are also intended to be encompassed within the term "antigen-binding fragment" of an antibody. These antibody fragments are obtained using conventional techniques known to those with skill in the art, and the fragments are screened for utility in the same manner as are intact antibodies.

Antibody fragments, such as Fv, $F(ab')_2$ and Fab may be prepared by cleavage of the intact protein, e.g. by protease or chemical cleavage.

In certain embodiments, all of the CDRs sequences or both the heavy and light chain, may be used in another antibody molecule, e.g., in a CDR-grafted, humanized, or chimeric antibody molecule.

Embodiments include an antibody molecule that comprises sufficient CDRs, e.g., all six CDRs from one of the rabbit hybridoma antibodies described herein to allow binding to cytoplasmic pSYK.

In an embodiment the CDRs, e.g., all of the HCDRs, or all of the LCDRs, or all six, are embedded in human or human derived framework region(s). Examples of human framework regions include human germline framework sequences, human germline sequences that have been affinity matured (either in vivo or in vitro), or synthetic human sequences, e.g., consensus sequences. In an embodiment the heavy chain framework is an IgG1 or IgG2 framework. In an embodiment the light chain framework is a kappa framework.

In an embodiment the anti-pSYK antibody molecule, e.g., a CDR-grafted or humanized antibody molecule, comprises sufficient CDRs, e.g., all six CDRs from one of the antibodies described herein, e.g., sequences listed in Table 5, to allow binding to pSYK. (Exemplary nucleic acid sequences which encode the CDR amino acid sequences listed in Table 5, are provided, in Table 6 herein). In particular embodiments, an anti-pSYK antibody molecule comprises CDRs from MIL81-1-8.

Antibody fragments for in vivo therapeutic or diagnostic use may benefit from modifications which improve their serum half-lives. Suitable organic moieties intended to increase the in vivo serum half-life of the antibody may include one, two or more linear or branched moiety selected from a hydrophilic polymeric group (e.g., a linear or a branched polymer (e.g., a polyalkane glycol such as polyethylene glycol, monomethoxy-polyethylene glycol and the like), a carbohydrate (e.g., a dextran, a cellulose, a polysaccharide and the like), a polymer of a hydrophilic amino acid (e.g., polylysine, polyaspartate and the like), a polyalkane oxide and polyvinyl pyrrolidone), a fatty acid group (e.g., a mono-carboxylic acid or a di-carboxylic acid), a fatty acid ester group, a lipid group (e.g., diacylglycerol group, sphingolipid group (e.g., ceramidyl)) or a phospholipid group (e.g., phosphatidyl ethanolamine group).

In certain embodiments, the organic moiety is bound to a predetermined site where the organic moiety does not impair the function (e.g., decrease the antigen binding affinity) of the resulting immunoconjugate compared to the non-conjugated antibody moiety. The organic moiety may have a molecular weight of about 500 Da to about 50,000 Da, about 2000, about 5000, about 10,000 or about 20,000 Da. Examples and methods for modifying polypeptides, e.g., antibodies, with organic moieties may be found, for example, in U.S. Pat. Nos. 4,179,337 and 5,612,460, PCT Publication Nos. WO 95/06058 and WO 00/26256, and U.S. Patent Application Publication No. 20030026805.

An anti-pSYK antibody molecule may comprise all, or an antigen binding fragment of the variable region, of one or both, the heavy and light chain, of one of the above-referenced rabbit hybridoma antibodies.

In an embodiment, the light chain amino acid sequence of SEQ ID NO:10 may differ from one of the reference amino acid sequence(s) referred to in (a)(i-ii) by as many as 1, 2, 3, 4, 5, 10, or 15 residues. In certain embodiments the differences are conservative substitutions. In certain embodiments, the differences are in the framework regions. In an embodiment the heavy chain amino acid sequence of SEQ ID NO: 8 may differ from one of the reference amino acid sequence(s) referred to in (b)(i-ii) by as many as 1, 2, 3, 4, 5, 10, or 15 residues. In certain embodiments the differences are conservative substitutions. In certain embodiments the differences are in the framework regions.

In an embodiment, the anti-pSYK antibody molecule comprises both of: (a) a light chain amino acid sequence of all, or an antigen binding fragment of, either, (i) a light chain variable region amino acid sequence from Table 3, SEQ ID NO:10, or (ii) a light chain variable region amino acid encoded by a nucleotide sequence from Table 4, e.g., SEQ ID NO:9; and (b) a heavy chain amino acid sequence of all, or an antigen binding fragment of, either (i) a heavy chain variable region amino acid sequence from Table 3, SEQ ID NO:8, or (ii) a heavy chain amino acid sequence encoded by a nucleotide sequence from Table 4, e.g., SEQ ID NO:7.

In an embodiment the anti-pSYK antibody molecule comprises one or both of: a) a light chain variable region, or an antigen binding fragment thereof, having at least 85, 90, 95, 97 or 99% homology with the light chain variable region of an anti-pSYK antibody molecule of the invention; and (b) a heavy chain variable region, or an antigen binding fragment thereof, having at least 85, 90, 95, 97 or 99% homology with the heavy chain variable region of an anti-pSYK antibody molecule of the invention.

Amino acid sequences of the variable regions of the anti-pSYK antibodies of the invention can be found in Table 3.

In one approach, consensus sequences encoding the heavy and light chain J regions may be used to design oligonucleotides for use as primers to introduce useful restriction sites into the J region for subsequent linkage of V region segments to human C region segments. C region cDNA may be modified by site directed mutagenesis to place a restriction site at the analogous position in the human sequence.

Expression vectors include plasmids, retroviruses, cosmids, YACs, EBV derived episomes, and the like. A convenient vector is one that encodes a functionally complete human CH or CL immunoglobulin sequence, with appropriate restriction sites engineered so that any VH or VL sequence may be easily inserted and expressed. In such vectors, splicing usually occurs between the splice donor site in the inserted J region and the splice acceptor site preceding the human C region, and also at the splice regions that occur within the human CH exons. Suitable expression vectors may contain a number of components, for example, an origin of replication, a selectable marker gene, one or more expression control elements, such as a transcription control element (e.g., promoter, enhancer, terminator) and/or one or more translation signals, a signal sequence or leader sequence, and the like. Polyadenylation and transcription termination occur at native chromosomal sites downstream of the coding regions. The resulting chimeric antibody may be joined to any strong promoter. Examples of suitable vectors that may be used include those that are suitable for mammalian hosts and based on viral replication systems, such as simian virus 40 (SV40), Rous sarcoma virus (RSV), adenovirus 2, bovine papilloma virus (BPV), papovavirus BK mutant (BKV), or mouse and human cytomegalovirus (CMV), and moloney murine leukemia virus (MMLV), native Ig promoters, etc. A variety of suitable vectors are known in the art, including vectors which are maintained in single copy or multiple copies, or which become integrated into the host cell chromosome, e.g., via LTRs, or via artificial chromosomes engineered with multiple integration sites (Lindenbaum et al. Nucleic Acids Res. 32:el72 (2004), Kennard et al. Biotechnol. Bioeng. Online May 20, 2009). Additional examples of suitable vectors are listed in a later section.

Thus, disclosed herein is an expression vector comprising a nucleic acid encoding an antibody, antigen-binding fragment of an antibody (e.g., a humanized, chimeric antibody or antigen-binding fragment of any of the foregoing), antibody chain (e.g., heavy chain, light chain) or antigen-binding portion of an antibody chain that binds a pSYK protein. Expression in eukaryotic host cells is useful because such cells are more likely than prokaryotic cells to assemble and secrete a properly folded and immunologically active antibody. However, any antibody produced that is inactive due to improper folding may be renaturable according to known methods (Kim and Baldwin, "Specific Intermediates in the Folding Reactions of Small Proteins and the Mechanism of Protein Folding", Ann. Rev. Biochem. 51, pp. 459-89 (1982)). It is possible that the host cells will produce portions of intact antibodies, such as light chain dimers or heavy chain dimers, which also are antibody homologs according to the present invention.

Further, as described elsewhere herein, antibodies or antibodies from human or non-human species may be generated through display-type technologies, including, without limitation, phage display, retroviral display, ribosomal display, and other techniques, using techniques well known in the art and the resulting molecules may be subjected to additional maturation, such as affinity maturation, as such techniques are known in the art. Winter and Harris Immunol Today 14:43-46 (1993) and Wright et al. Crit. Reviews in Immunol. 12125-168 (1992), Hanes and Plucthau PNAS USA 94:4937-4942 (1997) (ribosomal display), Parmley and Smith Gene 73:305-318 (1988) (phage display), Scott TIBS 17:241-245 (1992), Cwirla et al. Proc Natl Acad Sci USA 87:6378-6382 (1990), Russel et al. Nucl. Acids Research 21: 1081-1085 (1993), Hoganboom et al. Immunol. Reviews 130:43-68 (1992), Chiswell and McCafferty TIBTECH 10:80-84 (1992), and U.S. Pat. No. 5,733,743. If display technologies are utilized to produce antibodies that are not human, such antibodies may be humanized as described above.

It will be appreciated that antibodies that are generated need not initially possess a particular desired isotype but, rather, the antibody as generated may possess any isotype and still possess desired binding to the pSYK molecule. For example, the antibody produced by the MIL81-1-8 rabbit hybridoma has a rabbit IgG isotype. The isotype of the antibody may be switched thereafter, e.g., to human IgG1, IgG2, or IgG3, using conventional techniques that are known in the art. Such techniques include the use of direct recombinant techniques (see e.g., U.S. Pat. No. 4,816,397), cell-cell fusion techniques (see e.g., U.S. Pat No 5,916,771), among others. In the cell-cell fusion technique, a myeloma or other cell line is prepared that possesses a heavy chain with any desired isotype and another myeloma or other cell line is prepared that possesses the light chain. Such cells may, thereafter, be fused and a cell line expressing an intact antibody may be isolated.

Accordingly, as antibody candidates are generated that meet desired "structural" attributes as discussed above, they may generally be provided with at least certain additional "functional" attributes that are desired through isotype switching.

In an embodiment the variable region or antigen binding fragment thereof may be coupled to a constant region (or fragment thereof) other than the constant region it was generated with, e.g., a constant region (or fragment thereof) from another antibody or to a synthetic constant region (or fragment thereof). In certain embodiments the constant region is an IgGl or IgG2 constant region (or fragment thereof). Sequence changes may be made in the variable or constant regions to modify effector activity of the antibody molecule.

Design and Generation of Other pSYK Binding Agents

The antibodies that are produced and characterized herein with respect to pSYK provide for the design of other therapeutic or diagnostic modalities including other antibodies, other antagonists, or chemical moieties other than antibodies. Such modalities include, without limitation, antibodies having similar binding activity or functionality, advanced antibody therapeutic and diagnostic agents, such as bispecific antibodies, immunoconjugates, and radiolabeled agents, generation of peptide agents, particularly intrabodies, and small molecules. Furthermore, as discussed above, the effector function of the antibodies of the invention may be changed by isotype switching to an IgG1, IgG2, IgG3, IgG4, IgD, IgAl, IgA2, IgE, or IgM for various in vivo uses such as for therapy or imaging.

In connection with bispecific antibodies, bispecific antibodies may be generated that comprise (i) two antibodies, one with a specificity to pSYK and another to a second molecule that are conjugated together, (ii) a single antibody that has one chain specific to pSYK and a second chain specific to a second molecule, or (iii) a single chain antibody that has specificity to pSYK and the other molecule. Such bispecific antibodies may be generated using techniques that are known. For example, bispecific antibodies may be produced by crosslinking two or more antibodies (of the same type or of different types). Suitable crosslinkers include those that are heterobifunctional, having two distinctly reactive groups separated by an appropriate spacer (e.g., m-maleimidobenzoyl-N-hydroxysuccinimide ester) or homobifunctional (e.g.,

disuccinimidyl suberate). Such linkers are available from Pierce Chemical Company, Rockford, 111. See also, e.g., Fanger et al. Immunomethods 4:72-81 (1994) and Winter and Harris Immunol Today 14:43-46 (1993) and Wright et al. Crit. Reviews in Immunol. 12125-168 (1992) and in connection with (iii) see e.g., Traunecker et al. Int. J. Cancer (Suppl.) 7:51-52 (1992). Songsivilai & Lachmann Clin. Exp. Immunol. 79: 315-321 (1990), Kostelny et al. J. Immunol. 148:1547-1553 (1992).

In addition, "Kappabodies" (Ill. et al. "Design and construction of a hybrid immunoglobulin domain with properties of both heavy and light chain variable regions" Protein Eng 10:949-57 (1997)), "Minibodies" (Martin et al. EMBO J. 13:5303-9 (1994), U.S. Pat. No. 5,837,821), "Diabodies" (Holliger et al. P roc Natl Acad Sci USA 90:6444-6448 (1993)), or "Janusins" (Traunecker et al. EMBO J. 10:3655-3659 (1991) and Traunecker et al. Int J Cancer Suppl 7:51-52 (1992)) may also be prepared.

Nucleic Acids and Polypeptides

In another embodiment, the present invention relates to polynucleotide and polypeptide sequences that encode for or represent the anti-SYK antibody molecules of the invention. Such polynucleotides encode for both the variable and constant regions of each of the heavy and light chains, although other combinations are also contemplated herein in accordance with the compositions described herein. Disclosed herein are also oligonucleotide fragments derived from the disclosed polynucleotides and nucleic acid sequences complementary to these polynucleotides.

The polynucleotides can be in the form of RNA or DNA. Polynucleotides in the form of DNA, cDNA, genomic DNA, nucleic acid analogs and synthetic DNA are within the scope of the present invention. The DNA may be double-stranded or single-stranded, and if single stranded, may be the coding (sense) strand or non-coding (anti-sense) strand. The coding sequence that encodes the polypeptide may be identical to the coding sequence provided herein or may be a different coding sequence which coding sequence, as a result of the redundancy or degeneracy of the genetic code, encodes the same polypeptide as the DNA provided herein.

In certain embodiments provided, polynucleotides encode at least one heavy chain variable region and at least one light chain variable region of the present invention, e.g., as summarized in Table 4.

The present invention also includes variant polynucleotides containing modifications such as polynucleotide deletions, substitutions or additions, and any polypeptide modification resulting from the variant polynucleotide sequence. A polynucleotide of the present invention may also have a coding sequence that is a variant of the coding sequence provided herein. For example, a variant polynucleotide may have at least 50%, 60%, 70%, 75%, 80%, 85%, 90%, 95% or 97% identity with a polynucleotide listed in Table 4. In certain embodiments, the variant polynucleotide encodes for an anti-pSYK antibody molecule.

The present disclosure further relates to polypeptides that represent the antibodies of the present invention as well as fragments, analogs and derivatives of such polypeptides. The polypeptides may be recombinant polypeptides, naturally produced polypeptides or synthetic polypeptides. The fragment, derivative or analogs of the polypeptides may be one in which one or more of the amino acid residues is substituted with a conserved or non-conserved amino acid residue and such substituted amino acid residue may or may not be one encoded by the genetic code; or it may be one in which one or more of the amino acid residues includes a substituent group; or it may be one in which the polypeptide is fused with another compound, such as a compound to increase the half-life of the polypeptide (for example, polyethylene glycol); or it may be one in which the additional amino acids are fused to the polypeptide, such as a leader or secretory sequence or a sequence that is employed for purification of the polypeptide or a proprotein sequence. Such fragments, derivatives and analogs are within the scope of the present invention. In various aspects, the polypeptides of the invention may be partially purified, or purified, product.

A polypeptide disclosed herein can have an amino acid sequence that is identical to that of the antibodies described herein, e.g., summarized in Tables 2 or 3, or that is different by minor variations due to one or more amino acid substitutions. The variation may be a "conservative change" typically in the range of about 1 to 5 amino acids, wherein the substituted amino acid has similar structural or chemical properties, e.g., replacement of leucine with isoleucine or threonine with serine; replacement of lysine with arginine or histidine. In contrast, variations may include nonconservative changes, e.g., replacement of a glycine with a tryptophan. Similar minor variations may also include amino acid deletions or insertions or both.

Guidance in determining which and how many amino acid residues may be substituted, inserted, or deleted without changing biological or immunological activity may be found using computer programs known in the art, for example DNASTAR software (DNASTAR, Inc., Madison, Wis.). the

In another aspect, the invention features, isolated nucleic acids encoding the anti-pSYK antibody molecules of the invention. Nucleic acids may encode one or more of an antibody molecule, a heavy chain, a light chain, a light chain variable region, a heavy chain variable region, portions of the heavy chains and light chains of the antibody molecules described herein (e.g., a light chain variable region fragment which when paired with a full length heavy chain variable region is antigen binding, or a heavy chain variable region fragment which when paired with a full length light chain variable region is antigen binding), and CDRs. Examples include such nucleic acids disposed in vectors, e.g., expression vectors. Still further, the invention encompasses antibody molecules produced by host cells, e.g., expressing the antibody molecules encoded by such plasmids.

Disclosed herein is a vector, e.g., an expression vector, comprising one or both of: sequences encoding a light chain variable region, e.g., a light chain variable region described in Table 3, e.g., a sequence listed in Table 4, an antigen binding fragment thereof, or one, two or three CDRs from a light chain (and optionally a framework region), described herein, e.g., CDRs described in Table 5, e.g., a CDR encoding sequence in Table 6; and sequences encoding a heavy chain variable region, e.g., a heavy chain variable region described in Table 3, e.g., a sequence listed in Table 4, an antigen binding fragment thereof, or one, two or three CDRs from a heavy chain (and optionally a framework region), described herein, e.g., CDRs described in Table 5, e.g., a CDR encoding sequence in Table 6.

In certain embodiments provided, polynucleotides encode at least one heavy chain variable region or at least one light chain variable region of the antibodies of the present invention. In certain embodiments provided herein, polynucleotides may encode at least one heavy chain variable region and one light chain variable region of the antibodies of the present invention. In an embodiment the anti-pSYK antibody molecule comprises one or both of: (a) a light chain variable region, or an antigen binding fragment thereof, encoded by a nucleic acid that hybridizes under selected stringency conditions with, (i) the complement of an anti-pSYK antibody molecule-encoding-nucleic acid sequence described herein, e.g., in Table 4, or (ii) any nucleic acid sequence that encodes a light chain of an anti-pSYK antibody molecule the invention, e.g., one of the above-referenced rabbit antibodies summarized in Tables 1 and 2; and (b) a heavy chain variable region, or an antigen binding fragment thereof, encoded by a nucleic acid that hybridizes under selected stringency conditions with, (i) the complement of an anti-pSYK antibody molecule-encoding-nucleic acid sequence described herein, e.g., in Table 4, or (ii) any nucleic acid sequence that encodes a heavy chain of an anti-pSYK antibody molecule, e.g., one of the above-referenced rabbit antibodies summarized in Tables 1 and 2.

In an embodiment selected stringency conditions are high stringency or very high stringency conditions, e.g., as those conditions are described herein.

The present invention also provides isolated nucleic acid sequences that encode the anti-SYK antibody molecule of the present invention, host cells which are genetically engineered with vectors comprising isolated nucleic acid sequences of the present invention and the production of the antibodies of the present invention by recombinant techniques.

The appropriate DNA sequence may be inserted into the vector by a variety of procedures. In general, the DNA sequence is inserted into appropriate restriction endonuclease sites by procedures known in the art. The polynucleotide sequence in the expression vector is operatively linked to an appropriate expression control sequence (i.e. promoter) to direct mRNA synthesis. Examples of such promoters include, but are not limited to, the Rous sarcoma virus LTR or the early or late SV40 promoter, the E. coli lac or trp, the phage lambda PL promoter and other promoters known to control expression of genes in prokaryotic (e.g., tac, T3, T7 promoters for E. coli) or eukaryotic (e.g., cytomegalovirus promoter, adenovirus late promoter, EF-la promoter) cells or their viruses. The expression vector also contains a ribosome binding site for translation initiation and a transcription terminator. The vector may also include appropriate sequences for amplifying expression. For example, the vector may contain enhancers, which are transcription-stimulating DNA sequences of viral origin, such as those derived form simian virus such as SV40, polyoma virus, cytomegalovirus, bovine papilloma virus or Moloney sarcoma virus, or genomic, origin. The vector may also contain an origin of replication. The vector may be constructed to contain an exogenous origin of replication or, such an origin of replication may be derived from SV40 or another viral source, or by the host cell chromosomal replication mechanism. In addition, the vectors optionally contain a marker gene for selection of transfected host cells such as dihydrofolate reductase marker genes to permit selection with methotrexate in a variety of hosts, or antibiotics, such as β-lactamase gene (ampicillin resistance), Tet gene (for tetracycline resistance) used in prokaryotic cells or neomycin, GA418 (geneticin, a neomycin-derivative) gpt (mycophenolic acid), ampicillin, or hygromycin resistance genes, or genes which complement a genetic lesion of the host cells such as the absence of thymidine kinase, hypoxanthine phosphoribosyl transferase, dihydrofolate reductase, etc. Genes encoding the gene product of auxotrophic markers of the host (e.g., LEU2, URA3, HIS3) are often used as selectable markers in yeast.

In order to obtain the antibodies of the present invention, one or more polynucleotide sequences that encode for the light and heavy chain variable regions and light and heavy chain constant regions of the antibodies of the present invention should be incorporated into a vector. Polynucleotide sequences encoding the light and heavy chains of the antibodies of the present invention may be incorporated into one or multiple vectors and then incorporated into the host cells.

Suitable expression vectors for expression in mammalian cells include, for example, pCDM8, pcDNAl.l/amp, pcDNA3.1, pRc/RSV, pEF-1 (Invitrogen Life Technologies, Carlsbad, Calif.), pCMV-SCRIPT, pFB, pSG5, pXTI (Stratagene, La Jolla, Calif.), pCDEF3 (Goldman, L. A., et al., Biotechniques, 21: 1013-1015 (1996)), pSVSPORT (GIBCO division of Invitrogen Life Technologies, Carlsbad, Calif.), pEF-Bos (Mizushima, S., et al., Nucleic Acids Res., 18:5322 (1990)), Bicistronic GPEX® Retrovector (Gala Biotech, Middleton, Wis.), pTT5 (National Research Council, Ottawa, ON, Canada; U.S. Pat. Application Publication Nos. 20050170450 or 20100261275), and the like.

Expression vectors which are suitable for use in various expression hosts, such as prokaryotic cells (E. coli), insect cells (Drosophila Schnieder S2 cells, Sf9) and yeast (P. methanolica, P. pastoris, S. cerevisiae) are also available. Exemplary vectors are pLKTOK58 (wild type IgGl Fc sequence) and pLKTOK59 (mutated IgGl Fc sequence) (see U.S. Patent Application publication no. 20060147445).

As will be appreciated, anti-SYK antibody molecules in accordance with the present invention may be expressed in cell lines other than hybridoma cell lines. Sequences encoding the cDNAs or genomic clones for the particular antibodies may be used for a suitable mammalian or nonmammalian host cells. Transformation may be by any known method for introducing polynucleotides into a host cell, including, for example packaging the polynucleotide in a virus (or into a viral vector) and transducing a host cell with the virus (or vector) or by transfection procedures known in the art, for introducing heterologous polynucleotides into mammalian cells, e.g., dextran-mediated transfection, calcium phosphate precipitation, polybrene mediated transfection, protoplast fusion, electroporation, encapsulation of the polynucleotide(s) into liposomes and direct microinjection of the DNA molecule. The transformation procedure used depends upon the host to be transformed. Methods for introduction of heterologous polynucleotides into mammalian cells are known in the art and include, but are not limited to, dextran-mediated transfection, calcium phosphate precipitation, polybrene mediated transfection, protoplast fusion, electroporation, particle bombardment, encapsulation of the polynucleotide(s) in liposomes, peptide conjugates, dendrimers, and direct microinjection of the DNA into nuclei.

In another aspect, the invention features, a host cell comprising a nucleic acid described herein. In certain embodiments the cell expresses an antibody molecule, or component thereof, described herein. Still further embodiment provides a method of producing an antibody molecule, e.g., an anti-pSYK antibody molecule described herein, e.g. a rabbit antibody molecule, or a humanized version thereof, comprising maintaining the host cell under conditions appropriate for expression, whereby immunoglobulin chain(s) are expressed and an antibody molecule is produced. An additional embodiment provides a host cell comprising any of the foregoing expression vectors encoding heavy and light chain antibody sequences. The host cell may be a eukaryotic cell, e.g., a mammalian cell, an insect cell, a yeast cell, or a prokaryotic cell, e.g., E. coli. For example, the mammalian cell may be a cultured cell or a cell line. Exemplary mammalian cells include lymphocytic cell lines (e.g., NSO), Chinese hamster ovary cells (CHO), COS cells. In a particular embodiment, the cultured host cell is a CHO cell comprising nucleic acid sequences encoding a MIL81-1-8 antibody molecule. In another embodiment, the host cell is Hybridoma MIL81-1-8. Additionally cells include oocyte cells, and cells from a transgenic animal, e.g., mammary epithelial cell. For example, nucleic acids encoding an antibody molecule described herein may be expressed in a transgenic nonhuman animal.

Mammalian cell lines available as hosts for expression are known in the art and include many immortalized cell lines available from the American Type Culture Collection (ATCC, Manassas, VA), including but not limited to Chinese hamster ovary (CHO) cells, NSO cells, HeLa cells, baby hamster kidney (BHK) cells, monkey kidney cells (COS), human hepatocellular carcinoma cells (e.g., Hep G2), human embryonic kidney (HEK) 293 cells (e.g., 293-6E cells, see, e.g., U.S. Patent No. 8,551,774) and a number of other cell lines. Non-mammalian cells including but not limited to bacterial, yeast, insect, and plants may also be used to express recombinant antibodies. Site directed mutagenesis of the antibody CH2 domain to eliminate glycosylation may be used in order to prevent changes in either the immunogenicity, pharmacokinetic, and/or effector functions resulting from non-human glycosylation. The expression methods are selected by determining which system generates the highest expression levels and produce antibodies with constitutive pSYK binding properties.

A still further embodiment provides a method of producing an anti-pSYK antibody molecule, e.g., a rabbit antibody molecule or a humanized version thereof, comprising maintaining the host cell comprising nucleic acids described herein, e.g., one or more nucleic acid sequence listed in Table 4 or 6, under conditions appropriate for expression of an immunoglobulin, whereby immunoglobulin chains, are expressed and an antibody molecule, e.g., a rabbit antibody molecule, or a humanized version thereof, that binds pSYK, or a fragment or variant thereof, is produced. For example, methods of expression of antibody molecules include the use of host cells wherein a first recombinant nucleic acid molecule encoding an antibody molecule, e.g., a rabbit antibody light chain or a humanized version thereof, and a second recombinant nucleic acid molecule encoding an antibody molecule, e.g., a rabbit antibody heavy chain or a humanized version thereof, are comprised in a single expression vector. In other examples, they are in separate vectors. The method may further comprise the step of isolating or recovering the antibody, antigen-binding fragment of an antibody, antibody chain or antigen-binding fragment of an antibody chain, if desired.

For example, a nucleic acid molecule (i.e., one or more nucleic acid molecules) encoding the heavy and light chains of a rabbit (or humanized) antibody that binds a pSYK protein, or an expression construct (i.e., one or more constructs) comprising such nucleic acid molecule(s), may be introduced into a suitable host cell to create a recombinant host cell using any method appropriate to the host cell selected (e.g., transformation, transfection, electroporation, infection), such that the nucleic acid molecule(s) are operably linked to one or more expression control elements (e.g., in a vector, in a construct created by processes in the cell, integrated into the host cell genome). The resulting recombinant host cell may be maintained under conditions suitable for expression (e.g., in the presence of an inducer, in a suitable non-human animal, in suitable culture media supplemented with appropriate salts, growth factors, antibiotics, nutritional supplements, etc.), whereby the encoded polypeptide(s) are produced. If desired, the encoded protein may be isolated or recovered (e.g., from the animal, the host cell, medium, milk). This process encompasses expression in a host cell of a transgenic non-human animal (see, e.g., WO 92/03918, GenPharm International) or plant.

Further, expression of antibodies of the invention (or other moieties therefrom) from production cell lines may be enhanced using a number of known techniques. For example, the glutamine synthetase and DHFR gene expression systems are common approaches for enhancing expression under certain conditions. High expressing cell clones may be identified using conventional techniques, such as limited dilution cloning, Microdrop technology, or any other methods known in the art. The GS system is discussed in whole or part in connection with European Patent Nos. 0 216 846, 0 256 055, and 0 323 997 and European Patent Application No. 89303964.4.

In an exemplary system for recombinant expression of a modified antibody, or antigen-binding portion thereof a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to enhancer/promoter regulatory elements (e.g., derived from SV40, CMV, adenovirus and the like, such as a CMV enhancer/ AdMLP promoter regulatory element or an SV40 enhancer/ AdMLP promoter regulatory element) to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

Antibodies of the invention may also be produced transgenically through the generation of a mammal or plant that is transgenic for the immunoglobulin heavy and light chain sequences of interest and production of the antibody in a recoverable form therefrom. In connection with the transgenic production in mammals, antibodies may be produced in, and recovered from, the milk of goats, cows, or other mammals. See, e.g., U.S. Pat. Nos. 5,827,690, 5,756,687, 5,750,172, and 5,741,957.

The antibodies, antigen-binding fragments, antibody chains and antigen-binding portions thereof described herein also may be produced in a suitable in vitro expression system, by chemical synthesis or by any other suitable method.

Fusion Proteins and Immunoconjugates

The anti-pSYK antibodies described herein may be functionally linked by any suitable method (e.g., chemical coupling, genetic fusion, noncovalent association or otherwise) to one or more non-antibody molecular entities.

Fusion proteins may be produced in which an anti-pSYK antibody molecule as described herein and a non-antibody moiety are components of a single continuous polypeptide chain. The non-antibody moiety may be located N-terminally, C-terminally, or internally, with respect to the antibody moiety. For example, some embodiments may be produced by the insertion of a nucleic acid encoding immunoglobulin sequences into a suitable expression vector, such as a pET vector (e.g., pET-15b, Novagen), a phage vector (e.g., pCNATAB 5 E, Pharmacia), or other vector, e.g., pRIT2T Protein A fusion vector, Pharmacia). The resulting construct may be expressed to produce antibody chains that comprise a non-antibody moiety (e.g., Histidine tag, E tag, or Protein A IgG binding domain). Fusion proteins may be isolated or recovered using any suitable technique, such as chromatography using a suitable affinity matrix (see, e.g., Current Protocols in Molecular Biology (Ausubel, F. M et al., eds., Vol. 2, Suppl. 26, pp. 16.4.1-16.7.8 (1991)).

The invention provides anti-pSYK antibody molecules which are directed to and, in certain embodiments, are internalized into cells, e.g., permeabilized cells. They are capable of delivering therapeutic agents or detectable agents to or into cells expressing pSYK, but not to or into cells where the target is not expressed. Thus, the invention also provides anti-pSYK immunoconjugates comprising an anti-pSYK antibody molecule as described herein, which is conjugated to a therapeutic agent or a detectable agent. In certain embodiments, the affinity for pSYK of an anti-pSYK immunoconjugate is at least 10, 25, 50, 75, 80, 90, or 95% of that for the unconjugated antibody. This may be determined using cytoplasmic pSYK or isolated pSYK. In an embodiment the anti-pSYK antibody molecule, e.g., an immunoconjugate, has an LD50, as determined by an assay described herein, of less than 1,000, 500, 250, 100, or 50 pM.

The anti-pSYK antibody molecule may be modified to act as an immunoconjugate utilizing techniques that are known in the art. See e.g., Vitetta Immunol Today 14:252 (1993). See also U.S. Pat. No. 5,194,594. The preparation of radiolabeled antibodies may also be readily prepared utilizing techniques that are known in the art. See e.g., Junghans et al. in Cancer Chemotherapy and Biotherapy 655-686 (2nd edition, Chafner and Longo, eds., Lippincott Raven (1996)). See also U.S. Pat. Nos. 4,681,581, 4,735,210, 5,101,827, 5,102,990 (U.S. Re. Pat. No. 35,500), 5,648,471, and 5,697,902.

Anti-pSYK Antibody Sequences

Rabbit monoclonal anti-pSYK antibodies were generated as is discussed in more detail in the Examples. Briefly, rabbit monoclonal antibodies MIL81-1-8, MIL81-2-1 and MIL81-99-1 were generated by traditional immunization technology in rabbits. True rabbit-rabbit hybridomas were generated at Epitomics (Burlingame, CA) by fusing isolated B-cells from an immunized rabbit with Epitomics' fusion partner cell line (see U.S. Patent No. 7,429,487). Specificity of the antibodies against pSYK was tested by several methods, including ELISA, western blot, immunofluorescence, AlphaLISA, immunocytochemistry and immunohistochemistry.

Table 1 below summarizes the sequences of rabbit monoclonal anti-pSYK antibody MIL81-1-8, generated using an immunogen comprising a synthetic phosphopeptide corresponding to residues surrounding Tyr525/526 of human SYK and selected from screens comprising pSYK.

The sequences of the light and heavy chain variable regions were determined. Table 2 below is a summary of the SEQ ID NOs for the variable regions of several antibodies. The amino acid and nucleic acid sequences for the variable regions of each of the heavy and light chains for rabbit anti-pSYK antibodies are shown in Tables 3 and 4, respectively.

The amino acid and nucleic acid sequences for each of the CDRs of the heavy and light chains for anti-pSYK antibodies are shown in Tables 5 and 6, respectively.

Sequencing of the CDRs allowed determination of the abundance of residues that might serve as toxin conjugation sites. For example, an unpaired free cysteine in the antigen binding region could be a site for auristatin conjugation and a lysine could be a site for maytansine conjugation. Toxin conjugation to an amino acid of the CDR would raise the concern of altering the binding affinity of the antibody to pSYK. Thus, in certain embodiments the CDRs lack an amino acid which may be conjugated to a therapeutic agent.

Table 1: Summary of SEQ ID NOs for heavy and light chains of anti-pSYK rabbit mAb

| mAb | IgG Chain | Nucleic Acid SEQ ID NO | Amino Acid SEQ ID NO |
|---|---|---|---|
| MIL81-1-8 3H3 | Heavy | 3 | 4 |
| MIL81-1-8 3L2 | Light | 5 | 6 |

MIL81-1-8 3H3 Nucleic Acid (SEQ ID NO:3)

ATGGAGACTGGGCTGCGCTGGCTTCTCCTGGTCGCTGTGCTCAAAGGTGTCCAGTGTCAGTC
GGTGGAGGAGTCCGGGGGTCGCCTGGTCACGCCTGGGACACCCCTGACACTCACCTGCACAG
TCTCTGGAATCGACCTCAATAGTTATATAATGAGTTGGGTCCGCCAGGCTCCAGGGAAGGGG
CTGGAATGGATCGGAATCATTAGTCGTCGTGGTAACACATACTACGCGAGCTGGCCGAAAGG
CCGATTCACCATCTCCAAAACCTCGACCACGGTGGATCTGAAAATCACCAGTCCGACAACCG
AGGACACGGCCACCTATTTCTGTGCCAGAGCATATCTTTATACTAGTGGTACGATGAGCGTC
TGGGGCCCAGGCACCCTGGTCACCGTCTCCTCAGGGCAACCTAAGGCTCCATCAGTCTTCCC
ACTGGCCCCCTGCTGCGGGGACACACCCAGCTCCACGGTGACCCTGGGCTGCCTGGTCAAAG
GGTACCTCCCGGAGCCAGTGACCGTGACCTGGAACTCGGGCACCCTCACCAATGGGGTACGC
ACCTTCCCGTCCGTCCGGCAGTCCTCAGGCCTCTACTCGCTGAGCAGCGTGGTGAGCGTGAC
CTCAAGCAGCCAGCCCGTCACCTGCAACGTGGCCCACCCAGCCACCAACACCAAAGTGGACA
AGACCGTTGCGCCCTCGACATGCAGCAAGCCCACGTGCCCACCCCCTGAACTCCTGGGGGGA
CCGTCTGTCTTCATCTTCCCCCCAAAACCCAAGGACACCCTCATGATCTCACGCACCCCCGA
GGTCACATGCGTGGTGGTGGACGTGAGCCAGGATGACCCCGAGGTGCAGTTCACATGGTACA
TAAACAACGAGCAGGTGCGCACCGCCCGGCCGCCGCTACGGGAGCAGCAGTTCAACAGCACG

ATCCGCGTGGTCAGCACCCTCCCCATCGCGCACCAGGACTGGCTGAGGGGCAAGGAGTTCAA
GTGCAAAGTCCACAACAAGGCACTCCCGGCCCCCATCGAGAAAACCATCTCCAAAGCCAGAG
GGCAGCCCCTGGAGCCGAAGGTCTACACCATGGGCCCTCCCCGGGAGGAGCTGAGCAGCAGG
TCGGTCAGCCTGACCTGCATGATCAACGGCTTCTACCCTTCCGACATCTCGGTGGAGTGGGA
GAAGAACGGGAAGGCAGAGGACAACTACAAGACCACGCCGGCCGTGCTGGACAGCGACGGCT
CCTACTTCCTCTACAGCAAGCTCTCAGTGCCCACGAGTGAGTGGCAGCGGGGCGACGTCTTC
ACCTGCTCCGTGATGCACGAGGCCTTGCACAACCACTACACGCAGAAGTCCATCTCCCGCTC
TCCGGGTAAATGA

MIL81-1-8 3H3 Amino Acid (SEQ ID NO:4)

M E T G L R W L L L V A V L K G V Q C Q
S V E E S G G R L V T P G T P L T L T C
T V S G I D L N S Y I M S W V R Q A P G
K G L E W I G I I S R R G N T Y Y A S W
P K G R F T I S K T S T T V D L K I T S
P T T E D T A T Y F C A R A Y L Y T S G
T M S V W G P G T L V T V S S G Q P K A
P S V F P L A P C C G D T P S S T V T L
G C L V K G Y L P E P V T V T W N S G T
L T N G V R T F P S V R Q S S G L Y S L
S S V V S V T S S S Q P V T C N V A H P
A T N T K V D K T V A P S T C S K P T C
P P P E L L G G P S V F I F P P K P K D
T L M I S R T P E V T C V V V D V S Q D
D P E V Q F T W Y I N N E Q V R T A R P
P L R E Q Q F N S T I R V V S T L P I A
H Q D W L R G K E F K C K V H N K A L P
A P I E K T I S K A R G Q P L E P K V Y
T M G P P R E E L S S R S V S L T C M I
N G F Y P S D I S V E W E K N G K A E D
N Y K T T P A V L D S D G S Y F L Y S K
L S V P T S E W Q R G D V F T C S V M H
E A L H N H Y T Q K S I S R S P G K

MIL81-1-8 3L2 Nucleic Acid (SEQ ID NO:5)

ATGGACACGAGGGCCCCCACTCAGCTGCTGGGGCTCCTGCTGCTCTGGCTCCCAGGTGCCAG
ATGTGCTGACATTGTGATGACCCAGACTCCATCCCCCGTGGAGGCAGCTGTGGGAGGCACAG
TCACCATCAAGTGCCAGGCCAGTGAGAGCATTAGTAGTTACTTATCCTGGTATCAGCAGAAA
CCAGGGCAGCCTCCCAAACTCCTGATCTACAGGGCATCCACTCTGGTATCTGGGGTCCCATC

GCGGTTCAAAGGCAGTGGATCTGGGACAGATTTCACTCTCACCATCAGCGACCTGGAGTGTG
CCGATGCTGCCACTTACTATTGTCAACATACTTATTTTGGTAGTGATTATGTTGGTGGTTTC
GGCGGAGGGACCGAGGTGGTGGTCAAAGGTGATCCAGTTGCACCTACTGTCCTCATCTTCCC
ACCAGCTGCTGATCAGGTGGCAACTGGAACAGTCACCATCGTGTGTGTGGCGAATAAATACT
TTCCCGATGTCACCGTCACCTGGGAGGTGGATGGCACCACCCAAACAACTGGCATCGAGAAC
AGTAAAACACCGCAGAATTCTGCAGATTGTACCTACAACCTCAGCAGCACTCTGACACTGAC
CAGCACACAGTACAACAGCCACAAAGAGTACACCTGCAAGGTGACCCAGGGCACGACCTCAG
TCGTCCAGAGCTTCAATAGGGGTGACTGTTAG

MIL81-1-8 3L2 Amino Acid (SEQ ID NO:6)

M D T R A P T Q L L G L L L L W L P G A
R C A D I V M T Q T P S P V E A A V G G
T V T I K C Q A S E S I S S Y L S W Y Q
Q K P G Q P P K L L I Y R A S T L V S G
V P S R F K G S G S G T D F T L T I S D
L E C A D A A T Y Y C Q H T Y F G S D Y
V G G F G G G T E V V V K G D P V A P T
V L I F P P A A D Q V A T G T V T I V C
V A N K Y F P D V T V T W E V D G T T Q
T T G I E N S K T P Q N S A D C T Y N L
S S T L T L T S T Q Y N S H K E Y T C K
V T Q G T T S V V Q S F N R G D C

Table 2: Summary of SEQ ID NOs for variable regions of anti-pSYK rabbit mAb

| mAb | IgG Chain | Nucleic Acid SEQ ID NO | Amino Acid SEQ ID NO |
|---|---|---|---|
| MIL81-1-8 3H3 | Heavy | 7 | 8 |
| MIL81-1-8 3L2 | Light | 9 | 10 |

Table 3: Amino Acid Sequences of mAb variable regions of anti-pSYK rabbit mAb

| mAb | IgG Chain | SEQ ID NO | Amino Acid Sequence |
|---|---|---|---|
| MIL81-1-8 3H3 | Heavy | 8 | QCQSVEESGGRLVTPGTPLTLTCTVSGIDLNSYI MSWVRQAPGKGLEWIGIISRRGNTYYASWPKGRF TISKTSTTVDLKITSPTTEDTATYFCARAYLYTS GTMSVWGPGTLVTVSSGQ |
| MIL81-1-8 3L2 | Light | 10 | ADIVMTQTPSPVEAAVGGTVTIKCQASESISSYL SWYQQKPGQPPKLLIYRASTLVSGVPSRFKGSGS GTDFTLTISDLECADAATYYCQHTYFGSDYVGGF GGGTEVVVKGD |

Table 4: Nucleic Acid Sequences encoding mAb variable regions of anti-pSYK rabbit mAb

| mAb | IgG Chain | SEQ ID NO | Nucleic Acid Sequence |
|---|---|---|---|
| MIL81-1-8 3H3 | Heavy | 7 | cagtgtcagtcggtggaggagtccgggggtcgcc tggtcacgcctgggacacccctgacactcacctg cacagtctctggaatcgacctcaatagttatata atgagttgggtccgccaggctccagggaaggggc tggaatggatcggaatcattagtcgtcgtggtaa cacatactacgcgagctggccgaaaggccgattc accatctccaaaacctcgaccacggtggatctga aaatcaccagtccgacaaccgaggacacggccac ctatttctgtgccagagcatatctttatactagt ggtacgatgagcgtctggggcccaggcaccctgg tcaccgtctcctcagggcaa |
| MIL81-1-8 3L2 | Light | 9 | gctgacattgtgatgacccagactccatcccccg tggaggcagctgtgggaggcacagtcaccatcaa gtgccaggccagtgagagcattagtagttactta tcctggtatcagcagaaaccagggcagcctccca aactcctgatctacagggcatccactctggtatc tggggtcccatcgcggttcaaaggcagtggatct gggacagatttcactctcaccatcagcgacctgg agtgtgccgatgctgccacttactattgtcaaca tacttattttggtagtgattatgttggtggtttc ggcggagggaccgaggtggtggtcaaaggtgat |

Table 5: Amino Acid Sequences of CDRs of anti-pSYK rabbit mAb

| mAb | IgG Chain | SEQ ID NO | Amino Acid Sequence |
|---|---|---|---|
| MIL81-1-8 3H3 | VH CDR1 | 11 | IDLNSYIMS |
| MIL81-1-8 3H3 | VH CDR2 | 12 | IISRRGNTYYASWPKG |
| MIL81-1-8 3H3 | VH CDR3 | 13 | AYLYTSGTMSV |
| MIL81-1-8 3L2 | VK CDR1 | 14 | QASESISSYLS |
| MIL81-1-8 3L2 | VK CDR2 | 15 | RASTLVS |
| MIL81-1-8 3L2 | VK CDR3 | 16 | QHTYFGSDYVGG |

Table 6: Nucleic Acid Sequences of CDRs of anti-pSYK rabbit mAb

| mAb | IgG Chain | SEQ ID NO | Nucleic Acid Sequence |
|---|---|---|---|
| MIL81-1-8 3H3 | VH CDR1 | 17 | atcgacctcaatagttatataatgagt |
| MIL81-1-8 3H3 | VH CDR2 | 18 | atcattagtcgtcgtggtaacacatactacgcga gctggccgaaaggc |
| MIL81-1-8 3H3 | VH CDR3 | 19 | gcatatctttatactagtggtacgatgagcgtc |

(continued)

| mAb | IgG Chain | SEQ ID NO | Nucleic Acid Sequence |
|---|---|---|---|
| MIL81-1-8 3L2 | VK CDR1 | 20 | caggccagtgagagcattagtagttacttatcc |
| MIL81-1-8 3L2 | VK CDR2 | 21 | agggcatccactctggtatct |
| MIL81-1-8 3L2 | VK CDR3 | 22 | caacatacttattttggtagtgattatgttggtg gt |

Antibody Labeling and Detection

Anti-pSYK antibody molecules used in methods described herein, e.g., in the in vitro and in vivo detection, e.g., diagnostic, staging, or imaging methods, may be directly or indirectly labeled with a detectable agent to facilitate detection of the bound or unbound binding agent. Indirect labeling includes contacting with a secondary antibody, such as an anti-rabbit antibody, which binds the anti-pSYK antibody, e.g., the Fc portion of the antibody. The secondary antibody may be conjugated to a detectable agent or to a moiety which binds to a label or enzymatically converts a substance into a detectable substance. Suitable detectable agents include various biologically active enzymes, ligands, prosthetic groups, fluorescent materials, luminescent materials, chemiluminescent materials, bioluminescent materials, chromophoric materials, electron dense materials, paramagnetic (e.g., nuclear magnetic resonance active) materials, and radioactive materials.

In some embodiments, the anti-pSYK antibody molecule is coupled to a radioactive ion, e.g., indium ($^{111}In$), iodine ($^{131}I$ or $^{125}I$), yttrium ($^{90}Y$), lutetium ($^{177}Lu$), actinium ($^{225}Ac$), bismuth ($^{212}Bi$ or $^{213}Bi$), sulfur ($^{35}S$), carbon ($^{14}C$), tritium ($^{3}H$), rhodium ($^{188}Rh$), technetium ($^{99}$ mTc), praseodymium, or phosphorous ($^{32}P$); or a positron-emitting radionuclide, e.g., carbon-11 ($^{11}C$), potassium-40 ($^{40}K$), nitrogen-13 ($^{13}N$), oxygen-15 ($^{15}O$), fluorine-18 ($^{18}F$), gallium ($^{68}Ga$), and iodine-121 ($^{121}I$). Additional radioactive agents that may be conjugated to the antibodies of the invention for use in in vitro or in vivo diagnostic/detection methods are described below.

Exemplary labels include fluorophores such as rare earth chelates, such as comprising europium, samarium or terbium, or fluorescein and its derivatives, rhodamine and its derivatives, dansyl, umbelliferone, luceriferases, e.g., firefly luciferase and bacterial luciferase (U.S. Pat. No. 4,737,456), luciferin, cyanine (Cy) fluorescent dyes such as Cy3, or Cy5), Alexa Fluor 488, Alexa Fluor 592, Oregon green and 2,3-dihydrophthalazinediones. Other exemplary labels for direct or indirect detection of an anti-pSYK antibody include moieties which enzymatically convert a substance into a detectable substance, such as horseradish peroxidase (HRP), alkaline phosphatase, galactosidase, glucoamylase, lysozyme, saccharide oxidases, e.g., glucose oxidase, galactose oxidase, and glucose 6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, moieties which bind to a detectable substance, such as biotin which can bind to an avidin-conjugated label, spin labels, bacteriophage labels, stable free radicals, and the like.

Fluorophore and chromophore labeled antibody molecules may be prepared from standard moieties known in the art. Since antibodies and other proteins absorb light having wavelengths up to about 310 nm, the fluorescent moieties should be selected to have substantial absorption at wavelengths above 310 nm or above 400 nm. A variety of suitable fluorescent compounds and chromophores are described by Stryer Science, 162:526 (1968) and Brand, L. et al. Annual Review of Biochemistry, 41 : 843-868 (1972). The antibodies may be labeled with fluorescent chromophore groups by conventional procedures such as those disclosed in U.S. Pat. Nos. 3,940,475, 4,289,747, and 4,376,110.

One group of fluorescers having a number of the desirable properties described above is the xanthene dyes, which include the fluoresceins derived from 3,6-dihydroxy-9-henylxanthhydrol and resamines and rhodamines derived from 3,6-diamino-9-phenylxanthydrol and lissanime rhodamine B. The rhodamine and fluorescein derivatives of 9-o-carboxyphenylxanthhydrol have a 9-o-carboxyphenyl group. Fluorescein compounds having reactive coupling groups such as amino and isothiocyanate groups such as fluorescein isothiocyanate and fluorescamine are readily available. Another group of fluorescent compounds are the naphthylamines, having an amino group in the $\alpha$ or $\beta$ position.

Labeled antibody molecules may be used, for example, diagnostically and/or experimentally in a number of contexts, including (i) to isolate a predetermined antigen by standard techniques, such as affinity chromatography or immunoprecipitation; (ii) to detect a predetermined antigen (e.g., in a cellular lysate, tissue specimen or cell supernatant) in order to evaluate the abundance and pattern of expression of the protein; (iii) to monitor protein levels in tissue as part of a clinical testing procedure, e.g., to determine the efficacy of a given treatment regimen.

An assay for detecting pSYK may include a primary antibody enhancer for diluting the pSYK antibody in preparation for a detection assay. One skilled in the art will recognize that the primary antibody enhancer may be an anti-rabbit secondary antibody raised in a species other than rabbit (e.g., human, rat, goat, mouse, etc.) having the same isotype as the MIL81-1-8 rabbit mAb (rabbit IgG) or a similar reagent that is suitable to amplify the MIL81-1-8 signal. Several enhancers are commercially available (e.g., Pierce Biotechnology, Inc). An assay amplification system, such as Tyramide Signal Amplification (TSA) system, can be used to increase signal intensity and reduce background in both immunohistochemistry and immunofluorescent staining techniques. TSA systems are commercially available (e.g. Life Technologies, Grand Island, NY or PerkinElmer Co., Waltham, MA, Ventana Medical Systems, Tucson, AZ). The TSA signal can be further amplified by a further secondary detection of the TSA (using a haptenized electron donor), such as in the AMP HQ method (Ventana Medical Systems, Tucson, AZ).

In Vitro Diagnostics

The anti-pSYK antibodies and immunoconjugates described herein may be used to detect the presence or absence of pSYK, e.g., to detect the presence or absence of pSYK in an ex vivo biological sample obtained from a subject (i.e., in vitro detection), or to detect the presence or distribution or absence of pSYK in a subject (i.e., in vivo detection). Such detection methods are useful to detect or diagnose a variety of disorders, or to guide therapeutic or economic decisions or actions. The term "detecting" as used herein encompasses quantitative or qualitative detection. Detecting pSYK or SYK phosphoprotein, as used herein, means detecting intact pSYK protein or detecting a portion of the pSYK protein that comprises the epitope to which the anti-pSYK antibody molecule binds.

Accordingly, in another aspect, the invention comprises a method of detecting pSYK expression in a biological sample such as a cell or tissue, e.g., a tumor cell, or a tumor having one or more cells that express pSYK. The method comprises: contacting a biological sample with an anti-pSYK antibody molecule of the invention (e.g., MIL81-1-8), under conditions which allow formation of a complex between the anti-pSYK antibody molecule and pSYK protein; and detecting formation of a complex between the anti-pSYK antibody molecule and pSYK protein, to thereby detect the presence of pSYK protein, e.g., to detect a pSYK expressing cell or tumor.

In an embodiment, the anti-pSYK antibody molecule is an immunoconjugate comprising a detectable label. The detectable label may be a radioactive agent. Alternatively, the detectable label is a non-radioactive agent (e.g. a fluorophore or a chromophore as described above).

A sample for use in an assay to measure pSYK, such as the pSYK Y525/526 IHC assay can be a biological sample comprising cells obtained from a patient afflicted with cancer. In certain embodiments, the biological sample includes normal and/or cancerous cells or tissues that express pSYK. In particular embodiments, the normal and/or cancerous cells or tissues may express pSYK at higher levels relative to other cells or tissues. A sample can contain tumor cells or normal cells from the tissue or organ of cancer origin or a mixture of tumor cells and normal cells. A sample can be from a vicinity of a tumor tissue, such as a lymph node or from a distant tissue, such as liver or bone, to which the cancer may spread by metastasis. In some embodiments, the cancerous cells are leukemia cells, e.g., cells from AML or CLL, lymphoma cells, e.g., cells from DLBCL or FL, or solid tumor cells, e.g., cells from nasopharyngeal carcinoma or gastric carcinoma. In certain embodiments, the cancerous cells are selected from PTCL, DLBCL, FL, MCL, CLL, AML, MDS, nasopharyngeal carcinoma, lymphoma, gastric carcinoma, breast cancer, ovarian cancer, lung cancer (e.g., small cell lung cancer) and PT-LPD cells. In certain embodiments, the cancerous cells are selected from DLBCL, AML, and CLL cells. In certain embodiments, the DLBCL cells are selected from the group consisting of GCB DLBCL cells, ABC DLBCL cells and non-GCB DLBCL cells.

Methods of detection described herein, whether in vitro or in vivo, may be used to evaluate a disorder in a subject characterized by one or more pSYK-expressing cells. In certain embodiments, the disorder is a cell proliferative disorder, such as a cancer, tumor, lymphoma or leukemia. In certain embodiments, the cell proliferative disorder is selected from PTCL, DLBCL, FL, MCL, CLL, AML, MDS, nasopharyngeal carcinoma, lymphoma, gastric carcinoma, breast cancer, ovarian cancer, lung cancer (e.g., small cell lung cancer) and PT-LPD. In some embodiments, a cell proliferative disorder is AML, CLL or DLBCL. In some embodiments, the DLBCL disorder is classified by subtype. In some embodiments, the DLBCL subtype is GCB subtype. In some embodiments, the DLBCL subtype is the ABC subtype. In some embodiments, the DLBCL subtype is the non-GCB subtype.

In one aspect, the invention comprises a method for detecting the presence or absence of pSYK protein in a biological sample in vitro (e.g., in a cell or tissue biopsy obtained from a subject). The method comprises: (i) contacting a biological sample obtained from a subject with an anti-pSYK antibody molecule or immunoconjugate thereof and (ii) detecting formation of a complex between the anti-pSYK antibody molecule and pSYK protein. Complex formation is indicative of the presence or level of pSYK protein in the biological sample, whereas no complex formation is indicative of the absence of pSYK protein in the biological sample.

Tissue samples to assay for pSYK, such as in a pSYK Y525/526 IHC assay, can be obtained from a patient who is a candidate for treatment as described herein or from a commercial source. Commercial sources of tissue samples, such as tumor samples or control samples, include PhenoPath Laboratories, PLLC, Seattle, WA; Proteogenix SAS, Obershausbergen, France; and U.S. Biomax, Inc., Rockville, MD.

Exemplary biological samples for methods, including both in vitro and in vivo, e.g., companion diagnostic methods for SYK-targeting therapy, as described herein comprise a cell, cells, tissue or body fluid, and isolates thereof, such as an inflammatory exudate, blood, serum, plasma, urine, sputum, bone marrow aspirate, nipple aspirate, bowel fluid, stool sample or material obtained by swabbing mucosal tissues, such as of the mouth, throat or cervix. In particular embodiments, the biological sample comprises a cancerous cell(s) or tissue. For example, the sample may be a tumor biopsy, such as a lymph node biopsy, or from a tissue sample from any metastatic site thereof. In another embodiment, the sample is a blood sample or derived from a blood sample. In some embodiments, the blood sample comprises tumor cells released from a tumor, e.g., from lymphocytosis. In other embodiments, the biological sample may be blood or another fluid, where the fluid comprises a cancer cell. A biological sample may be obtained using any of a number of methods in the art. Further, a biological sample may be treated with a fixative such as formaldehyde and embedded in paraffin and sectioned for use. Alternatively, fresh or frozen tissue may be employed. In other embodiments, fine-needle aspirates may be used. In certain embodiments, the biological sample is a cell or a tissue biopsy. In certain embodiments, the cell or tissue biopsy is a tumor biopsy. In some embodiments, the tumor biopsy is a needle biopsy.

In hematological tumors of the bone marrow, e.g., myeloma tumors or leukemias, primary analysis of the tumor can be performed on bone marrow samples, e.g., samples which comprise myeloma tumor or leukemia tumor cells. However, some tumor cells, (e.g., clonotypic tumor cells, circulating endothelial cells), are a percentage of the cell population in whole blood. These cells also can be mobilized into the blood during treatment of the patient with granulocyte-colony stimulating factor (G-CSF) in preparation for a bone marrow transplant, a standard treatment for hematological tumors, e.g., leukemias, lymphomas and myelomas. These cells also can be mobilized from chemotherapy treatment, e.g., treatment by a BTK inhibitor, such as ibrutinib, treatment by a SYK inhibitor, such as fostamatinib or treatment by a PI3K$\delta$ inhibitor, such as idelalisib. Examples of circulating tumor cells in multiple myeloma have been studied e.g., by Pilarski et al. (2000) Blood 95:1056-65 and Rigolin et al. (2006) Blood 107:2531-5. Thus, noninvasive samples, e.g., for *in vitro* measurement of markers to determine outcome of treatment, can include peripheral blood samples. Accordingly, cells within peripheral blood can be tested to determine the amount of pSYK.

Blood collection containers can comprise an anti-coagulant, e.g., heparin or ethylenediaminetetraacetic acid (EDTA), sodium citrate or citrate solutions with additives to preserve blood integrity, such as dextrose or albumin or buffers, e.g., phosphate or Ringer's. A protein stabilizer, e.g., an agent that inhibits proteases, such as serine proteases, cysteine proteases, etc. can be added to the sample. Several protease inhibitors, such as cocktails of inhibitors for more than one protease are readily available. If the amount of marker is being measured by measuring the level of its DNA in the sample, a DNA stabilizer, e.g., an agent that inhibits DNAse, such as a DNAse I inhibitor, can be added to the sample. If the amount of marker is being measured by measuring the level of its RNA in the sample, an RNA stabilizer, e.g., an agent that inhibits RNAse, such as an inhibitor or RNAse A, RNAse B, or RNAse C, can be added to the sample. Several DNAse or RNAse inhibitors, such as cocktails of inhibitors for more than one DNAse or RNAse are readily available. Examples of blood collection containers comprising a stabilizer are PAXGENE® tubes (PREANALYTIX, Valencia, CA), useful for RNA stabilization upon blood collection, and CELLSAVE Preservation tubes (Janssen Diagnostics, LLC), useful for the stabilization of circulating tumor cells upon blood collection. Peripheral blood samples or tumor exudates can be modified, e.g., fractionated, sorted or concentrated (e.g., to result in samples enriched with tumor or depleted of tumor (e.g., for a reference sample)). Examples of modified samples include clonotypic myeloma cells, which can be collected by e.g., negative selection, e.g., separation of white blood cells from red blood cells (e.g., differential centrifugation through a dense sugar or polymer solution (e.g., FICOLL® solution (Amersham Biosciences division of GE healthcare, Piscataway, NJ) or HISTOPAQUE®-1077 solution, Sigma-Aldrich Biotechnology LP and Sigma-Aldrich Co., St. Louis, MO)) and/or positive selection by binding cells to a selection agent (e.g., a reagent which binds to a tumor cell marker, a B-cell marker or myeloid progenitor marker, such as CD5, CD19, CD20, CD34, CD38, CD117, CD138, CD133, or ZAP70 for direct isolation (e.g., the application of a magnetic field to solutions of cells comprising magnetic beads (e.g., from Miltenyi Biotec, Auburn, CA) which bind to the B cell markers) or fluorescent-activated cell sorting). In one embodiment, the differential centrifugation concentrates a cell layer comprising tumor cells. The tumor cell layer can be isolated and fixed for pSYK detection. Non-myeloma samples, e.g., tumor exudates from solid tumors, can be treated by similar methods as myeloma samples to enrich for tumor cells, e.g., using tumor cell selection markers known in the art. In some embodiments, selection by binding to a marker selected from CD5, CD19 and CD20 can enrich a tumor cell in a sample from a cancer patient, such as a lymphoma patient, e.g., a CML cancer patient. In some embodiments, selection by binding to a marker selected from CD34 and CD117 can enrich a tumor cell in a sample from a cancer patient, e.g., a myeloid tumor patient, e.g., an AML cancer patient.

The sample, e.g., tumor, e.g., biopsy or bone marrow, blood or modified blood, (e.g., a sample comprising tumor cells), tumor exudate and/or the reference, e.g., matched control (e.g., germline or nontumor), sample can be subjected to a variety of well-known post-collection preparative and storage techniques (e.g., lysis, nucleic acid and/or protein extraction, or isolation, fixation, storage, freezing, ultrafiltration, concentration, evaporation, centrifugation, *etc.*) prior to detecting or assessing the amount of pSYK in the sample.

In certain embodiments, a test cell or tissue is obtained from an individual suspected of having a disorder associated with pSYK expression. In certain embodiments, a test cell or tissue is obtained from an individual suspected of having a disorder associated with increased expression of pSYK.

In an embodiment, the level of pSYK, in a sample from the subject, or in the subject, is compared with a reference level, e.g., the level of pSYK in a control material, e.g., a normal cell of the same tissue origin as the subject's cell or a cell having pSYK at levels comparable to such a normal cell. For patients with hematological tumors, a control, reference sample for normal characteristic, e.g., unphosphorylated SYK or SYK phosphorylated at a residue that is not Y525 or Y526, can be obtained from skin or a buccal swab of the patient. For solid tumors, a typical sample comprising tumor cells is a biopsy of the primary tumor or neighboring lymph nodes. Solid tumor samples obtained by less invasive means e.g., shed or scraped from the tumor site include a cervical smear (e.g., from a cervical cancer patient), tumor exudate, e.g., lymph fluid, cystic fluid, nipple aspirate (e.g., from a breast cancer patient), ascites fluid, pleural fluid, sputum (e.g., from lung cancer patient), gynecological fluids (e.g., from an ovarian cancer patient), urine, stool (e.g., for colon cancer). For solid tumors, a control, reference sample for normal SYK expression can be obtained from blood of the patient, a skin sample, or a buccal swab from the patient.

The method may comprise, e.g., providing a diagnosis, a prognosis, an evaluation of the efficacy of treatment, or the staging of a disorder in response to the detected level of pSYK. A higher level of pSYK in the sample or subject, as compared to the control material, indicates the presence of a disorder associated with increased expression of pSYK. A higher level of pSYK in the sample or subject, e.g., 1.2 times, 1.5 times, 2.0 times, 3 times, 4 times, 5 times or 10 times or more times the level, as compared to the control material, may also indicate the relative lack of efficacy of a treatment, a relatively poorer prognosis, or a later stage of disease. The level of pSYK may also be used to evaluate or select future treatment, e.g., the need for more or less aggressive treatment, or the need to switch from one treatment regimen to another. In some embodiments, the methods further comprise selecting a SYK-targeted therapy, e.g., a SYK-targeted therapy described herein, based, at least in part, on the determined pSYK levels, and optionally administering the selected SYK-targeted therapy to the subject.

Complex formation between the anti-pSYK antibody molecule, such as a small molecule inhibitor of SYK and pSYK may be detected by measuring or visualizing either the antibody (or antibody fragment) bound to the pSYK antigen or unbound antibody molecule. One having ordinary skill in the art can readily appreciate the multitude of ways to detect binding of anti-pSYK antibodies to pSYK. Some methods are included in the Examples described herein. Such methods include, but are not limited to, antigen-binding assays that are known in the art, such as western blots, radioimmunoassays (RIA), ELISA (enzyme linked immunosorbent assay), or variations thereof, such as ALPHALISA® immunoassay (PerkinElmer, Waltham, MA), "sandwich" Immunoassays or variations thereof, such as DELFIA® immunodiagnostics system (PerkinElmer, Waltham MA), immunoprecipitation assays, fluorescent immunoassays, protein A immunoassays, flow cytometry, and immunohistochemistry (IHC). In certain embodiments, the method of pSYK detection is selected from flow cytometry, IHC, and fluorescence activated cell sorting (FACS). In certain embodiments, the method of pSYK detection is selected from flow cytometry and IHC. In certain embodiments, the method of pSYK detection is flow cytometry. In certain embodiments, the method of pSYK detection is IHC. In some embodiments, the IHC method uses signal enhancement or amplification, such as tyramide signal amplification, horseradish peroxidase multimer amplification, such as AMP HQ system (Ventana Medical Systems, Tucson, AZ) or tyramide signal amplification with AMP HQ amplification.

In a particular embodiment, pSYK is detected or measured by immunohistochemistry using an anti-pSYK antibody of the invention. Immunohistochemistry techniques may be used to identify and essentially stain cells that express pSYK. Such "staining" also allows for analysis of metastatic migration. Anti-pSYK antibodies such as those described herein are contacted with fixed cells and the pSYK present in the cells reacts with the antibodies. The antibodies are detectably labeled or detected indirectly using labeled second antibody or protein A to stain the cells. In one particular embodiment, the MIL81-1-8 antibody is used in an IHC assay to detect or measure pSYK expression in a biological sample.

In antigen binding assays, some variation can be due to possible variation in sample preparation methods and sample quality. Since the method contemplates the use of archival samples, some degradation may occur prior to the assay. A balance can be struck between detecting pSYK, e.g., pSYK Y525/526 and keeping a high signal relative to background staining by several art-known immunochemistry, such as IHC, techniques. For example, the practitioner optionally may undertake pre-assay choices and/or IHC assay choices. Examples of pre-assay choices include choice of antibody, the step of eliminating cross-reaction, e.g., by preadsorbtion of the antibody preparation against a protein whose detection is not desired, such as normal sample, e.g., normal human tissue or inactive SYK, choice of label or development reagent, and the method of fixation of the biological sample. Examples of IHC assay choices include method or choice of reagent for blocking non-specific binding, whether to heat the slide (e.g., 72°C or 95°C) to open the conformation of the fixed structures prior to antibody binding and the method or choice of reagent for washing tissue sections, e.g., washing with a solution comprising a nonionic detergent or surfactant, such as polysorbate, such as TWEEN 20. Optimization of conditions for IHC assays is well known in the art, with techniques guides available, for example Buchwalow and Böcker, Immunohistochemistry Basics and Methods (2010, Springer-Verlag, Berlin, Germany). IHC can be performed by automated machinery, such as Ventana BENCHMARK™ slide staining platform or Ventana Discovery XT (Ventana Medical Systems, Inc., Tuscon, AZ) or Leica BOND RX (Leica Biosystems, Buffalo Grove, IL or suitable diagnostic autostaining platform). The automated IHC assay is a useful tool for screening cancer patients for pSYK expressing tumors as a clinical trial enrollment criterion for a SYK-targeted cancer therapeutic, and generally as a screening tool for selecting patients (e.g., cancer patients) who should receive a SYK-targeted therapy.

In some embodiments, a pSYK antigen binding assay comprises treating the sample with a protein blocking reagent to reduce binding to background structures, e.g., proteins or structures which do not comprise pSYK Y525/526. Examples include rabbit IgG, such as IgG2, BSA, casein, HiBlock (Perkin Elmer, Waltham, MA) or protein block (Dako, Carpinteria, CA). In some embodiments, the sample is treated with peroxide to block endogenous tissue peroxidases, e.g., when using a peroxidase development method. In other embodiments, an alkaline phosphatase blocking reagent, such as EGTA, glycerophosphate or phenanthroline can inhibit dephosphorylation of pSYK. In some embodiments, the sample undergoes decalcification, such as with EDTA.

It is also possible to directly detect pSYK to anti-pSYK antibody molecule complex formation without further manipulation or labeling of either component (pSYK or antibody molecule), for example by utilizing the technique of fluorescence energy transfer (FET, see, for example, Lakowicz et al., U.S. Pat. No. 5,631,169; Stavrianopoulos, et al., U.S. Pat. No. 4,868,103). A fluorophore label on the first, "donor" molecule is selected such that, upon excitation with incident light of appropriate wavelength, its emitted fluorescent energy will be absorbed by a fluorescent label on a second "acceptor" molecule, which in turn is able to fluoresce due to the absorbed energy. Alternately, the "donor" protein molecule may simply utilize the natural fluorescent energy of tryptophan residues. Labels are chosen that emit different wavelengths of light, such that the "acceptor" molecule label may be differentiated from that of the "donor". Since the efficiency of energy transfer between the labels is related to the distance separating the molecules, spatial relationships between the molecules may be assessed. In a situation in which binding occurs between the molecules, the fluorescent emission of the ""acceptor" molecule label in the assay should be maximal. An FET binding event may be conveniently measured through standard fluorometric detection means well known in the art (e.g., using a fluorimeter).

In another example, determination of the ability of an antibody molecule to recognize pSYK may be accomplished without labeling either assay component (pSYK or antibody molecule) by utilizing a technology such as real-time Biomolecular Interaction Analysis (BIA) (see, e.g., Sjolander, S, and Urbaniczky, C, 1991, Anal. Chem. 63:2338-2345 and Szabo et al., 1995, Curr. Opin. Struct. Biol. 5:699-705). As used herein, "BIA" or "surface plasmon resonance" is a technology for studying biospecific interactions in real time, without labeling any of the interactants (e.g., BIACORE™). Changes in the mass at the binding surface (indicative of a binding event) result in alterations of the refractive index of light near the surface (the optical phenomenon of surface plasmon resonance (SPR)), resulting in a detectable signal which may be used as an indication of real-time reactions between biological molecules. Quantification of anti-pSYK antibody binding to a sample, cell or tumor can be aided by a computer program, such as image analysis software. Such a program can reduce noise from non-specific sample variations, can integrate the strength of the signal, can calculate the input derived from colors of multiple labels to determine the specific contribution by the specific label for detecting the pSYK antibody molecule and/or to quantify a label for a second marker, such as pBTK or pBLNK, being detected in the assay. Examples of computer programs, e.g., image analysis software, for IHC include Definiens Tissue Studio (Definiens Inc, Carlsbad, CA) or APERIO® SCANSCOPE™ slide scanner or EPATHOLOGY™ system (Leica Biosystems, Buffalo Grove, IL). Quantification methods, including computer-assisted quantification, can be calibrated using a calibration sample. A calibration sample can be prepared before the staining procedure or prepared during the staining procedure, e.g., to use as an assay control. Examples of calibration samples include an unstained sample or a sample to remain unstained or stained with normal IgG during the staining procedure; a sample stained by a non-pSYK antibody, such as a total SYK antibody, or a sample for staining with a non-pSYK antibody during the staining procedure; a phosphatase-treated sample stained by a pSYK antibody, such as a sample treated with T-cell protein phosphatase (New England Biolabs, Ipswich, MA), or a sample for pretreating with phosphatase during the staining procedure; a sample with high pSYK amounts, such as cell pellets of pervanadate treated or BCR-crosslinked lymphoma, such as WSU-DLCL2 cells, xenografts of TMD8 lymphoma tumors (Tohda et al. (2006) Leuk. Res. 30:1385-1390) or PHTX95L tumor xenografts, pre-stained by a pSYK antibody or for staining with a pSYK antibody during the staining procedure; a sample with low pSYK amounts, such as a pellet of untreated WSU-DLCL2 cells (Al-Katib et al. (1998) Clin. Cancer Res. 4:1305-1314) or OCI-LY10 tumor cell (National Cancer Institute, Bethesda, MD) xenografts or a sample of cells, such as NIH-3T3 cells, expressing a negative control protein such as a kinase dead (kd) mutant of SYK or RAS, such as cells transfected with a RAS recombinant construct, such as a chimera with RAS, e.g., ITK-RAS, pre-stained by a pSYK antibody or for staining with a pSYK antibody during the staining procedure. A calibration sample may help adjust the sensitivity of the practitioner, such as a cytotechnologist or pathologist, the scanner or the image analysis software by prespecifying the levels expected for positive or strong staining and for negative, weak or background staining.

In some embodiments, an assay to detect pSYK in a sample indicates pSYK presence if pSYK is detected in the cytoplasm. In some embodiments, an assay to detect pSYK in a sample indicates pSYK presence if pSYK is detected in the cytoplasm and the cell membrane. In some embodiments, an assay to detect pSYK in a sample indicates pSYK presence if pSYK is detected in the nucleus and the cytoplasm.

In some embodiments, an assay to detect pSYK in a sample, e.g., comprising tumor cells, can limit the detection of false positive results. In some embodiments, an assay to detect pSYK in a sample indicates pSYK absence if pSYK is detected only in the nucleus. In some embodiments, an assay to detect pSYK in a sample indicates pSYK absence if pSYK is detected only in the cell membrane. In some embodiments, an assay to detect pSYK in a sample indicates pSYK absence if pSYK is detected in the membrane or nucleus and a further step of contacting a portion of the sample with a reagent which binds total SYK does not detect total SYK. In some embodiments, an assay to detect pSYK in a sample indicates pSYK absence if pSYK is detected in the membrane or nucleus and a further step of contacting an anti-pSYK antibody on a portion of the sample after phosphatase treatment of the portion does not prevent binding by the anti-pSYK antibody. In some embodiments, an assay to detect pSYK in a sample indicates pSYK absence if pSYK is detected in the membrane or nucleus and a further step of staining a portion of the sample with anti-pSYK antibody preadsorbed with the pY525/526 peptide immunogen does not prevent binding by the preadsorbed anti-pSYK antibody.

In some embodiments, pSYK expression in a cell is quantified by measuring the amount of pSYK detected in the cytoplasm. In some embodiments, pSYK expression in a sample comprising cells is quantified by subtracting background staining, e.g., staining of nuclei or non-tumor cells, from positive staining, e.g., tumor cell cytoplasm staining. In some embodiments, pSYK expression in a sample comprising cells is quantified by the formula $(N_p + N_{sp})/N_{total}$, wherein $N_p$ is number of positive pixels, $N_{sp}$ is the number of strong positive pixels and $N_{total}$ is the number of strong positive pixels + positive pixels + weak positive pixels + negative pixels. In some embodiments, the quantification is performed on IHC samples at a magnification of 10x to 200x or 10x to 50x. In some embodiments, the quantification is performed on IHC samples at a magnification of 20x.

In some aspects, the disclosure features a reaction mixture that includes an antibody molecule described herein (e.g., MIL81-1-8, MIL81-2-1 or MIL81-99-1) or an immunoconjugate that includes an antibody molecule described herein and, e.g., a label and a biological sample, e.g., a biological sample described herein. In other embodiments, the reaction mixture may include an antibody molecule described herein (e.g., MIL81-1-8, MIL81-2-1 or MIL81-99-1) or an immunoconjugate that includes an antibody molecule described herein and, e.g., a label and pSYK obtained or isolated from a biological sample, e.g., a biological sample described herein.

In certain embodiments, a method, such as those described above, comprises detecting binding of an anti-pSYK antibody to pSYK in a membrane preparation obtained from a cell comprising pSYK. In other embodiments, a method further comprises detecting binding of a cell by a reagent which detects a non-SYK marker expressed on its surface. In such embodiments, a non-SYK marker can be selected from a tumor cell marker, a B-cell marker or myeloid progenitor marker, such as CD5, CD19, CD20, CD79, such as mutated CD79 (e.g., as bound by Leica Biosystems PA0192 antibody), CD34, CD38, CD117, CD138, CD133, LMP2A and ZAP70. In certain embodiments, a method comprising detecting pSYK further comprises detecting CD34 and/or CD117. In certain embodiments, the method comprises contacting a cell with an anti-pSYK antibody under conditions permissive for binding of the anti-pSYK antibody to pSYK, such as under permeabilization conditions, and detecting whether a complex is formed between the anti-pSYK antibody and pSYK. An exemplary assay for detecting binding of an anti-pSYK antibody to pSYK under permeabilization conditions is an immunofluorescence assay, such as a flow cytometry assay or a fluorescence-activated cell sorting, "FACS" assay, optionally including gating using a cell surface molecule, such as CD34 or CD117.

In Vivo Diagnostics

In still another embodiment, the invention provides a SYK-targeted therapeutic agent for use in a method of treating a patient having a disease characterized by one or more pSYK-expressing cells, the method comprising a method for detecting the presence or absence of pSYK-expressing cells or tissues in vivo. The method includes (i) administering to a subject (e.g., a patient having a cancer) an anti-pSYK antibody molecule of the invention (i.e., MIL81-1-8), or antigen binding fragment thereof, such as an antibody or antigen binding fragment thereof conjugated to a detectable label or marker; (ii) exposing the subject to a means for detecting said detectable label or marker to the pSYK-expressing tissues or cells. Such in vivo methods may be used for evaluation, diagnosis, staging and/or prognosis of a patient having a disorder such as cancer. The method comprises: (i) administering to a subject, an anti-pSYK antibody molecule or immunoconjugate thereof; and (ii) detecting formation of a complex between the anti-pSYK antibody molecule and pSYK protein. Complex formation is indicative of the presence or level of pSYK in the subject whereas no complex formation is indicative of the absence of pSYK in the subject.

Such individuals may be diagnosed as having metastasized pSYK-expressing cancer and the metastasized pSYK-expressing cancer cells may be detected by administering to the individual, such as by intravenous administration, a pharmaceutical composition that comprises a pharmaceutically acceptable carrier or diluent and a conjugated compound that comprises an anti-pSYK antibody molecule and an active moiety wherein the active moiety is a radioactive agent, and detecting the presence of a localized accumulation or aggregation of radioactivity, indicating the presence of cells expressing pSYK. In some embodiments of the present invention, the pharmaceutical composition comprises a pharmaceutically acceptable carrier or diluent and a conjugated compound that comprises an anti-pSYK antibody molecule and an active moiety wherein the active moiety is a radioactive agent and the anti-pSYK antibody molecule is the MIL81-1-8 antibody described herein, or fragments or derivatives thereof.

In one particular embodiment, radionuclides may be conjugated to an anti-pSYK antibody molecule of the invention for use as an imaging agent in in vivo imaging procedures. Imaging agents are useful diagnostic procedures as well as the procedures used to identify the location of metastasized cells. For example, individuals may be diagnosed as having metastasized cancer and the metastasized cancer cells may be detected by administering to the individual, such as by intravenous administration, a pharmaceutical composition that comprises a pharmaceutically acceptable carrier or diluent and a conjugated compound that comprises an anti-pSYK antibody molecule of the invention and an active moiety wherein the active moiety is a radionuclide and detecting the presence of a localized accumulation or aggregation of radioactivity, indicating the presence of cells that express pSYK.

Imaging may be performed by many procedures well-known to those having ordinary skill in the art and the appropriate imaging agent useful in such procedures may be conjugated to an anti-pSYK antibody molecule of the invention by well-known means. Examples of labels useful for diagnostic imaging in accordance with the present invention are radiolabels such as $^{32}$P, $^{3}$H, $^{14}$C, $^{188}$Rh, $^{43}$K, $^{52}$Fe, $^{57}$CO, $^{67}$Cu, $^{67}$Ga, $^{68}$Ga, $^{77}$Br, $^{81}$Rb/$^{81}$MKr, $^{87M}$Sr, $^{99}$Tc, $^{111}$In, $^{113M}$In, $^{123}$I,$^{125}$I, $^{127}$Cs, $^{129}$Cs, 1$^{31}$ I, 1$^{32}$I, $^{197}$Hg, 2$^{03}$Pb and 2$^{06}$Bi, and $^{213}$Bi; fluorescent labels such as fluorescein and rhodamine; nuclear magnetic resonance active labels; positron emitting isotopes of oxygen, nitrogen, iron, carbon, or gallium (e.g., $^{68}$Ga, $^{18}$F) detectable by a single photon emission computed tomography ("SPECT") detector or positron emission tomography ("PET") scanner; chemiluminescers such as luciferin; and enzymatic markers such as peroxidase or phosphatase. Short-range radiation emitters, such as isotopes detectable by short-range detector probes, such as a transrectal probe, may also be employed. Imaging may also be performed, for example, by radioscintigraphy, nuclear magnetic resonance imaging (MRI) or computed tomography (CT scan). Imaging by CT scan may employ a heavy metal such as iron chelates. MRI scanning may employ chelates of gadolinium or manganese.

The antibody may be labeled with such reagents using techniques known in the art. For example, Magerstadt, M. (1991) Antibody Conjugates And Malignant Disease, CRC Press, Boca Raton, Fla.; and Barchel, S. W. and Rhodes, B. H., (1983) Radioimaging and Radiotherapy, Elsevier, NY, N.Y., teach the conjugation of various therapeutic and diagnostic radionuclides to amino acids of antibodies. Such reactions may be applied to conjugate radionuclides to anti-pSYK antibody molecules of the invention with an appropriate chelating agent and/or linker. See also Wensel and Meares (1983) Radioimmunoimaging and Radioimmunotherapy, Elsevier, N.Y., for techniques relating to the radiolabeling of antibodies. See also, D. Colcher et al. Meth. Enzymol. 121 : 802-816 (1986).

In the case of a radiolabeled antibody, the antibody is administered to the patient, is localized to the tumor bearing the antigen with which the antibody reacts, and is detected or "imaged" in vivo using known techniques such as radionuclear scanning using e.g., a gamma camera or emission tomography or computed tomography. See e.g., A. R. Bradwell et al., "Developments in Antibody Imaging", Monoclonal Antibodies for Cancer Detection and Therapy, R. W. Baldwin et al., (eds.), pp 65-85 (Academic Press 1985). Alternatively, a positron emission transaxial tomography scanner, such as designated Pet VI located at Brookhaven National Laboratory, may be used where the radiolabel emits positrons (e.g., $^{11}$C, $^{18}$F, $^{15}$O, and $^{13}$N, $^{68}$Ga).

In other embodiments, the invention comprises methods for determining the dose, e.g., radiation dose, that different tissues are exposed to when a subject, e.g., a human subject, is administered an anti-pSYK antibody molecule that is conjugated to a radioactive isotope. The method includes: (i) administering an anti-pSYK antibody molecule as described herein, e.g., an anti-pSYK antibody molecule, that is labeled with a radioactive isotope to a subject; (ii) measuring the amount of radioactive isotope located in different tissues, e.g., tumor, or blood, at various time points until some or all of the radioactive isotope has been eliminated from the body of the subject; and (iii) calculating the total dose of radiation received by each tissue analyzed. The measurements may be taken at scheduled time points, e.g., day 1, 2, 3, 5, 7, and 12, following administration (at day 0) of the radioactively labeled anti-pSYK antibody molecule to the subject. The concentration of radioisotope present in a given tissue, integrated over time, and multiplied by the specific activity of the radioisotope may be used to calculate the dose that a given tissue receives. Pharmacological information generated using anti-pSYK antibody molecules labeled with one radioactive isotope, e.g., a gamma-emitter, e.g., $^{111}$In may be used to calculate the expected dose that the same tissue would receive from a different radioactive isotope which cannot be easily measured, e.g., a beta-emitter, e.g., $^{90}$Y.

Companion Diagnostic for SYK-Targeted Therapy

The in vitro and in vivo diagnostic methods described herein are useful to inform whether a patient having a proliferative disease such as cancer, should be treated or not with a SYK-targeted therapy, based on the presence or absence, respectively, of pSYK expression or the level of pSYK expression on the surface of or within the patient's cells or tissue. A patient having one more cells that express pSYK on the cell surface or within the cell may be a candidate for treatment with a SYK-targeted therapy.

In certain aspects, the invention provides a SYK-targeted therapeutic agent for use in a method of treating a patient having a disease characterized by one or more pSYK-expressing cells, the method comprising a method of determining sensitivity of a patient that has or is suspected of having a pSYK-expressing disease or disorder to a SYK-targeted therapy, comprising the steps of: (i) contacting a biological sample obtained from a subject with an anti-pSYK antibody molecule; (ii) detecting formation of a complex between the anti-pSYK antibody molecule and pSYK protein; wherein complex formation is indicative of the presence or level of pSYK protein in the biological sample, whereas no complex formation or non-specific complex formation is indicative of the absence of pSYK protein in the biological sample, thereby determining the sensitivity of the patient to a SYK-targeted therapy. In a particular embodiment, complex formation between the anti-pSYK antibody molecule and pSYK protein in the biological sample is detected via immunohistochemistry or immunofluorescence using an antibody molecule described herein, e.g., the MIL81-1-8 antibody described herein. In some examples, the anti-pSYK antibody is used in combination with one or more additional antibodies to determine sensitivity of the patient to a SYK-targeted therapy. Examples of antibodies to be used in combination with the anti-pSYK antibody include, but are not limted to, an anti-pBTK antibody, such as a BTK pY551 antibody (ABCAM®, Catalog No. ab40770) and an anti-pBLNK antibody, such as a BLNK pY96 antibody (Cell Signaling Technologies, Catalog No. 3601). Additional antibodies include antibodies to FLT3, VAV1, PLCG1, PI-3-kinase (PI3K$\delta$ and PI3K$\delta/\gamma$), and LCP2. Other examples of antibodies which may be used in combination with the anti-pSYK antibody include a non-SYK antibody, such as an antibody to a cell surface molecule, e.g., selected from CD5, CD19, CD20, CD79, such as mutated CD79 (e.g., as bound by Leica Biosystems PA0192 antibody), CD34, CD38, CD117, CD138, CD133, LMP2A and ZAP70. Such antibodies are well known to those skilled in the art and are readily available commercially. In one aspect of the invention, the anti-pSYK antibody is used in a method of evaluating the pharmacodynamics of a SYK-targeted therapy , wherein the method comprises some or all of the following steps: administering to a patient a SYK-target therapy; obtaining a biological sample comprising one or more cells suspected of expressing pSYK from the patient; contacting the biological sample with an anti-pSYK antibody; detecting formation of a complex between the anti-pSYK molecule and pSYK protein in the biological sample; quantifying pSYK expression in the biological sample, and, optionally one or more control or calibration samples; comparing the pSYK expression level against a database comprising pSYK expression levels in calibration samples or in studies of SYK-targeted therapy; and optionally, adjusting the dosing regimen based on the pSYK expression level.

In certain aspects, the invention provides a SYK-targeted therapeutic agent for use in a method of treating a patient having a disease characterized by one or more pSYK-expressing cells (e.g., a hematological malignancy such as chronic lymphocytic leukemia, acute myeloid leukemia, diffuse large B-cell lymphoma, or peripheral T-cell lymphoma), comprising: a. detecting pSYK protein expression in a biological sample obtained from the patient (e.g., a cell, a tissue biopsy, or a tumor biopsy); and b. administering a SYK-targeted therapeutic agent to the patient (e.g., a small molecule inhibitor of SYK) if the biological sample expresses pSYK. In certain such embodiments, the detection step comprises: i) contacting the biological sample with an anti-pSYK antibody molecule comprising three heavy chain complementarity determining regions (CDR1, CDR2, and CDR3) comprising amino acid sequences according to SEQ ID NOs: 11, 12 and 13, respectively; and three light chain complementarity determining regions (CDR1, CDR2, and CDR3) comprising amino acid sequences according to SEQ ID NOs: 14, 15 and 16, respectively; and ii) detecting formation of a complex between the anti-pSYK antibody molecule and pSYK protein. In certain such embodiments, the detection step is performed using immunohistochemistry.

In certain aspects, the invention provides a SYK-targeted therapeutic agent for use in a method of treating a patient having a disease characterized by one or more pSYK-expressing cells (e.g., a hematological malignancy such as chronic lymphocytic leukemia, acute myeloid leukemia, diffuse large B-cell lymphoma, or peripheral T-cell lymphoma), comprising a. detecting pSYK protein expression in a biological sample of the patient, b. administering administering a SYK-targeted therapeutic agent to the patient (e.g., a small molecule inhibitor of SYK). Thus, in certain aspects, the invention provides a SYK-targeted therapeutic agent for use in a method of treating a patient having a hematological malignancy selected from chronic lymphocytic leukemia, acute myeloid leukemia, diffuse large B-cell lymphoma, and peripheral T-cell lymphoma, comprising a detection step and administering a compound as disclosed herein. In some embodiments, the diffuse large B-cell lymphoma is the germinal center B cell-like (GCB) subtype. In some embodiments, the diffuse large B-cell lymphoma is the activated B cell-like (ABC) subtype. In some embodiments, the diffuse large B-cell lymphoma is the non-germinal center B cell-like (non-GCB) subtype. In certain aspects, the invention encompasses determining a SYK inhibition therapy regimen for treating a tumor in a patient comprising determining in a sample obtained from the patient the level of pSYK by contacting the sample with an anti-pSYK antibody molecule comprising three heavy chain complementarity determining regions (CDR1, CDR2, and CDR3) comprising amino acid sequences according to SEQ ID NOs: 11, 12 and 13, respectively; and three light chain complementarity determining regions (CDR1, CDR2, and CDR3) comprising amino acid sequences according to SEQ ID NOs: 14, 15 and 16, respectively; and ii) detecting formation of a complex between the anti-pSYK antibody molecule and pSYK protein.

Disclosed herein is also a method for the manufacture of a medicament, e.g., a SYK inhibitor, for use in treating a disorder or cancer characterized as having pSYK expression, e.g., expression detected by the antibodies and methods described herein.

Exemplary diseases/disorders that may be evaluated (e.g., diagnosed) and/or treated using the companion diagnostic methods described herein include, but are not limited to disorders, diseases, and conditions related to abnormal cell growth, including hematological malignancies, such as acute myeloid leukemia, B-cell chronic lymphocytic leukemia (BCLL), B-cell lymphoma (e.g., mantle cell lymphoma, follicular lymphoma, diffuse large B-cell lymphoma (DLBCL) (e.g., GCB DLBCL, ABC DLBCL or non GCB DLBCL), and T-cell lymphoma (e.g., peripheral T-cell lymphoma), as well as epithelial cancers (i.e., carcinomas), such as lung cancer (small cell lung cancer and non-small cell lung cancer), pancreatic cancer, and colon cancer. In addition to the hematological malignancies and epithelial cancers noted above, in some embodiments, the condition may include other types of cancer, including leukemia (chronic myelogenous leukemia and chronic lymphocytic leukemia (CLL); breast cancer, genitourinary cancer, skin cancer, bone cancer, prostate cancer, and liver cancer; brain cancer; cancer of the larynx, gall bladder, rectum, parathyroid, thyroid, adrenal, neural tissue, bladder, head, neck, stomach, bronchi, and kidneys; basal cell carcinoma, squamous cell carcinoma, metastatic skin carcinoma, osteosarcoma, Ewing's sarcoma, veticulum cell sarcoma, and Kaposi's sarcoma; myeloma, EBV-associated tumors and other solid tumors, giant cell tumor, islet cell tumor, acute and chronic lymphocytic and granulocytic tumors, hairy-cell tumor, adenoma, medullary carcinoma, pheochromocytoma, mucosal neuromas, intestinal ganglioneuromas, hyperplastic corneal nerve tumor, marfanoid habitus tumor, Wilms' tumor, seminoma, ovarian tumor, leiomyomater tumor, cervical dysplasia, neuroblastoma, retinoblastoma, myelodysplastic syndrome, rhabdomyosarcoma, astrocytoma, non-Hodgkin's lymphoma, malignant hypercalcemia, polycythermia vera, adenocarcinoma, glioblastoma multiforma, glioma, lymphomas, and malignant melanomas, among others. In addition to cancer, in some embodiments, the condition may include other diseases related to abnormal cell growth, including non-malignant proliferative diseases such as benign prostatic hypertrophy, restinosis, hyperplasia, synovial proliferation disorder, retinopathy or other neovascular disorders of the eye, among others. In some embodiments, the condition may include a symptom of such SYK expressing conditions; or a predisposition toward such SYK-expressing conditions.

In certain embodiments, the disease characterized by one or more pSYK-expressing cells is selected from PTCL, DLBCL, FL, MCL, CLL, AML, MDS, nasopharyngeal carcinoma, lymphoma, gastric carcinoma, breast cancer, ovarian cancer, lung cancer (e.g., small cell lung cancer) and PT-LPD. In certain embodiments, the DLBCL is the GCB subtype, the ABC subtype or the non GCB subtype. In certain embodiments, the disease characterized by one or more pSYK-expressing cells is a cancer, such as a hematological malignancy selected from a leukemia and a lymphoma. In certain such embodiments, the hematological malignancy is chronic lymphocytic leukemia (CLL). In certain such embodiments, the hematological malignancy is acute myeloid leukemia (AML). In certain such embodiments, the hematological malignancy is diffuse large B-cell lymphoma (DLBCL). In certain such embodiments, the disease characterized by one or more pSYK-expressing cells is selected from CLL, AML, DLBCL and EBV-lymphoid or solid tumor malignancy. In certain embodiments, the disease characterized by one or more pSYK-expressing cells is selected from CLL, AML, and DLBCL.

The invention encompasses the determination of whether to treat a patient with a SYK-targeted therapy. The methods disclosed herein also allow for the generation of a personalized treatment report, e.g., a personalized cancer treatment report, e.g., with a SYK-targeted therapy described herein, e.g., based on the presence or level of pSYK in a sample from tumor cells from a patient. The methods also determine payment for treatment of the disorder or cancer. Such methods may further comprise paying for treatment, e.g., treatment with a SYK-targeted therapy, of a patient whose cancer is characterized as having pSYK or having an increased level of pSYK, e.g., as indicated in the personalized cancer treatment report.

In a particular embodiment, the SYK-targeted therapeutic agent is anti-pSYK human IgGl monoclonal antibody conjugated to a cytotoxic agent, wherein the mAb includes a light chain variable region (VL) having the three light chain complementarity determining regions (CDR1, CDR2, and CDR3) and a heavy chain variable region (VH) having the three heavy chain complementarity determining regions (CDR1, CDR2, and CDR3) listed in Tables 5 (amino acid sequences) and 6 (corresponding nucleic acid sequences), and a heavy chain variable region and light chain variable region listed in Tables 3 (amino acid sequences) and 4 (corresponding nucleic acid sequence).

In some aspects of the invention, the SYK-targeted therapy is a SYK antagonist or a SYK inhibitor. In one emodiment, the SYK-targeted therapy is a peptide antagonist, an ATP competitor or a small molecule inhibitor of SYK. In certain embodiments, the SYK-targeted therapeutic agent comprises a SYK inhibiting compound, such as a small molecule inhibitor of SYK. In certain embodiments, the SYK-targeted therapy is a small molecule inhibitor of SYK. In certain such embodiments, the small molecule inhibitor of SYK comprises a fused heteroaromatic pyrrolidinone such as a pyrrolopyrimidinone (e.g., a 6,7-dihydro-5H-pyrrolo[3,4-c]pyrimidin-5-one) or pyrrolopyridinone (e.g., a 1H-pyrrolo[3,4-c]pyridine-3(2H)-one). In certain embodiments, the small molecule inhibitor of SYK may be found in US 8,440,689.

In some embodiments, the small molecule inhibitor of SYK comprises one or more of the following compounds:

6-((1R,2S)-2-Aminocyclohexylamino)-4-(m-tolylamino)-1H-pyrrolo[3,4-c]pyridin-3(2H)-one; 4-(1H-Indazol-6-ylamino)-6-((1R,2S)-2-aminocyclohexylamino)-1H-pyrrolo[3,4-c]pyridin-3(2H)-one;

6-((1R,2S)-2-Aminocyclohexylamino)-4-(4-fluoro-3-methylphenylamino)-1H-pyrrolo[3,4-c]pyridin-3(2H)-one;

6-((1R,2S)-2-Aminocyclohexylamino)-7-fluoro-4-(m-tolylamino)-1H-pyrrolo[3,4-c]pyridin-3(2H)-one;
6-((1R,2S)-2-Aminocyclohexylamino)-4-(1-methyl-1H-pyrazol-4-yl)-1H-pyrrolo[3,4-c]pyridin-3(2H)-one;
6-((3R,4R)-3-Aminotetrahydro-2H-pyran-4-ylamino)-4-(1-methyl-1H-pyrazol-4-yl)-1H-pyrrolo[3,4-c]pyridin-3(2H)-one;
6-((1R,2S)-2-Aminocyclohexylamino)-7-fluoro-4-(1-methyl-1H-pyrazol-4-yl)-1H-pyrrolo[3,4-c]pyridin-3(2H)-one;
6-((1R,2S)-2-Aminocyclohexylamino)-7-chloro-4-(1-methyl-1H-pyrazol-4-yl)-1H-pyrrolo[3,4-c]pyridin-3(2H)-one;
6-((1R,2S)-2-Aminocyclohexylamino)-7-fluoro-4-(pyrazolo[1,5-a]pyridin-3-yl)-1H-pyrrolo[3,4-c]pyridin-3(2H)-one;
6-((1R,2S)-2-Aminocyclohexylamino)-4-(3-(methylsulfonyl)phenylamino)-1H-pyrrolo[3,4-c]pyridin-3(2H)-one;
6-((1R,2S)-2-Aminocyclopentylamino)-4-(3-(methylsulfonyl)phenylamino)-1H-pyrrolo[3,4-c]pyridin-3(2H)-one;
(R)-4-Methyl-2-(4-(3-(methylsulfonyl)phenylamino)-3-oxo-2,3-dihydro-1H-pyrrolo[3,4-c]pyridin-6-ylamino)pentanamide;
(R)-4-Methyl-2-(4-(1-methyl-1H-pyrazol-4-yl)-3-oxo-2,3-dihydro-1H-pyrrolo[3,4-c]pyridin-6-ylamino)pentanamide;
6-((1R,2S)-2-Aminocyclohexylamino)-4-(benzofuran-3-yl)-7-fluoro-1H-pyrrolo[3,4-c]pyridin-3(2H)-one;
6-((1R,2S)-2-aminocyclohexylamino)-7-fluoro-4-(imidazo[1,2-a]pyridin-3-yl)-1H-pyrrolo[3,4-c]pyridin-3(2H)-one;
6-((1R,2S)-2-aminocyclohexylamino)-4-(benzo[b]thiophen-3-yl)-7-fluoro-1H-pyrrolo[3,4-c]pyridin-3(2H)-one;
6-((1S,2R)-2-Aminocyclohexylamino)-7-fluoro-4-(1-methyl-1H-pyrazol-4-yl)-1H-pyrrolo[3,4-c]pyridin-3(2H)-one;
(R)-6-(2-Amino-3-ethoxypropylamino)-4-(m-tolylamino)-1H-pyrrolo[3,4-c]pyridin-3(2H)-one; (R)-6-(2-Amino-3-ethoxypropylamino)-7-fluoro-4-(m-tolylamino)-1H-pyrrolo[3,4-c]pyridin-3(2H)-one;
6-(2-Amino-3,3,3-trifluoropropylamino)-4-(m-tolylamino)-1H-pyrrolo[3,4-c]pyridin-3(2H)-one; (R)-4-Methyl-2-(3-oxo-4-(m-tolylamino)-2,3-dihydro-1H-pyrrolo[3,4-c]pyridin-6-ylamino)pentanamide;
6-(cis-4-Aminotetrahydrofuran-3-ylamino)-4-(m-tolylamino)-1H-pyrrolo[3,4-c]pyridin-3(2H)-one;
6-((1R,2S)-2-Aminocyclohexylamino)-4-(1-ethyl-1H-pyrazol-4-yl)-1H-pyrrolo[3,4-c]pyridin-3(2H)-one;
6-((1R,2S)-2-Aminocyclohexylamino)-4-(1-ethyl-1H-pyrazol-4-yl)-7-fluoro-1H-pyrrolo[3,4-c]pyridin-3(2H)-one;
6-((1R,2S)-2-Aminocyclohexylamino)-4-(1-cyclopropyl-1H-pyrazol-4-yl)-1H-pyrrolo[3,4-c]pyridin-3(2H)-one;
6-((1R,2S)-2-Aminocyclohexylamino)-4-(1-(difluoromethyl)-1H-pyrazol-4-yl)-7-fluoro-1H-pyrrolo[3,4-c]pyridin-3(2H)-one;
6-((1R,2S)-2-Aminocyclohexylamino)-4-(1-cyclopropyl-1H-pyrazol-4-yl)-7-fluoro-1H-pyrrolo[3,4-c]pyridin-3(2H)-one;
cis-6-(2-Aminocyclohexylamino)-7-fluoro-4-(1-methyl-1H-pyrazol-4-yl)-1H-pyrrolo[3,4-c]pyridin-3(2H)-one;
6-((3R,4R)-3-Aminotetrahydro-2H-pyran-4-ylamino)-7-fluoro-4-(1-methyl-1H-pyrazol-4-yl)-1H-pyrrolo[3,4-c]pyridin-3(2H)-one;
6-(cis-2-Amino-4,4-difluorocyclopentylamino)-7-fluoro-4-(1-methyl-1H-pyrazol-4-yl)-1H-pyrrolo[3,4-c]pyridin-3(2H)-one;
6-(cis-2-Amino-3,3-difluorocyclohexylamino)-7-iluoro-4-(1-methyl-1H-pymzol-4-yl)-1H-pyrrolo[3,4-c]pyridin-3(2H)-one;
6-(cis-2-Amino-3,3-difluorocyclohexylamino)-4-(1-(difluoromethyl)-1H-pyrazol-4-yl)-7-fluoro-1H-pyrrolo[3,4-c]pyridin-3(2H)-one;
6-(cis-2-amino-3,3-difluorocyclohexylamino)-4-(1-cyclopropyl-1H-pyrazol-4-yl)-7-fluoro-1H-pyrrolo[3,4-c]pyridin-3(2H)-one;
(R)-2-(7-Fluoro-4-(1-methyl-1H-pyrazol-4-yl)-3-oxo-2,3-dihydro-1H-pyrrolo[3,4-c]pyridin-6-ylamino)-4-methylpentanamide;
(R)-2-(4-(1-(Difluoromethyl)-1H-pyrazol-4-yl)-7-fluoro-3-oxo-2,3-dihydro-1H-pyrrolo[3,4-c]pyridin-6-ylamino)-4-methylpentanamide;
(R)-2-(4-(1-Cyclopropyl-1H-pyrazol-4-yl)-7-fluoro-3-oxo-2,3-dihydro-1H-pyrrolo[3,4-c]pyridin-6-ylamino)-4-methylpentanamide;
(R)-2-(4-(Benzofuran-3-yl)-7-fluoro-3-oxo-2,3-dihydro-1H-pyrrolo[3,4-c]pyridin-6-ylamino)-4-methylpentanamide;
(R)-2-(7-Fluoro-3-oxo-4-(pyrazolo[1,5-a]pyridin-3-yl)-2,3-dihydro-1H-pyrrolo[3,4-c]pyridin-6-ylamino)-4-methylpentanamide;
6-((1R,2S)-2-Aminocyclohexylamino)-7-chloro-4-(m-tolylamino)-1H-pyrrolo[3,4-c]pyridin-3(2H)-one;
6-((1R,2S)-2-Aminocyclohexylamino)-3-oxo-4-(m-tolylamino)-2,3-dihydro-1H-pyrrolo[3,4-c]pyridine-7-carbonitrile;
(R)-6-(2-Amino-3-methoxypropylamino)-4-(m-tolylamino)-1H-pyrrolo[3,4-c]pyridin-3(2H)-one; (R)-6-(2-Amino-3-methoxypropylamino)-3-oxo-4-(m-tolylamino)-2,3-dihydro-1H-pyrrolo[3,4-c]pyridine-7-carbonitrile;
(R)-6-(2-Amino-3-methoxypropylamino)-7-fluoro-4-(m-tolylamino)-1H-pyrrolo[3,4-c]pyridin-3(2H)-one;
7-Acryloyl-6-((1R,2S)-2-aminocyclohexylamino)-4-(m-tolylamino)-1H-pyrrolo[3,4-c]pyridin-3(2H)-one;
6-((1R,2S)-2-Aminocyclohexylamino)-7-iodo-4-(m-tolylamino)-1H-pyrrolo[3,4-c]pyridin-3(2H)-one;
6-((1R,2S)-2-Aminocyclohexylamino)-7-(1H-pyrazol-4-yl)-4-(m-tolylamino)-1H-pyrrolo[3,4-c]pyridin-3(2H)-one;
6-(cis-2-Amino-3,3-difluorocyclohexylamino)-4-(m-tolylamino)-1H-pyrrolo[3,4-c]pyridin-3(2H)-one;
6-((1R,2S)-2-Aminocyclohexylamino)-7-(1-methyl-1H-pyrazol-5-yl)-4-(m-tolylamino)-1H-pyrrolo[3,4-c]pyridin-3(2H)-one;
6-((3R,4R)-3-Aminotetrahydro-2H-pyran-4-ylamino)-4-(m-tolylamino)-1H-pyrrolo[3,4-c]pyridin-3(2H)-one;
6-((3R,4R)-4-Aminotetrahydro-2H-pyran-3-ylamino)-4-(m-tolylamino)-1H-pyrrolo[3,4-c]pyridin-3(2H)-one;
6-((1R,2S)-2-Aminocyclohexylamino)-7-methyl-4-(m-tolylamino)-1H-pyrrolo[3,4-d]pyridin-3(2H)-one;
6-((3R,4R)-3-Aminotetrahydro-2H-pyran-4-ylamino)-7-fluoro-4-(m-tolylamino)-1H-pyrrolo[3,4-c]pyridin-3(2H)-one;
6-((1R,2S)-2-Aminocyclohexylamino)-7-methyl-4-(1-methyl-1H-pyrazol-4-yl)-1H-pyrrolo[3,4-c]pyridin-3(2H)-one;
(R)-6-(2-Amino-3-methoxypropylamino)-4-(1-methyl-1H-pyrazol-4-yl)-1H-pyrrolo[3,4-c]pyridin-3(2H)-one;
6-((3R,4R)-3-Aminotetrahydro-2H-pyran-4-ylamino)-7-fluoro-4-(pyrazolo[1,5-c]pyridin-3-yl)-1H-pyrrolo[3,4-d]pyridin-3(2H)-one;
6-((3R,4R)-3-Aminotetrahydro-2H-pyran-4-ylamino)-4-(1-(difluoromethyl)-1H-pyrazol-4-yl)-7-fluoro-1H-pyrrolo[3,4-c]pyridin-3(2H)-one;
6-((3R,4R)-3-Aminotetrahydro-2H-pyran-4-ylamino)-4-(benzofuran-3-yl)-7-fluoro-1H-pyrrolo[3,4-c]pyridin-3(2H)-one;
(S)-6-(3-Aminopyrrolidin-1-yl)-7-fluoro-4-(1-methyl-1H-pyrazol-4-yl)-1H-pyrrolo[3,4-c]pyridin-3(2H)-one;
(S)-6-(3-Aminopiperidin-1-yl)-7-fluoro-4-(1-methyl-1H-pyrazol-4-yl)-1H-pyrrolo[3,4-c]pyridin-3(2H)-one;
6-((1R,2S)-2-Aminocyclohexylamino)-7-fluoro-4-(1-isopropyl-1H-pyrazol-4-yl)-1H-pyrrolo[3,4-c]pyridin-3(2H)-one;
7-Fluoro-4,6-bis(1-methyl-1H-pyrazol-4-yl)-1H-pyrrolo[3,4-c]pyridin-3(2H)-one; 6-((1R,2S)-2-Aminocyclohexylamino)-7-bromo-4-(1-methyl-1H-pyrazol-4-yl)-1H-pyrrolo[3,4-c]pyridin-3(2H)-one;
(R)-6-(2-Amino-3-methoxypropylamino)-7-fluoro-4-(1-methyl-1H-pyrazol-4-yl)-1H-pyrrolo[3,4-c]pyridin-3(2H)-one;
6-((3R,4R)-3-Aminotetrahydro-2H-pyran-4-ylamino)-7-fluoro-4-(thiophen-3-yl)-1H-pyrrolo[3,4-c]pyridin-3(2H)-one;
6-((3R,4R)-3-Aminotetrahydro-2H-pyran-4-ylamino)-7-fluoro-4-(4-methylthiophen-2-yl)-1H-pyrrolo[3,4-c]pyridin-3(2H)-one;
6-((1R,2S)-2-Aminocyclohexylamino)-7-fluoro-4-(4-methylthiophen-2-yl)-1H-pyrrolo[3,4-c]pyridin-3(2H)-one;
6-((1R,2S)-2-Aminocyclohexylamino)-7-fluoro-4-(thiophen-3-yl)-1H-pyrrolo[3,4-c]pyridin-3(2H)-one;
(R)-2-(7-Fluoro-4-(1-methyl-1H-pyrazol-4-yl)-3-oxo-2,3-dihydro-1H-pyrrolo[3,4-c]pyridin-6-ylamino)-N,4-dimethylpentanamide;
6-((1R,2S)-2-Aminocyclohexylamino)-7-fluoro-4-(5-methylthiophen-2-yl)-1H-pyrrolo[3,4-c]pyridin-3(2H)-one;
(R)-2-(7-Fluoro-4-(4-methylthiophen-2-yl)-3-oxo-2,3-dihydro-1H-pyrrolo[3,4-c]pyridin-6-ylamino)-4-methylpentanamide;
(R)-2-(7-Fluoro-3-oxo-4-(thiophen-3-yl)-2,3-dihydro-1H-pyrrolo[3,4-c]pyridin-6-ylamino)-4-methylpentanamide;
(R)-2-(7-Fluoro-4-(5-methylthiophen-2-yl)-3-oxo-2,3-dihydro-1H-pyrrolo[3,4-c]pyridin-6-ylamino)-4-methylpentanamide;
6-((1R,2S)-2-Aminocyclohexylamino)-4-(2-aminothiazol-5-yl)-7-fluoro-1H-pyrrolo[3,4-c]pyridin-3(2H)-one;
(R)-2-(7-Fluoro-4-(furan-2-yl)-3-oxo-2,3-dihydro-1H-pyrrolo[3,4-c]pyridin-6-ylamino)-4-methylpentanamide;
(R)-2-(7-Fluoro-4-(furan-3-yl)-3-oxo-2,3-dihydro-1H-pyrrolo[3,4-c]pyridin-6-ylamino)-4-methylpentanamide;
(R)-2-(7-Fluoro-4-(5-methylfuran-2-yl)-3-oxo-2,3-dihydro-1H-pyrrolo[3,4-c]pyridin-6-ylamino)-4-methylpentanamide;
(R)-2-(4-(5-Cyanothiophen-2-yl)-7-fluoro-3-oxo-2,3-dihydro-1H-pyrrolo[3,4-c]pyridin-6-ylamino)-4-methylpentanamide;
(R)-2-(4-(4-Cyanothiophen-2-yl)-7-fluoro-3-oxo-2,3-dihydro-1H-pyrrolo[3,4-c]pyridin-6-ylamino)-4-methylpentanamide;
(R)-2-(7-Fluoro-3-oxo-4-(thiazol-5-yl)-2,3-dihydro-1H-pyrrolo[3,4-c]pyridin-6-ylamino)-4-methylpentanamide;
(R)-2-(7-Fluoro-4-(isothiazol-5-yl)-3-oxo-2,3-dihydro-1H-pyrrolo[3,4-c]pyridin-6-ylamino)-4-methylpentanamide;
6-((1R,2S)-2-Aminocyclohexylamino)-7-fluoro-1,1-dimethyl-4-(1-methyl-1H-pyrazol-4-yl)-1H-pyrrolo[3,4-c]pyridin-3(2H)-one;
((1R,2S)-2-Aminocyclohexylamino)-7-fluoro-4-(1-methyl-1H-pyrazol-3-yl)-1H-pyrrolo[3,4-c]pyridin-3(2H)-one;
6-((1R,2S)-2-Aminocyclohexylamino)-7-fluoro-4-(2-methylthiazol-5-yl)-1H-pyrrolo[3,4-c]pyridin-3(2H)-one;
6-((3R,4R)-3-Aminotetrahydro-2H-pyran-4-ylamino)-7-fluoro-4-(5-methylthiophen-2-yl)-1H-pyrrolo[3,4-c]pyridin-3(2H)-one;
6-((1R,2S)-2-Aminocyclohexylamino)-7-fluoro-1-methyl-4-(1-methyl-1H-pyrazol-4-yl)-1H-pyrrolo[3,4-c]pyridin-3(2H)-one;
(R)-2-(7-Fluoro-3-oxo-4-(thiophen-2-yl)-2,3-dihydro-1H-pyrrolo[3,4-c]pyridin-6-ylamino)-4-methylpentanamide;
6-((3R,4R)-3-Aminotetrahydro-2H-pyran-4-ylamino)-7-fluoro-4-(thiophen-2-yl)-1H-pyrrolo[3,4-c]pyridin-3(2H)-one;
6-((3R,4R)-3-Aminotetrahydro-2H-pyran-4-ylamino)-7-fluoro-4-(thiazol-5-yl)-1H-pyrrolo[3,4-c]pyridin-3(2H)-one;
6-((1R,2S)-2-Aminocyclohexylamino)-7-fluoro-4-(thiophen-2-yl)-1H-pyrrolo[3,4-c]pyridin-3(2H)-one;
6-((3R,4R)-3-Aminotetrahydro-2H-pyran-4-ylamino)-7-fluoro-4-(4-(trifluoromethyl)-1H-imidazol-1-yl)-1H-pyrrolo[3,4-c]pyridin-3(2H)-one;
6-((1R,2S)-2-Aminocyclohexylamino)-7-fluoro-4-(4-methyl-1H-imidazol-1-yl)-1H-pyrrolo[3,4-c]pyridin-3(2H)-one;

6-((3R,4R)-3-Aminotetrahydro-2H-pyran-4-ylamino)-7-fluoro-4-(3-methylisothiazol-5-yl)-1H-pyrrolo[3,4-c]pyridin-3(2H)-one;

6-((3R,4R)-3-Aminotetrahydro-2H-pyran-4-ylamino)-7-fluoro-4-(2-methylthiazol-5-yl)-1H-pyrrolo[3,4-c]pyridin-3(2H)-one;

(R)-2-(7-Fluoro-4-(2-methylthiazol-5-yl)-3-oxo-2,3-dihydro-1H-pyrrolo[3,4-c]pyridin-6-ylamino)-4-methylpentanamide;

6-((3R,4R)-3-Aminotetrahydro-2H-pyran-4-ylamino)-4-(5-chlorothiophen-2-yl)-7-fluoro-1H-pyrrolo[3,4-c]pyridin-3(2H)-one;

6-((3R,4R)-3-Aminotetrahydro-2H-pyran-4-ylamino)-4-(1-cyclopropyl-1H-pyrazol-4-yl)-7-fluoro-1H-pyrrolo[3,4-c]pyridin-3(2H)-one;

a stereoisomer of any of the aforementioned compounds; or a pharmaceutically acceptable salt of any of the aforementioned compounds or stereoisomers.

In some embodiments, the small molecule inhibitor of SYK is 6-((1R,2S)-2-Aminocyclohexylamino)-7-fluoro-4-(1-methyl-1H-pyrazol-4-yl)-1H-pyrrolo[3,4-c]pyridin-3(2H)-one or a pharmaceutically acceptable salt thereof (Compound A). In some embodiments, the small molecule inhibitor of SYK is 6-((1S,2R)-2-Aminocyclohexylamino)-7-fluoro-4-(1-methyl-1H-pyrazol-4-yl)-1H-pyrrolo[3,4-c]pyridin-3(2H)-one or a pharmaceutically acceptable salt thereof. In some embodiments, the small molecule inhibitor of SYK is 6-((1R,2S)-2-Aminocyclohexylamino)-4-(1-(difluoromethyl)-1H-pyrazol-4-yl)-7-fluoro-1H-pyrrolo[3,4-c]pyridin-3(2H)-one or a pharmaceutically acceptable salt thereof. In some embodiments, the small molecule inhibitor of SYK is cis-6-(2-Aminocyclohexylamino)-7-fluoro-4-(1-methyl-1H-pyrazol-4-yl)-1H-pyrrolo[3,4-c]pyridin-3 (2H)-one or a pharmaceutically acceptable salt thereof. In some embodiments, the small molecule inhibitor of SYK is 6-((3R,4R)-3-aminotetrahydro-2H-pyran-4-ylamino)-4-(1-(difluoromethyl)-1H-pyrazol-4-yl)-7-fluoro-1H-pyrrolo[3,4-c]pyridin-3(2H)-one or a pharmaceutically acceptable salt thereof. In some embodiments, the small molecule inhibitor of SYK is 6-((3R,4R)-3-Aminotetrahydro-2H-pyran-4-ylamino)-7-fluoro-4-(3-methylisothiazol-5-yl)-1H-pyrrolo[3,4-c]pyridin-3(2H)-one or a pharmaceutically acceptable salt thereof.In certain embodiments, such small molecule inhibitors of SYK may be administered orally (such as by administering tablets or capsules), intravenously, subcutaneously, or by any other suitable method. In certain embodiments, administration may be twice daily or once daily. In certain embodiments, administration may be seven, six, five, four, three, two, or one time per week.

In some embodiments, one or more inhibitors of SYK, BTK, BLNK, FLT3, VAV1, PLCG1, PI-3-kinase (PI3K$\delta$ and PI3K$\delta/\gamma$), and LCP2, or any combination thereof, are administered to the patient. In certain embodiments, an inhibitor of SYK may be administered in combination with an inhibitor of FLT3. In certain embodiments, an inhibitor, such as a small molecule inhibitor, may act as an inhibitor of both SYK and FLT3.

As discussed above, the antibody molecules described herein permit assessment of the presence of a pSYK protein in normal versus neoplastic tissues, through which the presence or severity of disease, disease progress and/or the efficacy of therapy may be assessed. For example, therapy may be monitored and efficacy assessed. In one example, a pSYK protein may be detected and/or measured in a first sample obtained from a subject having a proliferative disease and therapy may be initiated. Later, a second sample may be obtained from the subject and pSYK protein in the sample may be detected and/or measured. A decrease in the quantity of pSYK protein detected or measured in the second sample may be indicative of therapeutic efficacy.

## Kits

Also disclosed herein are kits comprising an anti-pSYK antibody molecule or immunoconjugate as described herein. A "kit" is any article of manufacture (e.g. a package or container) comprising at least one reagent, e.g. an antibody described herein, for specifically detecting pSYK. Further included are kits comprising liposome compositions comprising an anti-pSYK antibody molecule or immunoconjugate. The kit may include one or more other elements including: instructions for use; other reagents, e.g., a label, a therapeutic agent, or an agent useful for chelating, or otherwise coupling, an antibody to a label or therapeutic agent, or a radioprotective composition; devices or other materials for preparing the antibody for administration; pharmaceutically acceptable carriers; and devices or other materials for administration to a subject. Instructions for use may include instructions for diagnostic applications of the anti-pSYK antibody molecule or immunoconjugate to detect pSYK, in vitro, e.g., in a sample, e.g., a biopsy, fluid or cells from a patient having a cancer, or in vivo. The instructions may include guidance for therapeutic application including suggested dosages and/or modes of administration, e.g., in a patient with a cancer. Other instructions may include instructions on coupling of the antibody to a chelator, a label or a therapeutic agent, or for purification of a conjugated antibody, e.g., from unreacted conjugation components. As discussed above, the kit may include a label, e.g., any of the labels described herein and optionally may further include an amplification reagent. As discussed above, the kit may include a therapeutic agent, e.g., a therapeutic agent described herein. In some applications the antibody will be reacted with other components, e.g., a chelator or a label or therapeutic agent, e.g., a radioisotope, e.g., yttrium or lutetium. In such cases the kit may include one or more of a reaction vessel to carry out the reaction or a separation device, e.g., a chromatographic column, for use in separating the finished product from starting materials or reaction intermediates.

The kit may further contain at least one additional reagent, such as a diagnostic or therapeutic agent, e.g., a diagnostic or therapeutic agent as described herein, and/or one or more additional anti-pSYK antibody molecules or immunoconjugates, formulated as appropriate, in one or more separate pharmaceutical preparations. The kit may further contain at least one additional reagent to detect at least one additional protein. The additional protein may be pBTK, such as a BTK pY551, pBLNK, such as BLNK pY96 or pFLT3. The additional protein may be FLT3, VAV1, PLCG1, PI-3-kinase (PI3Kδ and PI3Kδ/γ) or LCP2. The additional protein may be a cell surface molecule, e.g., CD5, CD19, CD20, CD79, such as mutated CD79, CD34, CD38, CD117, CD138, CD133, LMP2A or ZAP70. Thus, the kit may comprise a pSYK antibody and a non-pSYK antibody, such as an antibody to an additional protein. The kit may further contain a reagent which stabilizes the sample, such as a protein stabilizer, a RNA stabilizer, a DNA stabilizer or a phosphate stabilizer. The kit may further contain a calibration sample as described herein. The kit may further contain a reagent to confirm specific staining, such as an immunogen peptide, e.g., SEQ ID NO:25, for preadsorption of pSYK antibody, or phosphatase. The kit may further contain a reagent for identifying tumor subtype. In some embodiments, the reagent for identifying tumor subtype identifies a diffuse B-cell lymphoma subtype. In some embodiments, the diffuse large B-cell lymphoma subtype is the germinal center B cell-like (GCB) subtype. In some embodiments, the diffuse large B-cell lymphoma subtype is the activated B cell-like (ABC) subtype. In some embodiments, the diffuse large B-cell lymphoma subtype is the non-germinal center B cell-like (non-GCB) subtype.

The kit may further contain a radioprotectant. The radiolytic nature of isotopes, e.g., $^{90}$Yttrium ($^{90}$Y) is known. In order to overcome this radiolysis, radioprotectants may be included, e.g., in the reaction buffer, as long as such radioprotectants are benign, meaning that they do not inhibit or otherwise adversely affect the labeling reaction, e.g., of an isotope, such as of $^{90}$Y, to the antibody. The formulation buffer may include a radioprotectant such as human serum albumin (HSA) or ascorbate, which minimize radiolysis due to yttrium or other strong radionuclides. Other radioprotectants are known in the art and may also be used in the formulation buffer of the present invention, i.e., free radical scavengers (phenol, sulfites, glutathione, cysteine, gentisic acid, nicotinic acid, ascorbyl palmitate, glycerol, $HOP(O)H_2$, sodium formaldehyde sulfoxylate, $Na_2S_2O$, $Na_2S_2O_3$, and $SO_2$, etc.).

A disclosed kit is one useful for radiolabeling a chelator-conjugated protein or peptide with a therapeutic radioisotope for administration to a patient. The kit includes (i) a vial containing chelator-conjugated antibody, (ii) a vial containing formulation buffer for stabilizing and administering the radiolabeled antibody to a patient, and (iii) instructions for performing the radiolabeling procedure. The kit provides for exposing a chelator-conjugated antibody to the radioisotope or a salt thereof for a sufficient amount of time under amiable conditions, e.g., as recommended in the instructions. A radiolabeled antibody having sufficient purity, specific activity and binding specificity is produced. The radiolabeled antibody may be diluted to an appropriate concentration, e.g., in formulation buffer, and administered directly to the patient with or without further purification. The chelator-conjugated antibody may be supplied in lyophilized form.

## Screening Assays

Disclosed herein are methods (also referred to herein as "screening assays") for identifying SYK inhibitors, i.e., candidate or test compounds or agents (e.g., proteins, peptides, peptidomimetics, peptoids, small molecules or other drugs) which have a inhibitory effect on, for example, SYK phosphorylation, expression or SYK pathway activity, or have a stimulatory or inhibitory effect on, for example, the expression or activity of a SYK substrate or proteins in the SYK pathway, or on the expression or activity of a downstream effector of SYK function, e.g., Akt/mTOR or NF-κB. Compounds thus identified can be used to modulate the activity of the target (e.g., pSYK) in a therapeutic protocol, to elaborate the biological function of the target, or to identify compounds that disrupt pSYK interactions. Compounds, e.g., SYK inhibitors, can be identified that cause the death, apoptosis or senescence of cells, e.g., cells from a hematological tumor or a solid tumor (e.g., PTCL, DLBCL (e.g., GCB subtype, ABC subtype or non-GCB subtype), FL, MCL, CLL, AML, MDS, nasopharyngeal carcinoma, lymphoma, gastric carcinoma, breast cancer, ovarian cancer, lung cancer (e.g., small cell lung cancer) and PT-LPD), or a cell line, e.g., cells grown from an explant of a tumor from a patient nonresponsive to Compound A, which have a mutant SYK gene, or an active SYK pathway.

In other examples, the assay can identify compounds which modulate one or more activity of a SYK, e.g., the ability to bind a ligand, e.g., selected from the group consisting of an ITAM domain, CD79a, CD79b, ATP, BLNK, the ability to bind a nucleotide, e.g., ATP or ADP; the ability to hydrolyze a nucleotide, e.g., ATP; the ability to bind CD79, the ability to bind a signalosome; the ability to phosphorylate tyrosine; the ability to autophosphorylate; the ability to phosphorylate BLNK; the ability to control the cell cycle, the ability to regulate cell signaling; and/or the ability to support tumor cell survival.

The test compounds can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, e.g., Zuckermann et al. (1994) J. Med. Chem. 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam (1997) Anticancer Drug Des. 12:145). Additional compounds can be synthesized from the guidance provided in the publications disclosing SYK inhibitors described in an earlier section. Libraries of compounds can be presented in solution (e.g., Houghten (1992) Biotechniques 13:412-421), or on beads (Lam (1991) Nature 354:82-84), chips (Fodor (1993) Nature 364:555-556), bacteria (Ladner, USP 5,223,409), spores (Ladner USP '409), plasmids (Cull et al. (1992) Proc Natl Acad Sci USA 89:1865-1869) or on phage (Scott and Smith (1990) Science 249:386-390; Devlin (1990) Science 249:404-406; Cwirla et al. (1990) Proc. Natl. Acad. Sci. 87:6378-6382; Felici (1991) J. Mol. Biol. 222:301-310; Ladner *supra*.).

For example, an assay is a cell-based assay in which a cell which expresses SYK protein, pSYK or biologically active portion thereof or ITK-SYK (Streubel et al. 2006 Leukemia 20:313-318) is contacted with a test compound, and the ability of the test compound to modulate SYK activity or the viability of the cell is determined. In another example, an in vitro cell-based assay is conducted on cells grown under nutrient-poor, e.g., low serum or low glucose, conditions. Determining the ability of the test compound to modulate SYK activity can be accomplished in a SYK functional assay, such as by monitoring, for example, the ability SYK to bind a ligand, e.g., selected from the group consisting of an ITAM domain, CD79a, CD79b, ATP, BLNK, the ability to bind a nucleotide, e.g., ATP or ADP; the ability of SYK to hydrolyze a nucleotide, e.g., ATP; the ability to bind CD79, the ability of SYK to bind a signalosome; the ability of SYK to phosphorylate tyrosine; the ability of SYK to autophosphorylate; the ability of SYK to phosphorylate BTK; the ability of SYK to phosphorylate BLNK; the ability of SYK to control the cell cycle, the ability of the cell to regulate cell signaling, and/or tumor cell survival. The effect of the test compound can be compared to a control cell not exposed to the test compound, a cell comprising a kinase dead mutant of SYK, such as kinase dead ITK-SYK, or a cell comprising a molecule which does not signal through the BCR pathway, such as RAS, a cell comprising ITK-RAS. In some examples, there can be a comparison of the activity of the SYK in the presence of the test agent with the activity in the presence of a SYK inhibitor, e.g., a fused heteroaromatic pyrrolidinone, such as a pyrrolopyrimidinone (e.g., a 6,7-dihydro-5H-pyrrolo[3,4-c]pyrimidin-5-one) or a pyrrolopyridinone (e.g., a 1H-pyrrolo[3,4-c]pyridine-3(2H)-one), such as Compound A, to which the cell comprising the pSYK has resistance. The cell, for example, can be of mammalian origin, e.g., human. In other examples, the assay can determine the ability of the test compound to modulate a variant of an enzyme structurally or mechanistically similar to pSYK in a drug resistant cell line *in vitro* or *in vivo,* e.g, in a xenograft tumor model. The compound is identified as modulator of drug resistance or a SYK inhibitor agent when the cell viability or cell growth is decreased.

Use of Information

In one method, information, e.g., about the SYK activation status of a patient's tumor, e.g., the presence or amount of pSYK measured as described herein, or about whether a patient is expected to have a favorable outcome, is provided (e.g., communicated, e.g., electronically communicated) to a third party, e.g., a hospital, clinic, a government entity, reimbursing party or insurance company (e.g., a life insurance company). For example, choice of medical procedure, whether to pay for a medical procedure, payment by a reimbursing party, or cost for a service or insurance can be function of the information. E.g., the third party receives the information, makes a determination based at least in part on the information, and optionally communicates the information or makes a choice of procedure, payment, level of payment, coverage, etc. based on the information. In the method, the presence or amount of pSYK, such as p525/526 is determined as described herein. The method may further comprise paying for a SYK therapy or billing for an insurance premium.

For example, a premium for insurance (e.g., life or medical) is evaluated as a function of information about the presence or level of pSYK associated with treatment outcome (e.g., the informative amount). For example, premiums can be increased (e.g., by a certain percentage) if the pSYK of a patient's cancer described herein is different between an insured candidate (or a candidate seeking insurance coverage) and a reference value (e.g., a non-afflicted person) or a reference sample, e.g., matched control. Premiums can also be scaled depending on the result of evaluating pSYK as described herein. For example, premiums can be assessed to distribute risk, e.g., as a function of pSYK, e.g., the result of evaluating a pSYK as described herein. In another example, premiums are assessed as a function of actuarial data that is obtained from patients that have known treatment outcomes. Information about SYK activity or pSYK expression, e.g., the result of evaluating a pSYK described herein (e.g., the informative amount), can be used, e.g., in an underwriting process for life insurance. The information can be incorporated into a profile about a subject. Other information in the profile can include, for example, date of birth, gender, marital status, banking information, credit information, children, and so forth. An insurance policy can be recommended as a function of the information on pSYK, e.g., the result of evaluating a pSYK described herein, along with one or more other items of information in the profile. An insurance premium or risk assessment can also be evaluated as function of the marker or marker set information. In one implementation, points are assigned on the basis of expected treatment outcome.

In one example, information about SYK, its activity or amount of pSYK, e.g., the result of evaluating pSYK described herein, is analyzed by a function that determines whether to authorize the transfer of funds to pay for a service or treatment provided to a subject (or make another decision referred to herein) and/or to pay for the service or treatment. For example, the results of analyzing pSYK described herein may indicate that a subject is expected to have a favorable outcome, suggesting that a treatment course is needed, thereby triggering a result that indicates or causes authorization to pay or pays for a service or treatment provided to a subject. In one example, pSYK amount measured in a sample comprising tumor cells, e.g., cells from a hematological cancer, such as AML, CLL or DLBCL, detected as described herein is determined and payment is authorized or is made if the pSYK amount identifies a favorable outcome. For example, an entity, e.g., a hospital, care giver, government entity, or an insurance company or other entity which pays for, or reimburses medical expenses, can use the result of a method described herein to determine whether a party, e.g., a party other than the subject patient, will pay for services (e.g., a particular therapy) or treatment provided to the patient. For example, a first entity, e.g., an insurance company, can use the outcome of a method described herein to determine whether to provide financial payment to, or on behalf of, a patient, e.g., whether to reimburse a third party, e.g., a vendor of goods or services, a hospital, physician, or other care-giver, for a service or treatment provided to a patient. For example, a first entity, e.g., an insurance company, can use the outcome of a method described herein to determine whether to continue, discontinue, enroll an individual in an insurance plan or program, e.g., a health insurance or life insurance plan or program.

In one aspect, the disclosure features a method of providing data. The method includes providing data described herein, e.g., generated by a method described herein, to provide a record, e.g., a record described herein, for determining if a payment will be provided. In some embodiments, the data is provided by computer, compact disc, telephone, facsimile, email, or letter. In some embodiments, the data is provided by a first party to a second party. In some examples, the first party is selected from the subject, a healthcare provider, a treating physician, a health maintenance organization (HMO), a hospital, a governmental entity, or an entity which sells or supplies the drug. In some examples, the second party is a third party payor, an insurance company, employer, employer sponsored health plan, HMO, or governmental entity. In some examples, the first party is selected from the subject, a healthcare provider, a treating physician, an HMO, a hospital, an insurance company, or an entity which sells or supplies the drug and the second party is a governmental entity. In some examples, the first party is selected from the subject, a healthcare provider, a treating physician, an HMO, a hospital, an insurance company, or an entity which sells or supplies the drug and the second party is an insurance company.

In another example, the disclosure features a record (e.g., computer readable record) or a personalized treatment report, e.g., a personalized cancer treatment report, which includes a list and value of SYK, e.g., activity, presence of p525/526 or amount of p525/526 for a patient. In some examples, the record includes more than one value for each aspect of SYK or pSYK values obtained at different times of treatment, e.g, before treatment and after treatment.

## Examples

### Example 1: SYK Inhibition

The ability of compounds to inhibit SYK activity may be assessed using a variety of methods, including in vitro and in vivo assays. An example of such an assay is disclosed in US 8,440,689. The following in vitro assay measures a test compound's ability to inhibit SYK-mediated phosphorylation of a FAM-labeled SYK-specific substrate (5FAM-KKKKEEIYFFFG-NH2, SEQ ID NO:23).

Briefly, SYK protein was prepared from cDNA encoding human spleen tyrosine kinase and was expressed in insect cells using a baculovirus expression vector. The cDNA was purchased from Open Biosystems. The SYK kinase domain (residues 356-635) was amplified via PCR and cloned into plasmid pFastBacl (Invitrogen) at BamHI/XbaI sites. Recombinant plasmid encoding Met-Ala-Lys-SYK(356-635)-HHHHHH (SEQ ID NO:24) was sequenced and transformed into E. coli DH10Bac strain. The recombinant bacmid DNA was isolated and transfected into Sf9 insect cells. Recombinant virus was harvested 72 h after transfection. High titer viral stock was prepared by infecting Sf9 cells at a multiplicity of infection (MOI) of approximately 0.01. A suspension of Sf9 cells (10 L) was infected with recombinant virus (MOI=5) and was incubated in a Wave Bioreactor (GE-Healthcare) for 48 h. The cells were then harvested and stored at -80° C.

To purify the expressed protein, the frozen Sf9 cells (10 L) were broken into small (<1 cm) particles and suspended in a lysis buffer (300 mL) containing 20 mM Tris (pH 7.6), 0.25 mM TCEP, 100 mM NaCl, 5% glycerol and a protease inhibitor. The suspension was stirred at RT until completely thawed, lysed an additional 2-4 min on a rotary blade homogenizer, and then centrifuged at 4200 g for 1 h. Following centrifugation, the supernatant was poured through cheese cloth and combined with a nickel chelating resin (PROBOND RESIN™, Invitrogen) which was pre-equilibrated in a wash buffer containing 10 mM Tris (pH 7.6), 0.25 mM TCEP, 300 mM NaCl, 5% glycerol, and 20 mM imidazole. The mixture was agitated for 3 h in a cold room and then centrifuged at 900 g for 10 min. The resin was dispersed in wash buffer (50 mL), centrifuged for 10 min at 900 g, re-dispersed in a small amount of wash buffer (5 mL), and then poured into a disposable Poly-Prep chromatography column, through which wash buffer was passed by gravity until no protein is observed in coomassie buffer (about 120 mL of wash buffer). An elution buffer (30 mL) containing 10 mM HEPES (pH 7.4), 150 mM NaCl, 10% glycerol, 5 mM DTT, and 400 mM imidazole was used to elute the SYK protein from the resin. The eluate was concentrated (5 mL) and further purified on a Superdex 200 column (1.2 mL/min for 160 min, 10 mM HEPES (pH 7.4), 10 mM NaCl, 10 mM MgCl, 0.1 mM EDTA, and 0.25 mM TCEP). The chromatographed fractions were run on SDS-PAGE and the requisite fractions were pooled and concentrated. Final delivery buffer was 10 mM HEPES (pH 7.4), 10 mM Methione, 150 mM NaCl, 10% glycerol, 5 mM DTT.

SYK inhibition was determined using a black 384 well plate format in buffer containing 50 mM HEPES, 10 mM NaCl, 10 mM MgCl2, 0.2 mM EDTA, 0.01% EDA (Brij 35), 1 mM DTT, and 0.1 mg/ml BSA at pH 7.3. Each test compound was prepared in DMSO using 2-fold serial dilutions for 11 data points, which were added to the buffer so that each dilution contained 3% DMSO. To each well was added 2 L of 3 M 5FAM-KKKKEEIYFFFG-NH2, SEQ ID NO:23, (in buffer), 2 L of diluted test compound (3% DMSO in buffer), and 2 L of 2.4 nM SYK and 45 M ATP (in buffer). The reaction mixture was incubated at RT for 60 min, and quenched by adding 50 mM Hepes, 30 mM EDTA, 0.1% Triton X-100 (pH 7.3). To quantify the fluorescent-labeled substrate and product following reaction, the test plate was loaded on a Caliper LC-3000, which measured percent of conversion by microfluidic-based separation. Corresponding IC50 values may be calculated by non-linear curve fitting of the compound concentrations and percent of inhibition to the standard IC50 equation and reported as pIC50, i.e., -log(IC50), where IC50 is molar concentration.

Example 2: Generation of rabbit monoclonal Antibodies

Rabbit monoclonal antibodies against the pSYK protein were generated using the RabMAb service provided by Epitomics (Burlingame, CA).

New Zealand White rabbits (2.5-3 months old, with body weight around 2.5 Kg, ID nos. 4784, 4785, 4786 and 4787) were immunized with a peptide covering residues 520-529 of hSYK and residues corresponding to tyrosine 525 and tyrosine 526 are phosphorylated (C-RADEN-pY-pY-KAQ, SEQ ID NO:25). This peptide was linked to KLH via the sulfhydryl moiety of the cysteine added to the N-terminus. Immunization was accomplished using conventional immunization techniques and employed adjuvant (SigmaAldrich, St. Louis, MO). The first injection used complete Freunds adjuvant (CFA) and subsequent injections used modified adjuvant (SFA). The immunogen was administered by subcutaneous injection at multiple sites on a schedule of 3 weeks + 2 weeks + 2 weeks + bleed 1 + 2 weeks +bleed 2.The rabbit with the highest serum titer, no. 4786, was chosen as a candidate for splenectomy and monoclonal fusion using Epitomics' fusion partner cell line 240E-W from US Patent 7429487and methods that included using polyethylene glycol (PEG).

Some lymphocytes from this rabbit (no. 4786) were used to produce a commercially available antibody (Epitomics 2175-1). Other lymphocytes from this rabbit were frozen and stored and later used for fusion and generation of pSYK antibodies described herein.

On two separate days (Day 1 and Day 2), two hundred million lymphocyte cells were fused with 100 million fusion partner cells and plated on 20X 96-well plates, respectively. The plates were kept in tissue culture incubators under standard conditions. Cell growth was examined 2-3 weeks after fusion and fusion efficiency computed using the number of wells with growth divided by the total number of wells examined. The fusion efficiency for the fusion on Day 1 was measured at 98% fusion efficiency, whereas the fusion efficiency on Day 2 was 98%.

Example 3: Generation of Screening Materials

The phosphopeptide Y525/526 immunogen was prepared by Epitomics. When tested by ELISA, this material was bound by Epitomics 2175-1, but was not bound by anti-hSYK antibody (Epitomics 1688-1).

GST-hSYK was expressed in baculovirus-infected Sf9 cells and affinity-purified by glutathione Sepharose affinity resin. For in vitro kinase (IVK) pSYK, isolated GST-SYK was incubated in 1 mM ATP, 1 mM $MgCl_2$ for 1 hr at room temperature to allow autophosphorylation to occur. The reaction was stopped by the addition of 5 mM EDTA and a small aliquot removed for treatment with 0.1% SDS, stored at 4° and used for AlphaScreen analysis or stored at -80° without further purification. This sample was later used at Epitomics for screening assays of hybridoma. An aliquot of the sample was analyzed by SDS-PAGE for Western blotting using anti-SYK (Cell Signaling Technologies #2712) and Epitomics 2175-1. The majority of the recombinant material was confirmed to be phosphorylated at Y525/526.

Some of the autophosphorylated GST-pSYK was treated with T-cell protein phosphatase (TC-PTP, New England Biolabs), a tyrosine-specific phosphatase, for confirming specificity of antibodies to pSYK. This material (PPase-pSYK or de-pSYK) retains its binding to an anti-hSYK antibody (Epitomics 1688-1 or Cell Signaling Technologies #2712) but loses most binding to Epitomics 2175-1 or Cell Signaling Technologies #2710.

Additional cell culture material was used to screen antibodies by utilizing WSU-DLCL2 (human diffuse large B-cell lymphoma) cells that were stimulated through crosslinking the B-cell receptor (AffiniPure F(ab')2 fragment of goat anti-human Ig, cat #109-006-127, JacksonImmuno Research Laboratories, Inc., West Grove, PA) or with pervanadate treatment (25 μL into 1mL of cells of the following; 50μL $Na_3VO_4$, 5.5μL 9.1M $H_2O_2$ (31%), 944.5μl $H_2O$) over a 60 min time course. At each time point cells were collected and lysed in MPER (Thermo: 7850) containing both a protease inhibitor cocktail I (Calbiochem:539131-10VL) and 1 X HALT a generic protein phosphatase inhibitor cocktail (Thermo: 78428).

Also LY10 (OCI-LY10) cells were used as lysates without or with stimulation with crosslinking or pervanadate as described above. Ly10 cells have weak SYK expression compared to WSUDLCL2 cells (Figure 1). This weak SYK and/or pSYK expression was confirmed in a western blot study comparing staining with different SYK and pSYK antibodies (pSYK, Epitomics 2175-1 and CST 2710; SYK pY352, CST 2717; SYK pY323, Epitomics 2173 and CST 2715) in 3 cell lines-WSU-DLCL2, LY10 and HBL1, untreated, treated for 10 min with crosslinker or treated with pervanadate, 2.5 nM, 10 min on WSU-DLCL2 cells and 5 nM for 30 min on Ly10 cells. This study also including staining of the blots with BLNK (CST 3575, SC8382 and SC15345) and pBLNK antibodies (BD558366, CST 36015 and SC28517-12). The Epitomics 2175 and 2173 antibodies and CST SYK pY352 antibody detected bands on crosslink-treated both WSU DLCL2 cells and HBL1 cells, but did not detect a band on Ly10 cells without the strongly enhancing pervanadate treatment. (BLNK was detected in every sample by CST 3575 and SC15345 antibodies, with the pBLNK detection paralleling the pSYK detection in the crosslinked and pervanadate-treated cells.) One conclusion of this study extended the nucleic acid results, and found weak pSYK signal in the Ly10 crosslinked sample.

Quality control of the screening materials was performed. An ELISA assay was performed at Epitomics (Burlingame, CA). The ELISA assays tested for binding by Epitomics 2175-1 compared to binding by the anti-SYK antibody (Epitomics 1688-1, raised against a peptide corresponding to a sequence near the kinase domain of SYK).

Western blot of cell lysates was performed using the two Epitomics commercial antibodies. Strong expression of total SYK was confirmed in both WSU-DLCL2 cells and LY10 cells, both unstimulated and stimulated. The positive control Epitomics #2175 bound the pSYK band only in the samples from the stimulated WSU-DLCL2 cells and LY10 cells (slightly lower intensity in LY10 than WSU-DLCL2).

Example 4: Antibody Screening

Initial Screening

A minimum of two plates were examined for each fusion as follows: All 40 plates were screened using standard ELISA methods with plates coated with 50 ng of GST-SYK-p/well. A bleed of rabbit 4786 at 1:10K dilution was used as a positive control. 103 clones having an O.D. greater than 0.5 were considered putatively positive and were further expanded into 24-well plates.

A subsequent confirmatory screen was performed by ELISA using plates coated with 50 ng of GST-SYK-p and GST-SYK-np. 30 clones were confirmed positive against GST-SYK-p and among them 29 were identified as GST-SYK-p specific, i.e., they were negative against negative control protein.

Second Level Screening

The 30 multi-clone supernatants were subjected to further testing by western blot, Alpha LISA, and immunofluorescence.

Western blots were prepared from SDS-PAGE gels of GST-pSYK (IVK), and phosphatase-treated GST-pSYK. Each hybridoma multiclone supernatant was tested for binding to the blots. Controls included anti-pSYK antibody, Epitomics 2175-1 or Cell Signaling Technologies, anti-SYK pY323 antibody, and anti-SYK pY352 antibody. By this method, multiclone hybridoma supernatants with good binding of phosphorylated relative to dephosphorylated pSYK were 1, 2, 19, 24, 39, 49, 52, 53, 55, 59, 63, 70, 81, 84, 85, 87, 98 and 99.

AlphaLISA® luminescence proximity assay (PerkinElmer Inc., Waltham, MA) was performed in duplicate on the AlphaScreen 96-well microtiter plates. For each individual hybridoma supernatant duplicate 25 μl of 2 nM SYK sample listed (IVK and PPase treated recombinant GST-SYK samples) was added to 5 μl of hybridoma supernatant and 25 μl of 25 μg/ml of glutathione donor (PerkinElmer # 6765300) and Protein A acceptor beads (PerkinElmer #6760137M) in HiBlock (PerkinElmer #AL004C) for 2 hrs at room temperature. By this method, there was good signal to background for supernatants of multiclones 1, 2, 19, 24, 39, 49, 53, 54, 55, 63, 70, 84, 85, 87, 96, 98 and 99. The supernatant of multiclone 94 had no phospho-specificity (strong binding to both phosphorylated and dephosphorylated samples).

Immunofluorescence assays of the multi-clone supernatants was performed on HeLa cells transfected for 24 hr with lipid:DNA complex (FuGENE 6, 3:1 ratio) of one of two versions of IL2-inducible T-cell kinase (ITK)-SYK fusion protein construct, wild-type (wt, Streubel et al. 2006 Leukemia 20:313-318) and kinase dead (kd) mutant thereof and plated at 3,000 cells per well of 96-well plates. Prior to the assay, the cells were treated 15 min with pervanadate, then fixed for antibody fluorescence staining by standard methods. Controls included staining by total SYK antibody, pSYK Y525/526 antibody, staining for hemagglutinin (HA), and DAPI (Hoescht reagent) DNA stain to confirm cell density. The best-staining multiclone supernatants were 1, 2, 24, 36, 53, 54 and 99. In the next tier, the good supernatants were 96, 55, 59, 63, 70, 84, 85 and 87.

The table below summarizes the results by these three tests.

| Antibody sample | Western blot | | AlphaLISA Signal | | Immuno fluorescence | |
|---|---|---|---|---|---|---|
| | pSYK | de-pSYK | pSYK | de-pSYK | wt SYK | KD SYK |
| multiclone 1 | ++++ | + | 86412 | 171 | ++++ | - |
| multiclone 2 | ++++ | + | 111834 | 523 | ++++ | - |
| multiclone 19 | +++ | + | 70728 | 333 | +++ | +/- |
| multiclone 24 | ++++ | + | 34419 | 922 | ++++ | - |
| multiclone 36 | - | - | 8778 | 1064 | ++++ | - |
| multiclone 39 | ++ | +/- | 22686 | 124 | +++ | - |
| multiclone 43 | +/- | - | 1511 | 200 | - | - |
| multiclone 49 | ++ | +/- | 11704 | 247 | ++ | +/- |
| multiclone 52 | ++ | +/- | 2565 | 257 | ++ | - |
| multiclone 53 | ++++ | + | 44080 | 333 | +++ | - |
| multiclone 54 | +/- | - | 48336 | 608 | ++ | - |
| multiclone 55 | ++++ | + | 64572 | 532 | ++++ | + |
| multiclone 57 | - | - | 333 | 390 | - | - |
| multiclone 59 | ++ | +/- | 4209 | 228 | + | - |
| multiclone 60 | - | - | 314 | 266 | - | +/- |
| multiclone 61 | +/- | - | 428 | 200 | - | + |
| multiclone 63 | ++ | +/- | 11752 | 162 | + | - |
| multiclone 67 | + | +/- | 5073 | 124 | - | - |
| multiclone 70 | +++ | +/- | 18725 | 152 | +/- | - |
| multiclone 79 | +/- | - | 8541 | 295 | + | - |
| multiclone 81 | +++ | +/- | 475 | 181 | +/- | - |
| multiclone 82 | +/- | - | 437 | 238 | - | - |
| multiclone 84 | ++++ | + | 189060 | 2860 | + | - |
| multiclone 85 | ++++ | + | 65094 | 466 | + | - |
| multiclone 87 | ++ | +/- | 21147 | 456 | +/- | - |
| multiclone 94 | - | - | 87343 | 135869 | - | - |
| multiclone 96 | +/- | - | 223868 | 4418 | ++ | - |
| multiclone 98 | ++++ | + | 20216 | 285 | ++ | ++++ |
| multiclone 99 | ++++ | + | 138862 | 1083 | ++++ | - |
| multiclone 102 | +/- | - | 2917 | 200 | - | - |
| Epitomics 2175-1 | ++++ | + | | | | |
| anti-SYK | +++ | ++++ | | | | |
| anti-pSYK Y525/526 CST | + | +/- | | | | |

(continued)

| Antibody sample | Western blot | | AlphaLISA Signal | | Immuno fluorescence | |
|---|---|---|---|---|---|---|
| | pSYK | de-pSYK | pSYK | de-pSYK | wt SYK | KD SYK |
| anti-SYK pY323 | ++++ | - | | | | |
| anti-SYK pY352 | +++ | +/- | | | | |

Some multiclone supernatants also were screened on western blots of cell lysates. Binding to SYK in lysates of WSU-DLCL2 cells and LY10 cells were compared. Antibodies from multiclones 1 and 2 moderately bound SYK on the sample from stimulated WSU-DLCL2 cells. Antibodies from multiclone 99 strongly bound SYK on the sample from stimulated WSU-DLCL2 cells and moderately bound SYK on the sample from stimulated LY10 cells. These antibodies also bound some extra bands in both the unstimulated and stimulated cells of both types. The commercial anti-pSYK Y525/526 antibody strongly bound SYK in the samples from both types of stimulated cells and did not bind any band in the samples from unstimulated cells.

After the above assays, 15 multiclone hybridoma supernatants were chosen for immunocytochemistry and immunohistochemistry. These were supernatants 1, 2, 19, 24, 36, 53, 54, 55, 59, 63, 70, 84, 85, 96 and 99.

Third Level Screening

Immunocytochemistry was performed on WSU-DLCL2 samples untreated or activated by Fab crosslinking. For activation, Fab crosslinking is performed by adding Fab to the cells while they are in culture. Fab crosslinks the surface proteins of the cell which activates the SYK pathway. A cell pellet of 200 x $10^6$ cells was formalin fixed as a pellet, paraffin embedded and sectioned for staining. The negative control was NIH 3T3 ITK-RAS (ITK fused to RAS). At one step, the supernatants were tested for binding the cell samples without or with prior heat treatment to expose epitopes (95°C, 8 min).

The results are provided in the Table below.

| | No Heat | | Heat | | |
|---|---|---|---|---|---|
| SUPE | NEG CTRL:NIH 3T3 ITK-Ras | POS CTRL:WSU-DLCL Fab Linked | NEG CTRL:NIH 3T3 ITK-Ras | POS CTRL:WSU-DLCL Fab Linked | Results |
| Epitomics 2175-1 | | | +/- | + | Good But Weak |
| 81-1 | +/- | ++ | +/- | ++ | Good |
| 81-2 | +/- | ++ | +/- | ++ | Good |
| 81-19 | + | ++ | +/- | ++ | OK |
| 81-24 | - | ++ | +/- | + | Good |
| 81-36 | +++ | +++ | +++ | +++ | Bad |
| 81-53 | +++ | +++ | +++ | +++ | Bad |
| 81-54 | +/- | ++ | +/- | ++ | OK |
| 81-55 | +++ | +++ | +++ | +++ | Bad |
| 81-59 | - | - | - | - | Bad |
| 81-63 | - | + | - | +/- | Bad |
| 81-70 | +++ | ++ | ++ | +++ | Bad |
| 81-84 | +/- | ++ | +/- | ++ | Good |
| 81-85 | +/- | ++ | +/- | ++ | Good |
| 81-96 | +/- | + | +/- | + | OK |
| 81-99 | + | +++ | + | +++ | Good |

Commercial antibody (Epitomics 2175-1), with heat, had slight rare staining background cell in the negative control pellet. Actual positive staining was mostly membranous with a very slight cytoplasmic haze. For supernatants 81-1, 81-2, 81-24, 81-54, 81-84, 81-85, 81-96 and 81-99, no heat was picked as the protocol. For supernatant 81-19, heat protocol had less unspecific background.

Since supernatants 81-1, 81-2, 81-24 showed very little to no background in negative controls and positive control showed a strong signal, these supernatants were used on xenografts. Since supernatants 81-19, 81-54, 81-84, 81-85 and 81-99 showed very little background in negative control and positive control showed strong signal, these supernatants were used on xenografts. Since supernatant 81-96 showed very little background in negative control and positive control showed weak signal, this supernatant was used on xenografts. Supernatants 81-36, 81-53, 81-55, and 81-70 had lots of unspecific staining, with both heat and no heat protocols. These supernatants were not pursued. For supernatants 81-59, no staining or 81-63, very weak staining, was seen by either the heat or no heat protocol. These 81-59 and 81-63 supernatants were not pursued.

In summary, good ICC staining was obtained by multiclone supernatants 1, 2, 24, 84, 85 and 99; OK ICC staining was obtained by supernatants 19, 54 and 96. Bad ICC staining was obtained by supernatants 36, 53, 55, 59, 63 and 70. The good staining and the OK staining supernatants were screened for staining on xenograft samples.

Fourth Level Screening

The 9 supernatants chosen for a good staining pattern in the ICC assay were screened using the PHTX-95L (primary human lymphoma tumor). In general, NOD SCID mice are implanted with primary human tumor pieces for growth to approx 200 $mm^3$ size. Formalin-fixed paraffin-embedded (FFPE) sections of the tumors were stained following standard Ventana Production protocol using the Ventana Discovery XT Automated IHC/ISH research slide staining system (Ventana Medical Systems, Inc. Tucson, AZ). The general protocol used manufacturer's reagents, EZ prep (Ventana 950-100), Reaction Buffer (Ventana 950-300), Cell Conditioning 1 (Ventana 950-124), hematoxylin (Ventant 760-2021) and Bluing reagent (Ventana 760-2037). All IHC using Epitomics #2175, was stained following a general protocol which used heat for incubating the antibody and ULTRAMAP™ anti-rabbit HRP reagent (Ventana 760-4315). The IHC assay for multiclone supernatant 81-19 IHC was performed following a general protocol which used heat for incubating the antibody and OMNIMAP™ anti-rabbit HRP reagent (Ventana 760-4311); and staining for binding of all other supernatants followed a general protocol which did not use heat for incubating the antibody and OMNIMAP™ anti-rabbit HRP reagent (Ventana 760-4311). Dako protein block (cat# X0909) was the block of choice used in all three protocols and Dako antibody diluent (cat# S0809) was used to dilute the Epitomics 2175-1 antibody.

Staining was compared among staining of WSU-DLCL DMSO section from a FFPE cell pellet (low SYK expressor) to compare with the ICC assay on these sups, WSU-DLCL Fab Crosslinked section from a FFPE cell pellet (medium SYK expressor), WSU-DLCL Pervandate, WSU-DLCL Pervandate section from a FFPE cell pellet (high SYK expressor). Pervanadate= 2.5μM for 15 minutes prior to cell harvest for pelleting. PHTX-95L-implanted mice which were untreated or treated by a single dose of an inhibitor of SYK kinase activity (Compound A) administered orally at 120 mg/kg. Two hours after dosing, tumors were collected for 10% formalin fixation and processing for IHC. The results are provided in the Table below.

| | WSU-DLCL DMSO | WSU-DLCL Fab Crosslinked | WSU-DLCL Pervandate | PHTX95L Untreated | PHTX95L Treated | Results |
|---|---|---|---|---|---|---|
| Epitomics 2175-1 | +/- | + | +++ | ++ | + | Good But Weak |
| 81-1 | + | ++ | +++ | ++ | + | Good |
| 81-2 | + | ++ | +++ | ++ | + | Good |
| 81-19 | + | ++ | +++ | ++ | ++ | Bad background |
| 81-24 | + | ++ | +++ | ++ | + | Good |
| 81-54 | + | ++ | +++ | + | + | Bad background |
| 81-84 | + | ++ | +++ | ++ | + | Good |
| 81-85 | + | ++ | +++ | ++ | + | Good |

(continued)

| | WSU-DLCL DMSO | WSU-DLCL Fab Crosslinked | WSU-DLCL Pervandate | PHTX95L Untreated | PHTX95L Treated | Results |
|---|---|---|---|---|---|---|
| 81-96 | + | + | +++ | + | + | Weak |
| 81-99 | + | +++ | +++ | ++ | ++ | Good |

In this assay, 81-1 and 81-2 had a decrease in staining intensity when comparing untreated versus treated xenografts. For supernatant 81-24, there was a decrease in intensity when comparing untreated versus treated xenografts, but it was not very strong. For 81-84, 81-85 supernatants, there was a decrease in intensity when comparing untreated versus treated xenografts, but there was some background in the negative control. For 81-99, there was a difference between treated and untreated. Staining was very strong, although significant background was observed. However, with purification and titration, it has the possibility to give the staining differential needed. The above supernatants were judged suitable for further consideration. The following supernatants were judged unsuitable for pursuing in further studies: For 81-54 supernatant, treated had some unspecific staining, also staining by this supernatant was very weak on tissue. For supernatant 81-19, the treated xenograft had some unspecific staining. For supernatant 81-96, there was no difference between untreated and treated. Also the staining was very weak.

In summary, from this IHC assay, two supernatants were eliminated as having bad background and one was eliminated as having weak staining. The remaining six supernatants were ranked: 1) 81-1, 2) 81-2, 3) 81-99, 4) 81-24, 5) 81-85 and 6) 81-84.

Following the multiclone supernatant evaluation, three of the p-Y525-SYK specific multiclones, 81-1, 81-2 and 81-99, were sub-cloned to generate 12 subclones each. Subcloning was done using the limited cell dilution method. Several subclone supernatatants were screened by AlphaLISA and IHC.

AlphaLISA assays of the subclones were performed in duplicate using 25 μl of a 2nM SYK sample diluted by ½ in final assay conditions sample (either recombinant in vitro autophosphorylated sample or with the same sample treated with a protein phosphatase , 5 μl of hybridoma supernatant and 25 μg/ml of glutathione donor (PerkinElmer: # 6765300) and Protein A acceptor beads (PerkinElmer #6760137M) in HiBlock (PerkinElmer #AL004C) for 2 hrs at room temperature. The results are in the table below.

| Subclone | IVK-pSYK | PPase-pSYK | Subclone | IVK-pSYK | PPase-pSYK | Subclone | IVK-pSYK | PPase-pSYK |
|---|---|---|---|---|---|---|---|---|
| 1-1 | 246449 | 12882 | 2-1 | 224789 | 3487 | 99-1 | 175019 | 6403 |
| 1-2 | 248596 | 14336 | 2-2 | 156294 | 3297 | 99-2 | 216581 | 8284 |
| 1-3 | 256130 | 12844 | 2-3 | 62957 | 627 | 99-3 | 166241 | 7990 |
| 1-4 | 162498 | 8883 | 2-4 | 131138 | 1501 | 99-4 | 206473 | 6536 |
| 1-5 | 199377 | 12379 | 2-5 | 182353 | 2926 | 99-5 | 187068 | 10070 |
| 1-6 | 214662 | 12322 | 2-6 | 18972 | 333 | 99-6 | 183559 | 9880 |
| 1-7 | 288050 | 12132 | 2-7 | 207110 | 1359 | 99-7 | 231278 | 7477 |
| 1-8 | 248948 | 13129 | 2-8 | 220619 | 1064 | 99-8 | 193724 | 5007 |
| 1-9 | 248226 | 8569 | 2-9 | 3553 | 418 | 99-9 | 258514 | 589 |
| 1-10 | 299203 | 17138 | 2-10 | 81311 | 399 | 99-10 | 209124 | 1406 |
| 1-11 | 265962 | 17813 | 2-11 | 182220 | 1929 | 99-11 | 267378 | 4123 |
| 1-12 | 268223 | 16198 | 2-12 | 169936 | 1121 | 99-12 | 240949 | 4703 |

Many of the subclones looked good in this assay (strong signal on the IVK treated sample and much weaker signal on the phosphatase treated sample). The subclones from multiclone 2 produced the lowest background levels.

IHC assays of the subclones were performed using Ventana Discovery XT following staining protocol followed a general protocol which did not use heat for incubating the antibody and OMNIMAP™ anti-rabbit HRP reagent (Ventana 760-4311) for all subclones and IHC using pSYK Y525/526 antibody, Epitomics 2175-1, was stained following a general protocol which used heat for incubating the antibody and ULTRAMAP™ anti-rabbit HRP reagent (Ventana 760-4315) as described in fourth level screening above). Samples Neg Control ITK-Ras FFPE cell pellet, Positive control (Neg. C.) WSU-DLCL Fab crosslinked FFPE cell pellet (Pos. C.).

The semiquantitative results of IHC screening of the subclones are in the table below.

| Subclone | Neg. C. | Pos. C. | Subclone | Neg. C. | Pos. C. | Subclone | Neg. C. | Pos. C. |
|---|---|---|---|---|---|---|---|---|
| 1-1 | + | +++ | 2-1 | - | ++ | 99-1 | +/- | +++ |
| 1-2 | + | +++ | 2-2 | - | ++ | 99-2 | +/- | +++ |
| 1-3 | +/- | +++ | 2-3 | - | ++ | 99-3 | + | +++ |
| 1-4 | +/- | +++ | 2-4 | - | ++ | 99-4 | + | +++ |
| 1-5 | +/- | +++ | 2-5 | +/- | ++ | 99-5 | + | +++ |
| 1-6 | +/- | +++ | 2-6 | - | ++ | 99-6 | + | +++ |
| 1-7 | +/- | +++ | 2-7 | - | ++ | 99-7 | + | +++ |
| 1-8 | - | +++ | 2-8 | +/- | ++ | 99-8 | + | +++ |
| 1-9 | + | +++ | 2-9 | - | + | 99-9 | +/- | +++ |
| 1-10 | +/- | +++ | 2-10 | +/- | ++ | 99-10 | +/- | +++ |
| 1-11 | + | +++ | 2-11 | - | ++ | 99-11 | +/- | +++ |
| 1-12 | +/- | +++ | 2-12 | - | ++ | 99-12 | +/- | +++ |

Taking into account strength of staining on the positive control compared to staining on the negative control, the subclones were ranked for each multiclone:

Multiclone 81-1 subclones, best to worst: 8, 10, 12, 5, 6, 7, 3, 4, 11, 1, 2, 9.
Multiclone 81-2 subclones, best to worst: 1, 2, 3, 4, 6, 7, 10, 11, 12, 5, 8, 9.
Multiclone 81-99 subclones, best to worst: 1, 10, 11, 12, 2, 9, 3, 4, 5, 6, 7, 8.

The hybridoma cells for subclones #81.1-8, 81.2-1 and 81.99-1 were selected for large scale-up and purification.

Hybridomas were grown in suspension in either CELLINE™ two-compartment disposable bioreactors (Integra Biosciences Corp., Hudson, NH) or standard tissue culture flasks. Culture supernatant underwent one-step purification (protein A chromatography followed by buffer exchange) to generate antibody for the later studies.

Final selection of antibody was performed by qualitative and quantitative assessment of IHC results of staining pSYK in PHTX 95L xenografts from untreated or SYK inhibitor-treated mice described above using the Ventana Discovery XT autostainer and the same staining protocols as previous assays (see Figure 2).

Quantitative analysis was performed with Aperio Spectrum analysis following Postitive Pixel Count v9 Algorithm at 20x magnification. The Positive pixel analysis was used rather than a signal-to-noise ratio because of program difficulties defining true cytoplasmic staining from background for positive amounts below 5%. The table below has the results of the quantitive analysis.

Table Quantifying the ratio of Percent Positive Pixel per total Pixel (Figure 3)

| Antibody | PHTX 95L Untreated | PHTX95L SYK Inhibitor |
|---|---|---|
| Epitomics 2175-1 | 8.21 | 0.56 |
| MIL81-1-8 | 25.19 | 11.30 |
| MIL81-2-1 | 22.28 | 15.50 |
| MIL81-99-1 | 15.27 | 9.92 |

Example 5: MIL81-1-8 Immunohistochemistry Studies

An IHC assay using the MIL81-1-8 antibody was developed to evaluate pSYK expression in HBL1 xenograft tissue and several primary human tissue xenografts (PHTX-95L, OCI LY10) derived from diffuse large B-cell lymphoma (DLBCL) patient samples in female SCID mice. pSYK protein levels in Formalin-Fixed, Paraffin-Embedded (FFPE) tissues were assessed on 5 m thick sections and incubated with MIL81-1-8 antibody (3.5 g/mL) for 1 hour on the Ventana Medical Systems (Tucson, AZ) Discovery XT automated stainer. Antibodies were incubated with OmniMap goat anti-rabbit HRP secondary antibody (Ventana Medical Systems) for 16 minutes and developed with the 3,3'-diaminobexidine (DAB) substrate map system. Slides were counterstained with hematoxylin and imaged using the Aperio whole slide scanning system (XT).

As noted above, pSYK levels differed significantly among these tissues with the highest scores in PHTX-95L (Figure 7). Stains of PHTX-95L xenografts using the commercially available antibody Epitomics pSYK Catalog No. 2175-1 (Y525/526) revealed that all 3 MIL81 clones tested performed significantly better than Epitomics 2175-1, with MIL81-1-8 performing best. Figure 3. As seen in Figures 3 and 4, MIL81-1-8 performance was superior to Epitomics 2175-1 due to its intensity and signal to noise ratio.

Similar results were obtained with the MIL81-1-8 subclone using an automated protocol on a Leica automated stainer. pSYK protein levels in Formalin-Fixed, Paraffin-Embedded tissues were incubated with MIL81-1-8 antibody (3.5 g/mL) for 30 minutes on the Leica Microsystems (Wetzlar, Germany) BOND RX automated stainer. Antibodies were incubated with a rabbit anti-goat HRP secondary antibody (Leica Laboratories) and developed with the 3,3'-diaminobexidine (DAB) substrate map system. Slides were counterstained with hematoxylin and imaged using the Aperio whole slide scanning system (XT). MIL81-1-8 antibody was compared to commercial pSYK antibody Epitomics pSYK Catalog No. 2175-1 (Y525/526) for staining specificity. MIL81-1-8 was able to be used at a higher dilution/lower concentration and had lower background (see Figure 4 staining a PHTX95L xenograft; MIL81-1-8 at 1:3200 minimal background; 2175 at 1:25, high background). This high background by 2175 was not eliminated by treatment of xenograft-bearing mice with a SYK inhibitor (Figure 5-HBL1 xenograft; Figure 6-Ly10 xenograft; summarized in Figure 7). The 2175 antibody was unable to distinguish tumors from untreated and Syk-inhibitor treated HBL1 or Ly-10 tumors (Figure 7). However, the MIL81-1-8 pSYK antibody was able to specifically detect the presence of pSYK in the medium- (HBL1) and low-expressing (Ly10) xenografts. A detectable trend toward reduction of pSYK staining also was seen by the anti-pSYK MIL81-1-8 antibody in medium- and low-expressing xenograft samples from mice treated with a SYK inhibitor (Figures 5 to 7).

The MIL81-1-8 staining judged as positive, specific for pSYK was cytoplasmic (see, e.g., Figures 3, 12). Sometimes, a nuclear stain is detected with MIL81-1-8 antibody. This staining is considered to be off-target because it is not detected in samples stained with total SYK antibody (Figure 8). This phenomenon is not limited to pSYK antibody, because it also was seen with anti-SYK p323 antibody (Figure 8). Nuclear staining by MIL81-1-8 antibody also was seen in old (at least 3 years old) archived samples, such as cores from tissue microarrays (see Figure 9-DLBCL TMA), cells from the interior of tissue samples (Figures 11 and 13) and samples from normal lymphoid tissues (spleen on Figure 10, lymph node on Figure 20). This periphery sample cytoplasmic staining-interior sample nuclear staining also was seen with SYK pY323 antibody (Figures 12 vs 8 and 13). Samples tested the instant study included samples that were cut and stored and samples that were cut fresh. The stability of the antigen in tissue samples and cut sections and the freshness of the cut samples over time will be considered for further studies.Other off-target staining by MIL81-1-8 antibody also was seen on xenografts of H1650 cell line (lung adenocarcinoma) (Figures 16 and 17), which were negative for SYK RNA (not shown). The total SYK antibody did not bind these cells, even at 4x concentration (1:100 instead of the typical 1:400 dilution, Figure 16). Other antibodies to an epitope of phosphorylated residues, such as anti-SYK pY323 and anti-pSYK (CST 2711), showed this staining which included membranous as well as cytoplasmic staining.

Validation of the specificity MIL81-1-8 on lymphoma cells used a pellet of pervanadate-treated WSU-DLCL cells, PHTX-95L xenograft or a TMA core of a DLBCL tumor. When peptide immunogen was added to the antibody prior to incubation with the tissue, the pSYK IHC staining was blocked (Figure 9). Another validation of the specificity of MIL81-1-8 staining used phosphatase treatment of the slides prior to antibody incubation. The phosphatase treatment eliminated epitopes for the pSYK antibody to bind, except for the nuclear staining of normal spleen (Figure 10). Due to this nuclear staining of phosphatase-treated samples, staining of only nuclei in samples was generally judged to be off-target and thus pSYK-negative.

Based on the results of the initial IHC experiments described above, MIL81-1-8 staining is much stronger and cleaner in recently biopsied DLBCL tissues than Epitomics 2175-1 giving MIL81-1-8 greater probability of identifying low pSYK Y525/6 expressing patients (Figures 14 and 15).

Example 6: Molecular Cloning of MIL81-1-8

The MIL81-1-8 antibody was cloned into a recombinant vector for production by transient transfection in mammalian cells and for sequencing.

cDNAs for both variable regions of heavy and light chains were RT-PCR amplified and cloned into pTT5 vector (National Research Council of Canada, see U.S. Pat. Application Publication Nos. 20050170450 or 20100261275) for production by transient transfection in mammalian cells and for sequencing. Five clones were produced. The plasmid is amplified in E. coli dH5$\alpha$ bacteria to to obtain sufficient material for transfection into mammalian cells (U.S. Patent No. 8,551,774). The plasmid holds only one chain, so a plasmid comprising the light chain is co-transfected with a plasmid comprising the heavy chain. After initial small scale transfection in 293 cells, the culture supernatants were tested by ELISA and sequenced. All the clones had the same sequences.

Binding activity of the molecular clone antibody (MIL81-1-8 3H3/3L2) was confirmed by western blot.

Samples included on the blot were GST-tagged recombinant SYK produced in the baculo/Sf9 system. The recombinant SYK was either untreated, treated with phosphatase or subjected to the in vitro kinase reaction (IVK) as described above. Other samples included lysates from WSU-DLCL2 cells, either resting or stimulated by 10 min crosslinking of the B-cell receptor. All samples bound the anti-hSYK antibody (Epitomics #1688). The positive control Epitomics 2175-1 bound all except the phosphatase-treated recombinant SYK and the unstimulated WSU-DLCL2 samples. The IVK sample had a stronger band than the untreated recombinant SYK. The molecular clone of the 81-1-8 antibody also stained the IVK-treated SYK band more intensely than the untreated SYK band. This antibody also stained the band from stimulated WSU-DLCL2 cells (and some extra bands). It slightly stained the band from phosphatase and the unstimulated WSU-DLCL2 cells.

The molecular clone antibody also was assessed by IHC. The immunohistochemical staining of antibody purified from MIL81-1-8 hybridoma supernatant as described above was compared to staining by MIL81-1-8 3H3/3L2 clone antibody purified from culture supernatant of transiently co-transfected HEK293 cells (also one-step purification -protein A chromatography followed by buffer exchange).

The samples used to compare these antibodies included WSU-DLBCL Cell pellets, Xenograft samples from TMD8 lymphoma cell line (DLBCL)-implanted mice (untreated or treated by a single dose of an inhibitor of SYK kinase activity (Compound A) administered orally at 120 mg/kg. Two hours after dosing, tumors were collected for 10% formalin fixation and processing for IHC.)

The molecular clone stains untreated samples and the membranous intensity is minutely weaker than the hybridoma in the treated samples (Figure 18). For both antibodies, the stain was diminished on the samples from Compound A treated TMD8 xenograft mice.

In another study was a comparison of staining samples which included an Epstein-Barr virus negative B-cell lymphoma cell line HBL1, xenograft samples from PHTX95L-implanted mice, untreated or treated with the SYK inhibitor as described above.

Both the molecular clone and the hybridoma antibodies stained HBL1 to a moderate extent and strongly stained PHTX95L untreated samples. For both antibodies, the stain was diminished on the samples from Compound A treated PHTX95L xenograft mice (Figure 19). The molecular clone antibody was compared to the hybridoma antibody for staining biopsies of human tissues. There were three DLBCL patient samples and one normal lymph node sample (non-tumorigenic) from a patient. The molecular clone nuclear intensity is remarkably reduced compared to staining by the hybridoma antibody, allowing for more cytoplasmic and membranous stain to be seen (Figure 20). If nuclear staining would be considered off-target and lead to a determination of absence of pSYK, then a version of MIL81-1-8, with less nuclear relative to cytoplasmic or membranous staining could lead to fewer false negative results.

In summary, the molecular clones staining pattern appears to be very similar to the hybridoma staining pattern. All the same regions and areas are staining for all cell pellets, xenografts and human biopsies tested. The molecular clone does appear to stain the membrane with a slightly weaker intensity, but its off-target nuclear staining is remarkably reduced. This allows easier viewing of the cytoplasmic and membrane staining. Overall, the molecular clone antibody appears to be slightly better than the hybridoma antibody.

Example 7: Compound A Demonstrates Anti-tumor Activity in DLBCL Models by Inhibiting SYK

Compound A is an investigational inhibitor of SYK that is currently being evaluated in a Phase I clinical trial. Compound A inhibits SYK with an IC50 of 3.2nM and has the ability to inhibit cellular proliferation in a subset of relevant models with an EC50 between 25 to 400 nM. In an expanded set of relevant models, Compound A was found to inhibit cellular proliferation with an EC50 between 25 nM to 4 μM (Figure 21).

| | Enzyme | | | | |
|---|---|---|---|---|---|
| Activity/$IC_{50}$ (nM) | SYK | FLT3 | ZAP70 | VEGFR | JAK3 |
| Compound A | 3.2 | 4.6 | 87 | 140 | 110 |

Daily oral administration of 60 mg/kg Compound A showed anti-tumor activity in DLBCL cell-line xenograft models representing ABC (OCI-LY-10 (TGI 50%), HBL-1 and a primary human tumor xenograft model, PHTX-95L (TGI 70% at 120 mg/kg), GCB (OCI-LY-19 (TGI 37%)) and non-ABC/GCB (WSU (TGI approximately 50%)) subtypes.

In another set of experiments, Compound A was administered orally once daily for 21 days to SCID mice bearing OCI-LY10 ABC DLBCL xenografts (Figure 22), PHTX-95L primary DLBCL tumor xenografts (Figure 23), and orally once daily for 14 days to femle SCID mice bearing OCI-LY19 GCB DLBCL xenografts (Figure 24). Mean tumor volumes ($mm^3$) ± SEM (N=8/group) are shown where treatment was initiated when mean tumor volume was approximately 200 mm3.

| Model | 60 mg/kg | 120 mg/kg |
|---|---|---|
| PHTX-95L (primary DLBCL) | 56% | 70% |
| OCI-LY10 (ABC) | 50% | 59% |
| HBL-1 (ABC) | 32% | 36% |
| TMD8 (ABC) | 45%* | - |
| WSU | 52% | - |
| OCI-LY19 (GCB) | 33%** | - |
| *TGI calculated on Day 14<br>**TGI calculated on Day 16 | | |

Interestingly, in the OCI-LY-19 GCB-type DLBCL model, 60 mg/kg Compound A showed increased activity over a BTK inhibitor (TGI 15%) suggesting the hypothesis that inhibition of BCR signaling upstream of BTK could be beneficial in treating a broader range of subtypes of B-cell malignancies (Figure 24).

The time course of pSYK (pSYK525) and pBLNK (pBLNK65) expression were assessed following single doses of 120 mg/kg of Compound A in NOD SCID mice bearing PHTX-95L primary DLBCL xenografts. Tumors were harvested over time, paraffin-embedded, and stained by immunohistochemistry for pSYK, pBLNK, and cleaved caspase 3 (Cl-Casp-3). Time dependent inhibition of these phospho-proteins and also increase in expression of cleaved caspase 3, an apoptosis marker, was observed in the DLBCL models studied here. Phenotypic assessment of 15 primary DLBCL samples for pSYK and other relevant pathway markers revealed that SYK activation occurs in a considerable number of molecularly heterogeneous DLBCL samples and is consistently associated with activation of the BCR pathway. These results together suggest that SYK activation occurs in various subsets of DLBCL samples and Compound A showed activity in pre-clinical models of the various subtypes of DLBCL.

Example 8: Generation of Amplified DAB IHC DLBCL Patient Selection Assay

An IHC assay using the Mil-81-1-8 3H3/3L2 clone and the Ventana Amp HQ DAB amplication system was developed to increase the staining intensity and decrease nonspecific staining of pSYK expression in human DLBCL biopsy samples as well as several primary human tissue xenografts (PHTX-95L, OCI-LY10). pSYK protein levels in Formalin-Fixed, Paraffin-Embedded (FFPE) tissues were assessed on 5 μm thick sections and incubated with MIL81-1-8 3H3/3L2 antibody (0.05 μg/mL) for 1 hour on the Ventana Medical Systems (Tucson, AZ) Discovery XT automated stainer. Antibodies were incubated with OmniMap goat anti-rabbit HRP secondary antibody (Ventana Medical Systems) for 16 minutes. A Discovery AMP TSA HQ kit (Ventana Medical Systems) was applied for 16 minutes followed by 16 minute incubation with the Discovery anti-HQ HRP (Ventana Medical Systems) antibody. The staining was then developed with the 3,3'-diaminobexidine (DAB) substrate map system. Slides were counterstained with hematoxylin and imaged using the Aperio whole slide scanning system (XT). A comparative staining was obtained with the MIL81-1-8 3H3/3L2 antibody using an automated protocol on a Leica automated stainer. pSYK protein levels in Formalin-Fixed, Paraffin-Embedded tissues were incubated with MIL81-1-8 3H3/3L2 antibody (0.16 g/mL) for 30 minutes on the Leica Microsystems (Wetzlar, Germany) BOND RX automated stainer. Antibodies were incubated with a rabbit anti-goat HRP secondary antibody (Leica Laboratories) and developed with the 3,3'-diaminobexidine (DAB) substrate map system. Slides were counterstained with hematoxylin and imaged using the Aperio whole slide scanning system (XT). The staining of the AMP HQ and the Leica IHC were compared to determine staining specificity. The AMP HQ was able to identify membrane staining with less cytoplasmic background, even when used at a higher dilution (standard (Leica) IHC used 1:3200 dilution and amplified (AMP HQ) IHC used 1:10,000 dilution)(see Figure 25 staining PHTX-95L). AMP HQ allowed clear assessment of the reduction of staining in a sample of primary human xenograft from an animal treated with (Compound A) compared to vehicle. Also the nuclear off target staining see in human DLBCL biopsies was greatly reduced allowing the determination of positive staining regions to be more easily identified (Figure 26). Serial sections of 82 human DLBCL biopsies were stained with both AMP HQ and Leica IHC. Each was scored either positive (membrane stain was found) or negative (no membrane staining was found). Out of these 82 samples, 24 were deemed positive by Leica IHC and 34 were deemed positive by AMP HQ. The amplified method identified ten more positive samples and missed only one sample identified by standard IHC as pSYK positive. When analyzing samples from DLBCL subtypes (germ cell B lymphocyte (GCB) or non-GCB), the amplified IHC system identified 24 of 28 samples as non-GCB, typically found to have activated SYK. The standard IHC system identified only 23 of 37 samples as non-GCB.

Table: Presence of membrane staining (+ or -)

| IHC | | | | | Sub-typing | | |
|---|---|---|---|---|---|---|---|
| | | + | - | Total | GCB | Non-GCB | Total |
| Amp HQ | + | 23 | 11 | 34 | 4 | 24 | 28 |
| | - | 1 | 47 | 48 | 14 | 23 | 37 |
| | Total | 24 | 58 | 82 | 18 | 47 | 65 |

Based on the results of this AMP HQ staining experiment described above, AMP HQ MIL81-1-8 staining was determined to be much stronger and cleaner in human DLBCL biopsied tissue and DLBCL xenograft tissue than the Leica IHC method, increasing the ability to identify pSYK Y525/6 expressing patients.

Example 9: Generation of Amplified Immunofluorecent AML Patient Selection Assay

An Ampilified Immunofluorescent AML patient selection assay was developed using peripheral blood mononuclear cells (PBMC) extracted from whole blood using a FICOLL® solution (Amersham Biosciences division of GE healthcare, Piscataway, NJ) and centrifugation at 1800 rpm for 22 minutes. The cloudy white layer was then extracted from the rest and fixed in 10% neutral buffered formalin for a minimum of 12 hours. The cells were embedded in histogel and then processed into a paraffin block. These AML PBMC blocks were assessed on 5 μm sections and incubated with MIL81-1-8 3H3/3L2 (0.10 μg/mL) for 1 hour on the Leica Bond RX (Leica Microsystems, Wetzlar, Germany) automated stainer. Antibodies were biotinylated with a goat anti-rabbit secondary antibody (Vector Labrotories). This biotinylated complex was then stained with a Tyramide Signal Amplification (TSA) molecule conjugated to a Alexa Fluor® 488 (Life Technologies) which convalently bound to the tissue immediately adjacent to the pSYK Y525/6 antigen. The tissue was heated to 95°C in a solution of pH 6.0 sodium citrate which stripped off the MIL81-1-8 3H3/3L2 and biotinylated secondary leaving only the convalently bound TSA and Alexa Fluor®. The slide was incubated with a cocktail of CD34 and CD117 commercially available antibodies (CD34 ab81289 and CD117 ab32363, Abcam, Cambridge, MA) for 1 hour. The antibodies were biotinylated with a goat anti-rabbit secondary antibody (Vector Labrotories). This biotinylated complex was then stained with a Tyramide Signal Amplification (TSA) molecule conjugated to Alexa Fluor® 594 (Life Technologies) which convalently bound to the tissue immediately adjacent to the CD34/CD117 antigens. The nuclear dye Hoeschst 33342 was added for 5 minutes allowing the cell nuclei to be visible. The slides were coverslipped using Prolong Gold (Life Technologies) mounting media and imaged with either Nikon WSI microscope or Aperio whole slide scanner (FL). The CD34/CD117 staining identified the AML tumor blasts and the cells that colocalized with MIL81-1-8 3H3/3L2 showed the AML tumor blasts that were pSYK Y525/6 upregulated. Analysis was done using 6 fields of view and Definiens Imgaing Analysis Software (Definiens, Munich, Germany).

Validation of the specificity of this technique was done using MV-4-11 AML cell pellet (known to have low CD34 expression), NCI-H82 lung cell pellet (negative control for pSYK and CD34/CD117), and KG-1 xenografts (positive control for pSYK and CD34/117). MV-4-11 showed high levels of pSYK and low levels of CD34/CD117, NCI-H82 showed no staining with either anibody, and the KG-1 xenograft showed high levels of both pSYK Y525/6 and CD34/CD117 which were the expected results (Figure 27).

Out of the 11 AML PBMCs collected 4 of these were identified to have an elevated level of pSYK Y525/6. Based on the results of the initial IF experiments described above, through the use of a dual MIL81-1-8 CD34/CD117 IF assay, a PBMC sample with elevated pSYK Y525/526 could be identified.

**Claims**

1. An anti-phospho-spleen tyrosine kinase (pSYK) antibody molecule comprising three heavy chain complementarity determining regions (CDR1, CDR2, and CDR3) comprising amino acid sequences SEQ ID NOs:11, 12 and 13, respectively; and three light chain complementarity determining regions (CDR1, CDR2, and CDR3) comprising amino acid sequences SEQ ID NOs:14, 15 and 16, respectively.

**2.** The anti-pSYK antibody molecule of claim 1, wherein said anti-pSYK antibody molecule is a monoclonal antibody.

**3.** The anti-pSYK antibody molecule of claim 1, wherein said anti-pSYK antibody molecule is a rabbit or rabbit-derived antibody, preferably a rabbit monoclonal antibody.

**4.** The anti-pSYK antibody molecule of claim 1, comprising a heavy chain variable region comprising an amino acid sequence according to SEQ ID NO:8, and a light chain variable region comprising an amino acid sequence according to SEQ ID NO:10.

**5.** The anti-pSYK antibody molecule of any one of claims 1 to 4, wherein said anti-pSYK antibody molecule is conjugated to a detectable label.

**6.** The anti-pSYK antibody molecule of claim 5, wherein said detectable label is:

(i) selected from the group consisting of horseradish peroxidase (HRP), alkaline phosphatase, galactosidase, glucoamylase, lysozyme, saccharide oxidases, heterocyclic oxidases, coupled with an enzyme that employs hydrogen peroxide to oxidize a dye, biotin/avidin, spin labels, bacteriophage labels, and stable free radicals;
(ii) a fluorophore selected from fluorescein or a derivatives thereof, rhodamine or a derivative thereof, dansyl, umbelliferone, a luciferase, luciferin, and 2,3-dihydrophthalazinediones; or
(iii) a radioactive agent selected from the group consisting of $^{32}P$, $^{3}H$, $^{14}C$, $^{188}Rh$, $^{43}K$, $^{52}Fe$, $^{57}Co$, $^{67}Cu$, $^{67}Ga$, $^{68}Ga$, $^{77}Br$, $^{81}Rb/^{81M}Kr$, $^{87M}Sr$, $^{99}Tc$, $^{111}In$, $^{113M}In$, $^{123}I$, $^{125}I$, $^{127}Cs$, $^{129}Cs$, $^{131}I$, $^{132}I$, $^{197}Hg$, $^{203}Pb$, $^{206}Bi$, and $^{213}Bi$.

**7.** An isolated nucleic acid sequence that encodes the anti-pSYK antibody molecule of any one of claims 1 or 4.

**8.** A cell comprising the isolated nucleic acid sequence of claim 7.

**9.** A method of producing the anti-pSYK antibody molecule of any one of claims 1 or 4, comprising culturing the cell of claim 8 under conditions that allow production of the anti-pSYK antibody molecule.

**10.** A SYK-targeted therapeutic agent for use in a method of treating a patient having a disease **characterized by** one or more pSYK-expressing cells, said method comprising:

a. detecting pSYK protein expression in a biological sample obtained from the patient using a method comprising:

contacting the biological sample with the anti-pSYK antibody molecule of any one of claims 1 to 7; and detecting formation of a complex between the anti-pSYK antibody molecule and pSYK protein; and

b. administering the SYK-targeted therapeutic agent to the patient if the biological sample expresses pSYK.

**11.** The SYK-targeted therapeutic agent for use of claim 10, comprising 6-((1S,2R)-2-aminocyclohexylamino)-7-fluoro-4-(1-methyl-1H-pyrazol-4-yl)-1H-pyrrolo[3,4-c]pyridin-3(2H)-one or a pharmaceutically acceptable salt thereof.

**12.** The SYK-targeted therapeutic agent for use of claim 10 or 11, wherein the patient is a cancer patient, and wherein the biological sample is a sample from tumor cells obtained from the cancer patient.

**13.** The SYK-targeted therapeutic agent for use of claim 10 or 11, wherein said disease is acute myeloid leukemia, or diffuse large B-cell lymphoma (DLBCL).

**14.** A method of evaluating the pharmacodynamics of a SYK-targeted therapy, said method comprising the steps of:

a. contacting the anti-pSYK antibody molecule of any one of claims 1 to 6 with a biological sample comprising one or more cells suspected of expressing pSYK from a patient who has received the SYK-targeted therapy according to a dosing regimen;
b. detecting formation of a complex between the anti-pSYK antibody molecule and pSYK protein in the biological sample;
c. quantifying pSYK expression in the biological sample to determine a pSYK expression level; and

d. comparing the pSYK expression level against a database comprising the SYK-targeted therapy.

**15.** The method of claim 14, further comprising adjusting the dosing regimen based on the pSYK expression level.

**Patentansprüche**

**1.** Anti-Phospho-Milz-Tyrosinkinase-(pSYK)-Antikörpermolekül, umfassend drei Komplementarität-bestimmende Regionen der schweren Kette (CDR1, CDR2 und CDR3), umfassend die Aminosäuresequenzen SEQ ID NRn.: 11, 12 bzw. 13; und drei Komplementarität-bestimmende Regionen der leichten Kette (CDR1, CDR2 und CDR3), umfassend die Aminosäuresequenzen SEQ ID NRn.: 14, 15 bzw. 16.

**2.** Anti-pSYK-Antikörpermolekül nach Anspruch 1, wobei das Anti-pSYK-Antikörpermolekül ein monoklonaler Antikörper ist.

**3.** Anti-pSYK-Antikörpermolekül nach Anspruch 1, wobei das Anti-pSYK-Antikörpermolekül ein Kaninchen-Antikörper oder ein von einem Kaninchen-stammender Antikörper, vorzugsweise ein monoklonaler Kaninchen-Antikörper, ist.

**4.** Anti-pSYK-Antikörpermolekül nach Anspruch 1, umfassend eine variable Region einer schweren Kette, umfassend eine Aminosäuresequenz gemäß SEQ ID NR.: 8, und eine variable Region einer leichten Kette, umfassend eine Aminosäuresequenz gemäß SEQ ID NR.: 10.

**5.** Anti-pSYK-Antikörpermolekül nach einem der Ansprüche 1 bis 4, wobei das Anti-pSYK-Antikörpermolekül mit einer nachweisbaren Markierung konjugiert ist.

**6.** Anti-pSYK-Antikörpermolekül nach Anspruch 5, wobei die nachweisbare Markierung ist:

(i) ausgewählt aus der Gruppe, bestehend aus Meerrettichperoxidase (HRP), alkalischer Phosphatase, Galactosidase, Glucoamylase, Lysozym, Saccharidoxidasen, heterozyklischen Oxidasen, gekoppelt mit einem Enzym, das Wasserstoffperoxid verwendet, um einen Farbstoff zu oxidieren, Biotin/Avidin, Spinmarkierungen, Bakteriophagenmarkierungen und stabilen freien Radikalen;
(ii) ein Fluorophor, ausgewählt aus Fluorescein oder einem Derivat davon, Rhodamin oder einem Derivat davon, Dansyl, Umbelliferon, einer Luciferase, Luciferin und 2,3-Dihydrophthalazindionen; oder
(iii) ein radioaktives Mittel, ausgewählt aus der Gruppe, bestehend aus $^{32}P$, $^{3}H$, $^{14}c$, $^{188}Rh$, $^{43}K$, $^{52}Fe$, $^{57}Co$, $^{67}Cu$, $^{67}Ga$, $^{68}Ga$, $^{77}Br$, $^{81}Rb$/$^{81M}Kr$, $^{87M}Sr$, $^{99}Tc$, $^{111}In$, $^{113M}In$, $^{123}I$, $^{125}I$, $^{127}Cs$, $^{129}Cs$, $^{131}I$, $^{132}I$, $^{197}Hg$, $^{203}Pb$, $^{206}Bi$ und $^{213}Bi$.

**7.** Isolierte Nukleinsäuresequenz, die für das Anti-pSYK-Antikörpermolekül nach einem der Ansprüche 1 oder 4 kodiert.

**8.** Zelle, umfassend die isolierte Nukleinsäuresequenz nach Anspruch 7.

**9.** Verfahren zur Herstellung des Anti-pSYK-Antikörpermoleküls nach einem der Ansprüche 1 oder 4, umfassend das Kultivieren der Zelle nach Anspruch 8 unter Bedingungen, die die Herstellung des Anti-pSYK-Antikörpermoleküls ermöglichen.

**10.** Auf SYK abgezieltes therapeutisches Mittel zur Verwendung in einem Verfahren zur Behandlung eines Patienten, der eine Krankheit aufweist, die durch eine oder mehrere pSYKexprimierende Zellen gekennzeichnet ist, wobei das Verfahren umfasst:

a. Nachweisen der pSYK-Proteinexpression in einer vom Patienten erhaltenen biologischen Probe unter Verwendung eines Verfahrens, umfassend:

Kontaktieren der biologischen Probe mit dem Anti-pSYK-Antikörpermolekül nach einem der Ansprüche 1 bis 7; und
Nachweisen der Bildung eines Komplexes zwischen dem Anti-pSYK-Antikörpermolekül und dem pSYK-Protein; und

b. Verabreichen des auf SYK abgezielten therapeutischen Mittels an den Patienten, insoweit die biologische Probe pSYK exprimiert.

11. Auf SYK abgezieltes therapeutisches Mittel zur Verwendung nach Anspruch 10, umfassend 6-((1S,2R)-2-Aminocyclohexylamino)-7-fluor-4-(1-methyl-1H-pyrazol-4-yl)-1H-pyrrolo[3,4-c]pyridin-3(2H)-on oder ein pharmazeutisch verträgliches Salz davon.

12. Auf SYK abgezieltes therapeutisches Mittel zur Verwendung nach Anspruch 10 oder 11, wobei der Patient ein Krebspatient ist und wobei die biologische Probe eine Probe aus Tumorzellen, die von einem Krebspatienten erhaltenen wurden, ist.

13. Auf SYK abgezieltes therapeutisches Mittel zur Verwendung nach Anspruch 10 oder 11, wobei die Krankheit akute myeloische Leukämie oder diffuses großes B-Zell-Lymphom (DLBCL) ist.

14. Verfahren zur Bewertung der Pharmakodynamik einer auf SYK abgezielten Therapie, wobei das Verfahren die Schritte umfasst:

a. Kontaktieren des Anti-pSYK-Antikörpermoleküls nach einem der Ansprüche 1 bis 6 mit einer biologischen Probe, die eine oder mehrere Zellen umfasst, die im Verdacht stehen, pSYK zu exprimieren, wobei die Probe von einem Patienten ist, der die auf SYK abgezielte Therapie gemäß einem Dosierungsschema erhalten hat;
b. Nachweisen der Bildung eines Komplexes zwischen dem Anti-pSYK-Antikörpermolekül und dem pSYK-Protein in der biologischen Probe;
c. Quantifizieren der pSYK-Expression in der biologischen Probe zur Bestimmung eines pSYK-Expressionsniveaus; und
d. Vergleichen des pSYK-Expressionsniveaus mit einer Datenbank, die die auf SYK abgezielte Therapie umfasst.

15. Verfahren nach Anspruch 14, weiter umfassend die Anpassung des Dosierungsschemas auf der Grundlage des pSYK-Expressionsniveaus.

## Revendications

1. Molécule d'anticorps anti-phospho-tyrosine kinase splénique (pSYK) comprenant trois régions déterminant la complémentarité de la chaîne lourde (CDR1, CDR2 et CDR3) comprenant les séquences d'acides aminés SEQ ID NOs: 11, 12 et 13, respectivement; et trois régions déterminant la complémentarité de la chaîne légère (CDR1, CDR2 et CDR3) comprenant les séquences d'acides aminés SEQ ID NOs: 14, 15 et 16, respectivement.

2. Molécule d'anticorps anti-pSYK selon la revendication 1, ladite molécule d'anticorps anti-pSYK étant un anticorps monoclonal.

3. Molécule d'anticorps anti-pSYK selon la revendication 1, ladite molécule d'anticorps anti-pSYK étant un anticorps de lapin ou dérivé de lapin, de préférence un anticorps monoclonal de lapin.

4. Molécule d'anticorps anti-pSYK selon la revendication 1, comprenant une région variable de chaîne lourde comprenant une séquence d'acides aminés selon SEQ ID NO: 8, et une région variable de chaîne légère comprenant une séquence d'acides aminés selon SEQ ID NO: 10.

5. Molécule d'anticorps anti-pSYK selon l'une quelconque des revendications 1 à 4, ladite molécule d'anticorps anti-pSYK étant conjuguée à un marqueur détectable.

6. Molécule d'anticorps anti-pSYK selon la revendication 5, où ledit marqueur est:

(i) choisi dans le groupe constitué par la peroxydase de raifort (HRP), la phosphatase alcaline, la galactosidase, la glucoamylase, le lysozyme, les glucide oxydases, les oxydases hétérocycliques, couplées à une enzyme qui emploie du peroxyde d'hydrogène pour oxyder un colorant, la biotine/avidine, les marqueurs de spin, les marqueurs de bactériophages, et les radicaux libres stables;
(ii) un fluorophore choisi parmi la fluorescéine ou un dérivé de celle-ci, la rhodamine ou un dérivé de celle-ci, le dansyle, l'ombelliférone, une lucériferase, la luciférine, et les 2,3-dihydrophtalazinediones; ou

(iii) un agent radioactif choisi dans le groupe constitué par $^{32}P$, $^{3}H$, $^{14}C$, $^{188}Rh$, $^{43}K$, $^{52}Pe$, $^{57}CO$, $^{67}Cu$, $^{67}Ga$, $^{68}Ga$, $^{77}Br$, $^{81}Rb/^{81M}Kr$, $^{87M}Sr$, $^{99}Tc$, $^{111}In$, $^{113M}In$, $^{123}I$, $^{125}I$, $^{127}Cs$, $^{129}Cs$, $^{131}I$, $^{132}I$, $^{197}Hg$, $^{203}Pb$, $^{206}Bi$, et $^{213}Bi$.

**7.** Séquence d'acides nucléiques isolée qui code pour la molécule d'anticorps anti-pSYK selon l'une quelconque des revendications 1 ou 4.

**8.** Cellule comprenant la séquence d'acides nucléiques isolée selon la revendication 7.

**9.** Méthode de production de la molécule d'anticorps anti-pSYK selon l'une quelconque des revendications 1 ou 4, comprenant la culture de la cellule selon la revendication 8 dans des conditions qui permettent la production de la molécule d'anticorps anti-pSYK.

**10.** Agent thérapeutique ciblé sur SYK pour l'utilisation dans une méthode de traitement d'un patient ayant une maladie **caractérisée par** une ou plusieurs cellules exprimant pSYK, ladite méthode comprenant:

a. la détection d'une expression de la protéine pSYK dans un échantillon biologique obtenu à partir du patient en utilisant une méthode comprenant:

la mise en contact de l'échantillon biologique avec la molécule d'anticorps anti-pSYK selon l'une quelconque des revendications 1 à 7; et
la détection de la formation d'un complexe entre la molécule d'anticorps anti-pSYK et la protéine pSYK; et

b. l'administration de l'agent thérapeutique ciblé sur SYK au patient si l'échantillon biologique exprime pSYK.

**11.** Agent thérapeutique ciblé sur SYK pour l'utilisation selon la revendication 10, comprenant de la 6-((1S,2R)-2-aminocyclohexylamino)-7-fluoro-4-(1-méthyl-1H-pyrazol-4-yl)-1H-pyrrolo[3,4-c]pyridin-3(2H)-one ou un sel pharmaceutiquement acceptable de celle-ci.

**12.** Agent thérapeutique ciblé sur SYK pour l'utilisation selon la revendication 10 ou 11, où le patient est un patient atteint de cancer, et où l'échantillon biologique est un échantillon provenant de cellules tumorales obtenues à partir du patient atteint de cancer.

**13.** Agent thérapeutique ciblé sur SYK pour l'utilisation selon la revendication 10 ou 11, où ladite maladie est une leucémie myéloïde aiguë, ou un lymphome diffus à grandes cellules B (DLBCL).

**14.** Méthode d'évaluation de la pharmacocinétique d'une thérapie ciblée sur SYK, ladite méthode comprenant les étapes consistant à:

a. mettre en contact la molécule d'anticorps anti-pSYK selon l'une quelconque des revendications 1 à 6 avec un échantillon biologique comprenant une ou plusieurs cellules suspectées d'exprimer pSYK provenant d'un patient qui a reçu la thérapie ciblée sur SYK selon un schéma posologique;
b. détecter la formation d'un complexe entre la molécule d'anticorps anti-pSYK et une protéine pSYK dans l'échantillon biologique;
c. quantifier l'expression de pSYK dans l'échantillon biologique afin de déterminer un niveau d'expression de pSYK; et
d. comparer le niveau d'expression de pSYK avec une base de données comprenant la thérapie ciblée sur SYK.

**15.** Méthode selon la revendication 14, comprenant en outre l'ajustement du schéma posologique sur la base du niveau d'expression de pSYK.

FIG. 1

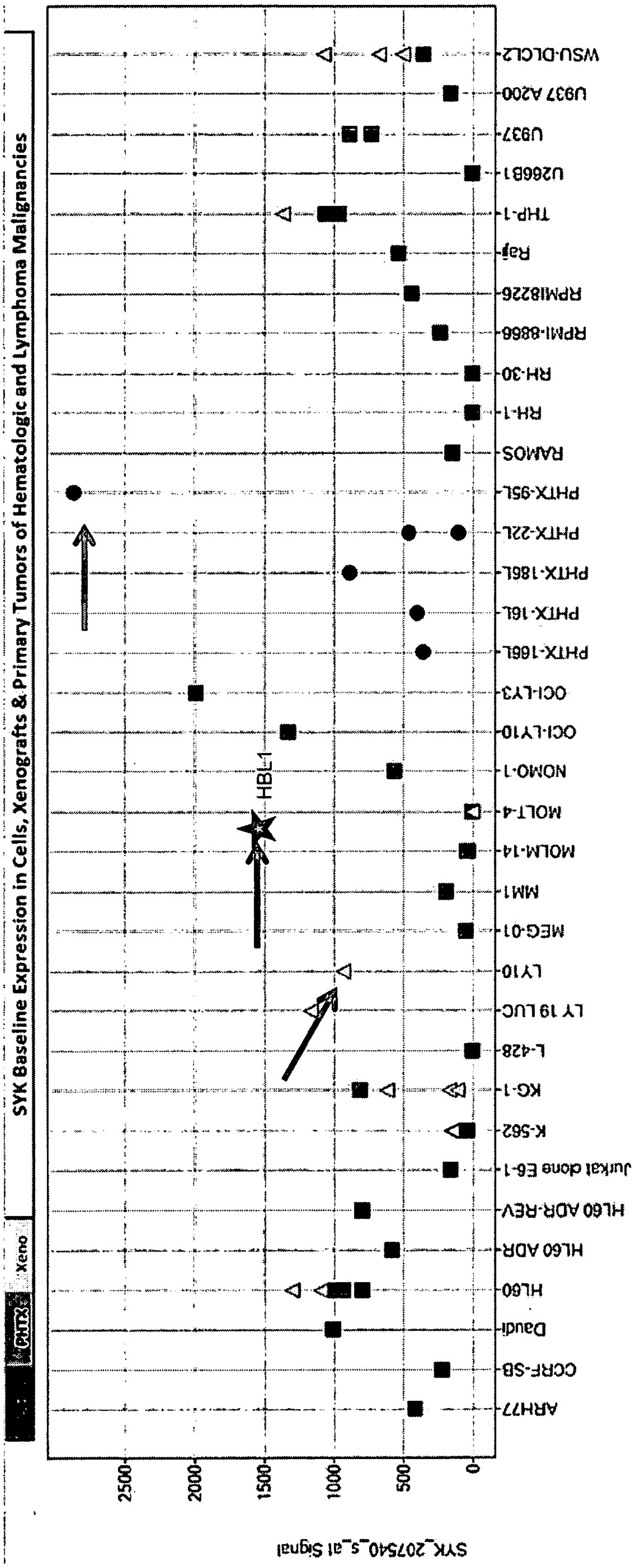
SYK Baseline Expression in Cells, Xenografts & Primary Tumors of Hematologic and Lymphoma Malignancies
PHTX
Xeno
HBL-1
SYK_207540_s_at Signal
0
500
1000
1500
2000
2500
WSU-DCL2
U937 A200
U937
U266B1
THP-1
Raji
RPMI8226
RPMI-8866
RH-30
RH-1
RAMOS
PHTX-95L
PHTX-22L
PHTX-186L
PHTX-16L
PHTX-166L
OCI-LY3
OCI-LY10
NOMO-1
MOLT-4
MOLM-14
MM1
MEG-01
LY10
LY 19 LUC
L-428
KG-1
K-562
Jurkat clone E6-1
HL60 ADR-REV
HL60 ADR
HL60
Daudi
CCRF-SB
ARH77

FIG. 2

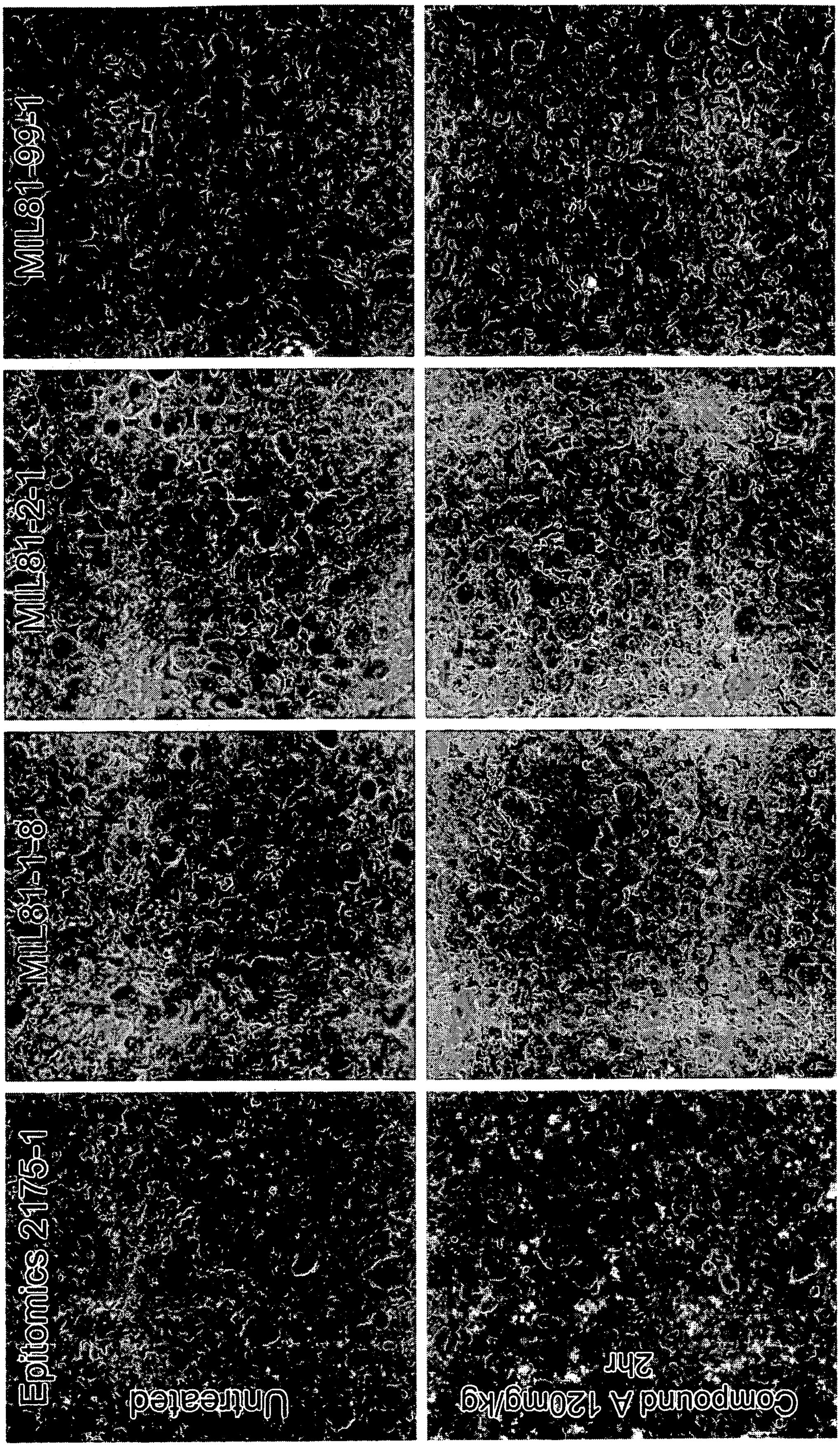
Epitomics 2175-1
MIL81-1-8
MIL81-2-1
MIL81-99-1
Untreated
Compound A 120mg/kg
2hr

FIG. 3

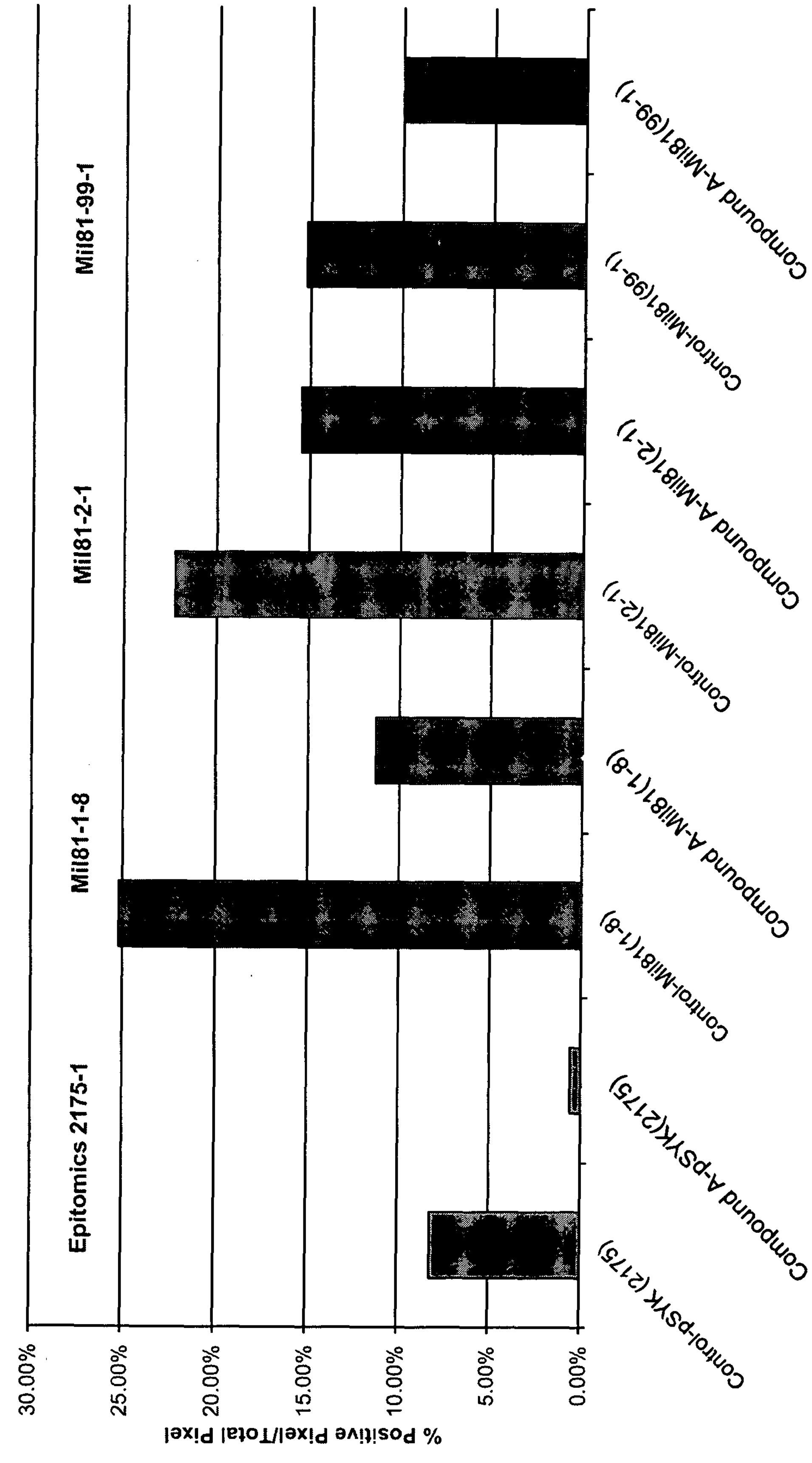
% Positive Pixel/Total Pixel
30.00%
25.00%
20.00%
15.00%
10.00%
5.00%
0.00%
Epitomics 2175-1
Mil81-1-8
Mil81-2-1
Mil81-99-1
Control-pSYK (2175)
Compound A-pSYK(2175)
Control-Mil81(1-8)
Compound A-Mil81(1-8)
Control-Mil81(2-1)
Compound A-Mil81(2-1)
Control-Mil81(99-1)
Compound A-Mil81(99-1)

FIG. 4

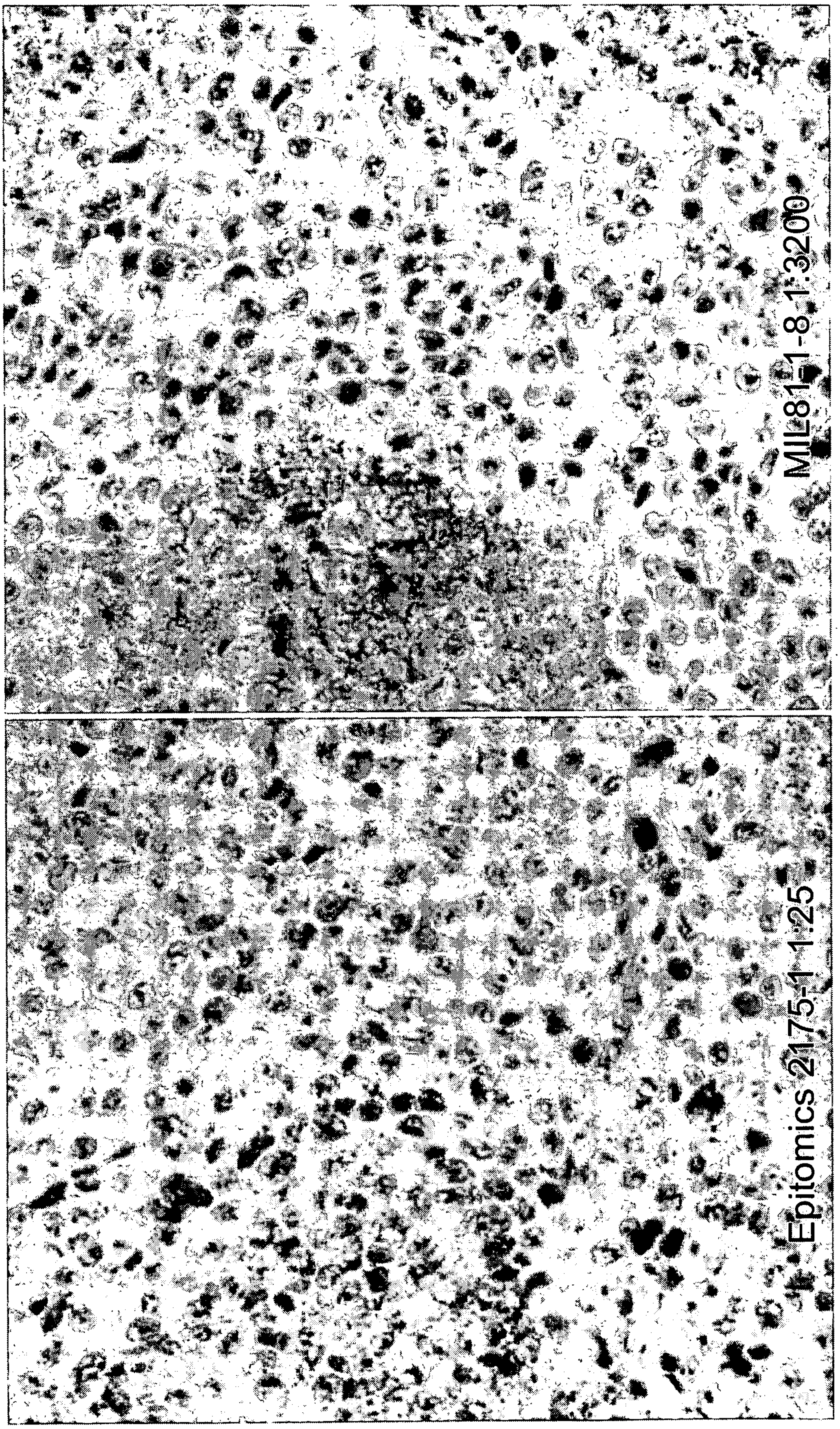

FIG. 5

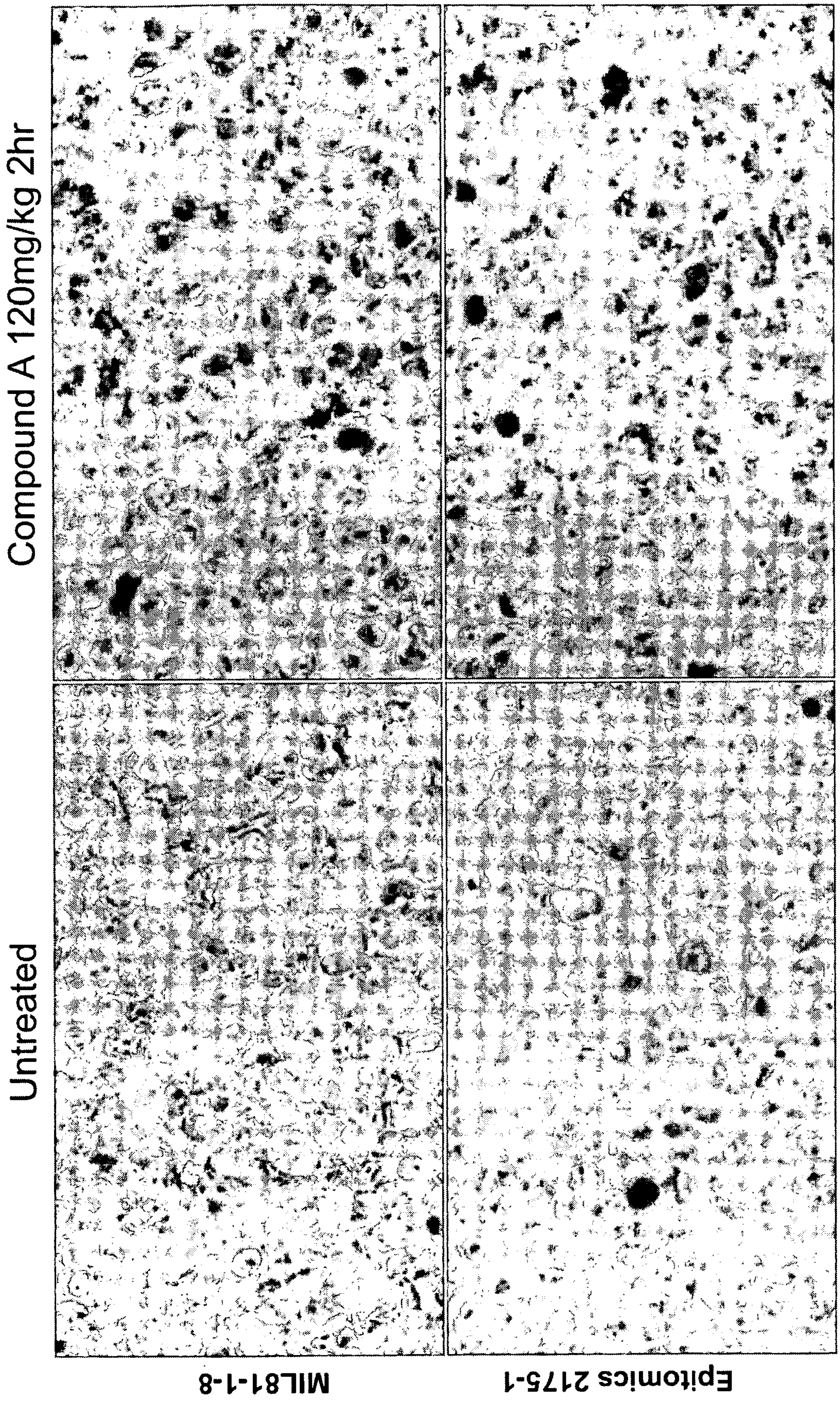

FIG. 6

Untreated

Compound A 120mg/kg 2hr

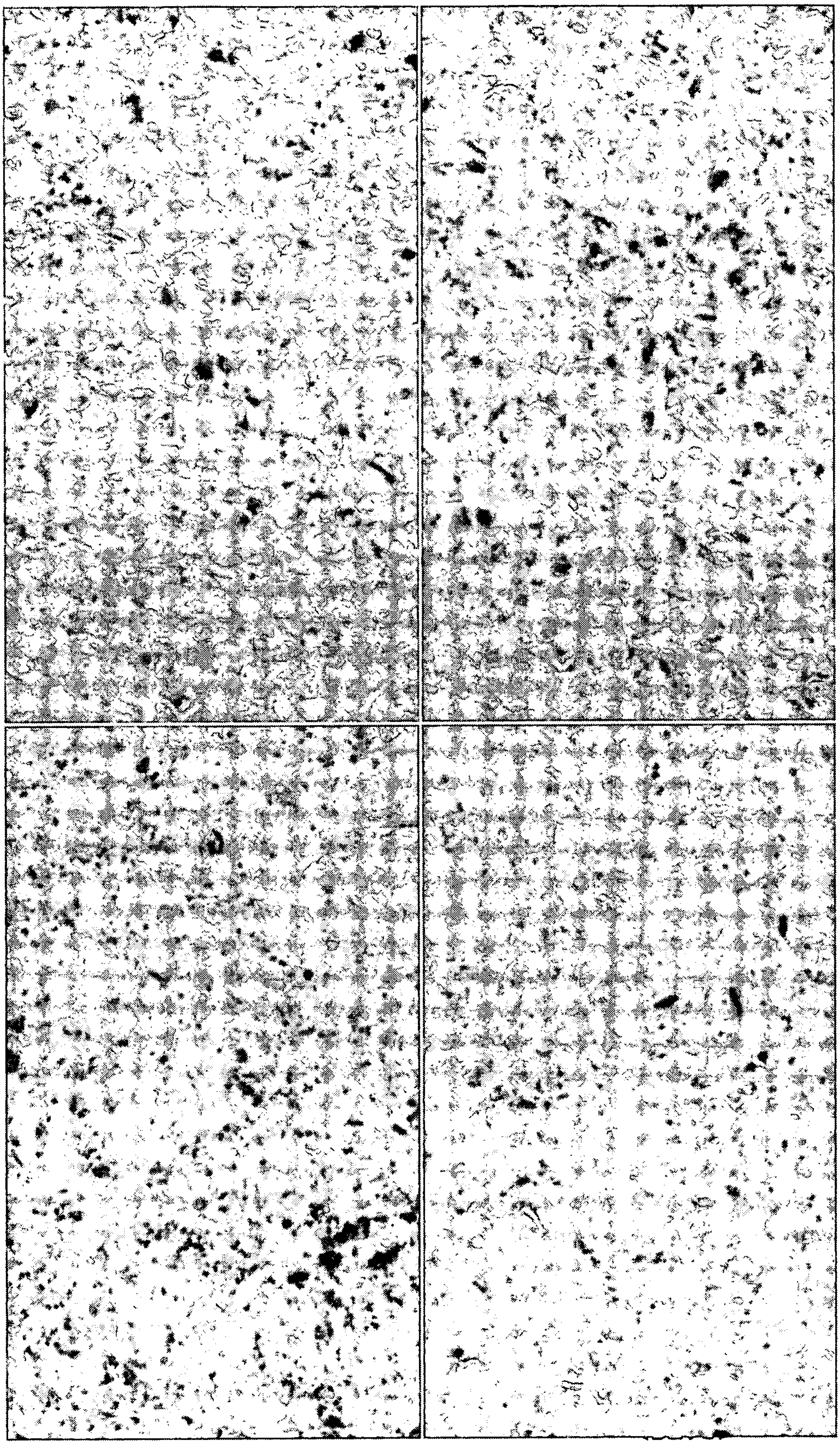

MIL81-1-8

Epitomics 2175-1

FIG. 7

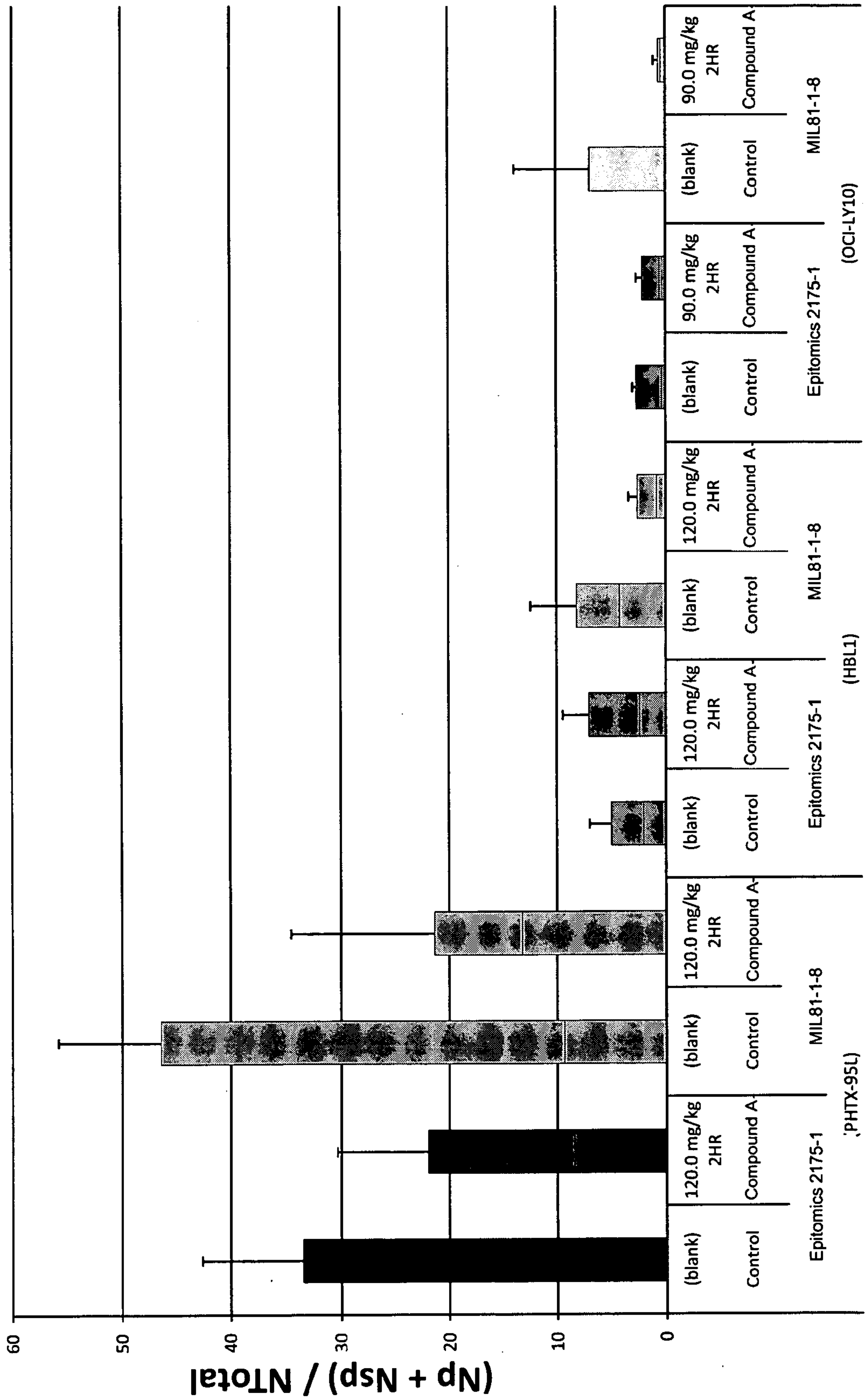
(Np + Nsp) / NTotal
0
10
20
30
40
50
60
(blank)
Control
120.0 mg/kg
2HR
Compound A
90.0 mg/kg
Epitomics 2175-1
MIL81-1-8
(PHTX-95L)
(HBL1)
(OCI-LY10)

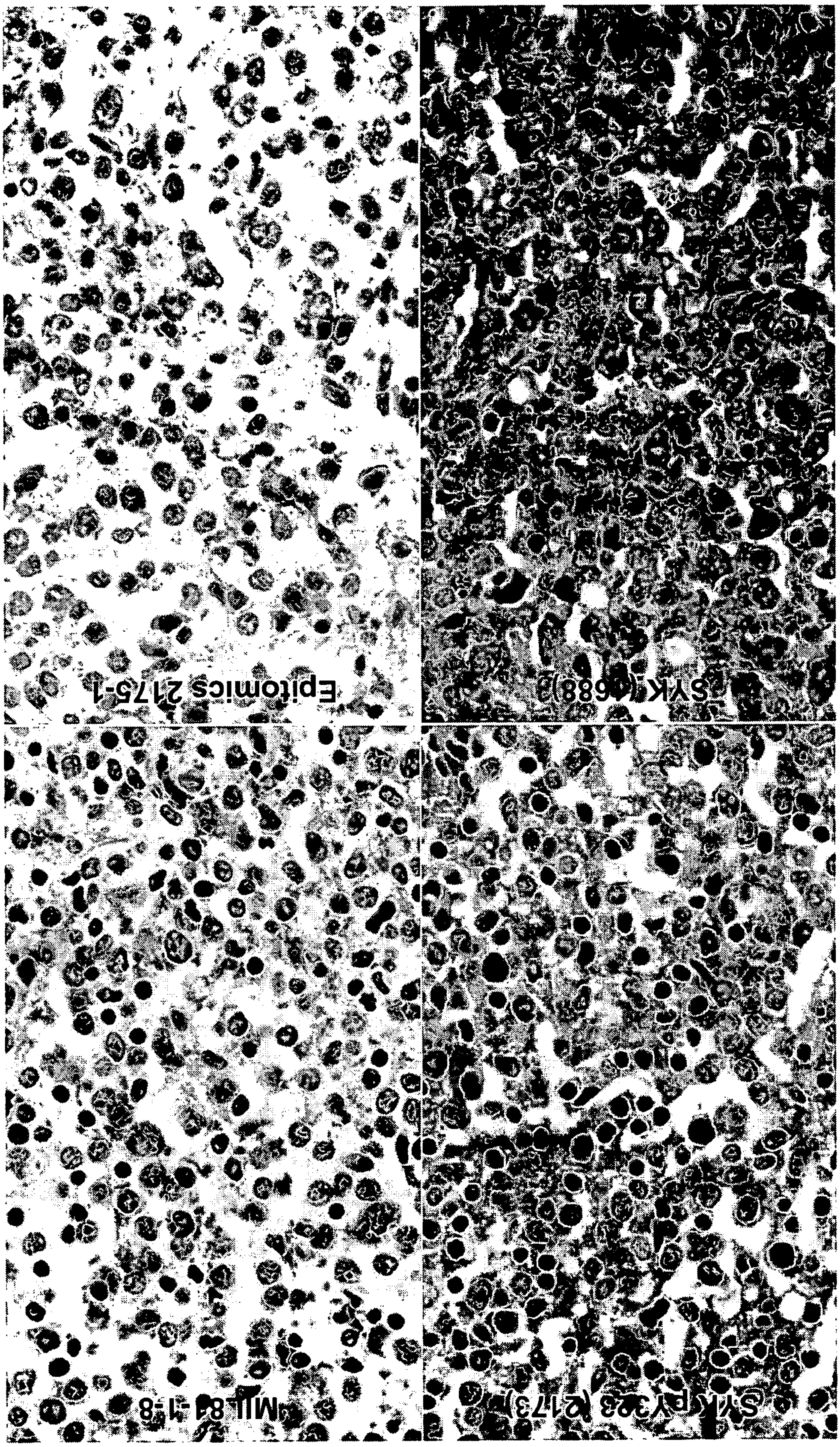


FIG. 8

FIG. 9

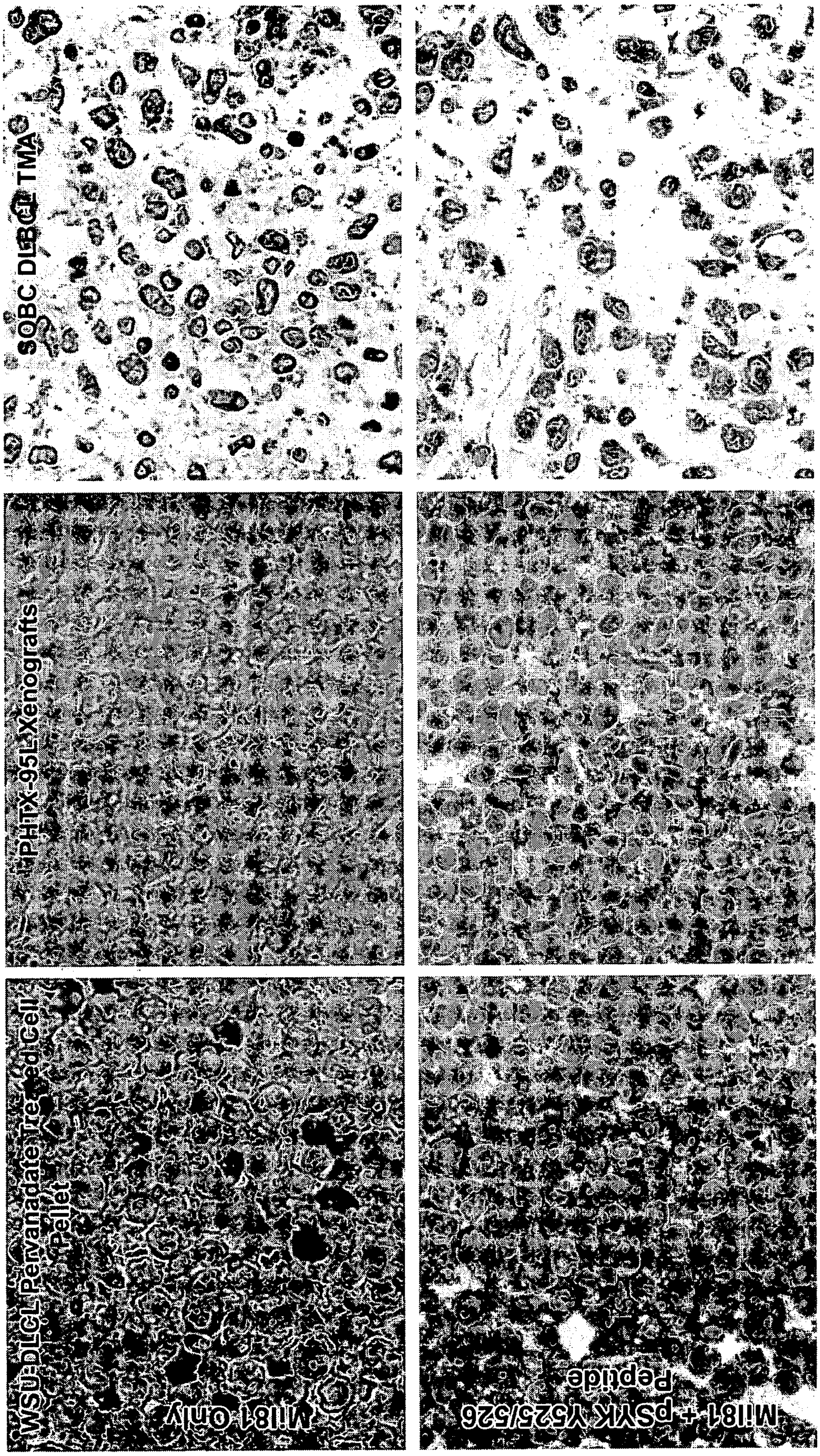
SOBC DLBCL TMA
PHTX-95L Xenografts
WSU-DLCL Pervanadate Treated Cell Pellet
Mil81 only
Mil81 + pSYK Y525/526 Peptide

FIG. 10

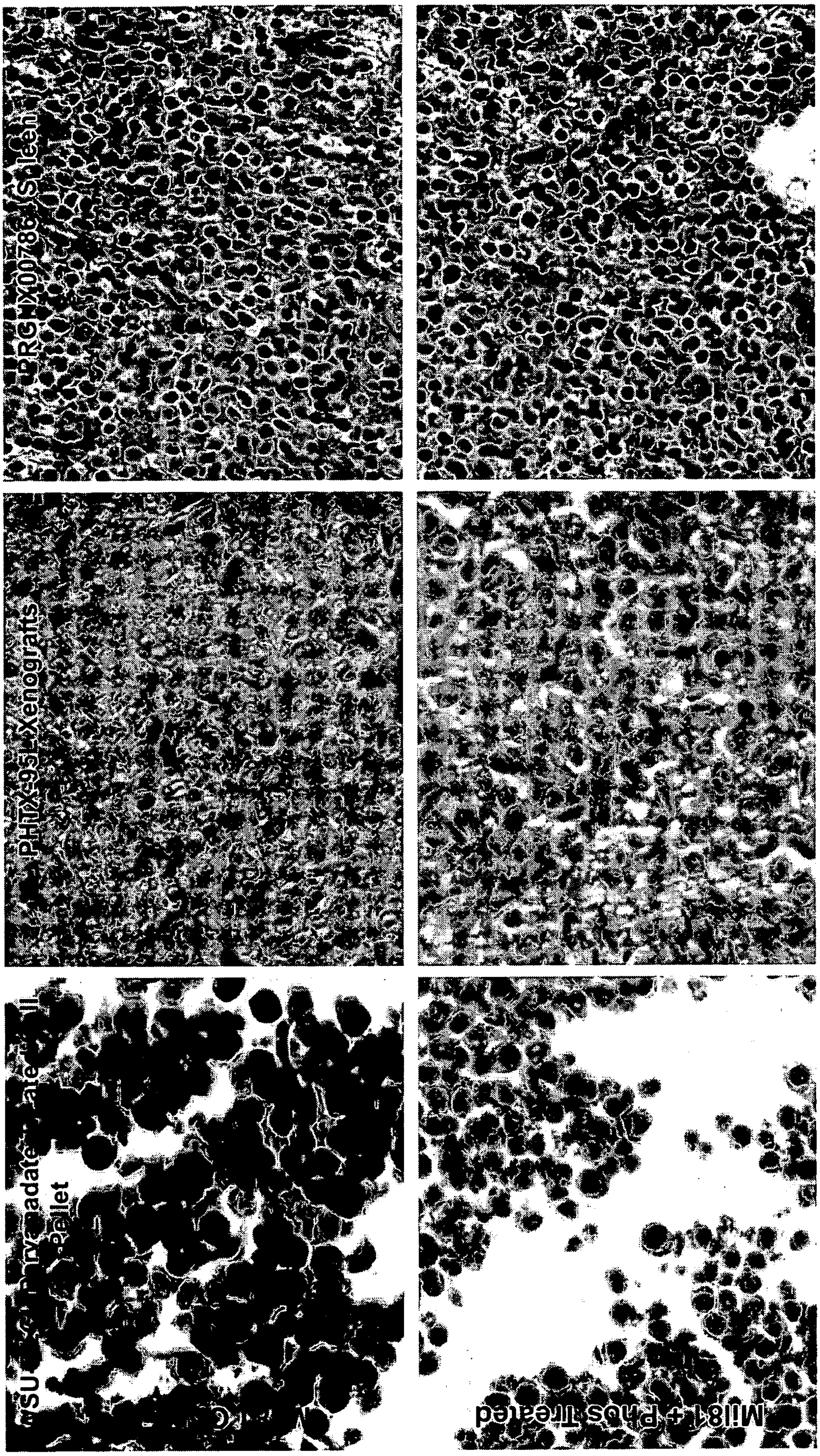
PRGHX00786 Spleen
PHTX-95L Xenografts
Pellet
Mil81 + Phos Treated

FIG. 11

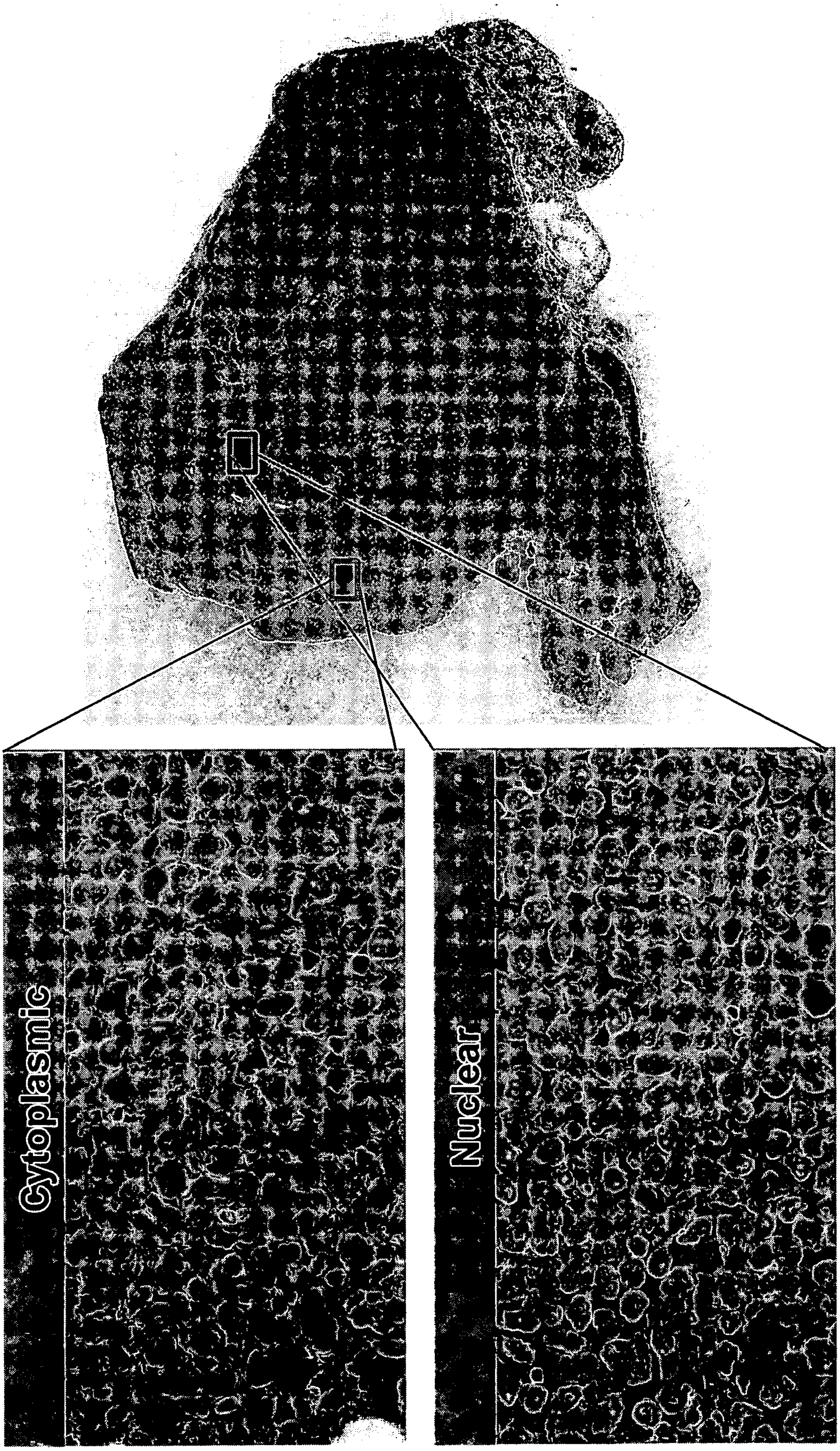

FIG. 12

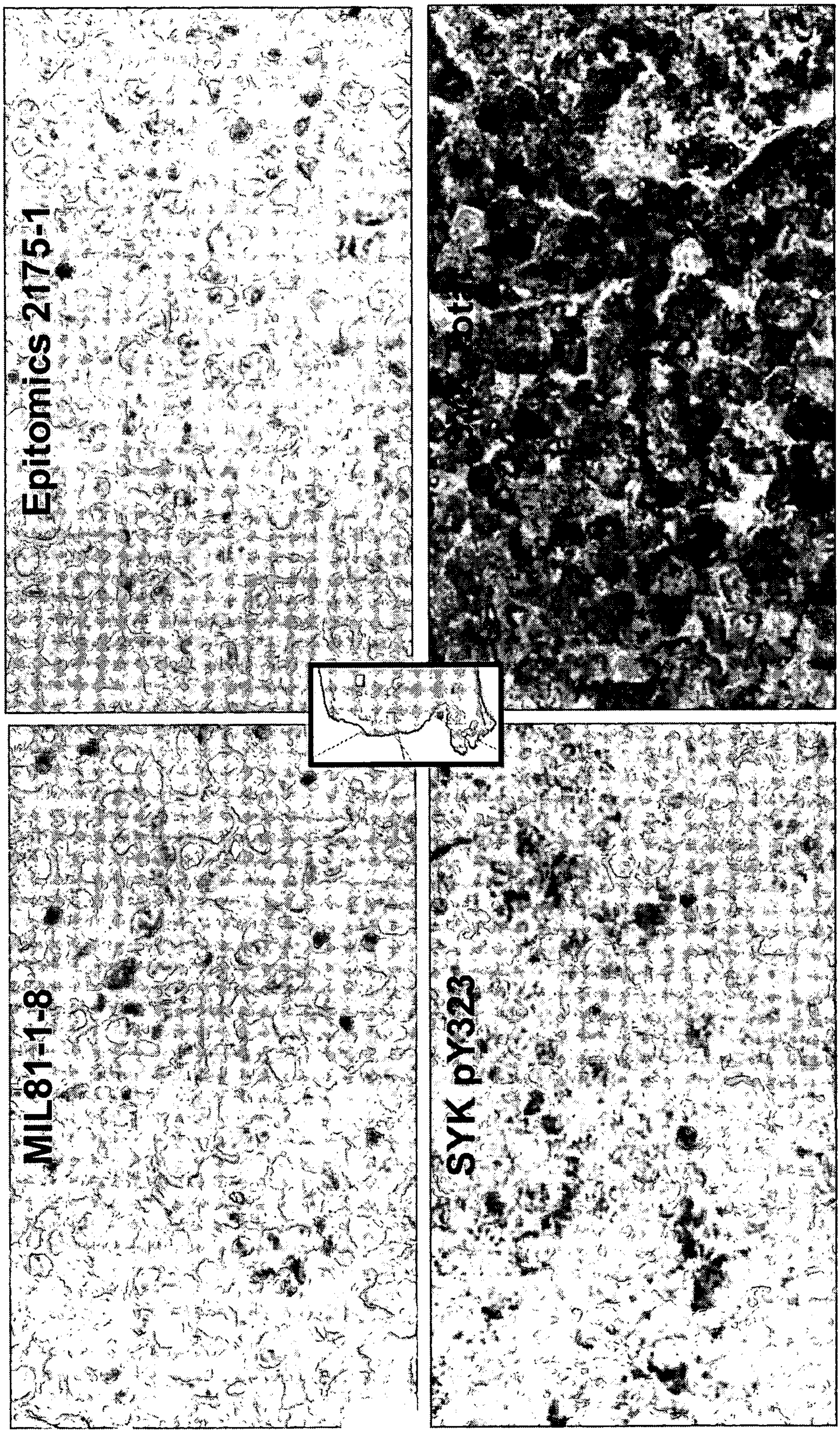

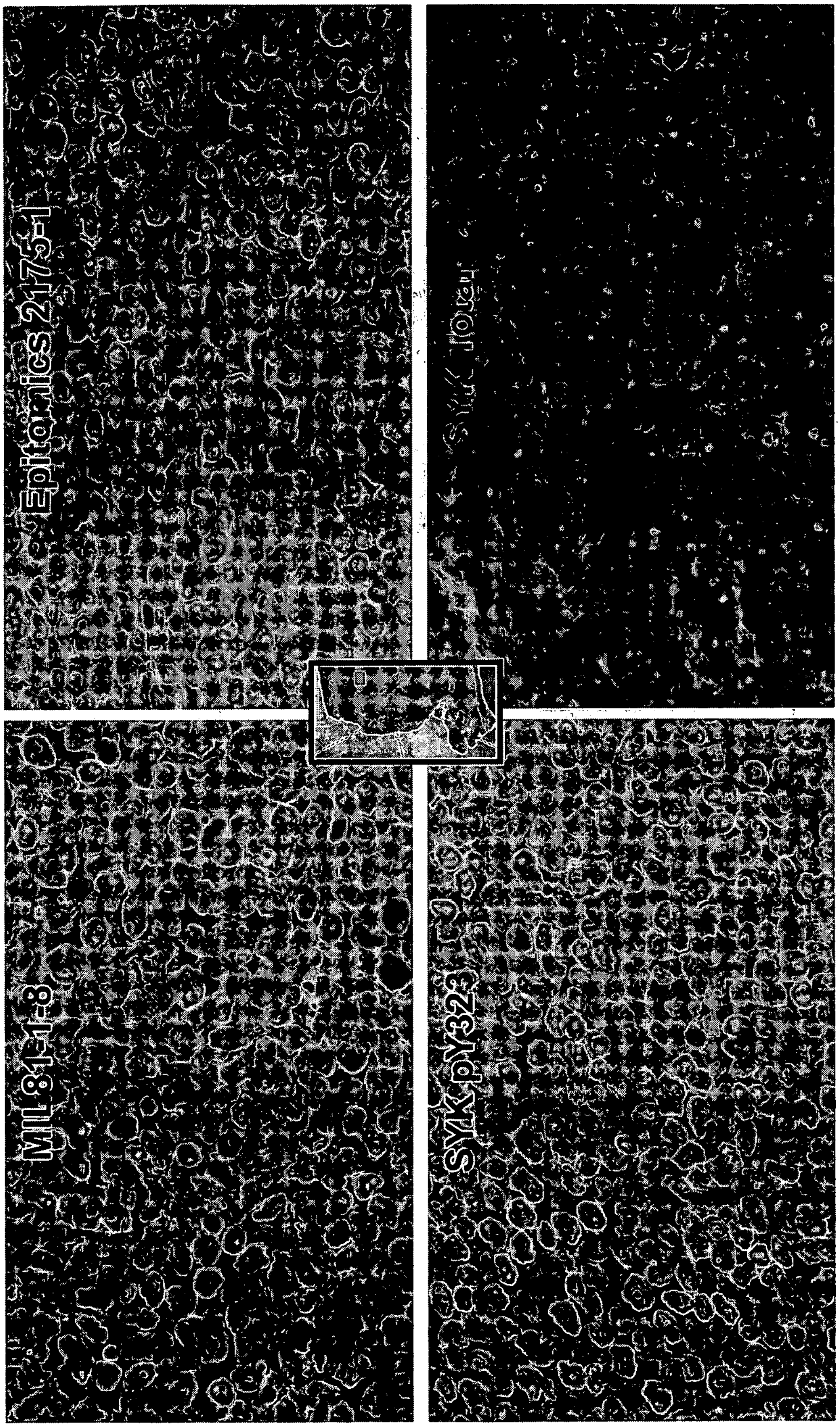


FIG. 13

FIG. 14

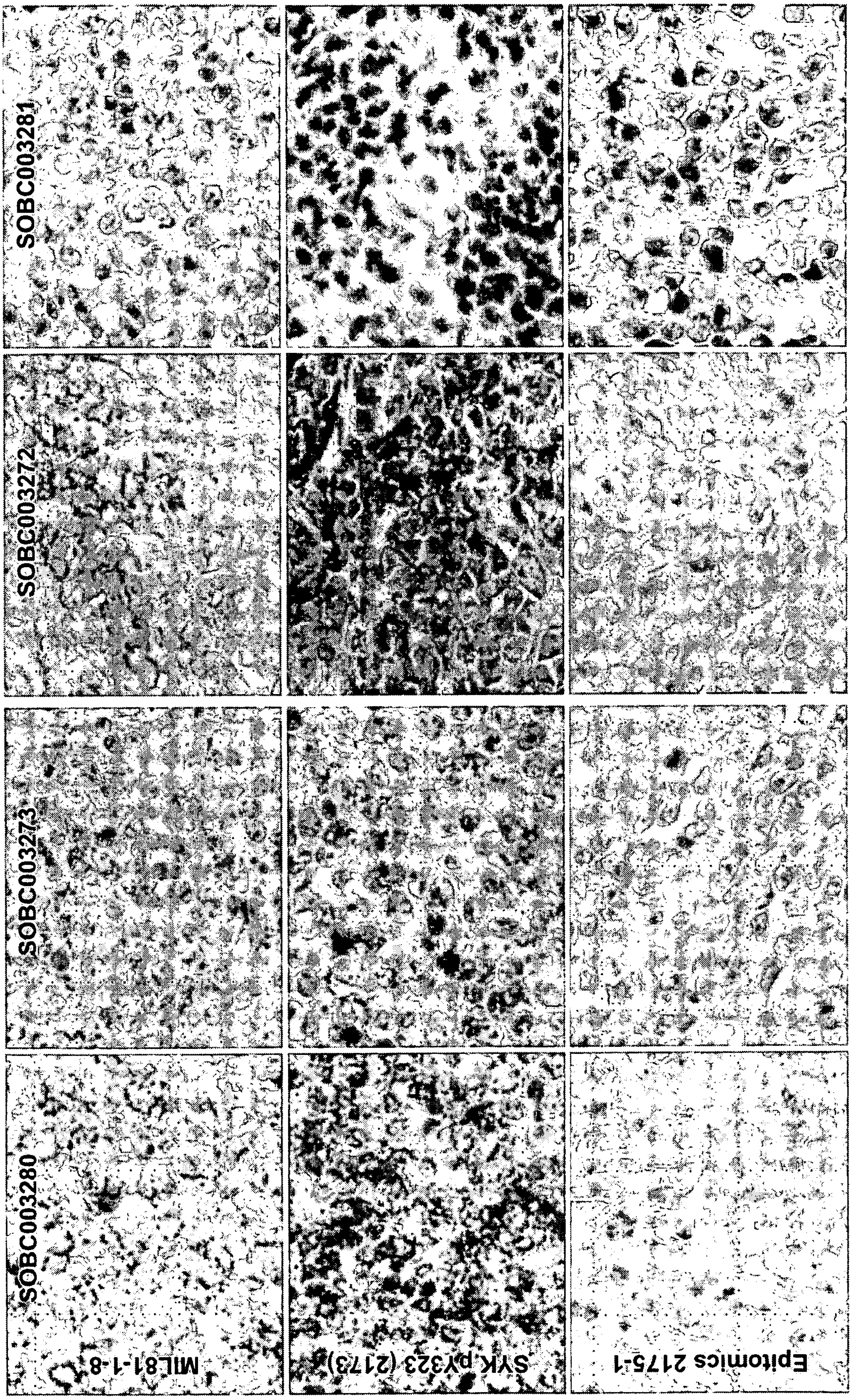
SOBC003281
SOBC003272
SOBC003273
SOBC003280
MIL81-1-8
SYK pY323 (2173)
Epitomics 2175-1

FIG. 15

| Sample ID | Total SYK | MIL81-1-8 | SYK pY323 (2173) Staining | Epitomics 2175-1 |
|---|---|---|---|---|
| SOBC003280 (2012) | +++ | ++ | ++ | + (Weak POS) |
| SOBC003273 (2012) | +++ | ++ | ++ | + (Weak POS) |
| SOBC003272 (2011) | +++ | ++ | ++ | + (Weak POS) |
| SOBC003281 (2012) | +++ | + | + | + (Weak POS) |
| SOBC003271 (2011) | +++ | + | + | + (Weak POS) |
| SOBC003270 (2012) | +++ | + | + | + (Weak POS) |
| SOBC003279 (2012) | +++ | +/- | - | - |
| SOBC003278 (2012) | +++ | +/- | +/- | - |
| SOBC003276 (2012) | +++ | +/- | +/- | +/- |
| SOBC003269 (2012) | +++ | +/- | - | +/- |
| SOBC003268 (2012) | +++ | +/- | - | - |
| SOBC003283 (2012) | +++ | - | - | +/- |
| SOBC003277 (2012) | +++ | - | - | - |
| SOBC003275 (2012) | +++ | - | +/- | +/- |
| SOBC003274 (2012) | +++ | - | - | - |
| % Positive | 100% | 40% | 40% | ~40% |

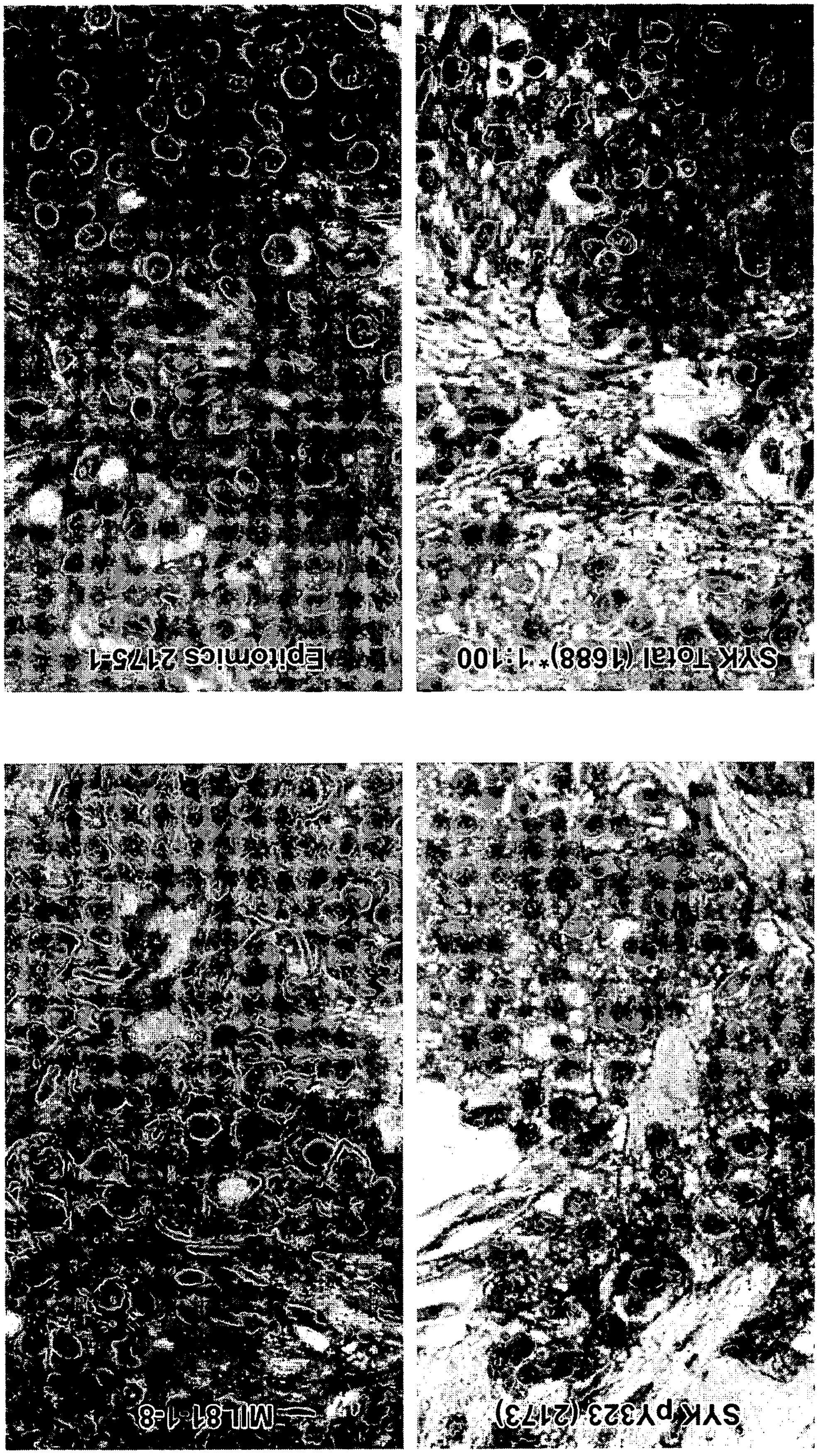


FIG. 16

FIG. 17

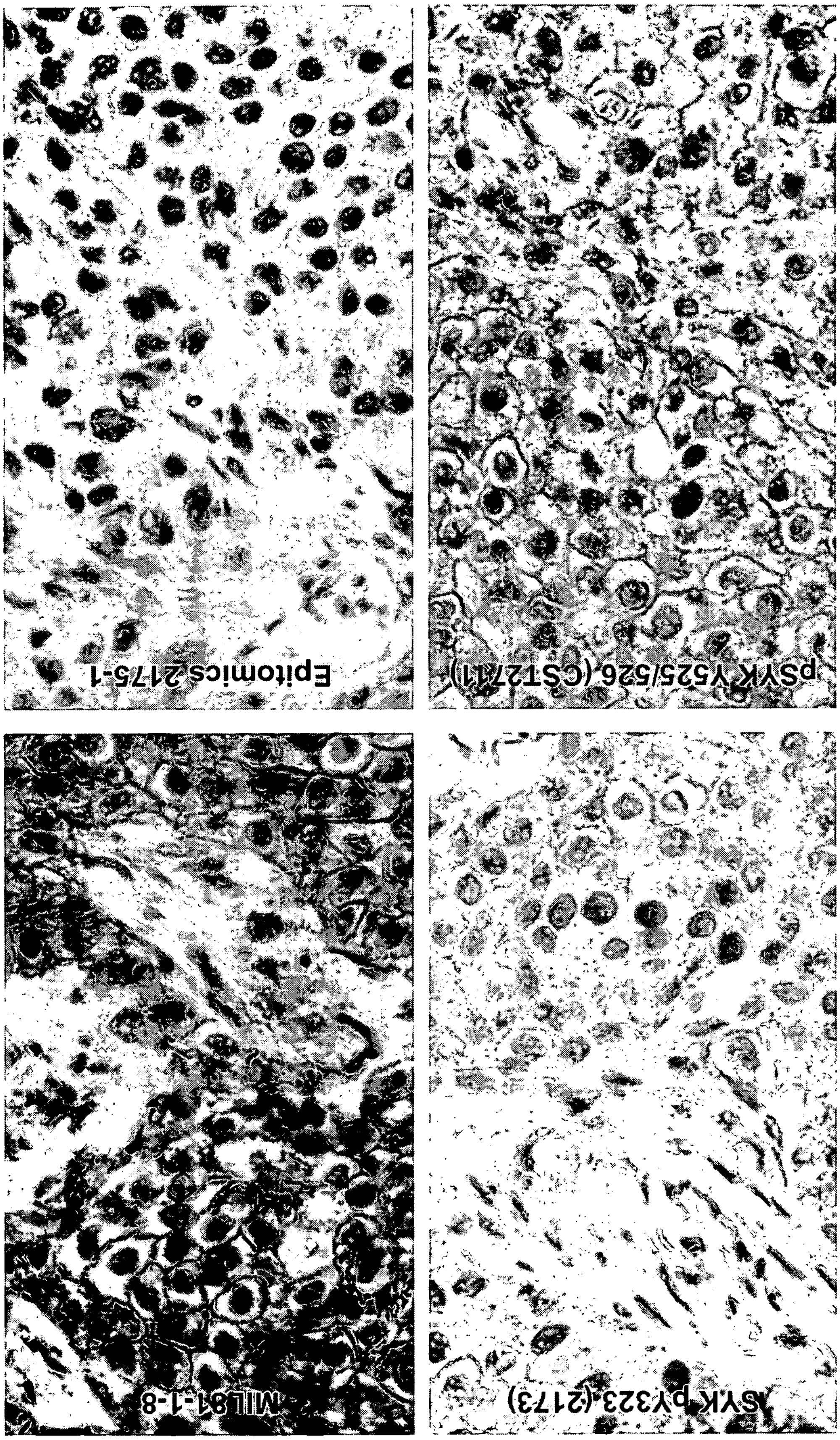
Epitomics 2175-1
pSYK Y525/526 (CST2711)
MIL81-1-8
SYK pY323 (2173)

FIG.18

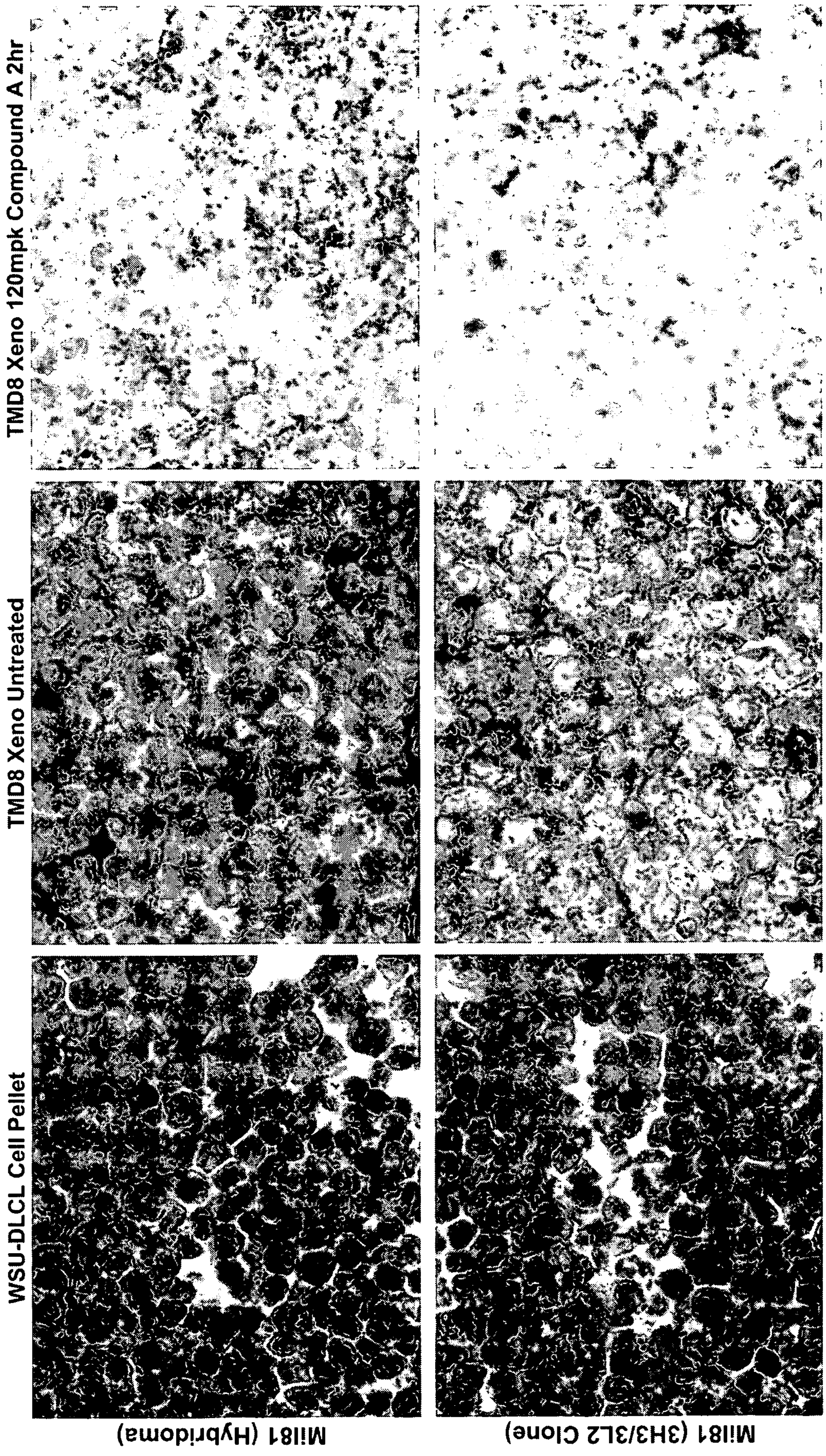
WSU-DLCL Cell Pellet
TMD8 Xeno Untreated
TMD8 Xeno 120mpk Compound A 2hr
Mil81 (Hybridoma)
Mil81 (3H3/3L2 Clone)

FIG. 19

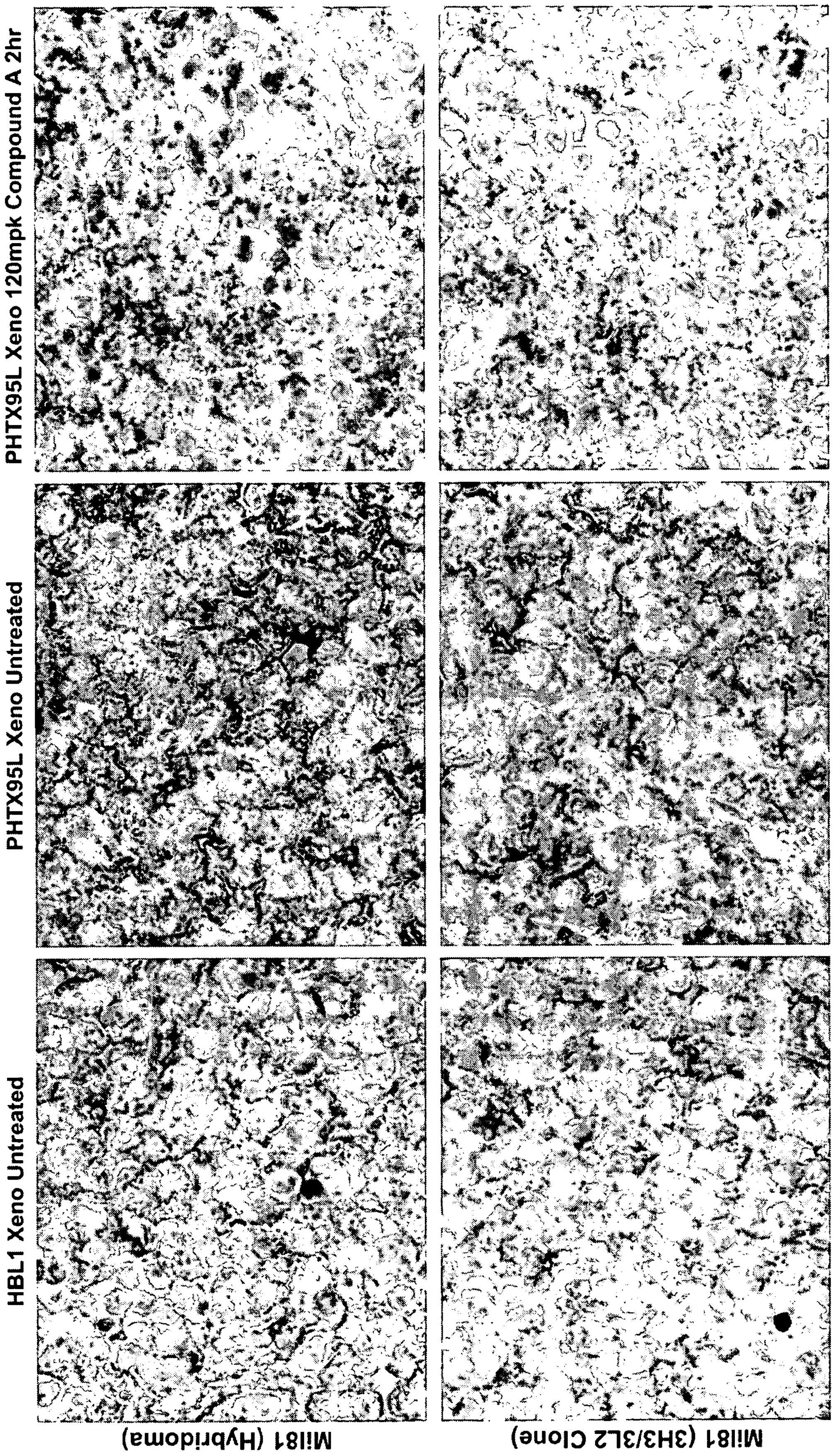
HBL1 Xeno Untreated
PHTX95L Xeno Untreated
PHTX95L Xeno 120mpk Compound A 2hr
Mil81 (Hybridoma)
Mil81 (3H3/3L2 Clone)

FIG. 20

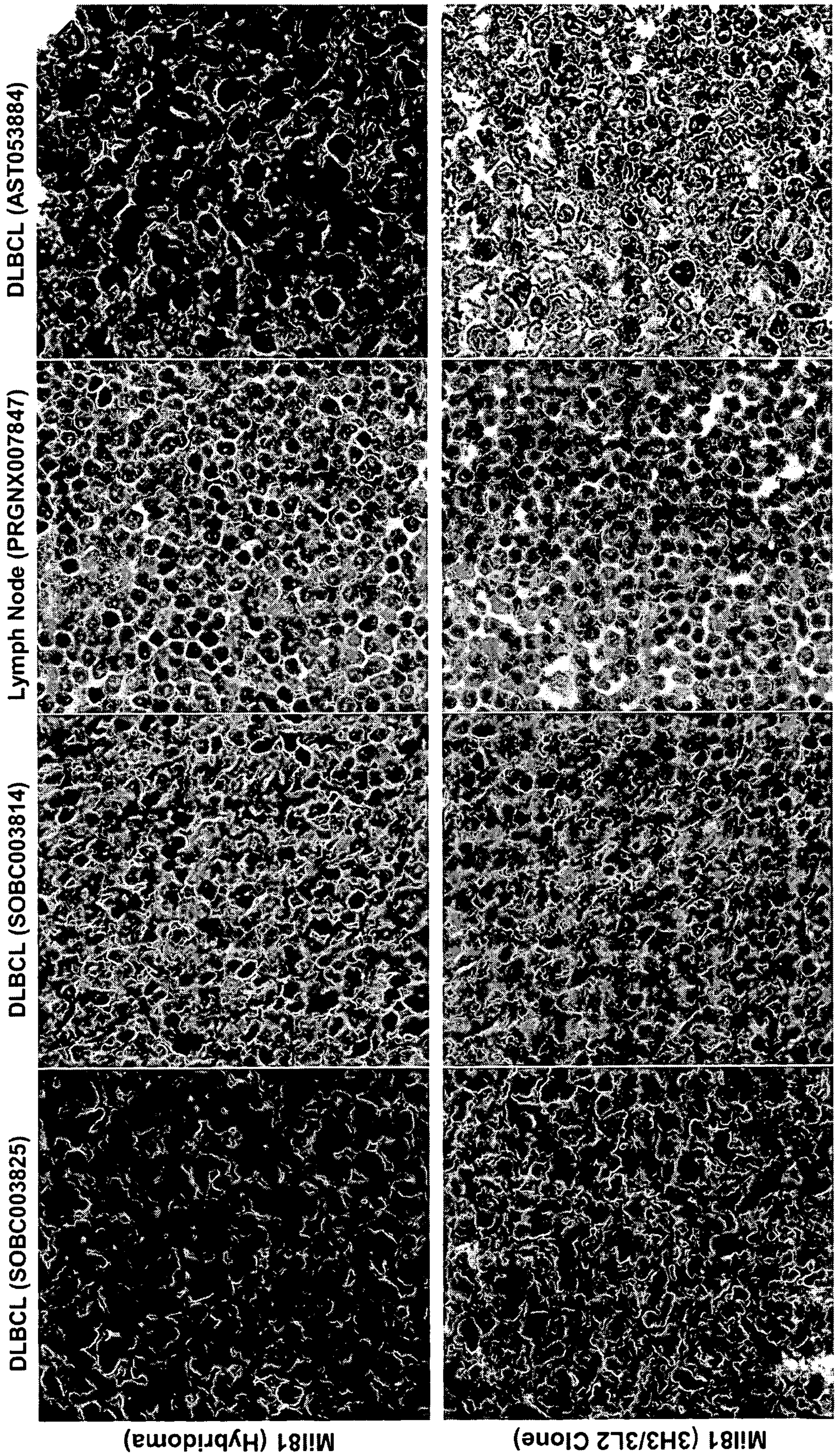
DLBCL (SOBC003825)
DLBCL (SOBC003814)
Lymph Node (PRGNX007847)
DLBCL (AST053884)
Mil81 (Hybridoma)
Mil81 (3H3/3L2 Clone)

FIG. 21

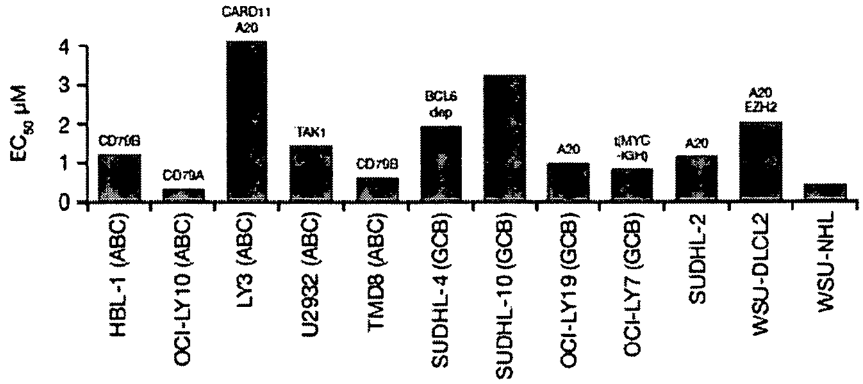


ABC, activated B-cell-like DLBCL subtype; GCB, germinal center B-cell-like DLBCL subtype.

FIG. 22

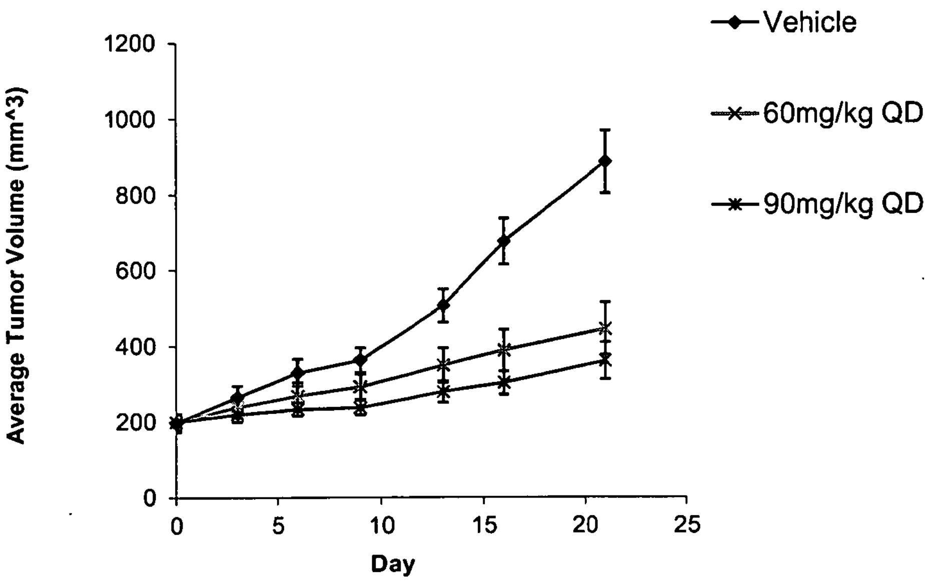

FIG. 23

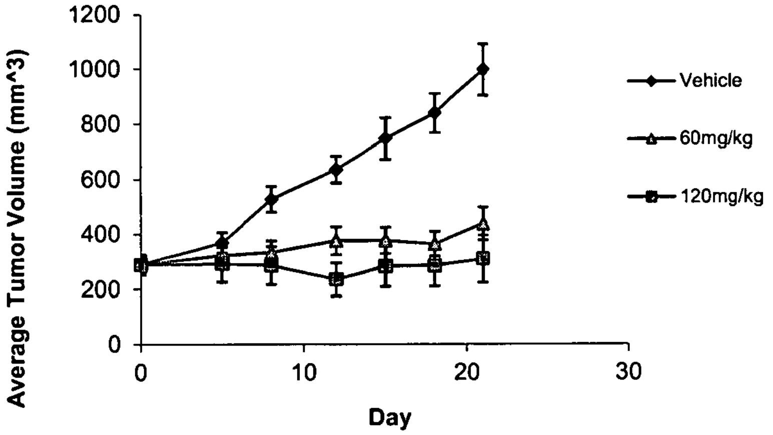
1200
1000
800
600
400
200
0
Average Tumor Volume (mm^3)
0
10
20
30
Day
Vehicle
60mg/kg
120mg/kg

FIG. 24

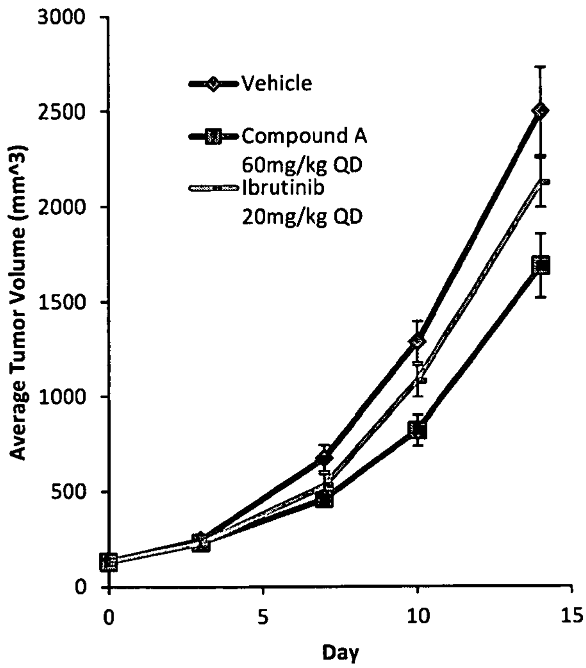
3000
2500
2000
1500
1000
500
0
Average Tumor Volume (mm^3)
0
5
10
15
Day
Vehicle
Compound A 60mg/kg QD
Ibrutinib 20mg/kg QD

FIG. 25

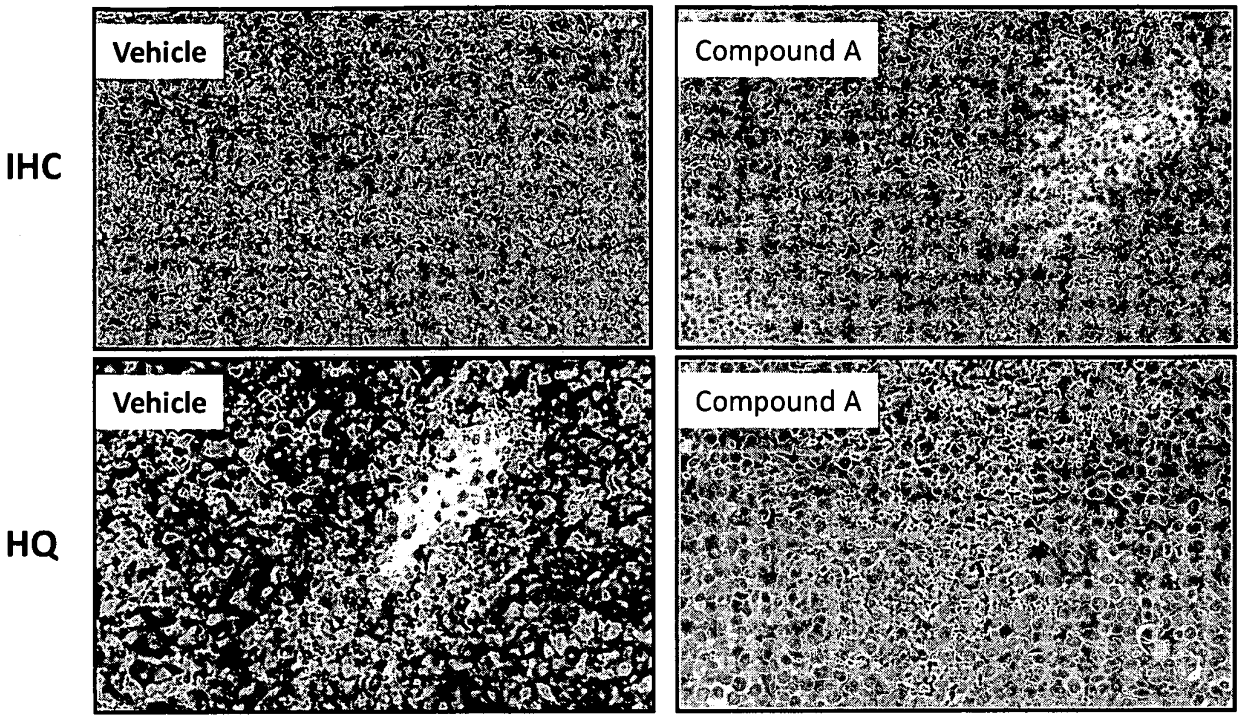

FIG. 26

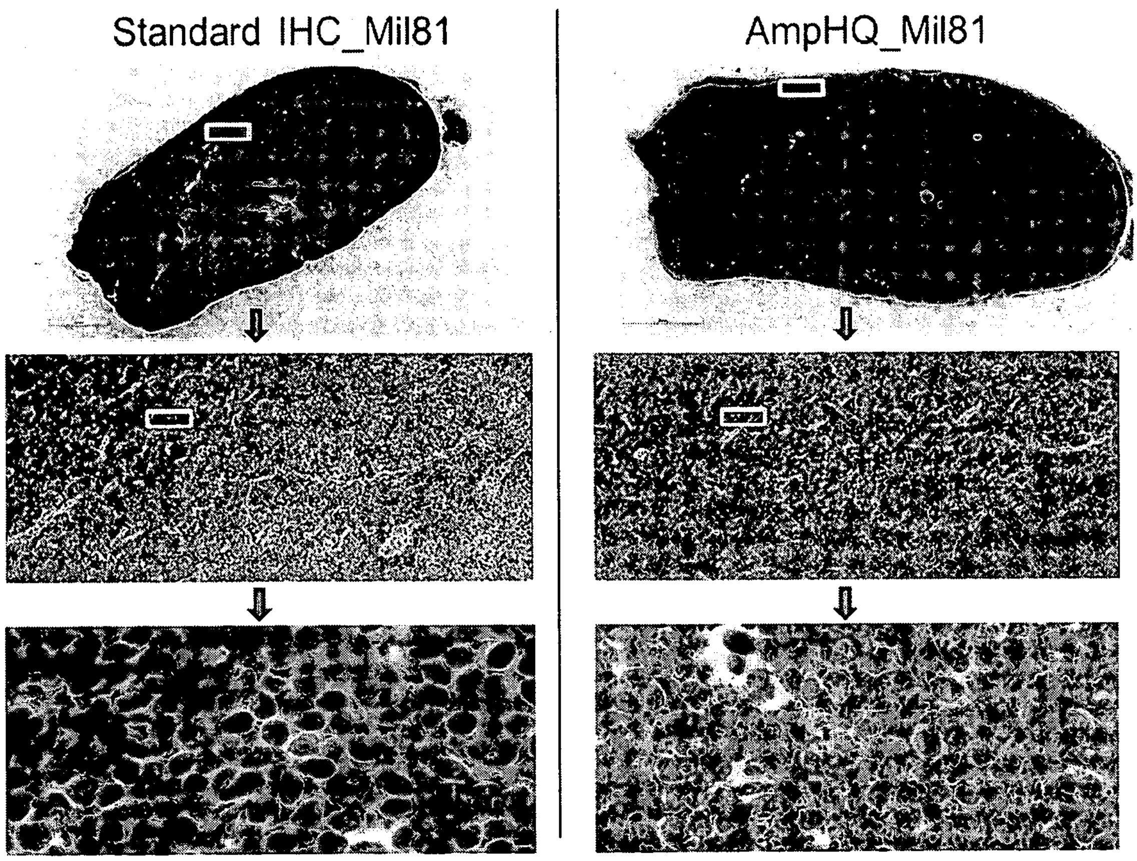

FIG. 27

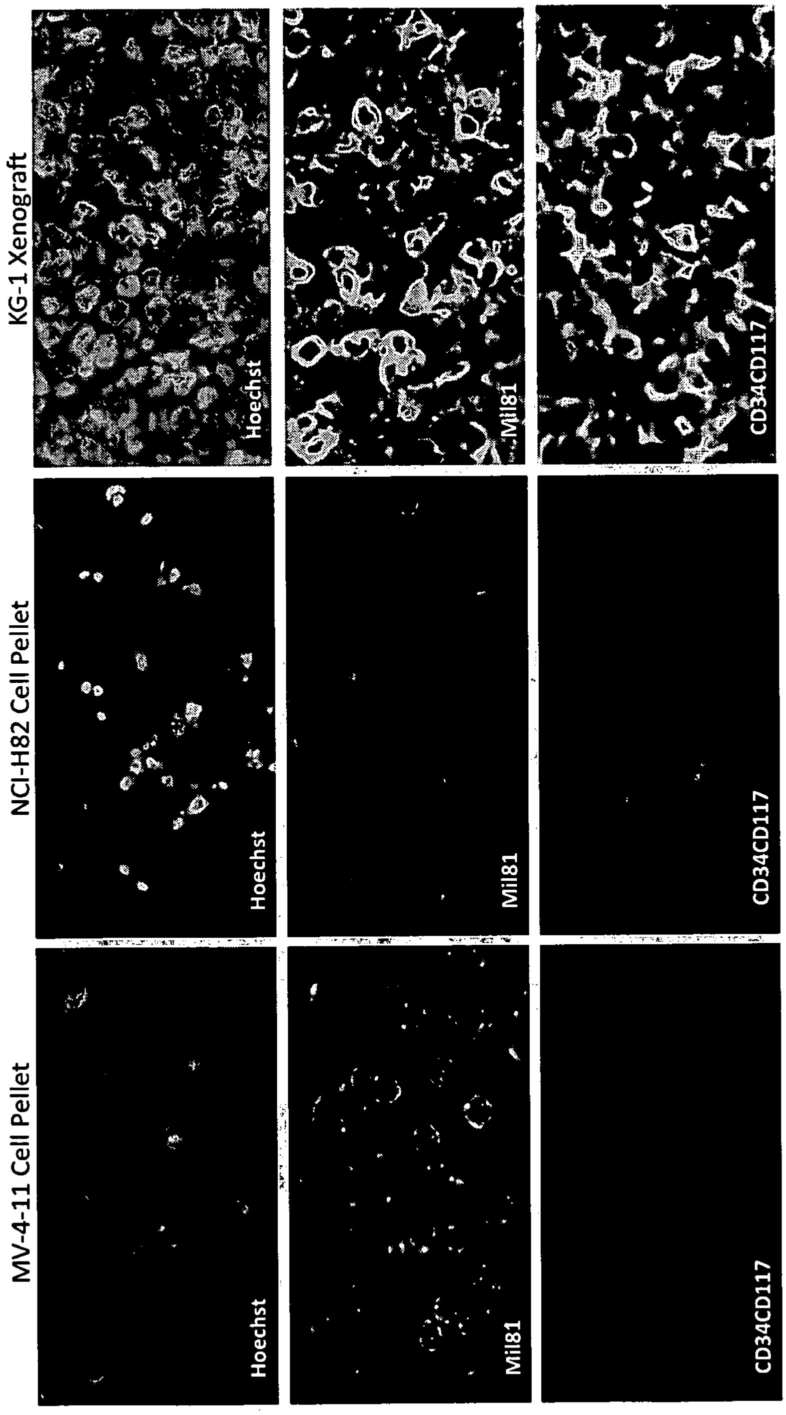

KG-1 Xenograft
NCI-H82 Cell Pellet
MV-4-11 Cell Pellet
Hoechst
Mil81
CD34CD117
Hoechst
Mil81
CD34CD117
Hoechst
Mil81
CD34CD117

FIG. 28

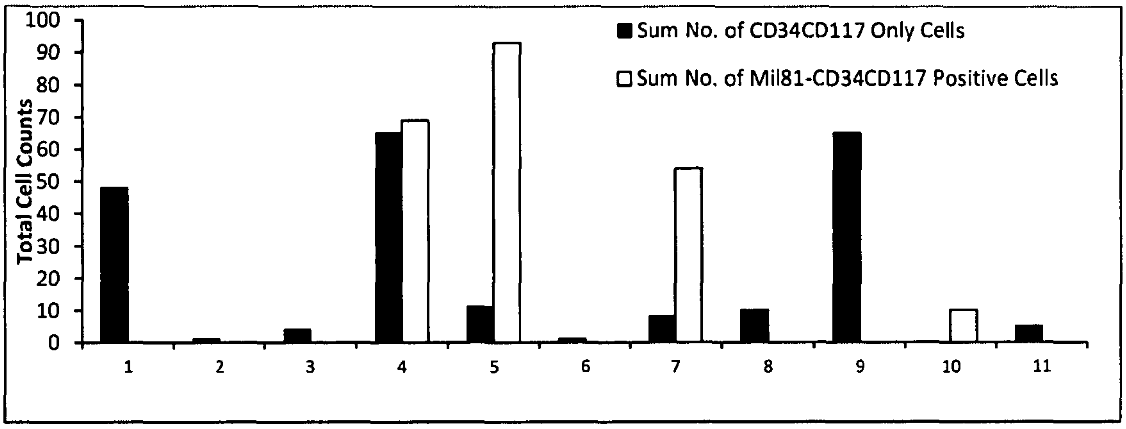
100
90
80
70
60
50
40
30
20
10
0
Total Cell Counts
1
2
3
4
5
6
7
8
9
10
11
Sum No. of CD34CD117 Only Cells
Sum No. of Mil81-CD34CD117 Positive Cells

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 199931073 A
- WO 2009136995 A
- WO 2009131687 A
- US 8440689 B
- US 4703004 A
- GB 2209757 B, Winter
- WO 8907142 A, Morrison
- WO 9429351 A, Morgan
- US 6020153 A
- US 6331415 B
- US 7402409 B
- US 7429487 B
- US 7462697 B
- US 7575896 B
- US 7732168 B
- US 8062867 B
- US 6197582 B, Trakht
- US 5675063 A
- WO 9202190 A
- US 5530101 A
- US 5585089 A
- US 5693761 A
- US 5693792 A
- US 5714350 A
- US 5777085 A
- US 4683195 A
- US 4683202 A
- US 5565332 A
- US 5573905 A
- US 6300064 B, Knappik
- US 7317091 B
- US 5624821 A
- WO 0042072 A
- US 5648260 A, Winter
- US 5834597 A, Tso
- US 20030157108 A, Presta
- US 20040093621 A
- US 4816567 A
- US 5225539 A
- US 5859205 A
- EP 519596 A1, Padlan
- WO 9852976 A
- WO 0034317 PCT
- US 6818749 B
- US 4179337 A
- US 5612460 A
- WO 9506058 A
- WO 0026256 PCT
- US 20030026805 PCT
- US 5733743 A
- US 4816397 A
- US 5916771 A
- US 5837821 A
- US 20050170450 A
- US 20100261275 A
- US 20060147445 A
- US 8551774 B
- WO 9203918 A
- EP 0216846 A
- EP 0256055 A
- EP 0323997 A
- EP 89303964 A
- US 5827690 A
- US 5756687 A
- US 5750172 A
- US 5741957 A
- US 5194594 A
- US 4681581 A
- US 4735210 A
- US 5101827 A
- US 5102990 A
- US 35500 A
- US 5648471 A
- US 5697902 A
- US 4737456 A
- US 3940475 A
- US 4289747 A
- US 4376110 A
- US 5631169 A, Lakowicz
- US 4868103 A, Stavrianopoulos
- US 5223409 A
- US 61982098 B
- US 62004496 B

### Non-patent literature cited in the description

- **MARTIN TURNER et al.** *Immunology Today,* 2000, vol. 21 (3), 148-54
- **MICHAEL P. SANDERSON et al.** *Inflammation & Allergy-Drug Targets,* 2009, vol. 8, 87-95

- **SUZUKI-INQUE, K. et al.** *Biochem. J.,* 2004, vol. 378, 1023-1029
- **SAMBROOK et al.** Molecular Cloning: A Laboratory Manual. Cold Spring Harbor Laboratory Press, 2000
- **HARLOW, E. ; LANE, D.** Antibodies: A Laboratory Manual. Cold Spring Harbor Laboratory Press, 1988
- **BUCHWALOW ; BÖCKER.** Immunohistochemistry Basics and Methods. Springer-Verlag, 2010
- **NEEDLEMAN ; WUNSCH.** *J. Mol. Biol.,* 1970, vol. 48, 444-453
- Current Protocols in Molecular Biology. John Wiley & Sons, 1989, 6.3.1-6.3.6
- **BOWIE, J U et al.** *Science,* 1990, vol. 247, 1306-1310
- **PADLAN et al.** *FASEB J.,* 1995, vol. 9, 133-139
- **SJOLANDER, S ; URBANICZKY, C.** *Anal. Chem.,* 1991, vol. 63, 2338-2345
- **SZABO et al.** *Curr. Opin. Struct. Biol.,* 1995, vol. 5, 699-705
- Fundamental Immunology. Raven Press, 1989
- Kabat Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 878-883
- **KOHLER ; MILSTEIN.** *Nature,* 1975, vol. 256, 495
- **NIEDBALA et al.** *Hybridoma,* 1998, vol. 17, 299-304
- **ZANELLA et al.** *J Immunol Methods,* 1992, vol. 156, 205-215
- **GUSTAFSSON et al.** *Hum Antibodies Hybridomas,* 1991, vol. 2, 26-32
- **WINTER ; HARRIS.** *Immunol Today,* 1993, vol. 14, 43-46
- **WRIGHT et al.** *Crit. Reviews in Immunol,* 1992, 12125-168
- **LIU et al.** *Proc Natl Acad Sci USA.,* 1987, vol. 84, 3439
- *J. Immunol.,* 1987, vol. 139, 3521
- **MCCAFFERTY et al.** *Nature,* 1990, vol. 348, 552-553
- **JOHNSON ; CHISWELL.** *Current Opinion in Structural Biology,* 1993, vol. 3, 564-571
- **CLACKSON et al.** *Nature,* 1991, vol. 352, 624-628
- **MARKS et al.** *J. Mol. Biol,* 1991, vol. 222, 581-597
- **GRIFFITH et al.** *EMBO J.,* 1993, vol. 12, 725-734
- **KABAT et al.** Sequences of Proteins of Immunological Interest. N.I.H, 1991
- **SHIELDS et al.** *J. Biol. Chem.,* 2001, vol. 276, 6591-6604
- **LAZAR et al.** *Proc. Natl. Acad. Sci. U.S.A.,* 2006, vol. 103, 4005-4010
- **SATOH et al.** *Expert Opin Biol. Ther.,* 2006, vol. 6, 1161-1173
- **KABAT, E. A. et al.** Sequences of Proteins of Immunological Interest. U.S. Department of Health and Human Services, 1991
- **CHOTHIA, C. et al.** *J. Mol. Biol.,* 1987, vol. 196, 901-917
- **THOMPSON J. D. et al.** *Nucleic Acids Res.,* 1994, vol. 22, 4673-4680
- **KABAT, E. A. et al.** Sequences of Proteins of Immunological Interest. U.S. Department of Health and Human Services, U.S. Government Printing Office, 1991
- **JOHNSON, G. ; WU, T. T.** *Nucleic Acids Research,* 2001, vol. 29, 205-206
- **KASHMIRI et al.** *Methods,* 2005, vol. 36, 25-34
- **TAN et al.** *J. Immunol.,* 2006, vol. 169, 1119-1125
- **HIGGINS D. G. et al.** *Meth. Enzymol.,* 1996, vol. 266, 383-402
- **WARD et al.** *Nature,* 1989, vol. 341, 544-546
- **CORTEZ-RETAMOZO et al.** *Cancer Res.,* 2004, vol. 64, 2853-2857
- **BIRD et al.** *Science,* 1988, vol. 242, 423-426
- **HUSTON et al.** *Proc. Natl. Acad. Sci. USA,* 1988, vol. 85, 5879-5883
- **LINDENBAUM et al.** *Nucleic Acids Res.,* 2004, vol. 32, el72
- **KENNARD et al.** *Biotechnol. Bioeng. Online,* 20 May 2009
- **KIM ; BALDWIN.** Specific Intermediates in the Folding Reactions of Small Proteins and the Mechanism of Protein Folding. *Ann. Rev. Biochem.,* 1982, vol. 51, 459-89
- **WRIGHT et al.** *Crit. Reviews in Immunol.,* 1992, 12125-168
- **HANES ; PLUCTHAU.** *PNAS USA,* 1997, vol. 94, 4937-4942
- **PARMLEY ; SMITH.** *Gene,* 1988, vol. 73, 305-318
- *Scott TIBS,* 1992, vol. 17, 241-245
- **CWIRLA et al.** *Proc Natl Acad Sci USA,* 1990, vol. 87, 6378-6382
- **RUSSEL et al.** *Nucl. Acids Research,* 1993, vol. 21, 1081-1085
- **HOGANBOOM et al.** *Immunol. Reviews,* 1992, vol. 130, 43-68
- **CHISWELL ; MCCAFFERTY.** *TIBTECH,* 1992, vol. 10, 80-84
- **FANGER et al.** *Immunomethods,* 1994, vol. 4, 72-81
- **TRAUNECKER et al.** *Int. J. Cancer (Suppl.),* 1992, vol. 7, 51-52

- **SONGSIVILAI ; LACHMANN.** *Clin. Exp. Immunol.,* 1990, vol. 79, 315-321
- **KOSTELNY et al.** *J. Immunol.,* 1992, vol. 148, 1547-1553
- **ILL. et al.** Design and construction of a hybrid immunoglobulin domain with properties of both heavy and light chain variable regions. *Protein Eng,* 1997, vol. 10, 949-57
- **MARTIN et al.** *EMBO J.,* 1994, vol. 13, 5303-9
- **HOLLIGER et al.** *P roc Natl Acad Sci USA,* 1993, vol. 90, 6444-6448
- **TRAUNECKER et al.** *EMBO J.,* 1991, vol. 10, 3655-3659
- **TRAUNECKER et al.** *Int J Cancer Suppl,* 1992, vol. 7, 51-52
- **GOLDMAN, L. A. et al.** *Biotechniques,* 1996, vol. 21, 1013-1015
- **MIZUSHIMA, S. et al.** *Nucleic Acids Res.,* 1990, vol. 18, 5322
- Current Protocols in Molecular Biology. 1991, vol. 2, 16.4.1-16.7.8
- *Vitetta Immunol Today,* 1993, vol. 14, 252
- **JUNGHANS et al.** Cancer Chemotherapy and Biotherapy. Lippincott Raven, 1996, 655-686
- **STRYER.** *Science,* 1968, vol. 162, 526
- **BRAND, L. et al.** *Annual Review of Biochemistry,* 1972, vol. 41, 843-868
- **PILARSKI et al.** *Blood,* 2000, vol. 95, 1056-65
- **RIGOLIN et al.** *Blood,* 2006, vol. 107, 2531-5
- **TOHDA et al.** *Leuk. Res.,* 2006, vol. 30, 1385-1390
- **AL-KATIB et al.** *Clin. Cancer Res.,* 1998, vol. 4, 1305-1314
- **MAGERSTADT, M.** Antibody Conjugates And Malignant Disease. CRC Press, 1991
- **BARCHEL, S. W. ; RHODES, B. H.** Radioimaging and Radiotherapy. Elsevier, 1983
- **WENSEL ; MEARES.** Radioimmunoimaging and Radioimmunotherapy. Elsevier, 1983
- **D. COLCHER et al.** *Meth. Enzymol.,* 1986, vol. 121, 802-816
- Developments in Antibody Imaging. **A. R. BRADWELL et al.** Monoclonal Antibodies for Cancer Detection and Therapy. Academic Press, 1985, 65-85
- **ZUCKERMANN et al.** *J. Med. Chem.,* 1994, vol. 37, 2678-85
- **LAM.** *Anticancer Drug Des.,* 1997, vol. 12, 145
- **HOUGHTEN.** *Biotechniques,* 1992, vol. 13, 412-421
- **LAM.** *Nature,* 1991, vol. 354, 82-84
- **FODOR.** *Nature,* 1993, vol. 364, 555-556
- **CULL et al.** *Proc Natl Acad Sci USA,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci.,* 1990, vol. 87, 6378-6382
- **FELICI.** *J. Mol. Biol.,* 1991, vol. 222, 301-310
- **STREUBEL et al.** *Leukemia,* 2006, vol. 20, 313-318